# EUROPEAN PATENT APPLICATION

(11) **EP 3 216 804 A2**
(43) Date of publication of application: **13.09.2017**
(21) Application number: 17157958.4
(22) Date of filing: 14.03.2014
(51) Int. Cl.: C07K 16/28

(54) **ANTI-CD25 ANTIBODIES AND THEIR USES**

(30) Priority: 15.03.2013 US 201361798235 P
(62) Divisional of application: 14729110.8
(71) Applicant: AbbVie Biotechnology Ltd., HM 11 Hamilton (BM)
(72) Inventor: AKAMATSU, Yoshiko, Palo Alto, CA 94306 (US); HARDING, Fiona A., Mountain View, CA 94041 (US); HINTON, Paul R., Sunnyvale, CA 94085 (US); XIONG, Mengli, Union City, CA 94587 (US); RAZO, Olivia Jennifer, Newark, CA 94560 (US); YE, Shiming, Palo Alto, CA 94306 (US)
(74) Representative: J A Kemp

(57) **Abstract**

The present disclosure relates to antibodies directed to CD25 and uses of such antibodies, for example to suppress organ transplant rejection or to treat multiple sclerosis.

SEQUENCE LISTING

<110>  ABBVIE BIOTECHNOLOGY LTD

<120>  ANTI-CD25 ANTIBODIES AND THEIR USES

<130>  N406439EP-A

<150>  US 61/798,235
<151>  2013-03-15

<160>  189

<170>  PatentIn version 3.5

<210>  1
<211>  116
<212>  PRT
<213>  Artificial Sequence

<220>
<223>  Synthetic polypeptide

<400>  1

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr Ser Tyr
            20                  25                  30

Arg Met His Trp Val Arg Gln Ala Pro Gly Gln Gly Leu Glu Trp Ile
        35                  40                  45

Gly Tyr Ile Asn Pro Ser Thr Gly Tyr Thr Glu Tyr Asn Gln Lys Phe
    50                  55                  60

Lys Asp Lys Ala Thr Ile Thr Ala Asp Glu Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gly Val Phe Asp Tyr Trp Gly Gln Gly Thr Leu Val
            100                 105                 110

Thr Val Ser Ser
        115

<210>  2
<211>  106
<212>  PRT
<213>  Artificial Sequence

<220>

<223>  Synthetic polypeptide

<400>  2

Asp Ile Gln Met Thr Gln Ser Pro Ser Thr Leu Ser Ala Ser Val Gly
1               5                   10                  15

Asp Arg Val Thr Ile Thr Cys Ser Ala Ser Ser Ser Ile Ser Tyr Met
            20                  25                  30

His Trp Tyr Gln Gln Lys Pro Gly Lys Ala Pro Lys Leu Leu Ile Tyr
        35                  40                  45

Thr Thr Ser Asn Leu Ala Ser Gly Val Pro Ala Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys His Gln Arg Ser Thr Tyr Pro Leu Thr
                85                  90                  95

Phe Gly Gln Gly Thr Lys Val Glu Val Lys
            100                 105

<210>  3
<211>  30
<212>  PRT
<213>  Artificial Sequence

<220>
<223>  Synthetic polypeptide

<400>  3

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Thr Phe Thr
            20                  25                  30

<210>  4
<211>  5
<212>  PRT
<213>  Artificial Sequence

<220>
<223>  Synthetic peptide

<400>  4

Ser Tyr Arg Met His
1               5

<210>  5
<211>  14
<212>  PRT
<213>  Artificial Sequence

<220>
<223>  Synthetic peptide

<400>  5

Trp Val Arg Gln Ala Pro Gly Gln Gly Leu Glu Trp Ile Gly
1               5                   10


<210>  6
<211>  17
<212>  PRT
<213>  Artificial Sequence

<220>
<223>  Synthetic peptide

<400>  6

Tyr Ile Asn Pro Ser Thr Gly Tyr Thr Glu Tyr Asn Gln Lys Phe Lys
1               5                   10                  15


Asp


<210>  7
<211>  32
<212>  PRT
<213>  Artificial Sequence

<220>
<223>  Synthetic polypeptide

<400>  7

Lys Ala Thr Ile Thr Ala Asp Glu Ser Thr Asn Thr Ala Tyr Met Glu
1               5                   10                  15


Leu Ser Ser Leu Arg Ser Glu Asp Thr Ala Val Tyr Tyr Cys Ala Arg
            20                  25                  30


<210>  8
<211>  7
<212>  PRT
<213>  Artificial Sequence

<220>
<223>  Synthetic peptide

<400>  8

Gly Gly Gly Val Phe Asp Tyr
1               5

<210>  9
<211>  11
<212>  PRT
<213>  Artificial Sequence

<220>
<223>  Synthetic peptide

<400>  9

Trp Gly Gln Gly Thr Leu Val Thr Val Ser Ser
1               5                   10


<210>  10
<211>  22
<212>  PRT
<213>  Artificial Sequence

<220>
<223>  Synthetic polypeptide

<400>  10

Asp Ile Gln Met Thr Gln Ser Pro Ser Thr Leu Ser Ala Val Gly Asp
1               5                   10                  15


Arg Val Thr Ile Thr Cys
            20


<210>  11
<211>  10
<212>  PRT
<213>  Artificial Sequence

<220>
<223>  Synthetic peptide

<400>  11

Ser Ala Ser Ser Ser Ile Ser Tyr Met His
1               5                   10


<210>  12
<211>  15
<212>  PRT
<213>  Artificial Sequence

<220>
<223>  Synthetic peptide

<400>  12

Trp Tyr Gln Gln Lys Pro Gly Lys Ala Pro Lys Leu Leu Ile Tyr
1               5                   10                  15


<210>  13

<211>  7
<212>  PRT
<213>  Artificial Sequence

<220>
<223>  Synthetic peptide

<400>  13

Thr Thr Ser Asn Leu Ala Ser
1               5


<210>  14
<211>  32
<212>  PRT
<213>  Artificial Sequence

<220>
<223>  Synthetic polypeptide

<400>  14

Gly Val Pro Ala Arg Phe Ser Gly Ser Gly Ser Gly Thr Glu Phe Thr
1               5                   10                  15


Leu Thr Ile Ser Ser Leu Gln Pro Asp Asp Phe Ala Thr Tyr Tyr Cys
            20                  25                  30


<210>  15
<211>  9
<212>  PRT
<213>  Artificial Sequence

<220>
<223>  Synthetic peptide

<400>  15

His Gln Arg Ser Thr Tyr Pro Leu Thr
1               5


<210>  16
<211>  10
<212>  PRT
<213>  Artificial Sequence

<220>
<223>  Synthetic peptide

<400>  16

Phe Gly Gln Gly Thr Lys Val Glu Val Lys
1               5                   10


<210>  17
<211>  227
<212>  PRT
<213>  Homo sapiens

<400>  17

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  18
<211>  15
<212>  PRT
<213>  Artificial Sequence

<220>
<223>  Synthetic peptide

<400>  18

Gly Gly Gly Gly Ser Gly Gly Gly Gly Ser Gly Gly Gly Gly Ser
1               5                   10                  15


<210>  19
<211>  5
<212>  PRT
<213>  Artificial Sequence

<220>
<223>  Synthetic peptide

<400>  19

Ala Asp Ala Ala Pro
1               5


<210>  20
<211>  5
<212>  PRT
<213>  Artificial Sequence

<220>
<223>  Synthetic peptide

<400>  20

Thr Val Ala Ala Pro
1               5


<210>  21
<211>  11
<212>  PRT
<213>  Artificial Sequence

<220>
<223>  Synthetic peptide

<400>  21

Ala Asp Ala Ala Pro Thr Val Ser Ile Phe Pro
1               5                   10


<210>  22
<211>  12
<212>  PRT
<213>  Artificial Sequence

<220>
<223>  Synthetic peptide

<400>  22

Thr Val Ala Ala Pro Ser Val Phe Ile Phe Pro Pro
1               5                   10


<210>  23
<211>  6
<212>  PRT
<213>  Artificial Sequence

<220>
<223>  Synthetic peptide

<400>  23

Gln Pro Lys Ala Ala Pro
1               5


<210>  24
<211>  13
<212>  PRT
<213>  Artificial Sequence

<220>
<223>  Synthetic peptide

<400>  24

Gln Pro Lys Ala Ala Pro Ser Val Thr Leu Phe Pro Pro
1               5                   10


<210>  25
<211>  5
<212>  PRT
<213>  Artificial Sequence

<220>
<223>  Synthetic peptide

<400>  25

Gly Gly Ser Gly Gly
1               5


<210>  26
<211>  9
<212>  PRT
<213>  Artificial Sequence

<220>
<223>  Synthetic peptide

<400>  26

Gly Gly Ser Gly Gly Gly Gly Ser Gly
1               5

<210>  27
<211>  13
<212>  PRT
<213>  Artificial Sequence

<220>
<223>  Synthetic peptide

<400>  27

Gly Gly Ser Gly Gly Gly Gly Ser Gly Gly Gly Gly Ser
1               5                   10


<210>  28
<211>  6
<212>  PRT
<213>  Artificial Sequence

<220>
<223>  Synthetic peptide

<400>  28

Ala Lys Thr Thr Ala Pro
1               5


<210>  29
<211>  6
<212>  PRT
<213>  Artificial Sequence

<220>
<223>  Synthetic peptide

<400>  29

Ala Ser Thr Lys Gly Pro
1               5


<210>  30
<211>  13
<212>  PRT
<213>  Artificial Sequence

<220>
<223>  Synthetic peptide

<400>  30

Ala Lys Thr Thr Ala Pro Ser Val Tyr Pro Leu Ala Pro
1               5                   10


<210>  31
<211>  13
<212>  PRT
<213>  Artificial Sequence

<220>
<223>  Synthetic peptide

<400>  31

Ala Ser Thr Lys Gly Pro Ser Val Phe Pro Leu Ala Pro
1               5                   10


<210>  32
<211>  6
<212>  PRT
<213>  Artificial Sequence

<220>
<223>  Synthetic peptide

<400>  32

Gly Gly Gly Gly Ser Gly
1               5


<210>  33
<211>  10
<212>  PRT
<213>  Artificial Sequence

<220>
<223>  Synthetic peptide

<400>  33

Gly Gly Gly Gly Ser Gly Gly Gly Gly Ser
1               5                   10


<210>  34
<211>  14
<212>  PRT
<213>  Artificial Sequence

<220>
<223>  Synthetic peptide

<400>  34

Gly Gly Gly Gly Ser Gly Gly Gly Gly Ser Gly Gly Gly Gly
1               5                   10


<210>  35
<211>  24
<212>  DNA
<213>  Artificial Sequence

<220>
<223>  Synthetic primer

<400>  35
tataacgcgt ccaccatgga ctcg                                              24


<210>  36
<211>  24

<212>  DNA
<213>  Artificial Sequence

<220>
<223>  Synthetic primer

<400>  36
tatagtcgac ggattaatat atcc                                              24


<210>  37
<211>  25
<212>  DNA
<213>  Artificial Sequence

<220>
<223>  Synthetic primer

<400>  37
gtgcaagagg aggaggagtc ttgac                                             25


<210>  38
<211>  26
<212>  DNA
<213>  Artificial Sequence

<220>
<223>  Synthetic primer

<400>  38
gtcaaagact cctcctcctc ttgcac                                            26


<210>  39
<211>  20
<212>  DNA
<213>  Artificial Sequence

<220>
<223>  Synthetic primer

<400>  39
ccatagaaga caccgggacc                                                   20


<210>  40
<211>  20
<212>  DNA
<213>  Artificial Sequence

<220>
<223>  Synthetic primer

<400>  40
tcaccttagc cccctccctg                                                   20


<210>  41
<211>  61
<212>  DNA
<213>  Artificial Sequence

<220>
<223>  Synthetic primer

<220>
<221>  modified_base
<222>  (23)..(23)
<223>  a, c, t or g

<220>
<221>  misc_feature
<222>  (23)..(23)
<223>  n is a, c, g, or t

<400>  41
ccctggacag ggtctggaat ggntgggata tattaatccg tcgactgggt atactgaata      60

c                                                                      61

<210>  42
<211>  22
<212>  DNA
<213>  Artificial Sequence

<220>
<223>  Synthetic primer

<400>  42
ccattccaga ccctgtccag gg                                               22

<210>  43
<211>  61
<212>  DNA
<213>  Artificial Sequence

<220>
<223>  Synthetic primer

<400>  43
ccctggacag ggtctggaat ggattggata tsycaatccg tcgactgggt atactgaata      60

c                                                                      61

<210>  44
<211>  61
<212>  DNA
<213>  Artificial Sequence

<220>
<223>  Synthetic primer

<400>  44
ccctggacag ggtctggaat ggattggata tattaatccg tcgkycgggt atactgaata      60

c                                                                      61

<210>  45
<211>  61
<212>  DNA

<213>  Artificial Sequence

<220>
<223>  Synthetic primer

<400>  45
ccctggacag ggtctggaat ggattggata tattaatccg tcgactgggg ccactgaata      60

c                                                                      61


<210>  46
<211>  26
<212>  DNA
<213>  Artificial Sequence

<220>
<223>  Synthetic primer

<400>  46
gtcaacgcgt ccaccatgga ctcgag                                           26


<210>  47
<211>  36
<212>  DNA
<213>  Artificial Sequence

<220>
<223>  Synthetic primer

<400>  47
gtactctaga ggttttaagg actcacctga ggagac                                36


<210>  48
<211>  30
<212>  DNA
<213>  Artificial Sequence

<220>
<223>  Synthetic primer

<400>  48
gtactctaga ggttttaagg actcacctga                                       30


<210>  49
<211>  61
<212>  DNA
<213>  Artificial Sequence

<220>
<223>  Synthetic primer

<400>  49
ccctggacag ggtctggaat ggatgggata tctgaatccg tcgactgggt atactgaata      60

c                                                                      61


<210>  50
<211>  61

<212>  DNA
<213>  Artificial Sequence

<220>
<223>  Synthetic primer

<400>  50
ccctggacag ggtctggaat ggatgggata tattaatccg tcgtccgggt atactgaata      60

c                                                                      61


<210>  51
<211>  61
<212>  DNA
<213>  Artificial Sequence

<220>
<223>  Synthetic primer

<400>  51
ccctggacag ggtctggaat gggtgggata tattaatccg tcgtccgggt atactgaata      60

c                                                                      61


<210>  52
<211>  116
<212>  PRT
<213>  Artificial Sequence

<220>
<223>  Synthetic polypeptide

<400>  52

Gln Val Gln Leu Gln Gln Ser Gly Ala Glu Leu Ala Lys Pro Gly Ala
1               5                   10                  15

Ser Val Lys Met Ser Cys Lys Ala Ser Gly Tyr Thr Phe Thr Ser Tyr
            20                  25                  30

Arg Met His Trp Val Lys Gln Arg Pro Gly Gln Gly Leu Glu Trp Ile
        35                  40                  45

Gly Tyr Ile Asn Pro Ser Thr Gly Tyr Thr Glu Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Phe Glu Asp Ser Ala Val Tyr Tyr Cys
                85                  90                  95

Ala Arg Gly Gly Gly Val Phe Asp Tyr Trp Gly Gln Gly Thr Thr Leu
            100                 105                 110

Thr Val Ser Ser
        115

<210>  53
<211>  116
<212>  PRT
<213>  Artificial Sequence

<220>
<223>  Synthetic polypeptide

<400>  53

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Arg Met His Trp Val Arg Gln Ala Pro Gly Gln Gly Leu Glu Trp Ile
        35                  40                  45

Gly Tyr Ile Asn Pro Ser Thr Gly Tyr Thr Glu Tyr Asn Gln Lys Phe
    50                  55                  60

Lys Asp Lys Ala Thr Met Thr Ala Asp Lys Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gly Val Phe Asp Tyr Trp Gly Gln Gly Thr Leu Val
            100                 105                 110

Thr Val Ser Ser
        115

<210>  54
<211>  89
<212>  PRT
<213>  Artificial Sequence

<220>
<223>  Synthetic polypeptide

<400>  54

Gln Val Gln Leu Val Gln Ser Gly Ala Glu Val Lys Lys Pro Gly Ala
1               5                   10                  15

Ser Val Lys Val Ser Cys Lys Thr Ser Gly Tyr Thr Phe Val Trp Val
            20                  25                  30

Arg Gln Ala Pro Gly Gln Gly Leu Gln Trp Met Gly Arg Val Thr Met
        35                  40                  45

Thr Arg Asp Thr Ser Ile Ser Thr Ala Tyr Met Glu Leu Ser Ser Leu
    50                  55                  60

Arg Ser Asp Asp Thr Ala Val Tyr Tyr Cys Ala Arg Asp Tyr Trp Gly
65                  70                  75                  80

Gln Gly Thr Leu Val Thr Val Ser Ser
                85

<210>  55
<211>  89
<212>  PRT
<213>  Artificial Sequence

<220>
<223>  Synthetic polypeptide

<400>  55

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Trp Val
            20                  25                  30

Arg Gln Ala Pro Gly Gln Gly Leu Glu Trp Met Gly Arg Val Thr Ile
        35                  40                  45

Thr Ala Asp Thr Ser Thr Ser Thr Ala Tyr Met Glu Leu Ser Ser Leu
    50                  55                  60

Arg Ser Glu Asp Thr Ala Val Tyr Tyr Cys Ala Arg Asp Tyr Trp Gly
65                  70                  75                  80

Gln Gly Thr Leu Val Thr Val Ser Ser
                85

<210>  56
<211>  106
<212>  PRT
<213>  Artificial Sequence

<220>
<223>  Synthetic polypeptide

<400>  56

Gln Ile Val Leu Thr Gln Ser Pro Ala Ile Met Ser Ala Ser Pro Gly

1               5                   10                  15

Glu Lys Val Thr Ile Thr Cys Ser Ala Ser Ser Ser Ile Ser Tyr Met
            20                  25                  30

His Trp Phe Gln Gln Lys Pro Gly Thr Ser Pro Lys Leu Trp Ile Tyr
        35                  40                  45

Thr Thr Ser Asn Leu Ala Ser Gly Val Pro Ala Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys His Gln Arg Ser Thr Tyr Pro Leu Thr
                85                  90                  95

Phe Gly Ser Gly Thr Lys Leu Glu Leu Lys
            100                 105

<210>  57
<211>  106
<212>  PRT
<213>  Artificial Sequence

<220>
<223>  Synthetic polypeptide

<400>  57

Glu Ile Val Leu Thr Gln Ser Pro Ala Thr Leu Ser Leu Ser Pro Gly
1               5                   10                  15

Glu Arg Ala Thr Leu Thr Cys Ser Ala Ser Ser Ser Ile Ser Tyr Met
            20                  25                  30

His Trp Phe Gln Gln Lys Pro Gly Gln Ala Pro Arg Leu Leu Ile Tyr
        35                  40                  45

Thr Thr Ser Asn Leu Ala Ser Gly Ile Pro Ala Arg Phe Ser Gly Ser
    50                  55                  60

Gly Ser Gly Thr Asp Tyr Thr Leu Thr Ile Ser Ser Leu Glu Pro Glu
65                  70                  75                  80

Asp Phe Ala Val Tyr Tyr Cys His Gln Arg Ser Thr Tyr Pro Leu Thr
                85                  90                  95

Phe Gly Gln Gly Thr Lys Val Glu Ile Lys
            100                 105

<210>  58
<211>  80
<212>  PRT
<213>  Artificial Sequence

<220>
<223>  Synthetic polypeptide

<400>  58

Glu Ile Val Leu Thr Gln Ser Pro Ala Thr Leu Ser Leu Ser Pro Gly
1               5                   10                  15

Glu Arg Ala Thr Leu Ser Cys Trp Tyr Gln Gln Lys Pro Gly Gln Ala
            20                  25                  30

Pro Arg Leu Leu Ile Tyr Gly Ile Pro Ala Arg Phe Ser Gly Ser Gly
        35                  40                  45

Ser Gly Thr Asp Phe Thr Leu Thr Ile Ser Ser Leu Glu Pro Glu Asp
    50                  55                  60

Phe Ala Val Tyr Tyr Cys Phe Gly Gln Gly Thr Lys Val Glu Ile Lys
65                  70                  75                  80

<210>  59
<211>  80
<212>  PRT
<213>  Artificial Sequence

<220>
<223>  Synthetic polypeptide

<400>  59

Glu Ile Val Leu Thr Gln Ser Pro Gly Thr Leu Ser Leu Ser Pro Gly
1               5                   10                  15

Glu Arg Ala Thr Leu Ser Cys Trp Tyr Gln Gln Lys Pro Gly Gln Ala
            20                  25                  30

Pro Arg Leu Leu Ile Tyr Gly Ile Pro Asp Arg Phe Ser Gly Ser Gly
        35                  40                  45

Ser Gly Thr Asp Phe Thr Leu Thr Ile Ser Arg Leu Glu Pro Glu Asp
    50                  55                  60

Phe Ala Val Tyr Tyr Cys Phe Gly Gln Gly Thr Lys Val Glu Ile Lys
65                  70                  75                  80

<210>  60

<211>  15
<212>  PRT
<213>  Artificial Sequence

<220>
<223>  Synthetic peptide

<400>  60

Asp Ile Gln Met Thr Gln Ser Pro Ser Thr Leu Ser Ala Ser Val
1               5                   10                  15


<210>  61
<211>  15
<212>  PRT
<213>  Artificial Sequence

<220>
<223>  Synthetic peptide

<400>  61

Met Thr Gln Ser Pro Ser Thr Leu Ser Ala Ser Val Gly Asp Arg
1               5                   10                  15


<210>  62
<211>  15
<212>  PRT
<213>  Artificial Sequence

<220>
<223>  Synthetic peptide

<400>  62

Ser Pro Ser Thr Leu Ser Ala Ser Val Gly Asp Arg Val Thr Ile
1               5                   10                  15


<210>  63
<211>  15
<212>  PRT
<213>  Artificial Sequence

<220>
<223>  Synthetic peptide

<400>  63

Thr Leu Ser Ala Ser Val Gly Asp Arg Val Thr Ile Thr Cys Ser
1               5                   10                  15


<210>  64
<211>  15
<212>  PRT
<213>  Artificial Sequence

<220>
<223>  Synthetic peptide

<400>  64

Ala Ser Val Gly Asp Arg Val Thr Ile Thr Cys Ser Ala Ser Ser
1               5                   10                  15


<210>  65
<211>  15
<212>  PRT
<213>  Artificial Sequence

<220>
<223>  Synthetic peptide

<400>  65

Gly Asp Arg Val Thr Ile Thr Cys Ser Ala Ser Ser Ser Ile Ser
1               5                   10                  15


<210>  66
<211>  15
<212>  PRT
<213>  Artificial Sequence

<220>
<223>  Synthetic peptide

<400>  66

Val Thr Ile Thr Cys Ser Ala Ser Ser Ser Ile Ser Tyr Met His
1               5                   10                  15


<210>  67
<211>  15
<212>  PRT
<213>  Artificial Sequence

<220>
<223>  Synthetic peptide

<400>  67

Thr Cys Ser Ala Ser Ser Ser Ile Ser Tyr Met His Trp Tyr Gln
1               5                   10                  15


<210>  68
<211>  15
<212>  PRT
<213>  Artificial Sequence

<220>
<223>  Synthetic peptide

<400>  68

Ala Ser Ser Ser Ile Ser Tyr Met His Trp Tyr Gln Gln Lys Pro
1               5                   10                  15


<210>  69

<211>  15
<212>  PRT
<213>  Artificial Sequence

<220>
<223>  Synthetic peptide

<400>  69

Ser Ile Ser Tyr Met His Trp Tyr Gln Gln Lys Pro Gly Lys Ala
1               5                   10                  15


<210>  70
<211>  15
<212>  PRT
<213>  Artificial Sequence

<220>
<223>  Synthetic peptide

<400>  70

Tyr Met His Trp Tyr Gln Gln Lys Pro Gly Lys Ala Pro Lys Leu
1               5                   10                  15


<210>  71
<211>  15
<212>  PRT
<213>  Artificial Sequence

<220>
<223>  Synthetic peptide

<400>  71

Trp Tyr Gln Gln Lys Pro Gly Lys Ala Pro Lys Leu Leu Ile Tyr
1               5                   10                  15


<210>  72
<211>  15
<212>  PRT
<213>  Artificial Sequence

<220>
<223>  Synthetic peptide

<400>  72

Gln Lys Pro Gly Lys Ala Pro Lys Leu Leu Ile Tyr Thr Thr Ser
1               5                   10                  15


<210>  73
<211>  15
<212>  PRT
<213>  Artificial Sequence

<220>
<223>  Synthetic peptide

<400>  73

Gly Lys Ala Pro Lys Leu Leu Ile Tyr Thr Thr Ser Asn Leu Ala
1               5                   10                  15


<210>  74
<211>  15
<212>  PRT
<213>  Artificial Sequence

<220>
<223>  Synthetic peptide

<400>  74

Pro Lys Leu Leu Ile Tyr Thr Thr Ser Asn Leu Ala Ser Gly Val
1               5                   10                  15


<210>  75
<211>  15
<212>  PRT
<213>  Artificial Sequence

<220>
<223>  Synthetic peptide

<400>  75

Leu Ile Tyr Thr Thr Ser Asn Leu Ala Ser Gly Val Pro Ala Arg
1               5                   10                  15


<210>  76
<211>  15
<212>  PRT
<213>  Artificial Sequence

<220>
<223>  Synthetic peptide

<400>  76

Thr Thr Ser Asn Leu Ala Ser Gly Val Pro Ala Arg Phe Ser Gly
1               5                   10                  15


<210>  77
<211>  15
<212>  PRT
<213>  Artificial Sequence

<220>
<223>  Synthetic peptide

<400>  77

Asn Leu Ala Ser Gly Val Pro Ala Arg Phe Ser Gly Ser Gly Ser
1               5                   10                  15


<210>  78

<211>  15
<212>  PRT
<213>  Artificial Sequence

<220>
<223>  Synthetic peptide

<400>  78

Ser Gly Val Pro Ala Arg Phe Ser Gly Ser Gly Ser Gly Thr Glu
1               5                   10                  15


<210>  79
<211>  15
<212>  PRT
<213>  Artificial Sequence

<220>
<223>  Synthetic peptide

<400>  79

Pro Ala Arg Phe Ser Gly Ser Gly Ser Gly Thr Glu Phe Thr Leu
1               5                   10                  15


<210>  80
<211>  15
<212>  PRT
<213>  Artificial Sequence

<220>
<223>  Synthetic peptide

<400>  80

Phe Ser Gly Ser Gly Ser Gly Thr Glu Phe Thr Leu Thr Ile Ser
1               5                   10                  15


<210>  81
<211>  15
<212>  PRT
<213>  Artificial Sequence

<220>
<223>  Synthetic peptide

<400>  81

Ser Gly Ser Gly Thr Glu Phe Thr Leu Thr Ile Ser Ser Leu Gln
1               5                   10                  15


<210>  82
<211>  15
<212>  PRT
<213>  Artificial Sequence

<220>
<223>  Synthetic peptide

<400>  82

Gly Thr Glu Phe Thr Leu Thr Ile Ser Ser Leu Gln Pro Asp Asp
1               5                   10                  15


<210>  83
<211>  15
<212>  PRT
<213>  Artificial Sequence

<220>
<223>  Synthetic peptide

<400>  83

Phe Thr Leu Thr Ile Ser Ser Leu Gln Pro Asp Asp Phe Ala Thr
1               5                   10                  15


<210>  84
<211>  15
<212>  PRT
<213>  Artificial Sequence

<220>
<223>  Synthetic peptide

<400>  84

Thr Ile Ser Ser Leu Gln Pro Asp Asp Phe Ala Thr Tyr Tyr Cys
1               5                   10                  15


<210>  85
<211>  15
<212>  PRT
<213>  Artificial Sequence

<220>
<223>  Synthetic peptide

<400>  85

Ser Leu Gln Pro Asp Asp Phe Ala Thr Tyr Tyr Cys His Gln Arg
1               5                   10                  15


<210>  86
<211>  15
<212>  PRT
<213>  Artificial Sequence

<220>
<223>  Synthetic peptide

<400>  86

Pro Asp Asp Phe Ala Thr Tyr Tyr Cys His Gln Arg Ser Thr Tyr
1               5                   10                  15


<210>  87

<211>  15
<212>  PRT
<213>  Artificial Sequence

<220>
<223>  Synthetic peptide

<400>  87

Phe Ala Thr Tyr Tyr Cys His Gln Arg Ser Thr Tyr Pro Leu Thr
1               5                   10                  15


<210>  88
<211>  15
<212>  PRT
<213>  Artificial Sequence

<220>
<223>  Synthetic peptide

<400>  88

Tyr Tyr Cys His Gln Arg Ser Thr Tyr Pro Leu Thr Phe Gly Gln
1               5                   10                  15


<210>  89
<211>  15
<212>  PRT
<213>  Artificial Sequence

<220>
<223>  Synthetic peptide

<400>  89

His Gln Arg Ser Thr Tyr Pro Leu Thr Phe Gly Gln Gly Thr Lys
1               5                   10                  15


<210>  90
<211>  15
<212>  PRT
<213>  Artificial Sequence

<220>
<223>  Synthetic peptide

<400>  90

Ser Thr Tyr Pro Leu Thr Phe Gly Gln Gly Thr Lys Val Glu Val
1               5                   10                  15


<210>  91
<211>  15
<212>  PRT
<213>  Artificial Sequence

<220>
<223>  Synthetic peptide

<400>  91

Pro Leu Thr Phe Gly Gln Gly Thr Lys Val Glu Val Lys Arg Thr
1               5                   10                  15


<210>  92
<211>  15
<212>  PRT
<213>  Artificial Sequence

<220>
<223>  Synthetic peptide

<400>  92

Val His Ser Gln Val Gln Leu Val Gln Ser Gly Ala Glu Val Lys
1               5                   10                  15


<210>  93
<211>  15
<212>  PRT
<213>  Artificial Sequence

<220>
<223>  Synthetic peptide

<400>  93

Gln Val Gln Leu Val Gln Ser Gly Ala Glu Val Lys Lys Pro Gly
1               5                   10                  15


<210>  94
<211>  15
<212>  PRT
<213>  Artificial Sequence

<220>
<223>  Synthetic peptide

<400>  94

Leu Val Gln Ser Gly Ala Glu Val Lys Lys Pro Gly Ser Ser Val
1               5                   10                  15


<210>  95
<211>  15
<212>  PRT
<213>  Artificial Sequence

<220>
<223>  Synthetic peptide

<400>  95

Ser Gly Ala Glu Val Lys Lys Pro Gly Ser Ser Val Lys Val Ser
1               5                   10                  15


<210>  96

<211>  15
<212>  PRT
<213>  Artificial Sequence

<220>
<223>  Synthetic peptide

<400>  96

Glu Val Lys Lys Pro Gly Ser Ser Val Lys Val Ser Cys Lys Ala
1               5                   10                  15


<210>  97
<211>  15
<212>  PRT
<213>  Artificial Sequence

<220>
<223>  Synthetic peptide

<400>  97

Lys Pro Gly Ser Ser Val Lys Val Ser Cys Lys Ala Ser Gly Tyr
1               5                   10                  15


<210>  98
<211>  15
<212>  PRT
<213>  Artificial Sequence

<220>
<223>  Synthetic peptide

<400>  98

Ser Ser Val Lys Val Ser Cys Lys Ala Ser Gly Tyr Thr Phe Thr
1               5                   10                  15


<210>  99
<211>  15
<212>  PRT
<213>  Artificial Sequence

<220>
<223>  Synthetic peptide

<400>  99

Lys Val Ser Cys Lys Ala Ser Gly Tyr Thr Phe Thr Ser Tyr Arg
1               5                   10                  15


<210>  100
<211>  15
<212>  PRT
<213>  Artificial Sequence

<220>
<223>  Synthetic peptide

<400>  100

Cys Lys Ala Ser Gly Tyr Thr Phe Thr Ser Tyr Arg Met His Trp
1               5                   10                  15


<210>  101
<211>  15
<212>  PRT
<213>  Artificial Sequence

<220>
<223>  Synthetic peptide

<400>  101

Ser Gly Tyr Thr Phe Thr Ser Tyr Arg Met His Trp Val Arg Gln
1               5                   10                  15


<210>  102
<211>  15
<212>  PRT
<213>  Artificial Sequence

<220>
<223>  Synthetic peptide

<400>  102

Thr Phe Thr Ser Tyr Arg Met His Trp Val Arg Gln Ala Pro Gly
1               5                   10                  15


<210>  103
<211>  15
<212>  PRT
<213>  Artificial Sequence

<220>
<223>  Synthetic peptide

<400>  103

Ser Tyr Arg Met His Trp Val Arg Gln Ala Pro Gly Gln Gly Leu
1               5                   10                  15


<210>  104
<211>  15
<212>  PRT
<213>  Artificial Sequence

<220>
<223>  Synthetic peptide

<400>  104

Met His Trp Val Arg Gln Ala Pro Gly Gln Gly Leu Glu Trp Ile
1               5                   10                  15


<210>  105

<211>  15
<212>  PRT
<213>  Artificial Sequence

<220>
<223>  Synthetic peptide

<400>  105

Val Arg Gln Ala Pro Gly Gln Gly Leu Glu Trp Ile Gly Tyr Ile
1               5                   10                  15


<210>  106
<211>  15
<212>  PRT
<213>  Artificial Sequence

<220>
<223>  Synthetic peptide

<400>  106

Ala Pro Gly Gln Gly Leu Glu Trp Ile Gly Tyr Ile Asn Pro Ser
1               5                   10                  15


<210>  107
<211>  15
<212>  PRT
<213>  Artificial Sequence

<220>
<223>  Synthetic peptide

<400>  107

Gln Gly Leu Glu Trp Ile Gly Tyr Ile Asn Pro Ser Thr Gly Tyr
1               5                   10                  15


<210>  108
<211>  15
<212>  PRT
<213>  Artificial Sequence

<220>
<223>  Synthetic peptide

<400>  108

Glu Trp Ile Gly Tyr Ile Asn Pro Ser Thr Gly Tyr Thr Glu Tyr
1               5                   10                  15


<210>  109
<211>  15
<212>  PRT
<213>  Artificial Sequence

<220>
<223>  Synthetic peptide

<400>  109

Gly Tyr Ile Asn Pro Ser Thr Gly Tyr Thr Glu Tyr Asn Gln Lys
1               5                   10                  15


<210>  110
<211>  15
<212>  PRT
<213>  Artificial Sequence

<220>
<223>  Synthetic peptide

<400>  110

Asn Pro Ser Thr Gly Tyr Thr Glu Tyr Asn Gln Lys Phe Lys Asp
1               5                   10                  15


<210>  111
<211>  15
<212>  PRT
<213>  Artificial Sequence

<220>
<223>  Synthetic peptide

<400>  111

Thr Gly Tyr Thr Glu Tyr Asn Gln Lys Phe Lys Asp Lys Ala Thr
1               5                   10                  15


<210>  112
<211>  15
<212>  PRT
<213>  Artificial Sequence

<220>
<223>  Synthetic peptide

<400>  112

Thr Glu Tyr Asn Gln Lys Phe Lys Asp Lys Ala Thr Ile Thr Ala
1               5                   10                  15


<210>  113
<211>  15
<212>  PRT
<213>  Artificial Sequence

<220>
<223>  Synthetic peptide

<400>  113

Asn Gln Lys Phe Lys Asp Lys Ala Thr Ile Thr Ala Asp Glu Ser
1               5                   10                  15


<210>  114

<211>  15
<212>  PRT
<213>  Artificial Sequence

<220>
<223>  Synthetic peptide

<400>  114

Phe Lys Asp Lys Ala Thr Ile Thr Ala Asp Glu Ser Thr Asn Thr
1               5                   10                  15


<210>  115
<211>  15
<212>  PRT
<213>  Artificial Sequence

<220>
<223>  Synthetic peptide

<400>  115

Lys Ala Thr Ile Thr Ala Asp Glu Ser Thr Asn Thr Ala Tyr Met
1               5                   10                  15


<210>  116
<211>  15
<212>  PRT
<213>  Artificial Sequence

<220>
<223>  Synthetic peptide

<400>  116

Ile Thr Ala Asp Glu Ser Thr Asn Thr Ala Tyr Met Glu Leu Ser
1               5                   10                  15


<210>  117
<211>  15
<212>  PRT
<213>  Artificial Sequence

<220>
<223>  Synthetic peptide

<400>  117

Asp Glu Ser Thr Asn Thr Ala Tyr Met Glu Leu Ser Ser Leu Arg
1               5                   10                  15


<210>  118
<211>  15
<212>  PRT
<213>  Artificial Sequence

<220>
<223>  Synthetic peptide

<400>  118

Thr Asn Thr Ala Tyr Met Glu Leu Ser Ser Leu Arg Ser Glu Asp
1               5                   10                  15


<210>  119
<211>  15
<212>  PRT
<213>  Artificial Sequence

<220>
<223>  Synthetic peptide

<400>  119

Ala Tyr Met Glu Leu Ser Ser Leu Arg Ser Glu Asp Thr Ala Val
1               5                   10                  15


<210>  120
<211>  15
<212>  PRT
<213>  Artificial Sequence

<220>
<223>  Synthetic peptide

<400>  120

Glu Leu Ser Ser Leu Arg Ser Glu Asp Thr Ala Val Tyr Tyr Cys
1               5                   10                  15


<210>  121
<211>  15
<212>  PRT
<213>  Artificial Sequence

<220>
<223>  Synthetic peptide

<400>  121

Ser Leu Arg Ser Glu Asp Thr Ala Val Tyr Tyr Cys Ala Arg Gly
1               5                   10                  15


<210>  122
<211>  15
<212>  PRT
<213>  Artificial Sequence

<220>
<223>  Synthetic peptide

<400>  122

Ser Glu Asp Thr Ala Val Tyr Tyr Cys Ala Arg Gly Gly Gly Val
1               5                   10                  15


<210>  123

<211>  15
<212>  PRT
<213>  Artificial Sequence

<220>
<223>  Synthetic peptide

<400>  123

Thr Ala Val Tyr Tyr Cys Ala Arg Gly Gly Gly Val Phe Asp Tyr
1               5                   10                  15


<210>  124
<211>  15
<212>  PRT
<213>  Artificial Sequence

<220>
<223>  Synthetic peptide

<400>  124

Tyr Tyr Cys Ala Arg Gly Gly Gly Val Phe Asp Tyr Trp Gly Gln
1               5                   10                  15


<210>  125
<211>  15
<212>  PRT
<213>  Artificial Sequence

<220>
<223>  Synthetic peptide

<400>  125

Ala Arg Gly Gly Gly Val Phe Asp Tyr Trp Gly Gln Gly Thr Leu
1               5                   10                  15


<210>  126
<211>  15
<212>  PRT
<213>  Artificial Sequence

<220>
<223>  Synthetic peptide

<400>  126

Gly Gly Val Phe Asp Tyr Trp Gly Gln Gly Thr Leu Val Thr Val
1               5                   10                  15


<210>  127
<211>  15
<212>  PRT
<213>  Artificial Sequence

<220>
<223>  Synthetic peptide

<400>  127

Phe Asp Tyr Trp Gly Gln Gly Thr Leu Val Thr Val Ser Ser Ala
1               5                   10                  15


<210>  128
<211>  75
<212>  DNA
<213>  Artificial Sequence

<220>
<223>  Synthetic oligonucleotide

<400>  128
tataacgcgt ccaccatgga ctcgaggctg aacttggtct tcctcgtgtt aattctcaag      60

ggcgtgcagt gtcag                                                        75


<210>  129
<211>  75
<212>  DNA
<213>  Artificial Sequence

<220>
<223>  Synthetic oligonucleotide

<400>  129
cttcacgctc gagccaggtt tcttgacttc agccccagac tggacaagct ggacctgaca      60

ctgcacgccc ttgag                                                        75


<210>  130
<211>  73
<212>  DNA
<213>  Artificial Sequence

<220>
<223>  Synthetic oligonucleotide

<400>  130
aaacctggct cgagcgtgaa ggtctcctgc aaggcttctg gctacacctt tactagctac      60

aggatgcact ggg                                                          73


<210>  131
<211>  77
<212>  DNA
<213>  Artificial Sequence

<220>
<223>  Synthetic oligonucleotide

<400>  131
tatagtcgac ggattaatat atccaatcca ttccagaccc tgtccagggg cctgccttac      60

ccagtgcatc ctgtagc                                                      77


<210>  132

<211>  18
<212>  PRT
<213>  Artificial Sequence

<220>
<223>  Synthetic peptide

<400>  132

Gln Gly Leu Glu Trp Ile Gly Tyr Ile Asn Pro Ser Thr Gly Tyr Thr
1               5                   10                  15

Glu Tyr

<210>  133
<211>  18
<212>  PRT
<213>  Artificial Sequence

<220>
<223>  Synthetic peptide

<400>  133

Gln Gly Leu Glu Trp Val Gly Tyr Ile Asn Pro Ser Thr Gly Tyr Thr
1               5                   10                  15

Glu Tyr

<210>  134
<211>  18
<212>  PRT
<213>  Artificial Sequence

<220>
<223>  Synthetic peptide

<400>  134

Gln Gly Leu Glu Trp Leu Gly Tyr Ile Asn Pro Ser Thr Gly Tyr Thr
1               5                   10                  15

Glu Tyr

<210>  135
<211>  18
<212>  PRT
<213>  Artificial Sequence

<220>
<223>  Synthetic peptide

<400>  135

Gln Gly Leu Glu Trp Met Gly Tyr Ile Asn Pro Ser Thr Gly Tyr Thr
1               5                   10                  15

Glu Tyr

<210>  136
<211>  18
<212>  PRT
<213>  Artificial Sequence

<220>
<223>  Synthetic peptide

<400>  136

Gln Gly Leu Glu Trp Ile Gly Tyr Leu Asn Pro Ser Thr Gly Tyr Thr
1               5                   10                  15

Glu Tyr

<210>  137
<211>  18
<212>  PRT
<213>  Artificial Sequence

<220>
<223>  Synthetic peptide

<400>  137

Gln Gly Leu Glu Trp Ile Gly Tyr Val Asn Pro Ser Thr Gly Tyr Thr
1               5                   10                  15

Glu Tyr

<210>  138
<211>  18
<212>  PRT
<213>  Artificial Sequence

<220>
<223>  Synthetic peptide

<400>  138

Gln Gly Leu Glu Trp Ile Gly Tyr Ala Asn Pro Ser Thr Gly Tyr Thr
1               5                   10                  15

Glu Tyr

<210>  139

<211>  18
<212>  PRT
<213>  Artificial Sequence

<220>
<223>  Synthetic peptide

<400>  139

Gln Gly Leu Glu Trp Ile Gly Tyr Ile Asn Pro Ser Ala Gly Tyr Thr
1               5                   10                  15


Glu Tyr




<210>  140
<211>  18
<212>  PRT
<213>  Artificial Sequence

<220>
<223>  Synthetic peptide

<400>  140

Gln Gly Leu Glu Trp Ile Gly Tyr Ile Asn Pro Ser Val Gly Tyr Thr
1               5                   10                  15


Glu Tyr




<210>  141
<211>  18
<212>  PRT
<213>  Artificial Sequence

<220>
<223>  Synthetic peptide

<400>  141

Gln Gly Leu Glu Trp Ile Gly Tyr Ile Asn Pro Ser Ser Gly Tyr Thr
1               5                   10                  15


Glu Tyr




<210>  142
<211>  18
<212>  PRT
<213>  Artificial Sequence

<220>
<223>  Synthetic peptide

<400>  142

Gln Gly Leu Glu Trp Ile Gly Tyr Ile Asn Pro Ser Thr Gly Ala Thr
1               5                   10                  15

Glu Tyr

<210>  143
<211>  18
<212>  PRT
<213>  Artificial Sequence

<220>
<223>  Synthetic peptide

<400>  143

Gln Gly Leu Glu Trp Val Gly Tyr Leu Asn Pro Ser Thr Gly Tyr Thr
1               5                   10                  15

Glu Tyr

<210>  144
<211>  18
<212>  PRT
<213>  Artificial Sequence

<220>
<223>  Synthetic peptide

<400>  144

Gln Gly Leu Glu Trp Val Gly Tyr Val Asn Pro Ser Thr Gly Tyr Thr
1               5                   10                  15

Glu Tyr

<210>  145
<211>  18
<212>  PRT
<213>  Artificial Sequence

<220>
<223>  Synthetic peptide

<400>  145

Gln Gly Leu Glu Trp Val Gly Tyr Ala Asn Pro Ser Thr Gly Tyr Thr
1               5                   10                  15

Glu Tyr

<210>  146

<211>  18
<212>  PRT
<213>  Artificial Sequence

<220>
<223>  Synthetic peptide

<400>  146

Gln Gly Leu Glu Trp Leu Gly Tyr Leu Asn Pro Ser Thr Gly Tyr Thr
1               5                   10                  15

Glu Tyr

<210>  147
<211>  18
<212>  PRT
<213>  Artificial Sequence

<220>
<223>  Synthetic peptide

<400>  147

Gln Gly Leu Glu Trp Leu Gly Tyr Val Asn Pro Ser Thr Gly Tyr Thr
1               5                   10                  15

Glu Tyr

<210>  148
<211>  18
<212>  PRT
<213>  Artificial Sequence

<220>
<223>  Synthetic peptide

<400>  148

Gln Gly Leu Glu Trp Leu Gly Tyr Ala Asn Pro Ser Thr Gly Tyr Thr
1               5                   10                  15

Glu Tyr

<210>  149
<211>  18
<212>  PRT
<213>  Artificial Sequence

<220>
<223>  Synthetic peptide

<400>  149

Gln Gly Leu Glu Trp Met Gly Tyr Leu Asn Pro Ser Thr Gly Tyr Thr
1               5                   10                  15

Glu Tyr

<210>  150
<211>  18
<212>  PRT
<213>  Artificial Sequence

<220>
<223>  Synthetic peptide

<400>  150

Gln Gly Leu Glu Trp Met Gly Tyr Val Asn Pro Ser Thr Gly Tyr Thr
1               5                   10                  15

Glu Tyr

<210>  151
<211>  18
<212>  PRT
<213>  Artificial Sequence

<220>
<223>  Synthetic peptide

<400>  151

Gln Gly Leu Glu Trp Met Gly Tyr Ala Asn Pro Ser Thr Gly Tyr Thr
1               5                   10                  15

Glu Tyr

<210>  152
<211>  18
<212>  PRT
<213>  Artificial Sequence

<220>
<223>  Synthetic peptide

<400>  152

Gln Gly Leu Glu Trp Val Gly Tyr Ile Asn Pro Ser Ala Gly Tyr Thr
1               5                   10                  15

Glu Tyr

<210>  153

<211>  18
<212>  PRT
<213>  Artificial Sequence

<220>
<223>  Synthetic peptide

<400>  153

Gln Gly Leu Glu Trp Val Gly Tyr Ile Asn Pro Ser Val Gly Tyr Thr
1               5                   10                  15

Glu Tyr

<210>  154
<211>  18
<212>  PRT
<213>  Artificial Sequence

<220>
<223>  Synthetic peptide

<400>  154

Gln Gly Leu Glu Trp Val Gly Tyr Ile Asn Pro Ser Ser Gly Tyr Thr
1               5                   10                  15

Glu Tyr

<210>  155
<211>  18
<212>  PRT
<213>  Artificial Sequence

<220>
<223>  Synthetic peptide

<400>  155

Gln Gly Leu Glu Trp Leu Gly Tyr Ile Asn Pro Ser Ala Gly Tyr Thr
1               5                   10                  15

Glu Tyr

<210>  156
<211>  18
<212>  PRT
<213>  Artificial Sequence

<220>
<223>  Synthetic peptide

<400>  156

Gln Gly Leu Glu Trp Leu Gly Tyr Ile Asn Pro Ser Val Gly Tyr Thr
1               5                   10                  15

Glu Tyr

<210>  157
<211>  18
<212>  PRT
<213>  Artificial Sequence

<220>
<223>  Synthetic peptide

<400>  157

Gln Gly Leu Glu Trp Leu Gly Tyr Ile Asn Pro Ser Ser Gly Tyr Thr
1               5                   10                  15

Glu Tyr

<210>  158
<211>  18
<212>  PRT
<213>  Artificial Sequence

<220>
<223>  Synthetic peptide

<400>  158

Gln Gly Leu Glu Trp Met Gly Tyr Ile Asn Pro Ser Ala Gly Tyr Thr
1               5                   10                  15

Glu Tyr

<210>  159
<211>  18
<212>  PRT
<213>  Artificial Sequence

<220>
<223>  Synthetic peptide

<400>  159

Gln Gly Leu Glu Trp Met Gly Tyr Ile Asn Pro Ser Val Gly Tyr Thr
1               5                   10                  15

Glu Tyr

<210>  160

<211>  18
<212>  PRT
<213>  Artificial Sequence

<220>
<223>  Synthetic peptide

<400>  160

Gln Gly Leu Glu Trp Met Gly Tyr Ile Asn Pro Ser Ser Gly Tyr Thr
1               5                   10                  15

Glu Tyr

<210>  161
<211>  18
<212>  PRT
<213>  Artificial Sequence

<220>
<223>  Synthetic peptide

<400>  161

Gln Gly Leu Glu Trp Val Gly Tyr Ile Asn Pro Ser Thr Gly Ala Thr
1               5                   10                  15

Glu Tyr

<210>  162
<211>  18
<212>  PRT
<213>  Artificial Sequence

<220>
<223>  Synthetic peptide

<400>  162

Gln Gly Leu Glu Trp Leu Gly Tyr Ile Asn Pro Ser Thr Gly Ala Thr
1               5                   10                  15

Glu Tyr

<210>  163
<211>  18
<212>  PRT
<213>  Artificial Sequence

<220>
<223>  Synthetic peptide

<400>  163

Gln Gly Leu Glu Trp Met Gly Tyr Ile Asn Pro Ser Thr Gly Ala Thr
1               5                   10                  15

Glu Tyr

<210>  164
<211>  18
<212>  PRT
<213>  Artificial Sequence

<220>
<223>  Synthetic peptide

<400>  164

Gln Gly Leu Glu Trp Ile Gly Tyr Leu Asn Pro Ser Ala Gly Tyr Thr
1               5                   10                  15

Glu Tyr

<210>  165
<211>  18
<212>  PRT
<213>  Artificial Sequence

<220>
<223>  Synthetic peptide

<400>  165

Gln Gly Leu Glu Trp Ile Gly Tyr Leu Asn Pro Ser Val Gly Tyr Thr
1               5                   10                  15

Glu Tyr

<210>  166
<211>  18
<212>  PRT
<213>  Artificial Sequence

<220>
<223>  Synthetic peptide

<400>  166

Gln Gly Leu Glu Trp Ile Gly Tyr Leu Asn Pro Ser Ser Gly Tyr Thr
1               5                   10                  15

Glu Tyr

<210>  167

<211>  18
<212>  PRT
<213>  Artificial Sequence

<220>
<223>  Synthetic peptide

<400>  167

Gln Gly Leu Glu Trp Ile Gly Tyr Val Asn Pro Ser Ala Gly Tyr Thr
1               5                   10                  15

Glu Tyr

<210>  168
<211>  18
<212>  PRT
<213>  Artificial Sequence

<220>
<223>  Synthetic peptide

<400>  168

Gln Gly Leu Glu Trp Ile Gly Tyr Val Asn Pro Ser Val Gly Tyr Thr
1               5                   10                  15

Glu Tyr

<210>  169
<211>  18
<212>  PRT
<213>  Artificial Sequence

<220>
<223>  Synthetic peptide

<400>  169

Gln Gly Leu Glu Trp Ile Gly Tyr Val Asn Pro Ser Ser Gly Tyr Thr
1               5                   10                  15

Glu Tyr

<210>  170
<211>  18
<212>  PRT
<213>  Artificial Sequence

<220>
<223>  Synthetic peptide

<400>  170

Gln Gly Leu Glu Trp Ile Gly Tyr Ala Asn Pro Ser Ala Gly Tyr Thr
1               5                   10                  15

Glu Tyr

<210>  171
<211>  18
<212>  PRT
<213>  Artificial Sequence

<220>
<223>  Synthetic peptide

<400>  171

Gln Gly Leu Glu Trp Ile Gly Tyr Ala Asn Pro Ser Val Gly Tyr Thr
1               5                   10                  15

Glu Tyr

<210>  172
<211>  18
<212>  PRT
<213>  Artificial Sequence

<220>
<223>  Synthetic peptide

<400>  172

Gln Gly Leu Glu Trp Ile Gly Tyr Ala Asn Pro Ser Ser Gly Tyr Thr
1               5                   10                  15

Glu Tyr

<210>  173
<211>  18
<212>  PRT
<213>  Artificial Sequence

<220>
<223>  Synthetic peptide

<400>  173

Gln Gly Leu Glu Trp Ile Gly Tyr Leu Asn Pro Ser Thr Gly Ala Thr
1               5                   10                  15

Glu Tyr

<210>  174

<211>  18
<212>  PRT
<213>  Artificial Sequence

<220>
<223>  Synthetic peptide

<400>  174

Gln Gly Leu Glu Trp Ile Gly Tyr Val Asn Pro Ser Thr Gly Ala Thr
1               5                   10                  15

Glu Tyr

<210>  175
<211>  18
<212>  PRT
<213>  Artificial Sequence

<220>
<223>  Synthetic peptide

<400>  175

Gln Gly Leu Glu Trp Ile Gly Tyr Ala Asn Pro Ser Thr Gly Ala Thr
1               5                   10                  15

Glu Tyr

<210>  176
<211>  18
<212>  PRT
<213>  Artificial Sequence

<220>
<223>  Synthetic peptide

<400>  176

Gln Gly Leu Glu Trp Ile Gly Tyr Ile Asn Pro Ser Ala Gly Ala Thr
1               5                   10                  15

Glu Tyr

<210>  177
<211>  18
<212>  PRT
<213>  Artificial Sequence

<220>
<223>  Synthetic peptide

<400>  177

Gln Gly Leu Glu Trp Ile Gly Tyr Ile Asn Pro Ser Val Gly Ala Thr
1               5                   10                  15

Glu Tyr

<210>  178
<211>  18
<212>  PRT
<213>  Artificial Sequence

<220>
<223>  Synthetic peptide

<400>  178

Gln Gly Leu Glu Trp Ile Gly Tyr Ile Asn Pro Ser Ser Gly Ala Thr
1               5                   10                  15

Glu Tyr

<210>  179
<211>  17
<212>  PRT
<213>  Artificial Sequence

<220>
<223>  Synthetic peptide

<400>  179

Gln Gly Leu Glu Trp Ile Gly Tyr Ile Asn Pro Ser Thr Gly Tyr Thr
1               5                   10                  15

Glu

<210>  180
<211>  17
<212>  PRT
<213>  Artificial Sequence

<220>
<223>  Synthetic peptide

<400>  180

Gln Gly Leu Glu Trp Met Gly Tyr Ile Asn Pro Ser Thr Gly Tyr Thr
1               5                   10                  15

Glu

<210>  181

<211>  17
<212>  PRT
<213>  Artificial Sequence

<220>
<223>  Synthetic peptide

<400>  181

Gln Gly Leu Glu Trp Met Gly Tyr Leu Asn Pro Ser Thr Gly Tyr Thr
1               5                   10                  15

Glu

<210>  182
<211>  17
<212>  PRT
<213>  Artificial Sequence

<220>
<223>  Synthetic peptide

<400>  182

Gln Gly Leu Glu Trp Val Gly Tyr Ile Asn Pro Ser Ser Gly Tyr Thr
1               5                   10                  15

Glu

<210>  183
<211>  17
<212>  PRT
<213>  Artificial Sequence

<220>
<223>  Synthetic peptide

<400>  183

Gln Gly Leu Glu Trp Met Gly Tyr Ile Asn Pro Ser Ser Gly Tyr Thr
1               5                   10                  15

Glu

<210>  184
<211>  4
<212>  PRT
<213>  Artificial Sequence

<220>
<223>  Synthetic peptide

<400>  184

Thr Thr Ser Asp
1

<210> 185
<211> 9
<212> PRT
<213> Artificial Sequence

<220>
<223> Synthetic peptide

<400> 185

Ser Ala Ser Ser Ser Ile Ser Tyr Met
1               5

<210> 186
<211> 4
<212> PRT
<213> Artificial Sequence

<220>
<223> Synthetic peptide

<400> 186

Val Phe Asp Tyr
1

<210> 187
<211> 9
<212> PRT
<213> Artificial Sequence

<220>
<223> Synthetic peptide

<400> 187

His Gln Arg Ser Thr Tyr Pro Leu Tyr
1               5

<210> 188
<211> 110
<212> PRT
<213> Homo sapiens

<400> 188

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys
            100                 105                 110

<210>  189
<211>  107
<212>  PRT
<213>  Homo sapiens

<400>  189

Gly Gln Pro Arg Glu Pro Gln Val Tyr Thr Leu Pro Pro Ser Arg Asp
1               5                   10                  15

Glu Leu Thr Lys Asn Gln Val Ser Leu Thr Cys Leu Val Lys Gly Phe
            20                  25                  30

Tyr Pro Ser Asp Ile Ala Val Glu Trp Glu Ser Asn Gly Gln Pro Glu
        35                  40                  45

Asn Asn Tyr Lys Thr Thr Pro Pro Val Leu Asp Ser Asp Gly Ser Phe
    50                  55                  60

Phe Leu Tyr Ser Lys Leu Thr Val Asp Lys Ser Arg Trp Gln Gln Gly
65                  70                  75                  80

Asn Val Phe Ser Cys Ser Val Met His Glu Ala Leu His Asn His Tyr
                85                  90                  95

Thr Gln Lys Ser Leu Ser Leu Ser Pro Gly Lys
            100                 105

## Description

### 1. CROSS REFERENCE TO RELATED APPLICATIONS

This application claims the benefit under 35 U.S.C. § 119(e) of provisional application no. 61/798,235, which was filed March 15, 2013, and is incorporated by reference in its entirety.

### 2. SEQUENCE LISTING

The application contains a Sequence Listing which has been submitted electronically in ASCII format and is hereby incorporated by reference in its entirety. Said ASCII copy, created on March 13, 2014, is named 381493-885WO (129682)_SL.txt and is 67,649 bytes in size.

### 3. FIELD OF THE INVENTION

The present invention relates to anti-CD25 antibodies, pharmaceutical compositions comprising anti-CD25 antibodies, and therapeutic uses of such antibodies.

### 4. BACKGROUND

The high affinity interleukin-2 receptor (IL2-R) is a heterotrimeric cell surface receptor composed of α, β, and γ_{c}-polypeptide chains (K_{D} 10⁻¹¹ M). The 55 kDa α-chain, also known as IL2-Rα, CD25, p55, and Tac (T cell activation) antigen, is unique to the IL2-R. The β (CD122; P75) and γ_{c} (CD132) chains are part of a cytokine receptor superfamily (hematopoietin receptors) and are functional components of other cytokine receptors, such as IL-15R (Waldmann, 1993, Immunol. Today 14(6):264-70; Ellery et al., 2002, Cytokine Growth Factor Rev. 13(1): 27-40). The intermediate affinity receptor is a dimer composed of a β- and a γ_{c}-chain (K_{D} 10⁻⁹ M) while the low affinity receptor consists of a monomeric α-subunit that has no signal transduction capacity (K_{D} 10⁻⁸ M) (Waldmann, 1993, Immunol. Today 14(6):264-70).

Resting T cells, B cells, and monocytes express few CD25 molecules. However, the receptor is rapidly transcribed and expressed upon activation (Ellery et al., 2002, Cytokine Growth Factor Rev. 13(1): 27-40; Morris et al., 2000, Ann. Rheum. Dis. 59 (Suppl. 1):1109-14). Cells expressing the high affinityIL2-R express CD25 (the CD25-subunit) in excess which leads to both high and low affinity IL2 binding profiles (Waldmann et al., 1993, Blood 82(6):1701-12; de Jong et al., 1996, J. Immunol. 156(4):1339-48). The anti-CD25 antibody daclizumab, which is a humanized anti-CD25 antibody previously marketed under the trade name ZENAPAX, has shown clinical efficacy in a variety of such conditions involving the immune system, such as organ transplant rejection (reviewed by Pascual et al., 2001, J. Heart Lung Transplant. 20(12):1282-90), asthma (see, *e.g.,* Busse et al., 2008, Am. J. Respir. Crit. Care Med. 178(10):1002-1008), multiple sclerosis (see, *e.g.,* Bielekova et al., 2009, Arch Neurol. 66(4):483-9), uveitis (Nussenblatt, 1999, Proc. Nat'l. Acad. USA 96:7462-7466), ocular inflammation (Bhat et al., 2009, Graefes Arch. Clin. Exp. Ophthalmol. 247:687-692) and human T cell leukemia virus-1 associated T-cell leukemia (Berkowitz et al., 2010, Journal of Clinical Oncology, 2010 ASCO Annual Meeting Proceedings 28 (May 20 Supplement):8043).

Citation or identification of any reference in Section 2 or in any other section of this application shall not be construed as an admission that such reference is available as prior art to the present disclosure.

### 5. SUMMARY

The present disclosure relates to anti-CD25 antibodies that are related in sequence to the anti-CD25 antibody daclizumab but are characterized by improved properties, such as increased affinity to CD25, increased inhibition of IL2 activity (such as the ability to inhibit IL2-induced T-cell proliferation), or reduced immunogenicity. Interestingly, the inventors have discovered that the ability to inhibit IL2 activity does not always correlate to affinity to CD25. Moreover, the present inventors have identified certain amino acids substitutions that reduce daclizumab's immunogenicity and improve its inhibition of IL2 activity.

The daclizumab heavy chain (SEQ ID NO:1) has a variable region containing 4 framework regions (FRs), referred to (in amino- to carboxy-terminal order) as FR-H1, FR-H2, FR-H3 and FR-H4, separated by three heavy chain complementarity determining regions (CDRs), referred to herein (in amino- to carboxy-terminal order) as CDR-H1, CDR-H2 and CDR-H3. The heavy chain CDR sequences of daclizumab are designated SEQ ID NO:4 (CDR-H1); SEQ ID NO:6 (CDR-H2); and SEQ ID NO:8 (CDR-H3). The heavy chain FR sequences of daclizumab are designated SEQ ID NO:3 (FR-H1); SEQ ID NO:5 (FR-H2); SEQ ID NO:7 (FR-H3); and SEQ ID NO:9 (FR-H4).

Likewise, the daclizumab light chain (SEQ ID NO:2) has a variable region containing four framework regions, referred to (in amino- to carboxy-terminal order) as FR-L1, FR-L2, FR-L3 and FR-L4, separated by three light chain CDRs referred to herein (in amino- to carboxy-terminal order) as CDR-L1, CDR-L2 and CDR-L3. The light chain CDR sequences of daclizumab are designated SEQ ID NO:11 (CDR-L1); SEQ ID NO:13 (CDR-L2) and SEQ ID NO:15 (CDR-L3). The FR sequences of daclizumab are designated SEQ ID NO:10 (FR-L1); SEQ ID NO:12 (FR-L2); SEQ ID NO:14 (FR-L3); and SEQ ID NO:16 (FR-L4).

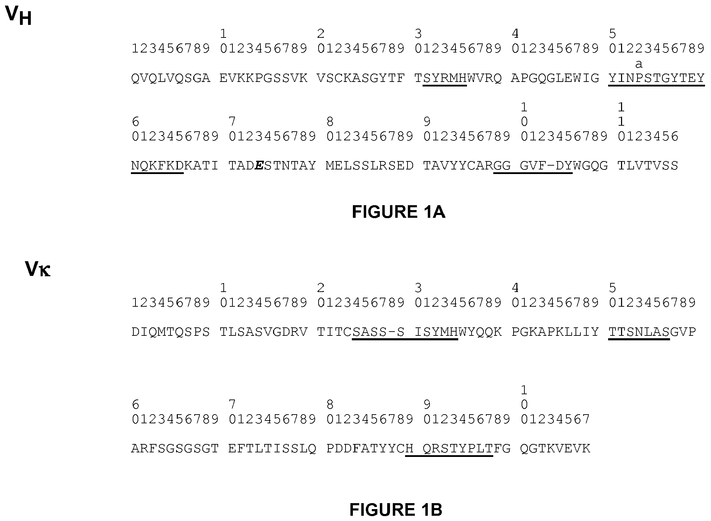

The present disclosure provides antibodies and binding fragments that are related in CDR sequence to the CDRs of daclizumab. The antibodies and binding fragments can also have FR sequences that are related to the FR sequences of daclizumab. Accordingly, in some aspects, the antibodies and fragments of the disclosure comprise V_{H} and V_{L} sequences that are related in sequence to the V_{H} and V_{L} regions of daclizumab. The sequences of the daclizumab variable regions are shown in Figure 1A and 1B, and the numbering of the CDRs and framework regions is set forth in Table 1 (for the heavy chain) and Table 2 (for the light chain).

In some embodiments, the anti-CD25 antibodies or anti-CD25 binding fragments of the disclosure (collectively termed "anti-CD25 antibodies") are characterized by one, two, three, four or all five of the following properties (a)(i) through (a)(v) and one or both properties (b)(i) through (b)(ii):
(a)
   (i) the anti-CD25 antibodies comprise altogether at least 2, at least 3, at least 4 or at least 5 amino acid substitutions as compared to the V_{H} and V_{L} sequences variable regions of SEQ ID NO:1 and SEQ ID NO:2;
   (ii) the six CDRs of the anti-CD25 antibodies altogether have up to 8, up to 7, up to 6, up to 5, or up to 4 amino acid substitutions as compared to the CDR sequences SEQ ID NOs:4, 6, 8, 11, 13, and 15;
   (iii) any individual CDR has no more than 3 amino acid substitutions as compared to the corresponding CDR sequence of an antibody having CDRs of SEQ ID NOs:4, 6, 8, 11, 13, and 15, or any individual CDR other than CDR-H2 has no more than 2 amino acid substitutions as compared to the corresponding CDR sequence of an antibody having CDRs of SEQ ID NOs:4, 6, 8, 11, 13, and 15;
   (iv) the individual framework regions have no more than 1, 2, 3, 4, or 5 amino acid substitutions as compared to the corresponding framework sequence of an antibody having framework sequences of SEQ ID NOs:3, 5, 7, 9, 10, 12, 14 and 16; and/or
   (v) the V_{H} and V_{L} sequences of the antibodies of the disclosure have at least 75% sequence identity (and in certain embodiments, at least 80%, at least 85%, at least 90%, at least 95%, at least 98%, or at least 99% sequence identity) to the V_{H} and V_{L} sequences of daclizumab (SEQ ID NO:1 and SEQ ID NO:2); and
(b)
   (i) the anti-CD25 antibodies include at least one amino acid substitution in at least one CDR as compared to daclizumab; and/or
   (ii) the anti-CD25 antibodies include at least one amino acid substitution in at least one framework region as compared to daclizumab.

Exemplary individual CDR and FR substitutions that can be incorporated into the anti-CD25 antibodies of the disclosure, alone or in combination, are set forth in Tables 6-8 and 11-21.

Preferably, the anti-CD25 antibodies of the disclosure include at least one amino acid substitution set forth in Table 6A and/or at least one combination of substitutions from Tables 7A-7C. Thus, in particular embodiments, the anti-CD25 antibodies of the disclosure include at least one substitution from S1, S2, S3, S4, S5, S6, S7, S8, S9, S10, S11, S12, S13, S14, S15, S16, S17, S18, S19, S20, S21, S22, S23, S24, S25, S26, S27, S28, S29, S30, S31, S32, S33, S34, S35, S36, S37, S38, S39, S40, S41, S42, S43, S44, S45, S46, S47, S48, S49, S50, S51, S52, S53, S54, S55, S56, S57, S58, S59, S60, S61, S62, S63, S64, S65, S66, S67, S68, S69, S70, S71, S72, S73, S74, S75, S76, S77, S78 and S79 (see Table 6A) and/or at least one combination of substitutions from C1, C2, C3, C4, C5, C6, C7, C8, C9, C10, C11, C12, C13, C14, C15, C16, C17, C18, C19, C20, C21, C22, C23, C24, C25, C26, C27, C28, C29, C30, C31, C32, C33, C34, C35, C36, C37, C38, C39, C40, C41, C42, C43, C44, C45, C46, C47, C48, C49, C50, C51, C52, C53, C54, C55, C56, C57, C58, C59, C60, C61, C62, and C63 (see Tables 7A-7C). Optionally, the antibodies of the disclosure can also include one or more substitutions or combinations of substutitions set forth in Tables 8, 11-21 and 22-1 through 22-9.

In specific embodiments, the percentage sequence identity for the heavy chain and the light chain compared to the V_{H} and V_{L} sequences of daclizumab is independently selected from at least 75%, at least 80%, at least 85%, at least 90%, at least 95% sequence identity, or at least 99% sequence identity. In certain aspects, the antibodies of the disclosure have V_{H} and/or V_{L} sequences having at least 95%, at least 98% or at least 99% sequence identity to the V_{H} and/or V_{L} sequences of daclizumab.

In various aspects, the antibodies of the disclosure have (a) up to 17 amino acid substitutions in their CDRs as compared to daclizumab and/or (b) up to 20 amino acid substitutions in their framework regions as compared to daclizumab. In specific embodiments of (a), the antibodies of the disclosure have up to 2, up to 3, up to 4, up to 5, up to 6, up to 7, up to 8, up to 9, up to 10, up to 11, up to 12, up to 13, up to 14, up to 15, up to 16, or up to 17 amino acid substitutions in their CDRs as compared to daclizumab. In specific embodiments of (b), the antibodies of the disclosure have up to 1, up to 2, up to 3, up to 4, up to 5, up to 6, up to 7, up to 8, up to 9, up to 10, up to 11, up to 12, up to 13, up to 14, up to 15, up to 16, up to 17, up to 18, up to 19 or up to amino acid substitutions in their CDRs as compared to daclizumab.

Activity of antibodies of the disclosure can be determined by measuring an IC₅₀ in an IL2-dependent T-cell proliferation assay, described further in Section 5.4. IC₅₀ measurements permit comparisons amongst various antibodies. Accordingly, in one aspect, the disclosure provides monoclonal anti-CD25 antibodies or an anti-CD25 binding fragments of monoclonal antibodies, which: (a) bind to human CD25; (b) comprise CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); and (c) have an IC₅₀ of up to 50% of the IC₅₀ of a corresponding antibody having CDRs of SEQ ID NOs:4, 6, 8, 11, 13, and 15 in an IL2-dependent T-cell proliferation assay.

In typical embodiments, the IC₅₀ can be up to 50%, up to 40%, or up to 30% the IC₅₀ of a corresponding antibody having CDRs of SEQ ID NOs:4, 6, 8, 11, 13, and 15 in an IL2-dependent T-cell proliferation assay.

In certain aspects, the anti-CD25 antibodies of the disclosure can comprise various amino acid substitutions that the inventors have shown to reduce daclizumab's immunogenicity and/or improve its inhibition of IL2 activity. In some embodiments, the anti-CD25 antibodies comprise the amino acid substitutions N52K and T54R in CDR-H2 as compared to CDR-H2 of SEQ ID NO:6. In some embodiments, the anti-CD25 antibodies comprise the amino acid substitution N53E in CDR-L2 as compared to CDR-L2 of SEQ ID NO:13. In some embodiments, the anti-CD25 antibodies comprise the amino acid substitutions N52S, S53R and T54K in CDR-H2 as compared to CDR-H2 of SEQ ID NO:6 and N53E in CDR-L2 as compared to CDR-L2 of SEQ ID NO:13.

Anti-CD25 antibodies may also comprise substitutions within their framework regions. In some embodiments, the anti-CD25 antibodies comprise framework regions with up to 4 amino acid substitutions as compared to frameworks of SEQ ID NO:3 (FR-H1), SEQ ID NO:5 (FR-H2), SEQ ID NO:7 (FR-H3), SEQ ID NO:9 (FR-H4), SEQ ID NO:10 (FR-L1), SEQ ID NO:12 (FR-L2), SEQ ID NO:14 (FR-L3) and SEQ ID NO:16 (FR-L4). In particular embodiments, the anti-CD25 antibodies have reduced T cell immunogenicity as compared to an anti-CD25 antibody or anti-CD25 binding fragment comprising heavy and light variable regions of SEQ ID NO:1 and SEQ ID NO:2.

In specific embodiments, the anti-CD25 antibodies comprise the amino acid substitution I48M in FR-H2 as compared to a FR-H2 of SEQ ID NO:5. In specific embodiments, the anti-CD25 antibodies Lck the amino acid substitution I48M in FR-H2 as compared to a FR-H2 of SEQ ID NO:5.

In another aspect, the anti-CD25 antibodies can be characterized in comparison to daclizumab. Thus, the disclosure provides anti-CD25 antibodies which (a) bind to human CD25; (b) comprise heavy and light chain variable regions having up to 12, up to 11, up to 10, up to 9, up to 8, up to 7, up to 6, up to 5 or up to 4 amino acid substitutions as compared to the heavy and variable regions of SEQ ID NO:1 and SEQ ID NO:2, respectively; and (c) have an IC₅₀ of up to 50% of the IC₅₀ of a corresponding antibody having the heavy and light variable regions of SEQ ID NO:1 and SEQ ID NO:2, respectively, in an IL2-dependent T-cell proliferation assay.

In typical embodiments, the IC₅₀ can be up to 50%, up to 40%, or up to 30% the IC₅₀ of a corresponding antibody having the heavy and light variable regions of SEQ ID NO:1 and SEQ ID NO:2, respectively, in an IL2-dependent T-cell proliferation assay.

In various embodiments, the anti-CD25 antibodies comprise one or more specific substitutions, including the amino acid substitution I48M in FR-H2 as compared to a FR-H2 of SEQ ID NO:5; the amino acid substitutions N52K and T54R in CDR-H2 as compared to CDR-H2 of SEQ ID NO:6 and S29K in CDR-L1 as compared to CDR-L1 of SEQ ID NO:11 and N53D in CDR-L2 as compared to CDR-L2 of SEQ ID NO:13; the amino acid substitutions N52K and T54R in CDR-H2 as compared to CDR-H2 of SEQ ID NO:6 and N53E in CDR-L2 as compared to CDR-L2 of SEQ ID NO:13; the amino acid substitutions N52S, S53R and T54K in CDR-H2 as compared to CDR-H2 of SEQ ID NO:6; the amino acid substitution T54S in CDR-H2 as compared to a CDR-H2 of SEQ ID NO:6; the amino acid substitutions S29K in CDR-L1 as compared to CDR-L1 of SEQ ID NO:11 and N53D in CDR-L2 as compared to CDR-L2 of SEQ ID NO:13; the amino acid substitutions S53R and T54K in CDR-H2 as compared to CDR-H2 of SEQ ID NO:6; the amino acid substitutions S29K in CDR-L1 as compared to CDR-L1 of SEQ ID NO:11 and N53D in CDR-L2 as compared to CDR-L2 of SEQ ID NO:13; and combinations thereof.

Anti-CD25 antibodies can include one or more of the single or double amino acid substitutions shown in Table 20 (for heavy chain substitutions) and/or Table 21 (for light chain substitutions). The single amino acid substitutions in Tables 20 and 21 have at least been shown to have no detrimental effect, and in some cases have a beneficial effect, on CD25 binding in at preliminary binding assays. Thus, in one aspect the disclosure provides monoclonal anti-CD25 antibodies that (a) bind to human CD25; (b) comprise CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); and (c) have, as compared to an antibody with CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3), (i) heavy chains CDRs comprising at least one substitution present in any of the CDR variants H1-H354 as shown in Table 20; and/or (ii) light chain CDRs comprising at least one substitution present in any of the CDR variants L1-L288 and L649 as shown in Table 21.

In some embodiments, the anti-CD25 antibodies comprise at least two substitutions present in any of the CDR variants H361-H369, H405-H443, H449-H487; H493-H531; H537-H572; H578-H613; H619-H654; H660-H690; H696-H726; H732-H762; H768-H798; H804-H834; H840-H865; H871-H896; H902-H927; H933-H958; H964-H989; H995-H1015; H1021-H1041; H107-H1067; H1073-H1093; H1099-H1119; H1125-H1141; H1147-H1163; H1169-H1185; H1191-H1207; H1213-H1226; H1232-H1245; H1251-H1264; H1270-H1280; H1286-H1296; H1302-H1312; H1316-H1327; H1333-H1341; H1347-H1351; H1357-H1361; H1367-H1371; H1377-H1381; H1387-H1391; H1425-H1476; H1478-H1517; and H1519-H1558 as shown in Table 20 and/or at least two substitutions present in any of the CDR variants L289-L648 and L650-L679 as shown in Table 21.

Also provided are anti-CD25 antibodies with up to 12, up to 11, up to 10, up to 9, up to 8, up to 7, up to 6, up to 5 or up to 4 amino acid substitutions in their heavy chains as compared to the heavy chain variable region of SEQ ID NO:1. In some embodiments, these anti-CD25 antibodies have up to 12, up to 11, up to 10, up to 9, up to 8, up to 7, up to 6, up to 5 or up to 4 amino acid substitutions in their heavy chains as compared to the heavy chain variable region of SEQ ID NO:1, in combination with specific heavy chain substitutions that reduce immunogenicity, such as I48M; I48V; I51L; T54S; I48M and I51L; I48V and T54S; I48M and T54S. In other embodiments, the anti-CD25 antibodies have up to 12, up to 11, up to 10, up to 9, up to 8, up to 7, up to 6, up to 5 or up to 4 amino acid substitutions as compared to the light chain variable region of SEQ ID NO:2.

In one aspect, the disclosure provides monoclonal anti-CD25 antibodies which: (a) bind to human CD25; (b) have a heavy chain variable region which has up to 12, up to 11, up to 10, up to 9, up to 8, up to 7, up to 6, up to 5 or up to 4 amino acid substitutions as compared to the heavy chain variable region of SEQ ID NO:1, said heavy chain comprising at least one substitution or combination of substitutions as compared to a heavy chain of SEQ ID NO:1 selected from: (i) I48M; (ii) I48V; (iii) I51L; (iv) T54S; (v) I48M and I51L; (vi) I48V and T54S; and (vii) I48M and T54S; (c) have a light chain variable region which has up to 12, up to 11, up to 10, up to 9, up to 8, up to 7, up to 6, up to 5 or up to 4 amino acid substitutions as compared to the heavy chain variable region of SEQ ID NO:2.

In another aspect, the disclosure provides monoclonal anti-CD25 antibodies which: (a) bind to human CD25; (b) comprise heavy and light chain variable regions having up to 12, up to 11, up to 10, up to 9, up to 8, up to 7, up to 6, up to 5 or up to 4 amino acid substitutions as compared to the heavy and light variable regions of SEQ ID NO:1 and SEQ ID NO:2, respectively; and (c) comprise the amino acid substitutions present in any of the combination variants as shown in Tables 7A-7C, for example variants C1-C19, C21 and C24-C63.

In some embodiments, the anti-CD25 antibodies comprise at least one light chain CDR substitution from Table 8A and/or at least one heavy chain CDR substitution from Table 8B. In specific embodiments, the at least one light chain CDR substitution from Table 8A includes one or more of: (a) S24V in CDR-L1; (b) A25I, A25T or A25M in CDR-L1; (c) S26L in CDR-L1; (d) S27K, S27R, S27A, or S27N in CDR-L1; (e) S29A, S29K or S29R in CDR-L1; (f) M33G in CDR-L1; (g) T50A in CDR-L2; (h) S52A, S52V, S52D, S52E or S52M in CDR-L2; (i) N53A, N53D, N53E, N53F or N53Y in CDR-L2; (j) L54H in CDR-L2; (k) S56A in CDR-L2; (1) T93Q, T93R, T93M in CDR-L3; and (m) T97S in CDR-L3.

In specific embodiments, the at least one heavy chain CDR substitution from Table 8B includes one or more of: (a) S31F, S31K, S31R or S31W in CDR-H1; (b) Y32S, Y32T or Y32V in CDR-H1; (c) M34A, M34T or M34V in CDR-H1; (d) I51W, I51L, I51A, I51K or I51V in in CDR-H2; (e) N52A, N52K, N52R, N52S or N52V in CDR-H2; (f) S53K, S53T, S53P or S53A in CDR-H2; (g) T54A, T54K, T54S or T54V in CDR-H2; (h) Y56K, Y56R or Y56A in CDR-H2; (i) T57A, T57D or T57G in CDR-H2; (j) Y59E in CDR-H2; (k) F63S; (1) K64A, K64D, K64V or K64G in CDR-H2; (m) D101G in CDR-H3; and/or (n) Y102D, Y102K, Y102Q or Y102T in CDR-H3.

In some embodiments, the anti CD-25 antibodies of the disclosure comprise the amino acid substitutions (i) N52S, N52K, N52R or N52V and (ii) T54R, T54S or T54K in CDR-H2 as compared to CDR-H2 of SEQ ID NO:6, and, optionally, one or more of: (iii) S53R, S53K or S53N in CDR-H2 as compared to CDR-H2 of SEQ ID NO:6, (iv) Y56R in CDR-H2 as compared to CDR-H2 of SEQ ID NO:6, (v) E58Q in CDR-H2 as compared to CDR-H2 of SEQ ID NO:6, (vi) E73K in CDR-H2 as compared to CDR-H2 of SEQ ID NO:6, (vii) S29K in CDR-L1 as compared to CDR-L1 of SEQ ID NO:11 and (viii) N53D or N53E in CDR-L2 as compared to CDR-L2 of SEQ ID NO:13.

In some embodiments, the anti CD-25 antibodies of the disclosure comprise the amino acid substitutions (i) N52S, N52K, N52R or N52V, (ii) S53K, S53R or S53N and (iii) T54R, T54S or T54K in CDR-H2 as compared to CDR-H2 of SEQ ID NO:6, (iv) S29K in CDR-L1 as compared to CDR-L1 of SEQ ID NO:11 and (v) N53D or N53E in CDR-L2 as compared to CDR-L2 of SEQ ID NO:13, and, optionally, further comprises the amino acid substitution (iv) Y56R in CDR-H2 as compared to CDR-H2 of SEQ ID NO:6.

In some embodiments, the anti CD-25 antibodies of the disclosure comprise the amino acid substitutions (i) N52S, (ii) S53R or S53K, (iii) T54S or T54K, and (iv) Y56R in CDR-H2 as compared to CDR-H2 of SEQ ID NO:6, (v) S29K in CDR-L1 as compared to CDR-L1 of SEQ ID NO:11 and (vi) N53D in CDR-L2 as compared to CDR-L2 of SEQ ID NO:13.

In certain embodiments, the anti-CD25 antibodies comprise at least one light chain CDR substitution from Table 8A and/or at least one heavy chain CDR substitution from Table 8B in which a wild type non-histidine residue is substituted with histidine.

In certain specific embodiments, the anti-CD25 antibodies of the disclosure are characterized by the absence of particular amino acid substitutions. For example,in certain embodiments, the anti-CD25 antibodies of the disclosure are characterized by one or a combination of any two, three, four, five or all six of the following features:
(a) the V_{H} sequence does not consist of the V_{H} sequence of any of the variants XH1 to XH16 as shown Tables 22-1 to 22-3;
(b) the V_{L} sequence does not consist of the V_{L} sequence of any of the variants XL1 to XL25 as shown in Tables 22-4 to 22-8;
(c) the V_{H} and V_{L} sequences do not consist of the V_{H} and V_{L} sequences of antibodies XF1 through XF15 as shown in Table 22-9;
(d) the V_{H} sequence does not include the substitution E73K;
(e) the V_{H} of an anti-CD25 antibody of the disclosure does not include one, two, three or all for of the substitutions (i) S31K in CDR-L1; (ii) S31R in CDR-L1; (iii) S92K in CDR-L3 and (iv) S92R in CDR-L3 or, if such substitutions are present, the anti-CD25 antibody includes one or more other substitutions selected from Tables 6-8, 20 and 21; and
(e) the V_{L} of an anti-CD25 antibody of the disclosure does not include one, two, three or all for of the substitutions (i) N52K in CDR-H2; (ii) N52R in CDR-H2; (iii) S53R in CDR-H2 and (iv) T54R in CDR-H2 or, if such substitutions are present, the anti-CD25 antibody includes one or more other substitutions selected from Tables 6-8, 20 and 21.

Antibodies of the disclosure may be human or humanized antibodies, or anti-CD25 binding fragments thereof. In some embodiments, the antibodies are IgG, including IgG1, IgG2, IgG2 M3, and IgG4. The antibodies can be isotype IgG1 fa, but in specific embodiments, the antibodies are not isotype IgG1 fa. Disclosed antibodies can have Fc domains which comprise the substitution M428L and, optionally, further comprise the substitution T250Q. In some embodiments, the Fc domains comprise one or more substitutions selected from V263L, V266L, V273C, V273E, V273F, V273L, V273M, V273S, V273Y, V305K, and V305W.

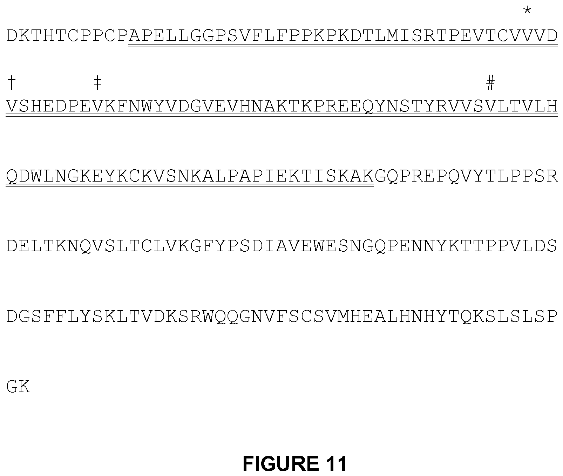

The anti-CD25 antibodies can have modifications relating to their Fc regions. The wild type Fc domain, from human IgG1 (SEQ ID NO:17) is shown in Figure 11. The CH2 domain (SEQ ID NO:188) of the wild type Fc domain is double underlined in Figure 11 and the CH3 domain (SEQ ID NO:189) of the wild type Fc domain is bolded in Figure 11. Accordingly, some disclosed anti-CD25 antibodies include one or more mutations in the Fc region that increases ADCC activity. In other embodiments, the anti-CD25 antibodies include one or more mutations in the Fc region that decreases ADCC activity (e.g., V263L, V273E, V273F, V273M, V273S, and V273Y). Antibodies of the disclosure may be non-fucosylated, and may include one or more mutations in the Fc region that increases binding to FcγR, decreases binding to FcγR, or increases binding to FcRn.

In some embodiments, anti-CD25 antibodies of the disclosure comprise variant Fc domains having up to 20, up to 15, up to 12, up to 10, up to 9, up to 8, up to 7, up to 6, up to 5 or up to 4 amino acid substitutions as compared to the CH2 domain of the Fc domain of SEQ ID NO:17

In some embodiments, anti-CD25 antibodies of the disclosure comprise variant Fc domains having at least 70%, at least 75%, at least 80%, at least 85%, at least 90%, at least 95%, at least 96%, at least 97%, at least 98% or at least 99% sequence identity to the Fc domain of SEQ ID NO:17.

In various embodiments, anti-CD25 antibodies of the disclosure comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (a) a V263 substitution that increases affinity towards FcγRIIB and decreases affinity towards FcγRIIIA; (b) a V266 substitution that increases affinity towards FcγRIIB and decreases affinity towards FcγRIIIA; and (c) a V273 substitution that increases affinity towards FcγRIIB and decreases affinity towards FcγRIIIA.

In other embodiments, anti-CD25 antibodies of the disclosure comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (a) V263 substitution that increases affinity towards FcγRIIB and decreases affinity towards FcγRIIIA; and (b) a V273 substitution that increases affinity towards FcγRIIB and decreases affinity towards FcγRIIIA.

In other embodiments, anti-CD25 antibodies of the disclosure comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In still other embodiments, anti-CD25 antibodies of the disclosure comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) a V273 substitution selected from V273C, V273E, V273F, V273L, V273M, V273S, V273Y.

In still other embodiments, anti-CD25 antibodies of the disclosure (a) comprise CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); (b) comprise the amino acid substitutions (i) N52S, N52K, N52R or N52V and (ii) T54R, T54S or T54K in CDR-H2 as compared to CDR-H2 of SEQ ID NO:6, and, optionally, one or more of: (iii) S53R, S53K or S53N in CDR-H2 as compared to CDR-H2 of SEQ ID NO:6, (iv) Y56R in CDR-H2 as compared to CDR-H2 of SEQ ID NO:6, (v) E58Q in CDR-H2 as compared to CDR-H2 of SEQ ID NO:6, (vi) E73K in CDR-H2 as compared to CDR-H2 of SEQ ID NO:6, (vii) S29K in CDR-L1 as compared to CDR-L1 of SEQ ID NO:11 and (viii) N53D or N53E in CDR-L2 as compared to CDR-L2 of SEQ ID NO:13; and, optionally (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In yet other embodiments, anti-CD25 antibodies of the disclosure (a) comprise CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); (b) comprise the amino acid substitutions (i) N52S, N52K, N52R or N52V, (ii) S53K, S53R or S53N and (iii) T54R, T54S or T54K in CDR-H2 as compared to CDR-H2 of SEQ ID NO:6, (iv) S29K in CDR-L1 as compared to CDR-L1 of SEQ ID NO:11 and (v) N53D or N53E in CDR-L2 as compared to CDR-L2 of SEQ ID NO:13, and, optionally, further comprises the amino acid substitution (iv) Y56R in CDR-H2 as compared to CDR-H2 of SEQ ID NO:6; and, optionally (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In yet other embodiments, anti-CD25 antibodies of the disclosure (a) comprise CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); (b) comprise the amino acid substitutions (i) N52S, (ii) S53R or S53K, (iii) T54S or T54K, and (iv) Y56R in CDR-H2 as compared to CDR-H2 of SEQ ID NO:6, (v) S29K in CDR-L1 as compared to CDR-L1 of SEQ ID NO:11 and (vi) N53D in CDR-L2 as compared to CDR-L2 of SEQ ID NO:13; and, optionally (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In yet other embodiments, anti-CD25 antibodies of the disclosure (a) comprise heavy and light chain variable regions having up to 12, up to 11, up to 10, up to 9, up to 8, up to 7, up to 6, up to 5 or up to 4 amino acid substitutions as compared to the heavy and light variable regions of SEQ ID NO:and SEQ ID NO:2, respectively; (b) has an IC₅₀ of up to 50% of the IC₅₀ of a corresponding antibody having the heavy and light variable regions of SEQ ID NO:1 and SEQ ID NO:2, respectively, in an IL2-dependent T-cell proliferation assay; and, optionally, (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In yet other embodiments, anti-CD25 antibodies of the disclosure (a) comprise CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); (b) has, as compared to an antibody with CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3), (i) heavy chains CDRs comprising at least one substitution present in any of the CDR variants H1-H354 as shown in Table 20; and/or (ii) light chain CDRs comprising at least one substitution present in any of the CDR variants L1-L288 and L649 as shown in Table 21; and, optionally, (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In yet other embodiments, anti-CD25 antibodies of the disclosure (a) comprise a heavy chain variable region which has up to 12, up to 11, up to 10, up to 9, up to 8, up to 7, up to 6, up to 5 or up to 4 amino acid substitutions as compared to the heavy chain variable region of SEQ ID NO:1, said heavy chain comprising at least one substitution or combination of substitutions as compared to a heavy chain of SEQ ID NO:1 selected from: (i) I48M; (ii) I48V; (iii) I51L; (iv) T54S; (v) I48M and I51L; (vi) I48V and T54S; and (vii) I48M and T54S; (b) has a light chain variable region which has up to 12, up to 11, up to 10, up to 9, up to 8, up to 7, up to 6, up to 5 or up to 4 amino acid substitutions as compared to the heavy chain variable region of SEQ ID NO:2; and, optionally, (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In yet other embodiments, anti-CD25 antibodies of the disclosure (a) comprise heavy and light chain variable regions having up to 12, up to 11, up to 10, up to 9, up to 8, up to 7, up to 6, up to 5 or up to 4 amino acid substitutions as compared to the heavy and light variable regions of SEQ ID NO:and SEQ ID NO:2, respectively; (b) comprise the amino acid substitutions present in any of the combination variants C1-C19, C21 and C24-C63, as shown in Tables 7A-7C; and, optionally, (c) comprise a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from: (i) the substitution V263L; and/or (ii) the substitution V266L; and/or (iii) a V273 substitution selected from V273C, V273E, V273F, V273L, V273M, V273S, V273Y; and/or (iv) a V305 substitution selected from V305K and V305W.

In one aspect, anti-CD25 antibodies of the disclosure exhibit improved affinity to CD25 compared to daclizumab. Accordingly, the anti-CD25 antibodies may have an affinity to CD25 that is 2-to 100-fold that of the affinity to CD25 of a corresponding antibody having V_{H} sequence corresponding to SEQ ID NO:1 and a V_{L} sequence corresponding to SEQ ID NO:2. In some embodiments, the antibodies of the disclosure exhibit improved affinity to CD25 by at least 1.2-fold, at least 1.5-fold, at least 2-fold, 3-fold, at least 4-fold at least 5-fold, at least 10-fold, at least 20-fold, at least 30-fold, at least 50-fold, at least 60-fold, at least 70-fold, at least 80-fold or at least 90-fold of a corresponding antibody having V_{H} sequence corresponding to SEQ ID NO:1 and a V_{L} sequence corresponding to SEQ ID NO:2, or exhibit a range of affinity between any pair of the foregoing values of improvement (*e*.*g*., 10-fold to 50-fold, 5-fold to 70-fold, 2-fold to 30-fold, 3-fold to 15-fold or 5-fold to 10-fold).

Anti-CD25 antibodies may be purified, and in some embodiments, purified to at least 85%, at least 90%, at least 95% or at least 98% homogeneity.

The present disclosure provides pharmaceutical compositions comprising the variant anti-CD25 antibodies of the disclosure, as well as antibody-drug conjugates comprising anti-CD25 antibodies of the disclosure.

Nucleic acids comprising nucleotide sequences encoding the anti-CD25 antibodies of the disclosure are provided herein, as are vectors comprising nucleic acids. Additionally, prokaryotic and eukaryotic host cells transformed with a vector comprising a nucleotide sequence encoding an anti-CD25 antibody are provided herein, as well as eukaryotic (such as mammalian) host cells engineered to express the nucleotide sequences. Methods of producing anti-CD25 antibodies by culturing host cells are also provided.

The anti-CD25 antibodies of the disclosure are useful in the treatment of a variety of immune conditions and cancers, such as organ transplant rejection, asthma, multiple sclerosis, uveitis, ocular inflammation and human T cell leukemia virus-1 associated T-cell leukemia.

It should be noted that the indefinite articles "a" and "an" and the definite article "the" are used in the present application, as is common in patent applications, to mean one or more unless the context clearly dictates otherwise. Further, the term "or" is used in the present application, as is common in patent applications, to mean the disjunctive "or" or the conjunctive "and."

All publications mentioned in this specification are herein incorporated by reference. Any discussion of documents, acts, materials, devices, articles or the like that has been included in this specification is solely for the purpose of providing a context for the present disclosure. It is not to be taken as an admission that any or all of these matters form part of the prior art base or were common general knowledge in the field relevant to the present disclosure as it existed anywhere before the priority date of this application.

The features and advantages of the disclosure will become further apparent from the following detailed description of embodiments thereof.

### 6. BRIEF DESCRIPTION OF THE TABLES AND FIGURES

The present application includes Tables and Figures in 5 separate parts: one part containing all the Figures; one part containing Tables 1-19; one part containing Table 20; one part containing Table 21; and one part containing Tables 22-1 to 22-9. All the parts are incorporated by reference herein.
**Table 1** shows the numbering of the amino acids in the heavy chain variable region of daclizumab.
**Table 2** shows the numbering of the amino acids in the light chain variable region of daclizumab.
**Table 3** shows a list of the amino acids incorporated into daclizumab combinatorial library. The amino acid complexity for V_{L} and V_{H} libraries are 69,984 and 34,848, respectively. The bold amino acids on the top of each column indicate the wild type. The amino acids enriched more than 3-times or more than 2 but less than 3-times than theoretical percentage after the final enrichment are underlined with double line or single line, respectively. The amino acid reduced to less than 0.5 of theoretical percentage after enrichment were shown in italic.
**Table 4** shows binding kinetics and biological function of daclizumab variants. For high affinity daclizumab variants, amino acid combination of V_{H} positions #52, 53, 54 and V_{L} #29, 53 are shown. Mutant amino acids were indicated in bold letters. Parental V_{H} -V_{L} (used as a transfection control) is denoted as NST-SN. V_{H} position #56 and 58 are not shown because they were heavily biased to parental amino acids after enrichment. For alanine mutations, wild type amino acid and the position substituted to alanine is shown (*e*.*g*., serine #31 changed to alanine is denoted as S31A). Association (kₒₙ) and dissociation (k_{off}) rate constant were determined using surface plasmon resonance in a BIAcore. Average numbers of at least three separate determinations are shown. The dissociation constant (K_{D}) was calculated from kₒₙ/k_{off}. Functional improvement was measured by the inhibition of proliferation of Kit225/K6 cells (n=2-3). The IC₅₀ value of parental daclizumab in functional assay was in a range of 0.12-0.23 nM for each set of experiment. The K_{D} and IC₅₀ values of daclizumab variants were normalized with those obtained from wild type daclizumab to calculate improvement in affinity and function, respectively. n.d.: not determined.
**Table 5** shows a dissection of daclizumab variants. Association (*k*ₒₙ) and dissociation (*k*_{off}) rate constant were determined using surface plasmon resonance in a BIAcore. Average numbers of at least three separate determinations are shown. The dissociation constant (K_{D}) was calculated from *k*_{off}/*k*ₒₙ n.d.: not determined. All variants and NST-SN (control) antibodies were expressed by cotransfecting a pair of heavy and light chain expression vectors after subcloning. (Fold improvement/ mutation). Functional improvement was measured by the inhibition of proliferation of Kit225/K6 cells. FACS binding, ELISA competition and proliferation inhibition assays were based on an average of two, 3 and 3-5 independent experiments, respectively.
**Table 6A-6B. Table 6A** summarizes the characteristics of variants of daclizumab with single CDR or framework amino acid substitutions that result in beneficial properties. * = similar to WT. **Table 6B** summarizes the results of testing of additional single amino acid substitutions tested in the heavy chain by ELISA direct binding to plate coated CD25.
**Tables 7A-7D. Tables 7A-7C** describes 63 variants (variants C1 through C63) of daclizumab with combinations of CDR and framework substitutions. The variants were grafted onto different constant regions, which are reflected in the "isotype" column. **Table 7D** provides kinetic and biological activities of selected combination variants. "ELISA" means improved binding in an ELISA competition assay. "FACS" means relative binding to Hut/Kit225 cells as measured by FACS. "Kit225" means improvement in inhibition of IL2-induced proliferation of Kit225 cells. CD56 NK expansion" means fold increase in the number of CD56^{bright} NK cells after culture of human PBMC with rhIL2 and the indicated anti-CD25 antibody variant. "Fold potency MLR" means fold improvement in inhibition of a human cell-based mixed lymphocyte response. The figures for the ELISA, KIT225, MLR and CD56 assays represent the improvement over combination variant C27 (having the substitutions I48M (in framework 2 of the daclizumab heavy chain) and T54S (in CDR2 of the daclizumab heavy chain)).
**Tables 8A-8B** shows mutations in the daclizumab CDRs that do not significantly impact binding when assessed in the context of a population assay. **Table 8A:** mutations in the daclizumab heavy chain CDRs that do not substantially impact CD25 binding and can be incorporated into the antibodies of the disclosure. **Table 8B:** mutations in the daclizumab light chain CDRs that do not substantially impact CD25 binding and can be incorporated into the antibodies of the disclosure. Table 8A discloses the wild-type sequences as SEQ ID NOS 185, 13 and 15, and **Table 8B** discloses the wild-type sequences as SEQ ID NOS 6 and 186, all respectively, in order of appearance.
**Table 9** shows daclizumab VH and VL peptides as tested in the I-mune Assay™. Each peptide is 15 amino acids in length, offset by three amino acids. CDR amino acids are underlined. Table 9 discloses the "VL peptides" as SEQ ID NOS 60-91 and the "VH peptides" as SEQ ID NOS 92-127, all respectively, in order of appearance.
**Table 10** shows the sequences of E.HAT-VH synthetic oligonucleotides (SEQ ID NOS: 128-131, respectively, in order of appearance).
**Table 11** shows VH epitope region amino acid variants selected for testing in the I-mune Assay. "Percent" designates the percentage of the total donors tested (n=78) with stimulation indexes equal to or grater an 2.95. "Ave SI" is the average stimulation index for all donors tested. S.e.m. is the standard error of the mean of the average stimulation index. **Table 11** discloses SEQ ID NOS 132-178 and 132, respectively, in order of appearance.
**Table 12** shows compiled proliferative response data for single amino acid variants of the daclizumab VH epitope region. "P" designates the parental epitope peptide sequence. The number greater than 2.95 indicates the total number of donor samples tested that proliferated with a stimulation index (SI) of 2.95 or greater. The percent of responders indicates the percent of donors whose CD4+ T cells responded with a stimulation index of 2.95 or greater. The average SI is the average stimulation index of all tested donors. The t-test is a comparison of the stimulation index results for the I48M variant compared to responses for the parental peptide.
**Table 13** shows compiled proliferation response data for double amino acid variants of the daclizumab VH epitope region. "P" designates the parental epitope peptide sequence. The number greater than 2.95 indicates the total number of donor samples tested that proliferated with a stimulation index (SI) of 2.95 or greater. The percent of responders indicates the percent of donors whose CD4+ T cells responded with a stimulation index of 2.95 or greater. The average SI is the average stimulation index of all tested donors. The t-test is a comparison of the stimulation index results for the designated variant compared to responses for the parental peptide.
**Table 14** shows compiled response data for four selected daclizumab epitope region variants. The top panel is data compiled from all 78 tested donors. The bottom panel is data from donors showing a response of 2.95 or greater to the parent peptide (n=18). The number greater than 2.95 indicates the total number of donor samples tested that proliferated with a stimulation index (SI) of 2.95 or greater. The percent of responders indicates the percent of donors whose CD4+ T cells responded with a stimulation index of 2.95 or greater. The average SI is the average stimulation index of all tested donors. The t-test is a comparison of the stimulation index results for the designated variant compared to responses for the parental peptide. **Table 14** discloses SEQ ID NOS 132, 135, 149, 154, 160, 132 and 179-183, respectively, in order of appearance.
**Table 15** shows IL2-Rα (CD25) binding potency of daclizumab HYP (daclizumab manufactured by a high yield process), E.HAT and the single amino acid variants. Binding is measured in an ELISA format.
**Table 16** shows IL2-Rα binding potency of daclizumab HYP, E.HAT and the double amino acid variants. Binding is measured in an ELISA format.
**Table 17** shows affinity measurements of the single amino acid variant antibody molecules as measured by surface plasmon resonance.
**Table 18** shows affinity measurements of the double amino acid variant antibody molecules for human CD25 as measured by surface plasmon resonance.
**Table 19** shows affinity measurements of the double amino acid variant antibody molecules for cynomolgous monkey CD25 as measured by surface plasmon resonance.
**Table 20** shows the sequences exemplary species of heavy chain CDR and FR variants of daclizumab. **Table 20** discloses the wild-type sequences as SEQ ID NOS 6 and 186, respectively, in order of appearance.
**Table 21** shows the sequences exemplary species of light chain CDR variants of daclizumab. Table 21 discloses the wild-type sequences as SEQ ID NOS 11, 13 and 187, respectively, in order of appearance.
**Tables 22-1 to 22-9** shows the sequences anti-CD25 antibodies disclosed in U.S. Patent No. 8,314,213, incorporated by reference herein in its entirety. The 16 V_{H} variant sequences of U.S. Patent No. 8,314,213 are reproduced **Tables 22-1 to 22-3** and designated XH1 to XH16. The 24 V_{L} sequences of U.S. Patent No. 8,314,213 are reproduced in **Tables 22-4 to 22-8** and designated XL1 to XL25. The 25 variant antibody molecules generated in U.S. Patent No. 8,314,213 by combining different variant V_{H} and V_{L} sequences are set defined in **Table 22-9**, which designates the combinations XF1 through XF25. Table 22-1 discloses the wild-type sequences as SEQ ID NOS 4-5, **Table 22-2** discloses the wild-type sequence as SEQ ID NO:6, Table 22-3 discloses the wild-type sequence as SEQ ID NO:7, **Table 22-4** discloses the wild-type sequence as SEQ ID NO:10, **Table 22-5** discloses the wild-type sequences as SEQ ID NOS 11-12, Table 22-6 discloses the wild-type sequence as SEQ ID NO:13, Table 22-7 discloses the wild-type sequence as SEQ ID NO:14 and **Table 22-8** discloses the wild-type sequences as SEQ ID NOS 15-16, all respectively, in order of appearance.
**Figures 1A**-**1B** show the amino acid sequences of the daclizumab heavy and light chain variable regions, SEQ ID NO:1 and SEQ ID NO:2, respectively, with CDR regions in underlined text.
**Figures 2A-2D****.** **Figures 2A-2B** show the amino acid sequences utlized in the rehumanization of daclizumab (see Example 1). (**Figure 2A** discloses SEQ ID NOS 1 and 52-55, and Figure 2B discloses SEQ ID NOS 2 and 56-59, all respectively, in order of appearance). **Figure 2C** shows impact of rehumanization on affinity of daclizumab to CD25. **Figure 2D** shows impact of heavy chain substitutions on affinity of daclizumab to CD25.
**Figures 3A-3C** show the relationship between binding kinetics and biological function. Fold improvement in IL2 blocking activity of all daclizumab variants including alanine substitutes were plotted as a function of the affinity, K_{D} (Figure 3A), dissociation rate constant, *k*_{off} (**Figure 3B**) and association rate constant, *k*ₒₙ (Figure 3C).

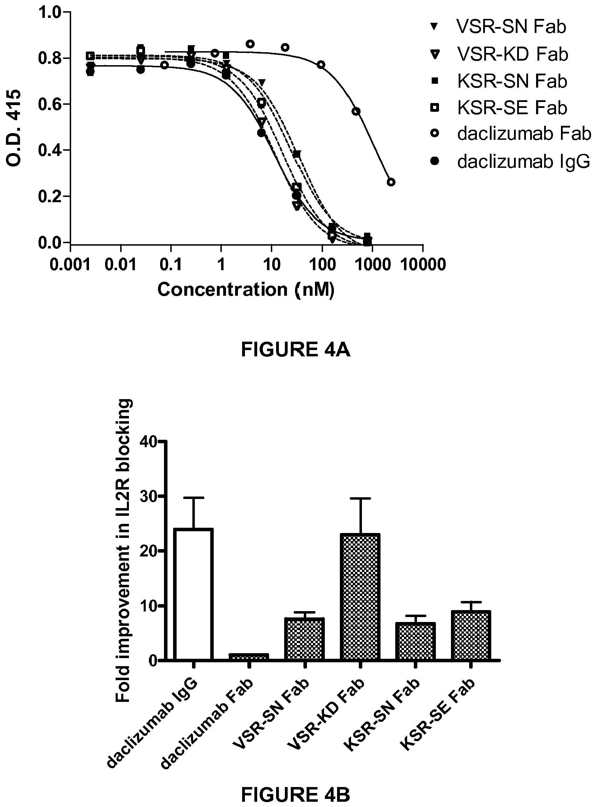

**Figures 4A-4B** **show a functional comparison of VKR-SN, VKR-KD, KSR-SN, KSR-SE in Fab.** Figure 4A: Competition ELISA to compare the affinities of Fab to CD25. The binding of biotinylated wild type daclizumab IgG to CD25 was analyzed in the presence of titrated amount of competitor Fab, generated form wild type or variant daclizumab. Figure 4B: IL2-R blocking activity using purified Fab. Receptor blocking was measured by proliferation of an IL2 dependent cell line, Kit225/K6. Data are normalized with an IC₅₀ value obtained from daclizumab Fab, shown as fold improvement in biological function.
**Figure 5** shows the results of daclizumab light chain V region peptides from Table 9 tested in the I-mune assay. Percent responses in 115 donor samples are shown.
**Figure 6** shows the results of daclizumab heavy chain V region peptides from Table 9 tested in the I-mune Assay. Percent responses in 115 donor samples are shown.
**Figure 7** shows average proliferative responses of human PBMC to E.HAT Fab and four variants. Heat inactivated Fab fragments from the E.HAT and four variant antibodies were cocultured with human PBMC for 6 days. Stimulation indexes were calculated for each donor at each concentration, and the results were averaged. Data is shown as average SI ± sem.
**Figure 8** shows the average stimulation index versus the percentage of donors responding with an SI > 1.99. Data for the 25 ug/ml concentration was selected, and was graphed versus the percent of donors whose proliferative response reached a value of 1.99 or greater.
**Figure 9** shows the average stimulation index for all tested variants from donors who responded with an SI greater than 1.99 to the E.HAT Fab in Figure 6. The proliferative responses from all donors whose responses were greater than 1.99 at the 25 µg/ml concentration were averaged. Data is shown as average SI + sem.
**Figure 10** shows the average stimulation index versus the percentage of donors responding with an SI greater than 1.99. Data for the 25 µg/ml concentration was selected, and was graphed versus the percent of donors whose proliferative response reached a value of 1.99 or greater.
**Figure 11** provides provides the sequence of a wild type Fc domain, from human IgG1 (SEQ ID NO:17). Within the Fc domain the CH2 domain (whose sequence is APELLGGPSVFLFPPKPKDTLMISRTPEVTCVWDVSHEDPEVKFNWYVDGVEVHNAKTKPREE QYNSTYRVVSVLTVLHQDWLNGKEYKCKVSNKALPAPIEKTISKAK; SEQ ID NO:188) is double underlined and the CH3 domain (whose sequence is GQPREPQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPGK; SEQ ID NO:189) is bolded. Residues 263, 266, 273, and 305 are indicated by asterisk (*), dagger (†), double dagger (‡) and the number sign (#), respectively.
**Figure 12** shows binding curves of WT and variant Fc region containing antibodies to FcγRIIB transfected CHO cells.
**Figure 13** shows binding curves of WT and variant Fc region containing antibodies to FcγRIIIA transfected CHO cells.
**Figure 14** shows Fc variants with little to no ADCC activity.
**Figure 15** shows Fc variants with lowest ADCC activity with retained/improved FcγRIIB binding in bold font.

### 7. DETAILED DESCRIPTION

### 7.1. Anti-CD25 Antibodies

The present disclosure provides anti-CD25 antibodies. Unless indicated otherwise, the term "antibody" (Ab) refers to an immunoglobulin molecule that specifically binds to, or is immunologically reactive with, a particular antigen, and includes polyclonal, monoclonal, genetically engineered and otherwise modified forms of antibodies, including but not limited to chimeric antibodies, humanized antibodies, heteroconjugate antibodies (*e*.*g*., bispecific antibodies, diabodies, triabodies, and tetrabodies), and antigen binding fragments of antibodies, including *e*.*g*., Fab', F(ab')₂, Fab, Fv, rIgG, and scFv fragments. Moreover, unless otherwise indicated, the term "monoclonal antibody" (mAb) is meant to include both intact molecules, as well as, antibody fragments (such as, for example, Fab and F(ab')₂ fragments) which are capable of specifically binding to a protein. Fab and F(ab')₂ fragments lack the Fc fragment of intact antibody, clear more rapidly from the circulation of the animal, and may have less nonspecific tissue binding than an intact antibody (Wahl et al., 1983, J. Nucl. Med. 24:316).

The term "scFv" refers to a single chain Fv antibody in which the variable domains of the heavy chain and the light chain from a traditional antibody have been joined to form one chain.

References to "VH" refer to the variable region of an immunoglobulin heavy chain of an antibody, including the heavy chain of an Fv, scFv, or Fab. References to "VL" refer to the variable region of an immunoglobulin light chain, including the light chain of an Fv, scFv, dsFv or Fab. Antibodies (Abs) and immunoglobulins (Igs) are glycoproteins having the same structural characteristics. While antibodies exhibit binding specificity to a specific target, immunoglobulins include both antibodies and other antibody-like molecules which lack target specificity. Native antibodies and immunoglobulins are usually heterotetrameric glycoproteins of about 150,000 daltons, composed of two identical light (L) chains and two identical heavy (H) chains. Each heavy chain has at the amino terminus a variable domain (V_{H}) followed by a number of constant domains. Each light chain has a variable domain at the amino terminus (V_{L}) and a constant domain at the carboxy terminus.

The anti-CD25 antibodies of the disclosure bind to human CD25 and inhibit its activity in a cell.

The anti-CD25 antibodies of the disclosure contain complementarity determining regions (CDRs) that are related in sequence to the CDRs of the antibody daclizumab.

CDRs are also known as hypervariable regions both in the light chain and the heavy chain variable domains. The more highly conserved portions of variable domains are called the framework (FR). As is known in the art, the amino acid position/boundary delineating a hypervariable region of an antibody can vary, depending on the context and the various definitions known in the art. Some positions within a variable domain may be viewed as hybrid hypervariable positions in that these positions can be deemed to be within a hypervariable region under one set of criteria while being deemed to be outside a hypervariable region under a different set of criteria. One or more of these positions can also be found in extended hypervariable regions. The disclosure provides antibodies comprising modifications in these hybrid hypervariable positions. The variable domains of native heavy and light chains each comprise four FR regions, largely by adopting a β-sheet configuration, connected by three CDRs, which form loops connecting, and in some cases forming part of, the β-sheet structure. The CDRs in each chain are held together in close proximity by the FR regions in the order FR1-CDR1-FR2-CDR2-FR3-CDR3-FR4 and, with the CDRs from the other chain, contribute to the formation of the target binding site of antibodies (see Kabat et al., Sequences of Proteins of Immunological Interest (National Institute of Health, Bethesda, Md. 1987)). As used herein, numbering of immunoglobulin amino acid residues is done according to the immunoglobulin amino acid residue numbering system of Kabat *et al.,* unless otherwise indicated.

The sequences of the heavy and light chain variable regions of daclizumab are represented by SEQ ID NO:1 and SEQ ID NO:2, respectively. The sequences of the heavy and light chain variable regions are also depicted in Figure 1A. The sequences of the CDRs of daclizumab, and their corresponding identifiers, are presented in Figure 1B. Any nucleotide sequences encoding SEQ ID NO:1 or SEQ ID NO:2 can be used in the compositions and methods of the present disclosure.

The present disclosure further provides anti-CD25 antibody fragments comprising CDR sequences that are related to the CDR sequences of daclizumab. The term "antibody fragment" refers to a portion of a full-length antibody, generally the target binding or variable region. Examples of antibody fragments include Fab, Fab', F(ab')₂ and Fv fragments. An "Fv" fragment is the minimum antibody fragment which contains a complete target recognition and binding site. This region consists of a dimer of one heavy and one light chain variable domain in a tight, noncovalent association (V_{H} -V_{L} dimer). It is in this configuration that the three CDRs of each variable domain interact to define a target binding site on the surface of the V_{H} -V_{L} dimer. Often, the six CDRs confer target binding specificity to the antibody. However, in some instances even a single variable domain (or half of an Fv comprising only three CDRs specific for a target) can have the ability to recognize and bind target. "Single chain Fv" or "scFv" antibody fragments comprise the V_{H} and V_{L} domains of an antibody in a single polypeptide chain. Generally, the Fv polypeptide further comprises a polypeptide linker between the V_{H} and V_{L} domains which enables the scFv to form the desired structure for target binding. "Single domain antibodies" are composed of a single V_{H} or V_{L} domain which exhibit sufficient affinity to the target. In a specific embodiment, the single domain antibody is a camelid antibody *(see, e.g.,* Riechmann, 1999, Journal of Immunological Methods 231:25-38).

The Fab fragment contains the constant domain of the light chain and the first constant domain (CH₁) of the heavy chain. Fab' fragments differ from Fab fragments by the addition of a few residues at the carboxyl terminus of the heavy chain CH₁ domain including one or more cysteines from the antibody hinge region. F(ab') fragments are produced by cleavage of the disulfide bond at the hinge cysteines of the F(ab')₂ pepsin digestion product. Additional chemical couplings of antibody fragments are known to those of ordinary skill in the art.

In certain embodiments, the anti-CD25 antibodies of the disclosure are monoclonal antibodies. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies useful in connection with the present disclosure can be prepared using a wide variety of techniques known in the art including the use of hybridoma, recombinant, and phage display technologies, or a combination thereof. The anti-CD25 antibodies of the disclosure include chimeric, primatized, humanized, or human antibodies.

The anti-CD25 antibodies of the disclosure can be chimeric antibodies. The term "chimeric" antibody as used herein refers to an antibody having variable sequences derived from a non-human immunoglobulin, such as rat or mouse antibody, and human immunoglobulin constant regions, typically chosen from a human immunoglobulin template. Methods for producing chimeric antibodies are known in the art. *See, e.g.,* Morrison, 1985, Science 229(4719):1202-7; Oi et al., 1986, BioTechniques 4:214-221; Gillies et al., 1985, J. Immunol. Methods 125:191-202; U.S. Patent Nos. 5,807,715; 4,816,567; and 4,816397, which are incorporated herein by reference in their entireties.

The anti-CD25 antibodies of the disclosure can be humanized. "Humanized" forms of non-human (e.g., murine) antibodies are chimeric immunoglobulins, immunoglobulin chains or fragments thereof (such as Fv, Fab, Fab', F(ab')₂ or other target-binding subdomains of antibodies) which contain minimal sequences derived from non-human immunoglobulin. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody can also comprise at least a portion of an immunoglobulin constant region (Fc), typically that of a human immunoglobulin consensus sequence. Methods of antibody humanization are known in the art. *See, e.g.,* Riechmann et al., 1988, Nature 332:323-7; U.S. Patent Nos: 5,530,101; 5,585,089; 5,693,761; 5,693,762; and 6,180,370 to Queen et al.*;* EP239400; PCT publication WO 91/09967; U.S. Patent No. 5,225,539; EP592106; EP519596; Padlan, 1991, Mol. Immunol., 28:489-498; Studnicka et al., 1994, Prot. Eng. 7:805-814; Roguska et al., 1994, Proc. Natl. Acad. Sci. 91:969-973; and U.S. Patent No. 5,565,332, all of which are hereby incorporated by reference in their entireties.

The anti-CD25 antibodies of the disclosure can be human antibodies. Completely "human" anti-CD25 antibodies can be desirable for therapeutic treatment of human patients. As used herein, "human antibodies" include antibodies having the amino acid sequence of a human immunoglobulin and include antibodies isolated from human immunoglobulin libraries or from animals transgenic for one or more human immunoglobulin and that do not express endogenous immunoglobulins. Human antibodies can be made by a variety of methods known in the art including phage display methods using antibody libraries derived from human immunoglobulin sequences. *See* U.S. Patent Nos. 4,444,887 and 4,716,111; and PCT publications WO 98/46645; WO 98/50433; WO 98/24893; WO 98/16654; WO 96/34096; WO 96/33735; and WO 91/10741, each of which is incorporated herein by reference in its entirety. Human antibodies can also be produced using transgenic mice which are incapable of expressing functional endogenous immunoglobulins, but which can express human immunoglobulin genes. *See, e.g.,* PCT publications WO 98/24893; WO 92/01047; WO 96/34096; WO 96/33735; U.S. Patent Nos. 5,413,923; 5,625,126; 5,633,425; 5,569,825; 5,661,016; 5,545,806; 5,814,318; 5,885,793; 5,916,771; and 5,939,598, which are incorporated by reference herein in their entireties. In addition, companies such as Medarex (Princeton, NJ), Astellas Pharma (Deerfield, IL), Amgen (Thousand Oaks, CA) and Regeneron (Tarrytown, NY) can be engaged to provide human antibodies directed against a selected antigen using technology similar to that described above. Completely human antibodies that recognize a selected epitope can be generated using a technique referred to as "guided selection." In this approach a selected non-human monoclonal antibody, *e*.*g*., a mouse antibody, is used to guide the selection of a completely human antibody recognizing the same epitope (Jespers et al., 1988, Biotechnology 12:899-903).

The anti-CD25 antibodies of the disclosure can be primatized. The term "primatized antibody" refers to an antibody comprising monkey variable regions and human constant regions. Methods for producing primatized antibodies are known in the art. *See e.g.,* U.S. Patent Nos. 5,658,570; 5,681,722; and 5,693,780, which are incorporated herein by reference in their entireties.

The anti-CD25 antibodies of the disclosure can be bispecific antibodies. Bispecific antibodies are monoclonal, often human or humanized, antibodies that have binding specificities for at least two different antigens. In the present disclosure, one of the binding specificities can be directed towards CD25, the other can be for any other antigen, *e*.*g*., for a cell-surface protein, receptor, receptor subunit, tissue-specific antigen, virally derived protein, virally encoded envelope protein, bacterially derived protein, or bacterial surface protein, *etc.*

The anti-CD25 antibodies of the disclosure include derivatized antibodies. For example, but not by way of limitation, derivatized antibodies are typically modified by glycosylation, acetylation, pegylation, phosphorylation, amidation, derivatization by known protecting/blocking groups, proteolytic cleavage, linkage to a cellular ligand or other protein (see Section 5.8 for a discussion of antibody conjugates), *etc.* Any of numerous chemical modifications can be carried out by known techniques, including, but not limited to, specific chemical cleavage, acetylation, formylation, metabolic synthesis of tunicamycin, *etc.* Additionally, the derivative can contain one or more non-natural amino acids, *e.g.,* using ambrx technology *(see, e.g.,* Wolfson, 2006, Chem. Biol. 13(10):1011-2).

The constant domains of the anti-CD25 antibodies of the disclosure can be selected with respect to the proposed function of the antibody, in particular with regard to the effector function which may be required. In some embodiments, the constant domains of the humanized antibodies of the invention are human IgA, IgE, IgG or IgM domains. In a specific embodiment, human IgG constant domains, especially of the IgG₁ and IgG₃ isotypes are used, especially when the anti-CD25 antibodies of the disclosure are intended for therapeutic uses and antibody effector functions are needed, for example in the treatment of CD25-expressing cancers. In alternative embodiments, IgG₂ and IgG₄ isotypes are used when the anti-CD25 antibody of the disclosure is intended for therapeutic purposes and antibody effector function is not required or even undesirable, for example in the treatment of multiple sclerosis or uveitis. The constant domains of the anti-CD25 antibodies of the disclosure can even be a hybrid of different isotypes from the same species or the same or different isotypes from different species. For example, the constant regions of ABT700 (anti-cMet), which contains a murine hinge in the context of a human IgG1, can be used.

The constant regions can also be modified to alter at least one constant region-mediated biological effector function relative to the corresponding wild type sequence.

For example, in some embodiments, an anti-CD25 antibody of the disclosure can be modified to reduce at least one constant region-mediated biological effector function relative to an unmodified antibody, *e*.*g*., reduced binding to the Fc receptor (FcγR). FcγR binding can be reduced by mutating the immunoglobulin constant region segment of the antibody at particular regions necessary for FcγR interactions (*see e.g.,* Canfïeld and Morrison, 1991, J. Exp. Med. 173:1483-1491; and Lund et al., 1991, J. Immunol. 147:2657-2662). Reduction in FcγR binding ability of the antibody can also reduce other effector functions which rely on FcγR interactions, such as opsonization, phagocytosis and antigen-dependent cellular cytotoxicity ("ADCC").

In other embodiments, an anti-CD25 antibody of the disclosure can be modified to acquire or improve at least one constant region-mediated biological effector function relative to an unmodified antibody, *e.g.,* to enhance FcγR interactions *(see, e.g.,* US 2006/0134709). For example, an anti-CD25 antibody of the disclosure can have a constant region that binds FcγRIIA, FcγRIIB and/or FcγRIIIA with greater affinity than the corresponding wild type constant region.

Thus, antibodies of the disclosure can have alterations in biological activity that result in increased or decreased opsonization, phagocytosis, or ADCC. Such alterations are known in the art. For example, modifications in antibodies that reduce ADCC activity are described in U.S. Patent No. 5,834,597. An exemplary ADCC lowering variant corresponds to "mutant 3" (or "M3") shown in Figure 4 of U.S. Patent No. 5,834,597, in which residue 236 is deleted and residues 234, 235 and 237 (using EU numbering) are substituted with alanines.

In some embodiments, the anti-CD25 antibodies of the disclosure have low levels of or lack fucose. Antibodies lacking fucose have been correlated with enhanced ADCC activity, especially at low doses of antibody. *See* Shields et al., 2002, J. Biol. Chem. 277:26733-26740; Shinkawa et al., 2003, J. Biol. Chem. 278:3466-73. Methods of preparing fucose-less antibodies include growth in rat myeloma YB2/0 cells (ATCC CRL 1662). YB2/0 cells express low levels of FUT8 mRNA, which encodes α-1,6-fucosyltransferase, an enzyme necessary for fucosylation of polypeptides.

In yet another aspect, the anti-CD25 antibodies or fragments thereof can be antibodies or antibody fragments that have been modified to increase or reduce their binding affinities to the fetal Fc receptor, FcRn, for example by mutating the immunoglobulin constant region segment at particular regions involved in FcRn interactions (see *e.g.,* WO 2005/123780). In particular embodiments, an anti-CD25 antibody of the IgG class is mutated such that at least one of amino acid residues 250, 314, and 428 of the heavy chain constant region is substituted alone, or in any combinations thereof, such as at positions 250 and 428, or at positions 250 and 314, or at positions 314 and 428, or at positions 250, 314, and 428, with positions 250 and 428 a specific combination. For position 250, the substituting amino acid residue can be any amino acid residue other than threonine, including, but not limited to, alanine, cysteine, aspartic acid, glutamic acid, phenylalanine, glycine, histidine, isoleucine, lysine, leucine, methionine, asparagine, proline, glutamine, arginine, serine, valine, tryptophan, or tyrosine. For position 314, the substituting amino acid residue can be any amino acid residue other than leucine, including, but not limited to, alanine, cysteine, aspartic acid, glutamic acid, phenylalanine, glycine, histidine, isoleucine, lysine, methionine, asparagine, proline, glutamine, arginine, serine, threonine, valine, tryptophan, or tyrosine. For position 428, the substituting amino acid residues can be any amino acid residue other than methionine, including, but not limited to, alanine, cysteine, aspartic acid, glutamic acid, phenylalanine, glycine, histidine, isoleucine, lysine, leucine, asparagine, proline, glutamine, arginine, serine, threonine, valine, tryptophan, or tyrosine. In yet further embodiments, the variant Fc domains have at least one or more modification that enhances the affinity to FcRn, *e.g.,* a modification of one or more amino acid residues 251-256, 285-290, 308-314, 385-389, and 428-436 (e.g., M428L), or a modification at positions 250 and 428 *(e.g.,* T250Q/M428L), see, *e.g.,* Hinton et al., 2004, J. Biol. Chem. 279(8): 6213-6; PCT Publication No. WO 97/34631; and WO 02/060919, all of which are incorporated herein by reference in their entirety. Such mutations increase the antibody's binding to FcRn, which protects the antibody from degradation and increases its half-life.

In yet other aspects, an anti-CD25 antibody has one or more amino acids inserted into one or more of its hypervariable regions, for example as described in S. Jung and A. Plückthun, 1997, Protein Engineering 10:959-966; Yazaki et al., 2004, Protein Eng Des Sel. 17(5):481-9.

In various embodiments, the anti-CD25 antibodies or fragments thereof can be antibodies or antibody fragments that have been modified for increased expression in heterologous hosts. In certain embodiments, the anti-CD25 antibodies or fragments thereof can be antibodies or antibody fragments that have been modified for increased expression in and/or secretion from heterologous host cells. In some embodiments, the anti-CD25 antibodies or fragments thereof are modified for increased expression in bacteria, such as *E. coli.* In other embodiments, the anti-CD25 antibodies or fragments thereof are modified for increased expression in yeast. (Kieke et al., 1999, Proc. Nat'l Acad. Sci. USA 96:5651-5656). In still other embodiments, the anti-CD25 antibodies or fragments thereof are modified for increased expression in insect cells. In additional embodiments, the anti-CD25 antibodies or fragments thereof are modified for increased expression in mammalian cells, such as CHO cells.

In certain embodiments, the anti-CD25 antibodies or fragments thereof can be antibodies or antibody fragments that have been modified to increase stability of the antibodies during production. In some embodiments, the antibodies or fragments thereof can be modified to replace one or more amino acids such as asparagine or glutamine that are susceptible to nonenzymatic deamidation with amino acids that do not undergo deamidation. (Huang et al., 2005, Anal. Chem. 77:1432-1439). In other embodiments, the antibodies or fragments thereof can be modified to replace one or more amino acids that is susceptible to oxidation, such as methionine, cysteine or tryptophan, with an amino acid that does not readily undergo oxidation. In still other embodiments, the antibodies or fragments thereof can be modified to replace one or more amino acids that are susceptible to cyclization, such as asparagine or glutamic acid, with an amino acid that does not readily undergo cyclization.

In some embodiments, the anti-CD25 antibodies or fragments of the disclosure are engineered to include one or more amino acid substitutions that increase susceptibility to pH sensitive antigen release to allow rapid dissociation from CD25 in the endosome. The rapid dissociation can improve antibody pharmacokinetic by release free antibody from within a cell back to the circulation. See Chaparro-Riggers et al., 2012, J. Biol. Chem. 287(14):11090-11097 and Igawa et al., 2010, Nature Biotechnology 28(11):1203-1208. Amino acid residues that increase susceptibility to pH sensitive antigen release include histidines. Exemplary histidine subsitutions can be selected from Table 8.

### 7.2. Nucleic Acids and Expression Systems

The present disclosure encompasses nucleic acid molecules and host cells encoding the anti-CD25 antibodies of the disclosure.

An anti-CD25 antibody of the disclosure can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, optionally, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Molecular Cloning; A Laboratory Manual, Second Edition (Sambrook, Fritsch and Maniatis (eds), Cold Spring Harbor, N. Y., 1989), Current Protocols in Molecular Biology (Ausubel, F.M. et al., eds., Greene Publishing Associates, 1989) and in U.S. Patent No. 4,816,397.

In one embodiment, the anti-CD25 antibodies are similar to daclizumab but for changes in one or more CDRs (referred to herein as having "daclizumab-related" sequences). In another embodiment, the anti-CD25 antibodies are similar to daclizumab but for changes in one or more framework regions. In yet another embodiment, the anti-CD25 antibodies are similar to daclizumab but for changes in one or more CDRs and in one or more framework regions. To generate nucleic acids encoding such anti-CD25 antibodies, DNA fragments encoding the light and heavy chain variable regions are first obtained. These DNAs can be obtained by amplification and modification of germline DNA or cDNA encoding light and heavy chain variable sequences, for example using the polymerase chain reaction (PCR). Germline DNA sequences for human heavy and light chain variable region genes are known in the art *(see e.g.,* the "VBASE" human germline sequence database; *see also* Kabat, E. A. et al., 1991, Sequences of Proteins of Immunological Interest, Fifth Edition, U.S. Department of Health and Human Services, NIH Publication No. 91-3242; Tomlinson et al., 1992, J. Mol. Biol. 22T:116-198; and Cox et al., 1994, Eur. J. Immunol. 24:827-836; the contents of each of which are incorporated herein by reference). A DNA fragment encoding the heavy or light chain variable region of daclizumab can be synthesized and used as a template for mutagenesis to generate a variant as described herein using routine mutagenesis techniques; alternatively, a DNA fragment encoding the variant can be directly synthesized.

Once DNA fragments encoding daclizumab or daclizumab-related VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked," as used in this context, is intended to mean that the two DNA fragments are joined such that the amino acid sequences encoded by the two DNA fragments remain in-frame.

The isolated DNA encoding the V_{H} region can be converted to a full-length heavy chain gene by operatively linking the V_{H}-encoding DNA to another DNA molecule encoding heavy chain constant regions (CH₁, CH₂, CH₃ and, optionally, CH₄). The sequences of human heavy chain constant region genes are known in the art (*see e.g.,* Kabat, E.A., et al., 1991, Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG₁, IgG₂, IgG₃, IgG₄, IgA, IgE, IgM or IgD constant region, but in certain embodiments is an IgG₁ constant region. For a Fab fragment heavy chain gene, the V_{H}-encoding DNA can be operatively linked to another DNA molecule encoding only the heavy chain CH₁ constant region.

The isolated DNA encoding the V_{L} region can be converted to a full-length light chain gene (as well as a Fab light chain gene) by operatively linking the V_{L}-encoding DNA to another DNA molecule encoding the light chain constant region, C_{L}. The sequences of human light chain constant region genes are known in the art *(see e.g.,* Kabat, E. A., et al., 1991, Sequences of Proteins of Immunological Interest, Fifth Edition (U.S. Department of Health and Human Services, NIH Publication No. 91-3242)) and DNA fragments encompassing these regions can be obtained by standard PCR amplification. The light chain constant region can be a kappa or lambda constant region, but in certain embodiments is a kappa constant region. To create a scFv gene, the V_{H} and V_{L}-encoding DNA fragments are operatively linked to another fragment encoding a flexible linker, *e*.*g*., encoding the amino acid sequence (Gly₄∼Ser)₃, (SEQ ID NO: 18), such that the V_{H} and V_{L} sequences can be expressed as a contiguous single-chain protein, with the V_{L} and V_{H} regions joined by the flexible linker (*see e.g.,* Bird et al., 1988, Science 242:423-426; Huston et al., 1988, Proc. Natl. Acad. Sci. USA 85:5879-5883; McCafferty et al., 1990, Nature 348:552-554).

To express the anti-CD25 antibodies of the disclosure, DNAs encoding partial or full-length light and heavy chains, obtained as described above, are inserted into expression vectors such that the genes are operatively linked to transcriptional and translational control sequences. In this context, the term "operatively linked" is intended to mean that an antibody coding sequence is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors or, more typically, both genes are inserted into the same expression vector.

The antibody genes are inserted into the expression vector by standard methods (*e*.*g*., ligation of complementary restriction sites on the antibody gene fragment and vector, or blunt end ligation if no restriction sites are present). Prior to insertion of the daclizumab or daclizumab-related light or heavy chain sequences, the expression vector can already carry antibody constant region sequences. For example, one approach to converting the daclizumab or daclizumab-related V_{H} and V_{L} sequences to full-length antibody genes is to insert them into expression vectors already encoding heavy chain constant and light chain constant regions, respectively, such that the V_{H} segment is operatively linked to the C_{H} segment(s) within the vector and the V_{L} segment is operatively linked to the C_{L} segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide *(i.e.,* a signal peptide from a non-immunoglobulin protein).

In addition to the antibody chain genes, the recombinant expression vectors of the disclosure carry regulatory sequences that control the expression of the antibody chain genes in a host cell. The term "regulatory sequence" is intended to include promoters, enhancers and other expression control elements (*e*.*g*., polyadenylation signals) that control the transcription or translation of the antibody chain genes. Such regulatory sequences are described, for example, in Goeddel, Gene Expression Technology: Methods in Enzymology 185 (Academic Press, San Diego, CA, 1990). It will be appreciated by those skilled in the art that the design of the expression vector, including the selection of regulatory sequences may depend on such factors as the choice of the host cell to be transformed, the level of expression of protein desired, *etc.* Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e*.*g*., the adenovirus major late promoter (AdMLP)) and polyoma. For further description of viral regulatory elements, and sequences thereof, *see e.g.,* U.S. Patent No. 5,168,062 by Stinski, U.S. Patent No. 4,510,245 by Bell et al.*,* and U.S. Patent No. 4,968,615 by Schaffner et al.

In addition to the antibody chain genes and regulatory sequences, the recombinant expression vectors of the disclosure can carry additional sequences, such as sequences that regulate replication of the vector in host cells (*e*.*g*., origins of replication) and selectable marker genes. The selectable marker gene facilitates selection of host cells into which the vector has been introduced *(see e.g.,* U.S. Patents Nos. 4,399,216, 4,634,665 and 5,179,017, all by Axel *et al*.). For example, typically the selectable marker gene confers resistance to drugs, such as G418, puromycin, blasticidin, hygromycin or methotrexate, on a host cell into which the vector has been introduced. Suitable selectable marker genes include the dihydrofolate reductase (DHFR) gene (for use in DHFR⁻ host cells with methotrexate selection/amplification) and the neo gene (for G418 selection). For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e*.*g*., electroporation, lipofection, calcium-phosphate precipitation, DEAE- dextran transfection and the like.

It is possible to express the antibodies of the disclosure in either prokaryotic or eukaryotic host cells. In certain embodiments, expression of antibodies is performed in eukaryotic cells, *e*.*g*., mammalian host cells, for optimal secretion of a properly folded and immunologically active antibody. Exemplary mammalian host cells for expressing the recombinant antibodies of the disclosure include Chinese Hamster Ovary (CHO cells) (including DHFR⁻ CHO cells, described in Urlaub and Chasin, 1980, Proc. Natl. Acad. Sci. USA 77:4216-4220, used with a DHFR selectable marker, *e*.*g*., as described in Kaufman and Sharp, 1982, Mol. Biol. 159:601-621), NS0 myeloma cells, COS cells, 293 cells and SP2/0 cells. When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods. Host cells can also be used to produce portions of intact antibodies, such as Fab fragments or scFv molecules. It is understood that variations on the above procedure are within the scope of the present disclosure. For example, it can be desirable to transfect a host cell with DNA encoding either the light chain or the heavy chain (but not both) of an anti-CD25 antibody of this disclosure.

Recombinant DNA technology can also be used to remove some or all of the DNA encoding either or both of the light and heavy chains that is not necessary for binding to CD25. The molecules expressed from such truncated DNA molecules are also encompassed by the antibodies of the disclosure.

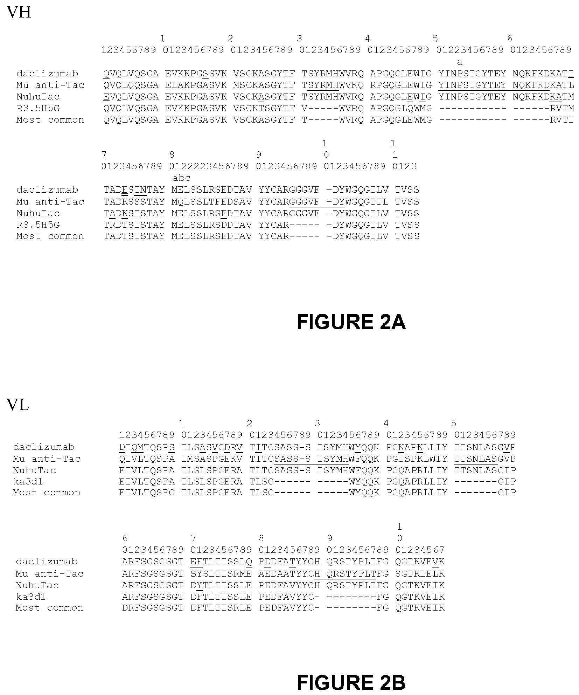

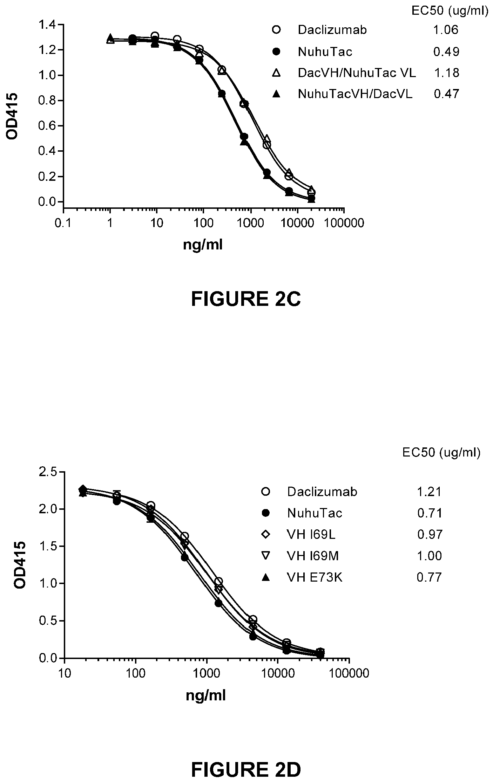

In addition, bifunctional antibodies can be produced in which one heavy and one light chain are an anti-CD25 antibody of the disclosure and the other heavy and light chain are specific for an antigen other than CD25, for example by crosslinking an antibody of the disclosure to a second antibody by standard chemical crosslinking methods. Bifunctional antibodies can also be made by expressing a nucleic acid engineered to encode a bifunctional antibody. Exemplary bifunctional antibody technologies that can be used to generate bifunctional antibodies are described by Kontermann, 2012, mAbs 4(2):182-197, particularly Figure 2.

In particular aspects the bifunctional antibodies are dual variable domain ("DVD") immunoglobulins ("DVD-Ig") (see, Gu & Ghayur, 2012, Methods in Enzymology 502:25-41, incorporated by reference herein in its entirety). A DVD-Ig combines the target-binding variable domains of two monoclonal antibodies via linkers to create a tetravalent, dual-targeting single agent. Suitable linkers for use in the light chains of the DVDs of the present disclosure include those identified on Table 2.1 on page 30 of Gu & Ghayur, 2012, Methods in Enzymology 502:25-41, incorporated by reference herein: the short κ chain linkers ADAAP (SEQ ID NO:19) (murine) and TVAAP (SEQ ID NO:20) (human); the long κ chain linkers ADAAPTVSIFP (SEQ ID NO:21) (murine) and TVAAPSVFIFPP (SEQ ID NO: 22) (human); the short λ chain linker QPKAAP (SEQ ID NO:23) (human); the long λ chain linker QPKAAPSVTLFPP (SEQ ID NO: 24) (human); the GS-short linker GGSGG (SEQ ID NO: 25), the GS-medium linker GGSGGGGSG (SEQ ID NO: 26), and the GS-long linker GGSGGGGSGGGGS (SEQ ID NO: 27) (all GS linkers are murine and human). Suitable linkers for use in the heavy chains of the DVDs of the present disclosure include those identified on Table 2.1 on page 30 of Gu & Ghayur, 2012, Methods in Enzymology 502:25-41, incorporated by reference herein: the short linkers AKTTAP (SEQ ID NO: 28) (murine) and ASTKGP (SEQ ID NO: 29) (human); the long linkers AKTTAPSVYPLAP (SEQ ID NO: 30) (murine) and ASTKGPSVFPLAP (SEQ ID NO: 31) (human); the GS-short linker GGGGSG (SEQ ID NO: 32), the GS-medium linker GGGGSGGGGS (SEQ ID NO: 33), and the GS-long linker GGGGSGGGGSGGGG (SEQ ID NO: 34) (all GS linkers are murine and human). Preferably human linkers are used for human or humanized DVD-Igs. Target binding domains of DVD immunoglobulins are typically arranged in tandem, with one variable domain stacked on top of another to form inner and outer Fv domains. The anti-CD25 variable domain can be the inner or outer Fv domain of a DVD.

In certain embodiments, dual specific antibodies, *i.e.,* antibodies that bind CD25 and an unrelated antigen using the same binding site, can be produced by mutating amino acid residues in the light chain and/or heavy chain CDRs. In various embodiments, dual specific antibodies that bind two antigens, such as CD25 and VEGF, can be produced by mutating amino acid residues in the periphery of the antigen binding site (Bostrom et al., 2009, Science 323:1610-1614). Dual functional antibodies can be made by expressing a nucleic acid engineered to encode a dual specific antibody.

For recombinant expression of an anti-CD25 antibody of the disclosure, the host cell can be co-transfected with two expression vectors of the disclosure, the first vector encoding a heavy chain derived polypeptide and the second vector encoding a light chain derived polypeptide. Typically, the two vectors each contain a separate selectable marker. Alternatively, a single vector can be used which encodes both heavy and light chain polypeptides.

Once a nucleic acid encoding one or more portions of daclizumab or of an anti-CD25 antibody with CDR sequences related to the CDR sequences of daclizumab is generated, further alterations or mutations can be introduced into the coding sequence, for example to generate nucleic acids encoding antibodies with different CDR sequences, antibodies with reduced affinity to the Fc receptor, or antibodies of different subclasses.

The anti-CD25 antibodies of the disclosure can also be produced by chemical synthesis (e.g., by the methods described in Solid Phase Peptide Synthesis, 2nd ed., 1984 The Pierce Chemical Co., Rockford, Ill.). Variant antibodies can also be generated using a cell-free platform (*see, e.g.,* Chu et al., Biochemia No. 2, 2001 (Roche Molecular Biologicals)).

Once an anti-CD25 antibody of the disclosure has been produced by recombinant expression, it can be purified by any method known in the art for purification of an immunoglobulin molecule, for example, by chromatography (*e*.*g*., ion exchange, affinity, particularly by affinity for CD25 after Protein A or Protein G selection, and sizing column chromatography), centrifugation, differential solubility, or by any other standard technique for the purification of proteins. Further, the anti-CD25 antibodies of the present disclosure or fragments thereof can be fused to heterologous polypeptide sequences described herein or otherwise known in the art to facilitate purification.

Once isolated, an anti-CD25 antibody can, if desired, be further purified, *e*.*g*., by high performance liquid chromatography (*See, e.g.,* Fisher, Laboratory Techniques In Biochemistry And Molecular Biology (Work and Burdon, eds., Elsevier, 1980)), or by gel filtration chromatography on a SuperdexTM 75 column (Pharmacia Biotech AB, Uppsala, Sweden).

### 7.3. Biological Activities of Anti-CD25 Antibodies

In certain embodiments, the anti-CD25 antibodies of the disclosure have certain biological activities, such as competing with daclizumab for binding to CD25 or neutralizing CD25 activity.

Accordingly, in certain embodiments, anti-CD25 antibodies of the disclosure compete with daclizumab for binding to CD25. The ability to compete for binding to CD25 can be tested using a competition assay, such as described in Section 8.3.1.1. Other formats for competition assays are known in the art and can be employed.

In other aspects, an anti-CD25 antibody of the disclosure inhibits (or neutralizes) CD25 activity in a range of *in vitro* assays, such as cell proliferation. For example, in one embodiment, the anti-CD25 antibody is assayed for the ability to inhibit T cell proliferation assays. Such assays can be carried out using known techniques. In one technique, human PBMCs are diluted in a suitable medium and then stimulated with, for example, an anti-CD3 antibody, before adding varying concentrations of the anti-CD25 antibodies to determine the effect they have on T cell proliferation. The PBMC proliferation assay can be carried out as described in Section 8.4.1.1 below. T cell proliferation of purified T cells can also be assessed in the presence of anti-CD3 and anti-CD28 monoclonal antibodies. In another technique, the ability of an anti-CD25 antibody of the disclosure to inhibit IL2-dependent proliferation of Kit225/K6 cells can be measured, as described in Section 8.3.1.3 below. Another assay that can be used is a mixed lymphocyte reaction, which shows the impact of anti-CD25 binding on an antigen-specific T cell proliferative responses. An exemplary mixed lymphocyte reaction can be performed as described in Section 6.4.1.6 below. Anti-CD25 antibodies block secretion of cytokines from antigen- and mitogen-activated PBMC. Supernatants from cultures activated with, for example, PHA can be tested for the presence of various cytokines and chemokines using known techniques such as ELISA assays, Luminex-based multiplex assays, and cytokine-dependent cell proliferation assays as readouts. In yet another assay, expansion of CD56bright NK cells by inclusion of anti-CD25 in cultures of human PBMC and recombinant human IL2 can be performed as described in Section 6.4.1.7 below.

Other formats for CD25 neutralization assays are known in the art and can be employed.

In various embodiments, an anti-CD25 antibody of the disclosure reduces the binding of labeled daclizumab by at least 30%, by at least 40%, by at least 50%, by at least 60%, by at least 70%, by at least 80%, by at least 90%, by at least 95%, by at least 99%, or by a percentage ranging between any of the foregoing values (e.g., an anti-CD25 antibody of the disclosure reduces the binding of labeled daclizumab by 50% to 70%) when the anti-CD25 antibody is used at a concentration of 0.08 µg/ml, 0.4 µg/ml, 2 µg/ml, 10 µg/ml, 50 µg/ml, 100 µg/ml or at a concentration ranging between any of the foregoing values *(e.g.,* at a concentration ranging from 2 µg/ml to 10 µg/ml).

In various embodiments, an anti-CD25 antibody of the disclosure neutralizes CD25 by at least 30%, by at least 40%, by at least 50%, by at least 60%, by at least 70%, by at least 80%, by at least 90%, or by a percentage ranging between any of the foregoing values (*e*.*g*., an anti-CD25 antibody of the disclosure neutralizes CD25 activity by 50% to 70%) when the anti-CD25 antibody is used at a concentration of 2 ng/ml, 5 ng/ml, 10 ng/ml, 20 ng/ml, 0.1 µg/ml, 0.2 µg/ml, 1 µg/ml, 2 µg/ml, 5 µg/ml, 10 µg/ml, 20 µg/ml, or at a concentration ranging between any of the foregoing values (e.g., at a concentration ranging from 1 µg/ml to 5 µg/ml).

In some embodiments, an anti-CD25 antibody of the disclosure is at least 0.7-fold as effective, 0.8-fold as effective, at least 0.9-fold as effective, at least 1-fold as effective, at least 1.1-fold as effective, at least 1.25-fold as effective, at least 1.5-fold as effective, at least 2-fold as effective, at least 3-fold as effective, at least 5-fold as effective, at least 10-fold as effective, at least 20-fold as effective, at least 50-fold as effective, at least 100-fold as effective, at least 200-fold as effective, at least 500-fold as effective, at least 1000-fold as effective as daclizumab at neutralizing CD25, or having an effectiveness at neutralizing CD25 relative to daclizumab ranging between any pair of the foregoing values (e.g., 0.9-fold to 5-fold as effective as daclizumab, 1-fold to 3-fold as effective as daclizumab, or 2-fold to 50-fold as effective as daclizumab in neutralizing CD25).

In some embodiments, the biological properties of an anti-CD25 antibody of the disclosure as compared to daclizumab are assessed in the context of full length immunoglobulin molecules (which can be any type of immunoglobulin, *e*.*g*., IgG, IgM, IgD, IgA, or IgE, but is preferably in the form of an immunoglobulin dimer). In other embodiments, the biological properties of an anti-CD25 antibody of the disclosure as compared to daclizumab are assessed in the context of Fab fragments. Thus, an anti-CD25 antibody of the disclosure can have improved affinity and/or improved IL2-blocking activity as compared to daclizumab in full length immunoglobulin form, in Fab form, or both.

### 7.4. Kinetic Properties of Anti-CD25 Antibodies

The anti-CD25 antibodies of the disclosure typically have an improved binding affinity for CD25 as compared to daclizumab.

In certain embodiments, an anti-CD25 antibody of the disclosure binds to CD25 with a K_{D} (k_{off}/kₒₙ) of less than 500 pM when assessed in the context of full length immunoglobulin molecules (which can be any type of immunoglobulin, *e*.*g*., IgG, IgM, IgD, IgA, or IgE, but is preferably in the form of an immunoglobulin dimer). In specific embodiments, the anti-CD25 antibodies of the disclosure have a K_{D} of 480 pM or less, 450 pM or less, 400 pM or less, 350 pM or less, 300 pM or less, 200 pM or less, 150 pM or less, 100 pM or less, 50 pM or less, or 25 pM or less. In yet other specific embodiments the K_{D} is at least 1 pM, at least 3 pM, at least 5 pM, at least 10 pM, at least 15 pM, or at least 20 pM. The K_{D} of the anti-CD25 antibodies of the disclosure can be defined in ranges, with the upper and lower bounds selected from any pair of the foregoing values (*e*.*g*., from 3 pM to 50 pM, from 5 pM to 200 pM, 10 pM to 100 pM; from 50 pM to 350 pM; from 15 pM to 150 pM; from 20 pM to 450 pM; from 10 pM to 200 pM; and so on an so forth).

In still other embodiments, an anti-CD25 antibody of the disclosure binds to CD25 with a K_{D} ranging from about 0.005x to 1x of the K_{D} of daclizumab, for example a K_{D} of 0.005x of the K_{D} of daclizumab, a K_{D} of 0.0075x of the K_{D} of daclizumab, a K_{D} of 0.01x of K_{D} of daclizumab, a K_{D} of 0.03x of the K_{D} of daclizumab, a K_{D} of 0.05x of the K_{D} of daclizumab, a K_{D} of 0.1x of the K_{D} of daclizumab, a K_{D} of 0.2x of the K_{D} of daclizumab, a K_{D} of 0.3x of the K_{D} of daclizumab, a K_{D} of 0.4x of the K_{D} of daclizumab, a K_{D} of 0.5x of the K_{D} of daclizumab, a K_{D} of 0.75x of the K_{D} of daclizumab, or a K_{D} ranging between any pair of the foregoing values, e.g., a K_{D} of 0.005x to 0.1x of the K_{D} of daclizumab, a K_{D} of 0.0075x to 0.3x of the K_{D} of daclizumab, a K_{D} of 0.1x to 0.4x of the K_{D} of daclizumab, a K_{D} of 0.05 to 1x of the K_{D} of daclizumab, *etc.* The relative affinity of an antibody of the disclosure as compared to daclizumab can be when assessed in the context of full length immunoglobulin molecules (which can be any type of immunoglobulin, *e*.*g*., IgG, IgM, IgD, IgA, or IgE, but is preferably in the form of an immunoglobulin dimer) or in the context of a Fab fragment.

The K_{D} (*k*_{off}/*k*ₒₙ) value can be determined by assays well known in the art, *e*.*g*., ELISA, FACS, isothermal titration calorimetry (ITC), fluorescent polarization assay or any other biosensors such as BIAcore. In various embodiments, binding constants for the interaction of the anti-CD25 antibodies with CD25 receptor extracellular domain can be determined using BIAcore or FACS binding assays such as described in Sections 8.3.1.2 and 8.3.1.4, respectively.

In some embodiments, an anti-CD25 antibody of the disclosure binds to CD25 and inhibits cell growth (for example in the Kit225 proliferation assay described in Section 8.3.1.3) with an IC₅₀ of 0.2 nM or less, 0.15 nM or less, less than 0.12 nM or less, 0.1 nM or less, 0.075 nM or less, 0.05 nM or less, 0.025 nM or less, 0.01 nM or less, 0.005 nM or less, 0.0025 nM or less, or 0.001 nM or less when assessed in the context of full length immunoglobulin molecules (which can be any type of immunoglobulin, *e*.*g*., IgG, IgM, IgD, IgA, or IgE, but is preferably in the form of an immunoglobulin dimer). The IC₅₀ of the anti-CD25 antibodies of the disclosure can be defined in ranges, with the upper and lower bounds selected from any pair of the foregoing values (*e*.*g*., from 0.001 nM to 0.2 nM, from 0.005 nM to 0.025 nM; from 0.001 nM to 0.1 nM, from 0.025 nM to 0.15 nM; and so on an so forth).

In some embodiments, an anti-CD25 antibody of the disclosure binds to CD25 and inhibits cell growth (for example in the Kit225 proliferation assay described in Section 8.3.1.3) with an IC₅₀ ranging from about 0.02x to 1x of the IC₅₀ of daclizumab, for example an IC₅₀ of 0.05x of the IC₅₀ of daclizumab, an IC₅₀ of 0.1x of the IC₅₀ of daclizumab, an IC₅₀ of 0.2x of the IC₅₀ of daclizumab, an IC₅₀ of 0.3x of the IC₅₀ of daclizumab, an IC₅₀ of 0.4x of the IC₅₀ of daclizumab, an IC₅₀ of 0.5x of the IC₅₀ of daclizumab, an IC₅₀ of 0.75x of the IC₅₀ of daclizumab, or an IC₅₀ ranging between any pair of the foregoing values, *e.g.,* an IC₅₀ of 0.1x to 0.4x of the IC₅₀ of daclizumab, an IC₅₀ of 0.05 to 1x of the IC₅₀ of daclizumab, *etc.* The relative IC₅₀ of an antibody of the disclosure as compared to daclizumab can be when assessed in the context of full length immunoglobulin molecules (which can be any type of immunoglobulin, *e*.*g*., IgG, IgM, IgD, IgA, or IgE, but is preferably in the form of an immunoglobulin dimer) or in the context of a Fab fragment.

### 7.5. Reduced Immunogenicity of Anti-CD25 Antibodies

In certain aspects, the present disclosure provides anti-CD25 antibodies having reduced immunogenicity as compared to daclizumab. The present disclosure provides anti-CD25 antibodies having single or multiple amino acid substitutions in their CDRs and/or framework regions as compared to the CDRs and/or framework regions of daclizumab, wherein at least one substitution reduces the immunogenicity of the antibody as compared to daclizumab. In certain embodiments, the reduced immunogenicity results from one or more amino acid substitutions that result in eliminating or mitigating one or more T cell epitopes.

In certain aspects, the anti-CD25 antibodies of the disclosure having reduced immunogenicity have comparable or improved biological activity as compared to daclizumab, *e*.*g*., affinity towards CD25 or neutralization of CD25 activity. Such properties can be tested, for example, by the methods described in Section 5.3 above.

In certain embodiments, the immunogenicity of an anti-CD25 antibody of the disclosure is reduced relative to daclizumab. In certain embodiments, a variant with "reduced immunogenicity" refers to an anti-CD25 antibody that elicits a reduced proliferative response in peripheral blood mononuclear cells as compared to the peptide PH16 or the peptide PH17 as set forth in Table 9. An exemplary proliferation assay that can be used to evaluate the proliferative response is set forth in Section 6.5.2 below. The reduced proliferative response can be reflected in terms of the percentage of responders, the stimulation index, or both.

In certain embodiments, anti-CD25 antibodies with reduced immunogenicity will have the substitution T54S in heavy chain CDR2 and/or I48M in heavy chain framework 2. The antibodies can also have one or more additional substitutions, for example substitutions that increase affinity towards CD25. Fab fragments derived from intact antibodies containing the substitutions will induce reduced proliferation. An exemplary proliferation assay that can be used to determine the relative immunogenicity of the fab fragments is set forth in section 6.5.2 below.

In other embodiments, as compared to the peptide PH16 or the peptide PH17 as set forth in Table 9, the variant sequence results in at least 25% fewer responders, in at least 30% fewer responders, in at least 35% fewer responders, in at least 40% fewer responders, in at least 45% fewer responders, in at least 50% fewer responders, in at least 60% fewer responders, in at least 65% fewer responders, in at least 70% fewer responders, in at least 75% fewer responders, in at least 80% fewer responders, in at least 85% fewer responders, in at least 90% fewer responders, in at least 95% fewer responders, in at least 100% fewer responders, or a reduction in responders in a range between any of the foregoing values, *e*.*g*., 25%-75% fewer responders, 50%-90% fewer responders, 60%-100% fewer responders, 70%-90% fewer responders, or the like.

In other embodiments, the variant sequence results in a stimulation index that is at least 5% less, at least 10% less, at least 15% less, at least 20% less, at least 25% less, at least 30% less, at least 35% less, or at least 40% less than the stimulation index elicited by the peptide PH16 or the peptide PH17 as set forth in Table 9, or results in a stimulation reduced by a range between any of the foregoing values as compared to a peptide of PH16 or PH69, *e.g.,* 5%-20% less, 10%-30% less, 30%-40% less, or the like.

Further exemplary embodiments of candidate anti-CD25 antibodies with reduced immunogenicity as compared to daclizumab comprise one or more of the CDR and/or framework substitutions or combination of substitutions set forth in Tables 11-19. Optionally, anti-CD25 antibodies with reduced immunogenicity as compared to daclizumab comprise one or more additional substitutions, such as one or more of the CDR mutations in any of Tables 6-8, 20 and 21.

### 7.6. Antibody Conjugates

The anti-CD25 antibodies of the disclosure include antibody conjugates that are modified, *e*.*g*., by the covalent attachment of any type of molecule to the antibody, such that covalent attachment does not interfere with binding to CD25.

In certain aspects, an anti-CD25 antibody of the disclosure can be conjugated to an effector moiety or a label. The term "effector moiety" as used herein includes, for example, antineoplastic agents, drugs, toxins, biologically active proteins, for example enzymes, other antibody or antibody fragments, synthetic or naturally occurring polymers, nucleic acids (*e*.*g*., DNA and RNA), radionuclides, particularly radioiodide, radioisotopes, chelated metals, nanoparticles and reporter groups such as fluorescent compounds or compounds which can be detected by NMR or ESR spectroscopy.

In one example, anti-CD25 antibodies can be conjugated to an effector moiety, such as a cytotoxic agent, a radionuclide or drug moiety to modify a given biological response. The effector moiety can be a protein or polypeptide, such as, for example and without limitation, a toxin (such as abrin, ricin A, *Pseudomonas* exotoxin, or *Diphtheria* toxin), a signaling molecule (such as α-interferon, β-interferon, nerve growth factor, platelet derived growth factor or tissue plasminogen activator), a thrombotic agent or an anti-angiogenic agent (*e*.*g*., angiostatin or endostatin) or a biological response modifier such as a cytokine or growth factor (*e*.*g*., interleukin-1 (IL-I), interleukin-6 (IL-6), granulocyte macrophage colony stimulating factor (GM-CSF), granulocyte colony stimulating factor (G-CSF), or nerve growth factor (NGF)).

In another example the effector moieties can be cytotoxins or cytotoxic agents. Examples of cytotoxins and cytotoxic agents include taxol, cytochalasin B, gramicidin D, ethidium bromide, emetine, mitomycin, etoposide, tenoposide, vincristine, vinblastine, colchicin, doxorubicin, daunorabicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector moieties also include, but are not limited to, antimetabolites (e.g. methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C5 and cis-dichlorodiamine platinum (II) (DDP) cisplatin), anthracyclines (e.g., daunorubicin (formerly daunomycin) and doxorubicin), antibiotics (*e*.*g*., dactinomycin (formerly actinomycin), bleomycin, mithramycin, anthramycin (AMC), calicheamicins or duocarmycins), and anti-mitotic agents (*e*.*g*., vincristine and vinblastine).

Other effector moieties can include radionuclides such as, but not limited to, ¹¹¹In and ⁹⁰Y, Lu¹⁷⁷, Bismuth²¹³, Californium²⁵², Iridium¹⁹² and Tungsten^{18s}/Rhenium¹⁸⁸ and drugs such as, but not limited to, alkylphosphocholines, topoisomerase I inhibitors, taxoids and suramin.

Techniques for conjugating such effector moieties to antibodies are well known in the art *(see, e.g.,* Hellstrom et al., Controlled Drug Delivery, 2nd Ed., at pp. 623-53 (Robinson et al., eds., 1987)); Thorpe et al., 1982, Immunol. Rev. 62:119-58 and Dubowchik et al., 1999, Pharmacology and Therapeutics 83:67-123).

In one example, the anti-CD25 antibody or fragment thereof is fused via a covalent bond *(e.g.,* a peptide bond), through the antibody's N-terminus or C-terminus or internally, to an amino acid sequence of another protein (or portion thereof; for example at least a 10, 20 or 50 amino acid portion of the protein). The antibody, or fragment thereof, can linked to the other protein at the N-terminus of the constant domain of the antibody. Recombinant DNA procedures can be used to create such fusions, for example as described in WO 86/01533 and EP0392745. In another example the effector molecule can increase half-life *in vivo,* and/or enhance the delivery of an antibody across an epithelial barrier to the immune system. Examples of suitable effector molecules of this type include polymers, albumin, albumin binding proteins or albumin binding compounds such as those described in WO 2005/117984.

In certain aspects, an anti-CD25 antibody is conjugated to a small molecule toxin. In certain exemplary embodiments, an anti-CD25 antibody of the disclosure is conjugated to a dolastatin or a dolostatin peptidic analogs or derivatives, *e*.*g*., an auristatin (U.S. Patent Nos. 5,635,483 and 5,780,588). The dolastatin or auristatin drug moiety may be attached to the antibody through its N (amino) terminus, C (carboxyl) terminus or internally (WO 02/088172). Exemplary auristatin embodiments include the N-terminus linked monomethylauristatin drug moieties DE and DF, as disclosed in U.S. Patent No. 7,498,298, which is hereby incorporated by reference in its entirety (disclosing, *e*.*g*., linkers and methods of preparing monomethylvaline compounds such as MMAE and MMAF conjugated to linkers).

In other exemplary embodiments, small molecule toxins include but are not limited to calicheamicin, maytansine (U.S. Patent No. 5,208,020), trichothene, and CC1065. In one embodiment of the disclosure, the antibody is conjugated to one or more maytansine molecules (*e*.*g*., about 1 to about 10 maytansine molecules per antibody molecule). Maytansine may, for example, be converted to May-SS-Me which may be reduced to May-SH3 and reacted with an antibody (Chari et al., 1992, Cancer Research 52: 127-131) to generate a maytansinoid-antibody or maytansinoid-Fc fusion conjugate. Structural analogues of calicheamicin that can also be used include but are not limited to γ₁¹, γ₃¹, γ₃¹, N-acetyl- γ₁¹, PSAG, and θ₁¹, (Hinman et al., 1993, Cancer Research 53:3336-3342; Lode et al., 1998, Cancer Research 58:2925-2928; U.S. Patent No. 5,714,586; U.S. Patent No. 5,712,374; U.S. Patent No. 5,264,586; U.S. Patent No. 5,773,001).

Antibodies of the disclosure can also be conjugated to liposomes for targeted delivery (*See, e.g.,* Park et al., 1997, Adv. Pharmacol. 40:399-435; Marty & Schwendener, 2004, Methods in Molecular Medicine 109:389-401).

In one example antibodies of the present disclosure can be attached to poly(ethyleneglycol) (PEG) moieties. In one particular example the antibody is an antibody fragment and the PEG moieties can be attached through any available amino acid side-chain or terminal amino acid functional group located in the antibody fragment, for example any free amino, imino, thiol, hydroxyl or carboxyl group. Such amino acids can occur naturally in the antibody fragment or can be engineered into the fragment using recombinant DNA methods. *See* for example U.S. Patent No. 5,219,996. Multiple sites can be used to attach two or more PEG molecules. PEG moieties can be covalently linked through a thiol group of at least one cysteine residue located in the antibody fragment. Where a thiol group is used as the point of attachment, appropriately activated effector moieties, for example thiol selective derivatives such as maleimides and cysteine derivatives, can be used.

In a specific example, an anti-CD25 antibody conjugate is a modified Fab' fragment which is PEGylated, *i.e.,* has PEG (poly(ethyleneglycol)) covalently attached thereto, *e.g.,* according to the method disclosed in EP0948544. *See also* Poly(ethyleneglycol) Chemistry, Biotechnical and Biomedical Applications, (J. Milton Harris (ed.), Plenum Press, New York, 1992); Poly(ethyleneglycol) Chemistry and Biological Applications, (J. Milton Harris and S. Zalipsky, eds., American Chemical Society, Washington D.C., 1997); and Bioconjugation Protein Coupling Techniques for the Biomedical Sciences, (M. Aslam and A. Dent, eds., Grove Publishers, New York, 1998); and Chapman, 2002, Advanced Drug Delivery Reviews 54:531-545. PEG can be attached to a cysteine in the hinge region. In one example, a PEG-modified Fab' fragment has a maleimide group covalently linked to a single thiol group in a modified hinge region. A lysine residue can be covalently linked to the maleimide group and to each of the amine groups on the lysine residue can be attached a methoxypoly(ethyleneglycol) polymer having a molecular weight of approximately 20,000 Da. The total molecular weight of the PEG attached to the Fab' fragment can therefore be approximately 40,000 Da.

The word "label" when used herein refers to a detectable compound or composition which can be conjugated directly or indirectly to an anti-CD25 antibody of the disclosure. The label can itself be detectable (e.g., radioisotope labels or fluorescent labels) or, in the case of an enzymatic label, can catalyze chemical alteration of a substrate compound or composition which is detectable. Useful fluorescent moieties include, but are not limited to, fluorescein, fluorescein isothiocyanate, rhodamine, 5-dimethylamine-1-napthalenesulfonyl chloride, phycoerythrin and the like. Useful enzymatic labels include, but are not limited to, alkaline phosphatase, horseradish peroxidase, glucose oxidase and the like.

Additional anti-CD25 antibody conjugates that are useful for, inter alia, diagnostic purposes, are described in Section 5.9 below.

### 7.7. Diagnostic Uses of Anti-CD25 Antibodies

The anti-CD25 antibodies of the disclosure, including those antibodies that have been modified, *e.g.,* by biotinylation, horseradish peroxidase, or any other detectable moiety, can be advantageously used for diagnostic purposes.

In particular, the anti-CD25 antibodies can be used, for example, but not limited to, to purify or detect CD25, including both *in vitro* and *in vivo* diagnostic methods. For example, the antibodies have use in immunoassays for qualitatively and quantitatively measuring levels of CD25 in biological samples. *See, e.g.,* Harlow et al., Antibodies: A Laboratory Manual, Second Edition (Cold Spring Harbor Laboratory Press, 1988), which is incorporated by reference herein in its entirety. In a specific embodiment, the anti-CD25 antibodies can be used for detecting and quantitating levels of CD25 in the serum, *i.e.,* levels of CD25 extracellular domain that has been shed from the surface of cells.

The present disclosure further encompasses antibodies or fragments thereof conjugated to a diagnostic agent. The antibodies can be used diagnostically, for example, to detect expression of a target of interest in specific cells, tissues, or serum; or to monitor the development or progression of an immunologic response as part of a clinical testing procedure to, *e*.*g*., determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance can be coupled or conjugated either directly to the antibody (or fragment thereof) or indirectly, through an intermediate (such as, for example, a linker known in the art) using techniques known in the art. Examples of enzymatic labels include luciferases (*e*.*g*., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, acetylcholinesterase, glucoamylase, lysozyme, saccharide oxidases (*e*.*g*., glucose oxidase, galactose oxidase, and glucose-6-phosphate dehydrogenase), heterocyclic oxidases (such as uricase and xanthine oxidase), lactoperoxidase, microperoxidase, and the like. Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include ¹²⁵I, ¹³¹I, ¹¹¹In or ⁹⁹Tc.

The disclosure provides for the detection of expression of CD25 comprising contacting a biological sample (cells, tissue, or body fluid of an individual) using one or more anti-CD25 antibodies of the disclosure (optionally conjugated to detectable moiety), and detecting whether or not the sample is positive for CD25 expression, or whether the sample has altered (e.g., reduced or increased) expression as compared to a control sample.

### 7.8. Therapeutic Methods Using Anti-CD25 Antibodies

### 7.8.1. Clinical Benefits

The anti-CD25 antibodies of the disclosure can be used to treat various immune conditions and cancers, such as organ transplant rejection, asthma, multiple sclerosis, uveitis, ocular inflammation and human T cell leukemia virus-1 associated T-cell leukemia.

Accordingly, the present disclosure provides methods of treating any of the foregoing diseases in a patient in need thereof, comprising: administering to the patient an anti-CD25 antibody of the disclosure. Optionally, said administration is repeated, *e*.*g*., after one day, two days, three days, five days, one week, two weeks, three weeks, one month, five weeks, six weeks, seven weeks, eight weeks, two months or three months. The repeated administration can be at the same dose or at a different dose. The administration can be repeated once, twice, three times, four times, five times, six times, seven times, eight times, nine times, ten times, or more. For example, according to certain dosage regimens a patient receives anti-CD25 therapy for a prolonged period of time, *e.g.,* 6 months, 1 year, 2 years or more, in some cases indefinitely when treating a chronic disease such as multiple sclerosis. In specific embodiments, the therapy is continued for 2 weeks to 6 months, from 3 months to 5 years, from 6 months to 1 or 2 years, from 8 months to 18 months, or the like. The therapeutic regimen can be a non-variable dose regimen or a multiple-variable dose regimen.

The amount of anti-CD25 antibody administered to the patient is in certain embodiments a therapeutically effective amount. As used herein, a "therapeutically effective" amount of CD25 antibody can be administered as a single dose or over the course of a therapeutic regimen, *e*.*g*., over the course of a week, two weeks, three weeks, one month, three months, six months, one year, or longer.

According to the present disclosure, treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom the anti-CD25 antibody of the disclosure is administered is preferably a mammal such as a non-primate (*e.g.,* cow, pig, horse, cat, dog, rat, *etc.*) or a primate (*e.g.,* monkey or human). In certain embodiments, the subject or patient is a human. In certain aspects, the human is an adult patient. In other aspects, the human is a pediatric patient.

In some embodiments, the constant domains of the humanized antibodies of the invention are human IgA, IgE, IgG or IgM domains. In a specific embodiment, human IgG constant domains, especially of the IgG1 and IgG3 isotypes are used, especially when the humanized antibodies of the invention are intended for therapeutic uses and antibody effector functions are needed.

### 7.9. Pharmaceutical Compositions and Routes of Administration

Compositions comprising an anti-CD25 antibody of the disclosure and, optionally one or more additional therapeutic agents, such as the combination therapeutic agents described in Section 7.10 below, are provided herein. The compositions will usually be supplied as part of a sterile, pharmaceutical composition that will normally include a pharmaceutically acceptable carrier. This composition can be in any suitable form (depending upon the desired method of administering it to a patient).

The anti-CD25 antibodies of the disclosure can be administered to a patient by a variety of routes such as orally, transdermally, subcutaneously, intranasally, intravenously, intramuscularly, intraocularly, topically, intrathecally and intracerebroventricularly. The most suitable route for administration in any given case will depend on the subject, and the nature and severity of the disease and the physical condition of the subject.

For treatment of indications described herein, the effective dose of an anti-CD25 antibody of the disclosure can range from about 0.1 to about 5 mg/kg per single (*e*.*g*., bolus) administration, multiple administrations or continuous administration, or any effective range or value therein depending on the condition being treated, the route of administration and the age, weight and condition of the subject. In certain embodiments, each dose can range from about 0.5 mg to about 2 mg per kilogram of body weight. In other embodiments, each dose can range from about 50 mg to 500 mg, and is in exemplary embodiments about 50 mg, 75 mg, 100 mg, 150 mg, 200 mg, 250 mg, 300 mg, 350 mg or 400 mg. The antibody can be formulated as an aqueous solution and administered by subcutaneous injection. In specific embodiments, the aqueous solution has a pH in the range of about pH 5.5 to about pH 6.5 and comprises about 20-60 mM succinate buffer, about 0.01% to about 0.1% (or about 0.02% - 0.04%) polysorbate, about 75-150 mM sodium chloride, and at least about 100 mg/ml (for example 125 mg/ml or 150 mg/ml) of the anti-CD25 antibody.

Pharmaceutical compositions can be conveniently presented in unit dose forms containing a predetermined amount of an anti-CD25 antibody of the disclosure per dose. Such a unit can contain for example but without limitation 0.1 mg to 0.5 g, for example 20 mg to 500 mg, 50 mg to 250 mg ot 100 mg to 300 mg. In specific embodiments, the unit dose comprises about 100 mg, 150 mg, 200 mg, 250 mg or 300 mg of an anti-CD25 antibody. Pharmaceutically acceptable carriers for use in the disclosure can take a wide variety of forms depending, *e*.*g*., on the condition to be treated or route of administration.

Therapeutic formulations of the anti-CD25 antibodies of the disclosure can be prepared for storage as lyophilized formulations or aqueous solutions by mixing the antibody having the desired degree of purity with optional pharmaceutically-acceptable carriers, excipients or stabilizers typically employed in the art (all of which are referred to herein as "carriers"), *i.e.,* buffering agents, stabilizing agents, preservatives, isotonifiers, non-ionic detergents, antioxidants, and other miscellaneous additives. *See,* Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can be present at concentration ranging from about 2 mM to about 50 mM. Suitable buffering agents for use with the present disclosure include both organic and inorganic acids and salts thereof such as citrate buffers (*e*.*g*., monosodium citrate-disodium citrate mixture, citric acid-trisodium citrate mixture, citric acid-monosodium citrate mixture, *etc*.), succinate buffers *(e.g.,* succinic acid monosodium succinate mixture, succinic acid-sodium hydroxide mixture, succinic acid disodium succinate mixture, *etc.),* tartrate buffers *(e.g.,* tartaric acid-sodium tartrate mixture, tartaric acid-potassium tartrate mixture, tartaric acid-sodium hydroxide mixture, *etc.),* fumarate buffers (*e.g.,* fumaric acid-monosodium fumarate mixture, fumaric acid disodium fumarate mixture, monosodium fumarate-disodium fumarate mixture, *etc.*), gluconate buffers (*e.g.,* gluconic acid-sodium glyconate mixture, gluconic acid-sodium hydroxide mixture, gluconic acid-potassium glyuconate mixture, *etc*.)*,* oxalate buffer (*e*.*g*., oxalic acid-sodium oxalate mixture, oxalic acid-sodium hydroxide mixture, oxalic acid-potassium oxalate mixture, *etc*.), lactate buffers (*e.g.,* lactic acid-sodium lactate mixture, lactic acid-sodium hydroxide mixture, lactic acid-potassium lactate mixture, *etc.)* and acetate buffers *(e.g.,* acetic acid-sodium acetate mixture, acetic acid-sodium hydroxide mixture, *etc.).* Additionally, phosphate buffers, histidine buffers and trimethylamine salts such as Tris can be used.

Preservatives can be added to retard microbial growth, and can be added in amounts ranging from 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (e.g., chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc*., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (e.g., peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, *etc.),* polyoxamers (184, 188 *etc.),* pluronic polyols, polyoxyethylene sorbitan monoethers (TWEEN®-20, TWEEN®-80, *etc.).* Nonionic surfactants can be present in a range of about 0.05 mg/ml to about 1.0 mg/ml, for example about 0.07 mg/ml to about 0.2 mg/ml.

Additional miscellaneous excipients include bulking agents *(e.g.,* starch), chelating agents *(e.g.,* EDTA), antioxidants (*e*.*g*., ascorbic acid, methionine, vitamin E), and cosolvents.

The anti-CD25 antibodies of the disclosure can be formulated into a stable pharmaceutical composition as described in U.S. Patent Publication 2011/0318343. In an exemplary embodiment, the pharmaceutical composition has a pH of pH 5.5 to pH 6.5 and comprises 20-60 mM succinate buffer, 0.02% - 0.04% polysorbate, 75-150 mM sodium chloride, and an anti-CD25 antibody at a concentration of 50 mg/ml or more.

The formulation herein can also contain a combination therapeutic agent in addition to the anti-CD25 antibody of the disclosure.

The dosing schedule for subcutaneous administration can vary from once every six months to daily depending on a number of clinical factors, including the type of disease, severity of disease, and the patient's sensitivity to the anti-CD25 antibody. In specific embodiments, the administration is weekly, monthly, or bimonthly.

The dosage of an anti-CD25 antibody of the disclosure to be administered will vary according to the particular antibody, the type of disease (*e*.*g*., immune disorder or cancer), the subject, and the severity of the disease, the physical condition of the subject, the therapeutic regimen (*e*.*g*., whether a combination therapeutic agent is used), and the selected route of administration; the appropriate dosage can be readily determined by a person skilled in the art.

It will be recognized by one of skill in the art that the optimal quantity and spacing of individual dosages of an anti-CD25 antibody of the disclosure will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop, the amount and/or frequency of the dosage can be altered or reduced, in accordance with normal clinical practice.

### 7.10. Combination Therapy

Described below are combinatorial methods in which the anti-CD25 antibodies of the disclosure can be utilized. The combinatorial methods of the disclosure involve the administration of at least two agents to a patient, the first of which is an anti- CD25 antibody of the disclosure, and the second of which is a combination therapeutic agent. The anti-CD25 antibody and the combination therapeutic agent can be administered simultaneously, sequentially or separately.

The combinatorial therapy methods of the present disclosure can result in a greater than additive effect, providing therapeutic benefits where neither the anti-CD25 antibody or combination therapeutic agent administered in an amount that is alone therapeutically effective.

In the present methods, the anti-CD25 antibody of the disclosure and the combination therapeutic agent can be administered concurrently, either simultaneously or successively. As used herein, the anti-CD25 antibody of the disclosure and the combination therapeutic agent are said to be administered successively if they are administered to the patient on the same day, for example during the same patient visit. Successive administration can occur 1, 2, 3, 4, 5, 6, 7 or 8 hours apart. In contrast, the anti-CD25 antibody of the disclosure and the combination therapeutic agent are said to be administered separately if they are administered to the patient on the different days, for example, the anti-CD25 antibody of the disclosure and the combination therapeutic agent can be administered at a 1-day, 2-day or 3-day, one-week, 2-week or monthly intervals. In the methods of the present disclosure, administration of the anti-CD25 antibody of the disclosure can precede or follow administration of the combination therapeutic agent.

As a non-limiting example, the anti-CD25 antibody of the disclosure and combination therapeutic agent can be administered concurrently for a period of time, followed by a second period of time in which the administration of the anti-CD25 antibody of the disclosure and the combination therapeutic agent is alternated.

Because of the potentially synergistic effects of administering an anti-CD25 antibody of the disclosure and a combination therapeutic agent, such agents can be administered in amounts that, if one or both of the agents is administered alone, is/are not therapeutically effective.

It is contemplated that when used to treat various diseases, the anti-CD25 antibodies of the disclosure can be combined with other therapeutic agents suitable for the same or similar diseases. In addition, because anti-CD25 antibodies target inflammatory pathways, they can be used in combination with anti-inflammatory agents such as acetaminophen, diphenhydramine, meperidine, dexamethasone, pentasa, mesalazine, asacol, codeine phosphate, benorylate, fenbufen, naprosyn, diclofenac, etodolac and indomethacin, aspirin and ibuprofen.

When used for treating cancer, antibodies of the present disclosure can be used in combination with conventional cancer therapies, such as surgery, radiotherapy, chemotherapy, anti-angiogenic agents, or combinations thereof.

Suitable chemotherapeutics include, but are not limited to, radioactive molecules, toxins, also referred to as cytotoxins or cytotoxic agents, which includes any agent that is detrimental to the viability of cells, agents, and liposomes or other vesicles containing chemotherapeutic compounds. Examples of suitable chemotherapeutic agents include but are not limited to 1-dehydrotestosterone, 5-fluorouracil decarbazine, 6-mercaptopurine, 6-thioguanine, actinomycin D, adriamycin, aldesleukin, an anti-α5β1 integrin antibody, alkylating agents, allopurinol sodium, altretamine, amifostine, anastrozole, anthramycin (AMC)), anti-mitotic agents, cisdichlorodiamine platinum (II) (DDP) cisplatin), diamino dichloro platinum, anthracyclines, antibiotics, antimetabolites, asparaginase, BCG live (intravesical), betamethasone sodium phosphate and betamethasone acetate, bicalutamide, bleomycin sulfate, busulfan, calcium leucouorin, calicheamicin, capecitabine, carboplatin, lomustine (CCNU), carmustine (BSNU), chlorambucil, cisplatin, cladribine, colchicin, conjugated estrogens, cyclophosphamide, cyclothosphamide, cytarabine, cytarabine, cytochalasin B, cytoxan, dacarbazine, dactinomycin, dactinomycin (formerly actinomycin), daunirubicin HCL, daunorucbicin citrate, denileukin diftitox, dexrazoxane, dibromomannitol, dihydroxy anthracin dione, docetaxel, dolasetron mesylate, doxorubicin HCL, dronabinol, *E. coli* L-asparaginase, eolociximab, emetine, epoetin-α, Erwinia Lasparaginase, esterified estrogens, estradiol, estramustine phosphate sodium, ethidium bromide, ethinyl estradiol, etidronate, etoposide citrororum factor, etoposide phosphate, filgrastim, floxuridine, fluconazole, fludarabine phosphate, fluorouracil, flutamide, folinic acid, gemcitabine HCL, glucocorticoids, goserelin acetate, gramicidin D, granisetron HCL, hydroxyurea, idarubicin HCL, ifosfamide, interferon α-2b, irinotecan HCL, letrozole, leucovorin calcium, leuprolide acetate, levamisole HCL, lidocaine, lomustine, maytansinoid, mechlorethamine HCL, medroxyprogesterone acetate, megestrol acetate, melphalan HCL, mercaptipurine, mesna, methotrexate, methyltestosterone, mithramycin, mitomycin C, mitotane, mitoxantrone, nilutamide, octreotide acetate, ondansetron HCL, paclitaxel, pamidronate disodium, pentostatin, pilocarpine HCL, plimycin, polifeprosan 20 with carmustine implant, porfimer sodium, procaine, procarbazine HCL, propranolol, rituximab, sargramostim, streptozotocin, tamoxifen, taxol, teniposide, tenoposide, testolactone, tetracaine, thioepa chlorambucil, thioguanine, thiotepa, topotecan HCL, toremifene citrate, tretinoin, valrubicin, vinblastine sulfate, vincristine sulfate, and vinorelbine tartrate.

Any anti-angiogenic agent can be used in conjunction with the anti-CD25 antibodies of the disclosure for the treatment of cancer, including those listed by Carmeliet and Jain, 2000, Nature 407:249-257. In certain embodiments, the anti-angiogenic agent is a VEGF antagonist or a VEGF receptor antagonist such as VEGF variants, soluble VEGF receptor fragments, aptamers capable of blocking VEGF or VEGFR, neutralizing anti-VEGFR antibodies, low molecule weight inhibitors ofVEGFR tyrosine kinases and any combinations thereof. Alternatively, or in addition, an anti-VEGF antibody may be co-administered to the patient.

When used for treating multiple sclerosis, antibodies of the disclosure can be used in combination with other targeted agents useful for treating multiple sclerosis, for example interferon β such as interferon β-1a (*e*.*g*., Avonex® or Rebif®) or interferon β-1b (*e*.*g*., Betaseron® or Extavia®); glatiramer acetate (*e.g.,* Copaxone®); fingolimod (*e.g.,* Gilenya®); mitoxantrone (*e.g.,* Novantrone®); natalizumab (*e*.*g*., Tysabri®); ocrelizumab (humanized anti-CD20 monoclonal antibody); pegylated interferon β-1a; dimethyl fumarate (tecfidera); fampridine (*e*.*g*., fampyra (prolonged-release fampridine tablets, marketed in the U.S. as Ampyra); alemtuzumab (*e.g.,* Lemtrada®); laquinimod; and teriflunomide (*e.g.,* Aubagio®).

When used for suppressing organ transplant rejection, antibodies of the disclosure can be used in combination with immunosuppressive agents such as corticosteroids; cyclosporin A; tacrolimus; rapamycin; mycophenolate mofetil; and azathioprine.

### 7.11. Diagnostic and Pharmaceutical Kits

Encompassed by the present disclosure are pharmaceutical kits containing the anti-CD25 antibodies (including antibody conjugates) of the disclosure. The pharmaceutical kit is a package comprising the anti-CD25 antibody of the disclosure (*e*.*g*., either in lyophilized form or as an aqueous solution) and one or more of the following:
A combination therapeutic agent, for example as described in Section 5.10 above;
A device for administering the anti-CD25 antibody, for example a pen, needle and/or syringe; and
Pharmaceutical grade water or buffer to resuspend the antibody if the antibody is in lyophilized form.

In certain aspects, each unit dose of the anti-CD25 antibody is packaged separately, and a kit can contain one or more unit doses (*e*.*g*., two unit doses, three unit doses, four unit doses, five unit doses, eight unit doses, ten unit doses, or more). In a specific embodiment, the one or more unit doses are each housed in a syringe or pen.

Diagnostic kits containing the anti-CD25 antibodies (including antibody conjugates) of the disclosure are also encompassed herein. The diagnostic kit is a package comprising the anti-CD25 antibody of the disclosure (*e*.*g*., either in lyophilized form or as an aqueous solution) and one or more reagents useful for performing a diagnostic assay. Where the anti-CD25 antibody is labeled with an enzyme, the kit can include substrates and cofactors required by the enzyme (*e*.*g*., a substrate precursor which provides the detectable chromophore or fluorophore). In addition, other additives can be included, such as stabilizers, buffers (*e*.*g*., a block buffer or lysis buffer), and the like. In certain embodiments, the anti-CD25 antibody included in a diagnostic kit is immobilized on a solid surface, or a solid surface (*e*.*g*., a slide) on which the antibody can be immobilized is included in the kit. The relative amounts of the various reagents can be varied widely to provide for concentrations in solution of the reagents which substantially optimize the sensitivity of the assay. In a specific embodiment, the antibody and one or more reagents can be provided (individually or combined) as dry powders, usually lyophilized, including excipients which on dissolution will provide a reagent solution having the appropriate concentration.

### 8. EXAMPLES

### 8.1. OVERVIEW

Daclizumab, a humanized IgG1 anti-CD25 monoclonal antibody, was subject to extensive mutational analysis to identify variants with beneficial properties. The generation of daclizumab was one of the earliest antibody humanizations conducted by the Queen method (Queen et al., 1989, Proc. Nat'l Acad. Sci. U.S.A. 86:10029-33). Although the humanized antibody maintained the function of the murine parental antibody (anti-Tac) and was approved for preventing rejection of kidney transplants, there was a 3-fold affinity loss after humanization in cell based binding assays (see Queen *et al., supra*). Given the availability of additional human framework sequences available and improvements in computer modeling since daclizumab was generated, anti-Tac was rehumanized to assess whether improved humanization designs existed that retain the affinity to CD25 and the functionality of the murine parental antibody (see Example 1). Further mutational analysis included affinity maturation and screening of combinatorial libraries of CDR mutants of daclizumab and characterization of individual clones (see Example 2, below), alanine scanning of CDR residues to identify residues important for binding (see Example 2), a comprehensive mutagenesis of CDR positions and analysis of variant behavior in a population to identify variants that show comparable or increased affinity to CD25, with subsequent confirmation of the binding and biological properties of representative individual variants (see Example 3), and an analysis of amino acids involved in T-cell immunogenicity of daclizumab and identification of "deimmunized" variants that maintain binding to CD25 (see Example 4). The results of these studies are summarized in Tables 6-8. Table 6A summarizes individual CDR or framework amino acid substitutions which were confirmed at the individual clone level to result in beneficial properties. Table 6B shows additional single amino acids substitutions within HC CDRs tested only by ELISA direct binding assay to plate coated CD25. Tables 7A-7C summarize the kinetic and biological properties of variants of daclizumab with multiple CDR substitutions as tested on the individual clone level. Tables 8A-8B identify individual CDR substitutions whose behavior in the context of a population of variants suggests have comparable or improved binding to CD25 as compared to daclizumab. In some cases the variants were grafted onto different constant regions than the IgG1 of daclizumab. The isotype of the non-IgG1 antibodies is reflected in the tables.

### 8.2. EXAMPLE 1: REHUMANIZATION OF MOUSE ANTI-TAC MONOCLONAL ANTIBODY

To rehumanize the VH of mouse anti-Tac, R3.5H5G (Manheimer-Lory et al., 1992, J. Exp. Med. 174:1639-1652) in subgroup I was used as a human framework (Figure 2A). Nine amino acids were predicted to be structurally critical and eight of them, except position 69, were substituted to corresponding mouse residues (underlined in Figure 2A) in NuhuTac. In the original humanization, in which Eu (Kabat et al., 1987, Sequences of Proteins of Immunological Interest, 4th edit., Public Health Service, N.I.H. Washington, DC) was used as a human framework, 12 residues were substituted to mouse residue. In addition to the humanizing substitutions in NuhuTac, Glu was selected as the N-terminal amino acid to avoid heterogeneity due to pyroglutamate formation from an N-terminal Gln (Chelius et al., 2006, Anal. Chem. 78: 2370-2376).

To rehumanize the VL of mouse anti-Tac Mab, ka3d1 (Qlee et al., 1992, J. Exp. Med. 175: 831-842) in subgroup III was used as a human framework. One amino acid was predicted to be structurally critical and was thus substituted to the corresponding mouse residue (underlined in Figure 2B) in NuhuTac. In the original humanization, in which Eu was used as a human framework, three residues were substituted to the corresponding mouse residue. Thus, the rehumanizing antibody had fewer murine residues and was predicted to be less immunogenic than daclizumab. In addition, due to the N-terminal Gln-to-Glu substitution, the rehumanized anti-Tac was predicted to be less heterogeneous than the original humanized anti-Tac.

Figure 2C shows the results of testing four combinations of daclizumab and NuhuTac heavy and light chains. An approximatley 2-fold improvement in binding by ELISA competition assay was observed in the rehumanized antibody (NuhuTac).

Combination antibodies were generated by combining (a) NuhuTacVH with daclizumabVL and (b) daclizumab VH and NuhuTacVL. The combination NuhuTacVH with daclizumabVL retained the higher affinity of NuhuTac whereas the combination of daclizumab VH and NuhuTacVL had a lower affinity than NuhuTac. Thus, the heavy chain substitutions likely gave rise to increased affinity of NuhuTac.

Next, the key substitution in the VH giving rise to improved affinity was identified. Of the six differences between VH of daclizumab and NuhuTac (underlined in the daclizumab VH sequence of Figure 2A), positions 69 and 73 (doublelined in the daclizmab VH sequence of Figure 2A) were postulated to be especially important as they are predicted to contact with CDR loop (Foote and Winter, 1992, J. Mol. Bio. 224: 487-499). Accordingly, the substitutions I69M, I69L and E73K were tested in the context of daclizumab. As shown in Figure 2D, E73K, but not I69M or I69L, proved to be important for the increased affinity of NuhuTac relative to daclizumab.

### 8.3. EXAMPLE 2: AFFINITY MATURATION OF A HUMANIZED ANTI-CD25 ANTIBODY USING MAMMALIAN CELL-BASED WHOLE IG_{G} DISPLAY LIBRARIES

Daclizumab was affinity matured for further improvement in its biological function, inhibiting IL2 from binding to IL2 receptor α chain. Using an EBV-based episomal vector, antibody libraries were displayed as whole IgG molecules on mammalian cell surface and screened for specific antigen binding by a combination of magnetic beads and fluorescence-activated cell sorting (Akamatsu et al., 2007, J. Immunol. Methods 327:40-52). V_{H} and V_{L} libraries with combinatorial mutations were screened separately to identify beneficial mutations. These mutations were then combined to generate a mini-library to identify combinations of V_{H} and V_{L} to achieve the highest binding affinity. As a result, high affinity variants were successfully identified, the highest being 14 pM in affinity, which is a 28-fold improvement over parental daclizumab. An improvement in IL2-receptor blocking activity of up to 3.9-fold was observed by introducing only three amino acids substitutions. Higher affinity (lower K_{D}) correlated with improved function in blocking IL2-receptor in general. Further break down of K_{D} indicated that both faster *k*ₒₙ values and slower *k*_{off} value deliver positive impact on function, however, faster *k*ₒₙ values showed stronger correlation with improved function than slower *k*_{off} value. Functional activity was more strongly correlated with K_{D} when they the variants were tested as Fab fragments.

### 8.3.1. Materials & Methods

### 8.3.1.1. ELISA competition assay

Daclizumab was biotinylated using NHS-LC-LC Biotin kit (Pierce, #21338). Wells of 96-well ELISA plates (Nunc-Immuno MaxiSorp plates, Nalge Nunc, Rochester, NY) were coated with 100 µL of 0.2 µg/mL CD25 (Pepro Tech Inc., Rocky Hill, New Jersey) in 0.2 M sodium carbonate-bicarbonate buffer (pH 9.4, Pierce, Rockford, IL) overnight at 4°C. After washing with Wash Buffer, wells were blocked with 200 µL of Superblock Blocking Buffer (Pierce, Rockford, IL) for 30 min and then washed. A mixture of sub-saturating amount of biotinylated daclizumab (80 ng/mL) and competitor antibody in serial dilution in ELISA Buffer was applied to wells in a final volume of 100 µL and incubated for 1hr at 37°C shaker. The plate was then washed with washing buffer three times. After washing, 100 µL of 1 µg/mL HRP-conjugated Streptavidin (Pierce) diluted in ELISA buffer was added to each well. After 30 minutes of incubation at room temperature, plates were washed and bound antibodies were detected by addition of ABTS substrate (Kirkegaard & Perry Laboratories, Gaithersburg, MD). The reaction was terminated by addition of 100 µL/well of 2% oxalic acid and the absorbance was measured at 415 nm using a VERSAmax microplate reader (Molecular Devices, Sunnyvale, CA). Binding inhibition curves were fitted using nonlinear regression with the software GRAPHPAD PRISM (GraphPad, San Diego) and reported as IC₅₀ wild type/IC₅₀ mutant (fold improvement over wild type control).

### 8.3.1.2. BIAcore assay

Binding affinities of daclizumab variants were measured by using a BIAcore 2000 and 3000 surface Plasmon resonance system (BIAcore, Neuchatel, Switzerland). Polyclonal goat anti-human Fc antibody (Jackson ImmunoResearch) was immobilized on a chip according to the manufacturer's instructions. Binding assays to study the binding of daclizumab and CD25 were run at a flow rate of 30 µL/min at room temperature. CD25 (Pepro Tech Inc.) in 8 different concentrations between 1-128 nM was injected over surfaces where daclizumab and its variants were captured, with a 3-minute association phase followed by 15-minute dissociation phase. Binding data were fit to the 1:1 Langmuir model to extract binding constants from the BIAevaluate software. All the binding kinetics data were analyzed by at least three separate determinations.

### 8.3.1.3. IL2-dependent Kit225/K6 proliferation assay

Kit225/K6 is an IL2 dependent T cell line derived from a patient suffering from T cell chronic leukemia (Hori, 1987, Blood 70:1069-1672). The cells are normally maintained in growth medium (RPMI-1640, 10% HI (heat inactivated)-FBS, 50 µg/ml gentamicin (Sigma) and 5 ng/mL of recombinant human IL2 ("rhIL2") (Roche Applied Science, Indianapolis, IN). On the day of assay, the cells were washed with RPMI-1640 three times and resuspended in IL2 free medium (RPMI 1640 medium containing 10% heat-inactivated FBS and 50 µg/mL gentamicin at the cell density of 50,000 cells/mL. Serially-diluted antibodies were prepared in rhIL2 containing assay medium (RPMI-1640, 10% heat-inactivated FBS, 50 µg/ml gentamicin and 0.2 ng/ml of rhIL2). Subsequently, 100 µL of diluted antibodies was mixed with 100 µL of previously prepared cells in 96-well sterile tissue culture plate. After 54 +/- 2 hours incubation at 37°C in a CO₂ incubator, 20 µL of AlamarBlue (Biosource International, Camarillo, CA) was added to each well and incubated overnight at 37°C in a CO₂ incubator in order to quantitatively measure the level of cell proliferation. After 18 +/- 1 hours of incubation, the signal was read spectrofluorometrically (excitation at 544 nM, emission at 590 nM) using a SPECTRAmax GEMINI SX microplate reader (Molecular Devices). ANOVA (analysis of variance) was used to analyze the statistical differences.

### 8.3.1.4. FACS binding assay

2x10⁵ of Kit225/K6 or HuT 102 (Gazdar, 1980, Blood 55: 409-17) expressing high-affinity IL2-R, were aliquoted in each well of a 96-well block (Corning, 2 ml capacity assay block). Cells were washed with 600 µL of FACS buffer (PBS + 1% BSA) twice. Daclizumab and its mutants were prepared at 5 µg/mL and diluted serially at 1:3 or 1:5 in FACS buffer. Then 100 µL (in some cases, 25 µL) of diluted antibodies were mixed with previously washed cells in each well and incubated for 1 hour on ice. Then the cells were washed again. 25 µL of Goat-anti-HuIgG-FITC conjugated antibody (Southern Biotech) diluted at 1:250 was added into each well and incubated for 30 minutes on ice at dark. After wash, the cells were suspended in 400 µL FACS buffer. The amount of antibody binding to the cell surface antigen was measured by flow cytometry Cyan (Dako).

### 8.3.2. Construction and enrichment of V_{H} and V_{L} library

CDR1 and CDR3 of the heavy chain variable domain (V_{H}) and CDR3 of the light chain variable domain (V_{L}) of daclizumab were considered to be critical for CD25 binding, while the remaining three CDR were thought to contribute to a lesser extent (Glaser, 1992, Journal of Immunol. 149:2607-2614). Because the affinity of daclizumab is subnanomolar level, binding center is likely to be near optimized though natural selection. To fine tune the periphery of binding surface, the CDRs that are considered to be less critical for binding were mutagenized. V_{L} and V_{H} libraries were constructed separately with limited choice in amino acids at the position of interest. Six positions in the V_{L} and five positions in the V_{H} thought to be highly variable and at the periphery of the binding surface (Wu and Kabat, 1970, J. Exp. Med. 132: 211-250) were chosen for mutagenesis. Conservative change, polar-to-apolar change and some charged amino acids were included so as to produce up to 10⁵ combinations of amino acid variants.

For the V_{L} library, two positions (29 and 31) from CDR1 and four positions (50, 51, 52 and 53) from CDR2 were chosen for mutagenesis. For the V_{H} library, five positions (52, 53, 54, 56 and 58) exclusively in CDR2 were selected. Amino acid variations at each position of interest in V_{L} and V_{H} are listed in Table 3.

Mutations at each position were introduced by PCR using primers containing degenerated codons. Library fragments were subcloned into an EBV-based episomal vector to display antibody variants in a form of IgG1/κ. To evaluate the quality of the library, miniprep DNA of 20-96 clones derived from each library were sequenced and confirmed that the mutations were introduced as at the positions it was designed (not shown).

The V_{L} and V_{H} library DNA, as well as control vectors, were transfected separately into 293c18 for IgG display. As a result, a VL and a VH library comprising approximately 2.9 x 10⁷ and 3.3 x 10⁶ independent clones were obtained, respectively.

The V_{L} library transfectants went through three rounds of FACS enrichment to select the clones expressing daclizumab variants that binds to human CD25 at higher affinity. At each round, cells were first incubated with an extracellular domain of CD25 fused with lambda light chain constant region (CD25-Cλ). After washing, the cells were double stained with PE-labeled goat anti-human lambda light chain antibody to detect cells bound to antigen fusion protein, and with PECy5-labeled anti-human gamma chains antibody to monitor the level of surface IgG. Antigen concentration was titrated to determine optimal binding condition for each round before sorting. To enrich clones displaying antibodies which affinity is higher than parental antibody, the sorting gate was set to double positive of above diagonal line based on staining of cells displaying daclizumab. Typically, 1-3% of total cells were sorted at each round of all libraries described in this study, unless otherwise stated. After each round of selection and culturing, cells were stained with CD25-Cλ to monitor the level of enrichment of CD25 binders. In the first round sorting, two different antigen concentrations were used, 3 nM and 1 nM. The resulting populations were cultured in the growth media separately for the 2nd round sorting using CD25-Cλ at 0.5 nM. Since these two populations looked similar in FACS staining (10 % and 7 % positive in binding, respectively (data not shown)), they were mixed to go through the 3rd round enrichment using CD25-Cλ at 0.3 nM. The cell transfected with a display vector without insert has no surface Ig and showed little nonspecific binding at 5 nM CD25-Cλ. When unsorted V_{L} library was stained at 3 nM antigen concentration, 4.4% of cells were double positive in binding and surface Ig expression. After the third round of enrichment, it became 72% positive, exceeding the percentage of positive cells transfected to display parental antibody (27%).

The V_{H} library went through three rounds of FACS enrichment and one negative selection against binding to an irrelevant antigen. In the first round of sorting, 5 nM CD25-Cλ was incubated with library cells at two different conditions, 1 hour or 2 minutes. At 2 minutes incubation, binding of wild type daclizumab is not saturated yet, thus the short incubation time was intended for enrichment of high affinity antibodies with some emphasis to faster association rate. The resulting cell populations collected from these conditions were cultured and used for the 2nd round sorting using CD25-Cλ at 0.5 nM for incubation 1 hour, or 3 nM for incubation 2 minutes, respectively. After the expansion of sorted populations, cells were to absorb non-specific binders using magnetic beads as described in Materials and Methods, and then enriched for 3rd round using CD25-Cλ at 0.1 nM for incubation 1 hour or 0.5 nM for 2 minutes, respectively. When unsorted V_{H} library was stained at the condition at 5 nM antigen concentration, 3.5% of cells showed positive binding to begin with. After the 3rd round of enrichment, it became 79% positive in antigen binding in either sorting condition, exceeding the percentage of positive cells transfected to display parental antibody (50%, data not shown).

During characterization of V_{L} variants, we experienced enrichment of some variants that seemed to gain nonspecific characteristics due to amino acid substitutions. Such clones can survive until the end of enrichment but can be difficult to purify, due to nonspecific binding to protein A column. From this experience, negative selection was introduced for V_{H} library enrichment using magnetic beads conjugated with irrelevant protein, to exclude non-specific binders from the population. As a result, no clone gained nonspecific characteristics were identified from V_{H} library.

### 8.3.3. Identification of V_{L} and V_{H} variants with higher affinity to CD25

After the final enrichment, cells were expanded and plasmid DNA was rescued as described. Several hundreds of independent colonies were obtained after electroporation. The plasmid prepared as mixture was converted to the form producing soluble IgG1, by removing the region encoding membrane tether domain with restriction enzyme digestion. The digested vector was then re-ligated and transformed into bacteria. The colonies were cultured individually in the 96-well format and plasmid DNA was isolated for sequencing analysis.

For V_{L} enrichment, plasmid DNA was rescued from each round and compared the progress of enrichment of particular mutations. A total of 86, 89 and 41 sequences were obtained from the first, second and third round of enrichment, respectively. The numbers of independent sequences were reduced from 52, 40 and 16, as it enriched (60%, 45% and 39%), indicating population was biased to certain combinations as enrichment proceeded. Frequency in observing R²⁹S³¹ or R²⁹T³¹ in CDR1 was consistently increased after first, second and third round of enrichment from 2%, 8% and 10%, and 7%, 9% and 10%, respectively. Similarly, the frequency of T⁵⁰T⁵¹S⁵²D⁵³ (SEQ ID NO: 184) in CDR2 was increased from 2%, 8% and 12%. None of other combination was enriched at a frequency of more than 5% at the final enrichment, except one showing nonspecific binding property. The plasmids containing these mutations in secretion from were transiently transfected for antibody expression and binding affinity of purified antibodies were compared by ELISA competition as initial characterization. All three variants showed an approximately 2-fold improvement in binding. Because S29R and S29R-S31T in CDR1 showed no significant difference in binding, S31T was excluded from further analysis. Since both positions 29 and 53 changed from a neutral to a charged residue, we decided to test another residue with similar characteristics to address if there charges were responsible to improvement in antigen binding. For position 29 in CDR1, another positively charged amino acid, Lys, was tested as well as Arg. For position 53 in CDR2, another negatively charged amino acid, Glu, was tested as well as Asp. Antibodies were purified from culture supernatant of transient expression and tested for competitive ELISA. Daclizumab and its variants were competed with biotinylated daclizumab for binding to CD25 in a concentration-dependent manner. Both mutations showed improvement in binding (approximately 3- and 5-fold improvement in IC₅₀ for N53E and S29K, respectively), even better than those originally identified from library (approximately 1.5- and 2-fold improvement in IC₅₀ for N53D and S29R, respectively). S29K was not identified from the library because it was not included as a choice at position 29. On the other hand, N53E was not identified as an enriched mutation even it was included as a choice at position 53 (Table 3, left). This is most likely due to the incomplete coverage of all the possible combination of amino acid substitutions at the transfection level. The Glu substitution at this position survived at low frequency (5%) at the end of enrichment, suggesting that cells expressing mutants with appropriate combination were not available in the initial population. Insufficient coverage of library population may be partly due to high background of parental sequence existed in V_{L} library. Percentage of parental sequence in V_{L} library was 18% and 31%, before and after enrichment, respectively. Due to the same reason, wild type residues looked enriched the most at each position, when enrichment was analyzed by position-by-position.

Because not all the combinations of these beneficial mutations from the V_{L} library were identified, a total of eight variants were generated individually in the light chain expression vector to evaluate the effect of combinatorial mutations. These plasmids were cotransfected into 293T with an expression vector expressing a parental heavy chain for production of antibodies as secretion from. The antibodies were purified from culture supernatant and their binding kinetics was analyzed by BIAcore.

At position 53, Glu showed slightly better affinity than Asp (NST-SE and NST-SD; 190 pM and 204 pM, respectively). At position 29, Lys showed better affinity than Arg (NST-KN and NST-RN; 227 pM and 262 pM, respectively). However, the combination of S29K and N53E did not result in the best V_{L} variants (See NST-KE). Although N53E was the highest in affinity (KD) within the identified V_{L} variants with single amino acid substitution, it does not seem to fully combine with mutation at position 29. Instead, N53D combined additively with either mutation at position 53 (S29R-N53D: 2.5 x 1.9 = ~4.9-fold; S29K-N53D: 2.5 x 2.2 = ~5.1-fold). In conclusion, the best V_{L} variants was identified to be S29K-N53D, with affinity to be 98 pM, up to 5.1-fold improvement in K_{D} over the parental antibody.

For V_{H} enrichment, plasmid DNA was rescued from the final rounds of enrichment and the enrichment of particular mutations were compared at each position. Because there was no significant difference in sequences obtained from two pools sorted in different staining conditions, the results were combined to analyze. Unlike the V_{L} library where final population was severely biased to certain combinations with significant amount of parental sequences, the V_{H} library was still diverse after the third round of enrichment as 67 independent sequences obtained out of 82 sequences (82%). No parental sequence was observed before and after enrichment from the number of sequences obtained (64 and 82, respectively). The most frequent combination of V_{H} mutation was VRKYQ (when parental VH positions N52,S53,T54,Y56,E58 is represented as NSTYE) occurring 6 times, followed by RRGFE (4 times) and RKGFE, RRGYE, RKGFN, SNKYL, QRKFH, RRKFE, VKRFQ occurring twice. To confirm the affinity of these mutants, the membrane tether was removed from the plasmid containing each mutation, soluble forms were expressed from transient transfection and proteins were analyzed by competitive ELISA. As a result, all of them turned out to be high affinity variants, ~10 fold over the parental antibody. Although it was difficult to identify the most enriched combination from this number of sequence data because of the large library size, positively charged sequences were preferred at positions 52, 53 and 54. When the enrichment ratio at each individual position was analyzed, Arg, Ser and Val were consistently enriched at position 52, at least 2-fold over theoretical percentage. On the other hand, most of other choices except Lys and Gln were excluded from the population, to be less than 0.3 in enrichment ratio. At position 53, Arg and Lys were enriched more than 3-fold, whereas Glu, Ile, and Thr were excluded. At position 54, Gly, Lys and Arg were enriched, however, most of other choices were excluded except Asp and Val. Position 56 did not show any preference to either choice, because nearly equal number of each amino acid was recovered (39 with Phe and 43 with Tyr, out of 82 sequences isolated from the third round of selection). At position 58, Glu and Gln were enriched 7- and 4-fold, respectively, whereas other choices except Asn, His and Gly were eliminated. Interestingly, even parental residues were eliminated in some cases such as N52 or T54, suggesting that some positions were not fully optimized during affinity maturation process *in vivo.*

To test if those amino acids enriched at each position of 52, 53 and 54 are responsible for improved affinity, four combinations of amino acids that were not identified from as a single clone, RKR, RRK, SRK and RKK (when parental antibody was denoted as NST at positions 52, 53 and 54), were subcloned into vector to express them secreted proteins. Positions 56 and 58 were left as they were in wild type (Y and E). Soluble form IgG was transiently expressed in 293T and purified antibodies were tested on BIAcore analysis. All of these variants showed two digits in K_{D} with 7-23 fold improvement in affinity over parental antibody. They all showed affinity better than the best V_{L} variants identified, which is 98 nM (5.1-fold improvement over daclizumab) with S29K-N53D mutation, suggesting that each of these three positions were likely to be responsible in affinity improvement. Taken together with ELISA results, several amino acid combinations can give rise to an affinity that is at least 10-fold higher than daclizumab.

### 8.3.4. Construction and enrichment of mini-library to combine V_{H} and V_{L} mutations

To isolate the combination of V*_{L}* and V_{H} to achieve highest affinity, mutations enriched in V*_{L}* and V_{H} library, as well as those confirmed as beneficial separately, were combined into one small library. Because not all the mutations may have additive or synergistic effect when they are combined, wild type amino acid was included at each position to achieve highest affinity with minimal number of mutation. Library complexity at amino acid level of the V_{H}-V*_{L}* mini library was 2,160 (2,592 at nucleotide level).

The 293c18 stable transfectants that contained the mini library went through 3 rounds of FACS-based enrichment to obtain V_{H}-V*_{L}* combinatorial variants with highest binding affinity to human CD25. The mini combinatorial library was stained and sorted in two distinct approaches: one with simple FACS binding with increasing stringency and another employed competitive binding for FACS staining. In the former approach, 1 nM, 0.07 nM and 0.02 nM CD25-Cλ were used for the initial, 2^{nd} round and the final round of sorting, respectively. For the latter approach, cells were sorted as usual without competitor for the first round of sorting at 1 nM. Then, the expanded cells were incubated with 0.1 nM CD25-Cλ in the presence of parental daclizumab for 2^{nd} round of enrichment. The concentration of competitor antibody has been optimized to be able to compete away 90% of daclizumab displayed on cell surface. After sorting, the cells were stained at 1 nM CD25-Cλ and analyzed by FACS to compare the level of enrichment. Little binding was observed to IL13Rα1-Cλ after 3^{rd} round of enrichment, indicating that the vast majority of the cells expressing IgG specific for the extracellular domain of CD25. No 3^{rd} round of enrichment was performed after competitive FACS enrichment, as binding percentage after the competitive sort looked comparable to what observed after the third round of enrichment without competition. From conventional three-round FACS enrichment method (FS3), 34 independent antibody sequences were obtained from 66 clones. From the enrichment method with competition (FS2C), 67 independent antibody sequences were obtained from 89 clones. The most frequently observed V_{H}-V_{L} combination from FS3 was RKTE-SE (7 times), followed by VKRE-RE (5 times) (parental V_{H}-V_{L} combination N⁵²S⁵³T⁵⁴E⁵⁸-S²⁹N⁵³ was denoted as NSTE-SN here). For FS3, the two most frequent V_{H} variants were RNRE (8 times) and RKTE (7 times) and for FS2C, they were VSRE (12 times) and KSRE (6 times). The most frequently observed V_{H}-V_{L} combinations from FS2C were VRRE-SE (4 times) and VSRE-KD (4 times). For V_{L}, The most preferred combination for either condition was SE (52 times in FS3; 24 times in FS2C), followed by RE (18 times for FS3; 20 times for FS2C). At position 29, a parental residue, Ser, seemed to be enriched in either condition while Lys was excluded in FS3. At position 53 of V*_{L}*, Glu was preferred over parental Asp residue at either condition. At position 52, V_{H}, Arg and Ver were most enriched in FS3 and FS2C, respectively. On the other hand, Asp, Glu, Gly are clearly excluded, generally reproduced the results of V_{H} library (Table 1, right). At, position 54, both Lys and Arg seemed to be preferred over parental residue, Thr, with preference in Arg in FS2C. Although position 53 seems to have no preference in choice, position 58 was heavily biased Glu in either condition. Based on these results, six variants containing enriched amino acids combination, and a combination of the highest affinity V_{H} identified in V_{H} library (S⁵²R⁵³K⁵⁴) and the most enriched V*_{L}* in mini library (S²⁹E⁵³) were chosen for characterization. The library members were transiently expressed and purified though protein A column. Binding affinities of these variants were in the range of 14-40 pM in *K*_{D}, which is 13-36 fold improvement from parental antibody. The members were also tested for functional assay, measured by proliferation of inhibition of IL2 dependent cell line, Kit225/K6, to compare the ability to block the IL2-R from binding to its ligand, IL2. The variants with improved IL2-R blockade should require less amount of antibody to inhibit proliferation. The IC₅₀ value of variants were normalized with that of parental antibody and shown as functional improvement. Interestingly, not all of them were improved in function even all the 7 variants were high in affinity, suggesting involvement of other factors involved in the efficiency of translation of affinity into biological function.

### 8.3.5. Correlation between binding kinetics and biological function

To understand the relationship between affinity and biological function better, we next attempted to identify some mutations reducing affinity of daclizumab. To identify mutations likely to moderately reduce but not completely knock down antigen binding, alanine substitutions were constructed on eight positions within V_{L} CDR1 and CDR2, predicted to be exposed in solution (S27A, S29A, S31A, Y32A, T50A, T51A, S52A and N53A). After prescreening in ELISA competition assay, four antibodies showed reduction in binding increasing in IC₅₀ at least 2-fold (S31A, Y32A, T50A and T51A, data not shown). These were further tested to measure binding kinetics and ability to inhibit proliferation of Kit225/K6 cells. Most of alanine substitutes of tested showed significant reduction in function, except T50A which showed binding affinity equivalent to parental antibody. In conclusion, reduction in affinity seemed to result in reduction in biological function, at least for those tested.

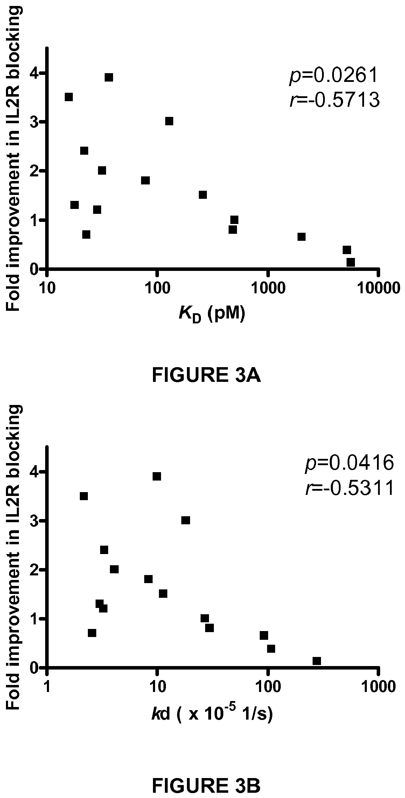

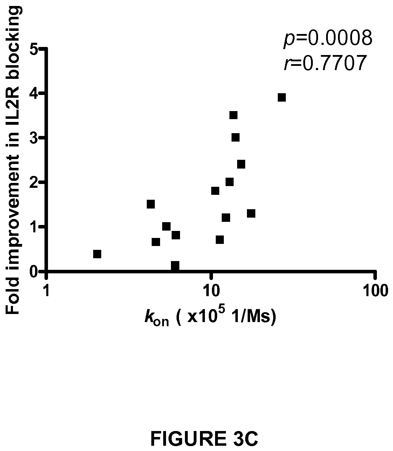

All the data containing both biological function and binding kinetics obtained in this study were plotted in graphs (Figures 3A-3C). Improvement in receptor blocking activity was correlated with smaller value in K_{D} (*p*=0.0261), indicating having higher affinity helps biological function of daclizumab in general (Figure 3A). The correlations were still true, even the affinity data was broken down into *kₒₙ* and *k*_{off} (Figures 3B and 3C). Larger value in *k*ₒₙ (*p*=0.0008) correlated more strongly with biological function than smaller *k*_{off}, (*p*=0.0416) indicating that faster binding was preferred over slower dissociation to improve efficacy of daclizumab.

### 8.3.6. Dissection of two variants with fastest on-rate and slowest off-rate

Although higher affinity generally correlated with better biological function, affinity alone is not responsible for the improvement in biological activity. For example, V⁵²S⁵³R⁵³ - K²⁹D⁵³ (VSR-KD) showed the highest affinity among all, due to its slowest *k*_{off}, however, this variant did not give the maximal improvement in biological activity. On the other hand, K⁵²S⁵³R⁵³ - S²⁹E⁵³ (KSR-SE) showed the best improvement in function, possibly due to its fastest *k*ₒₙ, even it was not show the best improvement in affinity.

To understand the factors involved in determining daclizumab function better, these two variants were selected to further analysis. The DNA fragments encoding heavy and light chains were subcloned separately into vectors to evaluate the contribution of V_{H} and V*_{L}* mutations. Combinations of different V_{H} and V*_{L}* mutations were easily addressed by co-transfecting them. Variant antibodies of 6 combinations with and without V*_{L}* mutations were expressed, and purified antibodies were subjected to competitive ELISA. Interestingly, antibodies containing KSR V_{H} showed better binding than those containing VSR V_{H}. Although contributions of V_{L} mutations looked small in ELISA, they contributed to binding affinity measured by BIAcore (Table 5). In fact, contribution of V_{L} mutation was additive to V_{H} mutation, for both antibodies (for VSR-KD: 6.4 x 4.6 = ~28; for KSR-SE: 3.9 x 2.6 = ~14). The discrepancy between BIAcore and ELISA data is likely to be due to the dissociation rate of VSR VH being too slow for binding to reach equilibrium under the binding condition employed in ELISA (1hr at 37°C).

To compare the activities based on pure affinity, Fab fragments were generated from whole antibody and their function was compared by competitive ELISA and proliferation inhibition assay (Figure 4). The IC50 value of daclizumab Fab in competitive ELISA was about 2-order higher than that in IgG, indicating significant avidity effect in binding by being bivalent. Unlike ELISA in IgG format, the order among variants was consistent with their intrinsic affinity, showing the best binding in VSR-KD (Figure 4A). Similarly, proliferation inhibition activity using Fab correlates with their intrinsic affinity (Figure 4B). Thus, in this experimental seeting, the ability of an anti-CD25 antibody to block IL2-R correlates with IL2 inhibition.

### 8.4. EXAMPLE 3: IDENTIFICATION & CHARACTERIZATION OF FURTHER VARIANTS OF DACLIZUMAB

In another study, daclizumab was subjected to comprehensive mutagenesis in its CDRs to produce a population of variants with single point mutations. The variant population was then screened to identify point mutants that resulted in increased binding affinity to CD25 based on an antibody's behavior in the population.

53 variants were identified whose behavior in the population indicated a higher binding affinity than daclizumab to CD25 including those identified by Example 2. The mutagenesis also identified variants whose behavior in the population indicated did not significantly vary from daclizumab in binding to CD25. To confirm that the behavior of the variants in the context of the population reflected their actual affinity to CD25, some of variants were further analyzed by FACS and/or competition ELISA. Additionally, some of the variants were further analyzed for activity in a Kit225 proliferation assay and/or a PBMC proliferation assay.

### 8.4.1. Materials & Methods

### 8.4.1.1. IL2 induced PHA blast proliferation inhibition assay

PBMC were isolated from human whole blood by Ficoll-Paque Plus (GE Healthcare, Uppsala, Sweden) density gradient centrifugation following the manufacturer's instructions of Leucosep (Greiner Bio-One, Germany) and resuspend at 10⁶/mL in RPMI1640 supplemented with1mM NaPyrubate (Invitrogen), 10mM HEPES (HyClone, Utah), 1x Non-essential amino acids (HyClone), 0.055mM 2-Mercaptoethanol (Invitrogen), 1x L-Glutamine (HyClone), 100 U/ml Penicillin-Streptomycin (HyClone) and 10% heat-inactivated FBS. PHA was added at 10µg/mL (Sigma) and cultured for 72 hrs at 37°C in 5% CO₂. Harvested PBMC blasts were washed 3 times with plain RPMI1640 and resuspended in completed RPMI at 10⁶/mL. To set up the assay plates, 3-fold dilutions of antibodies were prepared in completed RPMI1640 containing 2x final concentration of IL2 (1ng/mL, for final concentration to be 0.5ng/mL) and dispensed at 100µL per well in 96-well round bottom plates at duplication. Dilutions were started from 40µg/mL at final concentration to be 20µg/mL (200µL/well). 100µL each of cells were added and incubated for total of 72 hrs. 16hrs before harvesting, the plates were pulsed with 0.5 µCi/well of [³H]-thymidine. Cells were harvested with a cell harvester (Filtermate Omnifilter-96 Harvester, PerkinElmer) using the manufacturer's recommended conditions and beta particle emission from thymidine incorporation was measured using a scintillation counter (Wallac Trilux). Data were analyzed as total counts per minute of [³H]-thymidine-associated emission and persent inhibition relative to IL2 stimulation only control. Inhibition curves were fitted using nonlinear regression with the software GRAPHPAD PRISM (GraphPad, San Diego) and reported as IC₅₀ wild type/IC₅₀ mutant (fold improvement over wild type control).

### 8.4.1.2. Competition ELISA

Competition ELISA was performed as described in Section 6.2.1.

### 8.4.1.3. KIT225 Assay

The KIT225 assay was performed as described in Section 6.2.3.

### 8.4.1.4. BIAcore

Affinity measurements were carried out on BIAcore model 2000 or T100 (Biacore, GE Healthcare) at 25°C using HBS-EP+ with 0.1 mg/ml BSA as running buffer. A CM5 sensor chip was amine-coupled with polyclonal goat anti-human Fc antibody (Pierce) in all 4 flow cells at ~10,000 RU to capture daclizumab or its variants at 10mL/min (~60RU) by injecting 5uL of 1ug/mL antibodies. Binding to antigen were carried out by injecting 0.195-25 nM CD25 (R&D systems) at a flow rate of 50 µL/min. Association was monitored for 5 min followed by 15-minute dissociation phase. Surface was regenerated by two consecutive pulses of 50uL of 10mM glycine (pH 1.5) at 100mL/min. Kinetic analysis was done by simultaneously fitting the association and dissociation phases of the sensorgram using 1:1 model to extract binding constants from the BIAevaluate software.

### 8.4.1.5. FACS binding

FACS binding was performed as described in Section 6.2.4.

### 8.4.1.6. Mixed lymphocyte reactions

Mixed lymphocyte reactions (MLR) were performed using in vitro derived moDC and allogeneic CD4+ T cells from human PBMC donors. Briefly, dendritic cells were matured form human PBMC as described in section 6.5.1.4. CD4+ T cells were isolated from frozen aliquots of an allogeneic donor as described in section 6.5.1.5. Purified CD4 T cells and dendritic cells were cocultured at a 10:1 ratio in serum free AIM V media with a titrateding concentration of anti-CD25 antibodies. On day 5, cultures were pulsed with tritiated thymidine. Cultures were harvested to filtermats and tritiated thymidine incorporation was detected using a scintillation counter (Wallac Betamax 1450; the Wallac TriLux system (Uppsala, Finland)). An EC50 of inhibition was calculated. Multiple donors were tested with each variant and an average EC50 was calculated. The compiled EC50 of each variant was benchmarked against parametric data for the parent antibody to provide a fold potency value.

### 8.4.1.7. 6.4.1.7 NK cell expansion

CD56^{bright} NK cells specifically expand in the presence of rhIL2 and anti-CD25 antibodies (Martin et al., 2010, J. Immunol.185:1311-1320; Sheridan et al., 2011, Multiple Sclerosis J. 17:1441-1448). PBMC from human donors were co-cultured with 10 ng rhIL2 (Prometheus) and 2.5 µg/ml of anti-CD25 antibodies in RPMI1640 (Invitrogen) containing 10% super low Ig fetal bovine serum (HyClone), and supplemented with L-glutamine (HyClone), sodium bicarbonate (BioWhittaker), sodium pyruvate (GIBCO), non-essential amino acids (HyClone), penicillin and streptomycin (BioWhittaker), and beta-mercaptoethanol (GIBCO) for 10 days. PMBC were assayed at 4x10^6 cells per well in 24-well plates. Every two to three days 1 mL of the media was replaced with fresh IL2 and antibody-containing complete media. On day 10 the cell cultures collected, washed and were subjected to flow cytometry to enumerate the number of CD56bright NK cells present. The markers used to identify CD56^{bright} NK cells were fluorescently tagged anti-CD3, anti-CD16, and anti-CD56 (all from BD Biosciences). CD56^{bright} cells were identified as CD3 negative, CD16 low, CD56 bright. The day 10 results were compared to the percentage of CD56^{bright} cells present on the day of culture initiation (dD=0). Results were obtained from 25 donors and benchmarked to the result from cultures containing the parent anti-CD25. Only variant C54 showed statistically significant enhancement for CD56^{bright} NK cell induction *in vitro.*

### 8.4.2. RESULTS

A total of 580 (29 positions x 20 a.a.) and 520 (26 positions x 20 a.a.) single a.a. substitutions of V_{H} and V_{L}, respectively, were ranked by affinity. 33 out of 580 (5.7%) VH mutations and 20 out of 520 (3.8%) VL mutations were proven to show improved affinity with at least 1.2-fold improvement in binding either BIAcore or ELISA. Within total of 53 point mutations on CDRs, the best mutation was Y56R in VH, which displays 13.6-fold improvent in affinity based on BIAcore.

### 8.5. EXAMPLE 4: IDENTIFICATION OF DEIMMUNIZED VARIANTS OF DACLIZUMAB

### 8.5.1. Materials & Methods

### 8.5.1.1. Peptides

Peptides were synthesized using a multi-pin format by PepScan (Lelystad, the Netherlands) or Mimotopes (Adelaide, Australia). The sequences of the daclizumab light and heavy chain V regions were synthesized as 15-mer peptides overlapping by 12 amino acids (Table 9). The first peptide in the heavy chain peptide set includes three additional amino acids (VHS) known to occur at a small frequency due to incorrect signal peptide cleavage. Peptide PH2 represents the first 15 amino acids of the correctly cleaved VH protein (Table 9). Epitope region peptide variants were synthesized as 18-mers in order to encompass both identified peptides of interest. Peptides arrived lyophilized and were resuspended in DMSO (Sigma-Aldrich) at approximately 1-2 mg/ml. Stock peptides were kept frozen at -20°C.

### 8.5.1.2. Human Peripheral Blood Mononuclear Cells

Community donor buffy coat products were purchased from the Stanford Blood Center, Palo Alto, Calif. Buffy coat material was diluted 1:1 v:v with DPBS containing no calcium or magnesium. Diluted buffy coat material (25-35 mls) was underlayed in 50 ml conical centrifuge tubes (Sarsted or Costar) with 12.5 mls of FicollPaque-PLUS (GE Healthcare). The samples were centrifuged at 900 g for 30 minutes at room temperature. Peripheral blood mononuclear cells (PBMC) were collected from the interface. DPBS was added to bring the final volume to 50 mls and the cells were centrifuged at 350 g for 5 minutes. Pelleted cells were resuspended in DPBS and counted.

### 8.5.1.3. HLA analysis

DNA was isolated from frozen aliquots of human PBMC using a commercially available kit (Qiagen). PCR-based SSO typing of HLA-DRβ1 and HLA-DQβ alleles was performed as per the manufacturer's recommendations (Invitrogen: Dynal RELI SSO typing system). HLA allelotype assignment was performed by hand.

### 8.5.1.4. Dendritic Cells

For isolation of dendritic cells, T75 culture flasks (Costar) were seeded with 10⁸ freshly isolated PBMC in a total volume of 30 mls AIM V media (Invitrogen). Excess PBMC were frozen at -80°C in 90% fetal calf serum (FCS), 10% DMSO at 5X10⁷ cells/ml. T75 flasks were incubated at 37°C in 5% CO₂ for 2 hours. Nonadherent cells were removed, and the adherent monolayer was washed with DPBS. To differentiate dendritic cells from monocytes, 30 mls of AIM V media containing 800 units/ml of GM-CSF (R and D Systems) and 500 units/ml IL-4 (R and D Systems) was added. Flasks were incubated for 5 days. On day 5 IL-1α (Endogen) and TNF-α (Endogen) were added to 50 pg/ml and 0.2 ng/ml. Flasks were incubated two more days. On day 7, dendritic cells were collected by the addition of 3 mls of 100 mM EDTA containing 0.5 to 1.0 mg Mitomycin C (Sigma-Aldrich) for a final concentration of 10 mM EDTA and 16.5 to 33 µg/ml Mitomycin C. Alternatively, dendritic cells can be irradiated with 4,000 rads for fixation. Flasks were incubated an additional hour at 37°C and 5% CO₂. Dendritic cells were collected, and washed in AIM V media 2-3 times.

### 8.5.1.5. Cell Culture

On day 7, previously frozen autologous PBMC were thawed quickly in a 37°C water bath. Cells were immediately diluted into DPBS or AIM V media and centrifuged at 350 g for 5 minutes. CD4⁺ cells were enriched by negative selection using magnetic beads (Easy-Sep CD4⁺ kit, Stem Cell Technologies). Autologous CD4⁺ T cells and dendritic cells were cocultured at 2X10⁵ CD4+ T cells per 2X10⁴ dendritic cells per well in 96 well round bottomed plates (Costar 9077). Peptides were added at ~5 mg/ml. Control wells contained the DMSO (Sigma) vehicle alone at 0.25% v:v. Positive control wells contained DMSO at 0.25% and tetanus toxoid (List Biologicals or CalBioChem) at 1 mg/ml. Cultures were incubated for 5 days. On day 5, 0.25 µCi per well of tritiated thymidine (Amersham or GE Healthcare) was added. Cultures were harvested on day 6 to filtermats using a Packard Filtermate Cell harvester. Scintillation counting was performed using a Wallac MicroBeta 1450 scintillation counter (Perkin Elmer).

### 8.5.1.6. Data Analysis

Average background CPM values were calculated by averaging negative control well results from 6 to 12 replicates. The CPM values of the four positive control wells were averaged. Replicate or triplicate wells for each peptide were averaged. Stimulation index values for the positive control and the peptide wells were calculated by dividing the average experimental CPM values by the average negative control values. In order to be included in the dataset, a stimulation index of approximately 3 in the tetanus toxoid positive control wells was required. A response was noted for any peptide resulting in a stimulation index of 2.95 or greater. Peptides were tested using peripheral blood samples from a group of 115 donors. Responses to all peptides were compiled. For each peptide tested, the percentage of the donor set that responded with a stimulation index of 2.95 or greater was calculated. In addition, the average stimulation index for all donors was also calculated.

### 8.5.1.7. Generation of antibody variants

The humanized anti-Tac (HAT) light chain V region gene described by Queen *et al.* (Queen et al., 1989, Proc. Natl. Acad. Sci. USA 86:10029-10033) was subcloned as an XbaI-XbaI fragment into pVk.rg (Cole et al., 1997, J. Immunol. 159:3613-3621). The expression vector was further modified by replacing the bacterial replication origin with the high copy number bacterial replication origin from pUC18 (Yanisch-Perron et al., 1985, Gene 33:103-119).

The HAT heavy chain V region gene described by Queen *et al*., *supra*, was modified by replacing the signal peptide with that from the mouse heavy chain V region gene of EP-5C7 (He et al., 1998, J. Immunol. 160: 1029-1035). An MluI-SalI restriction fragment comprising the modified signal peptide and the N-terminal half of the HAT-VH gene was constructed and amplified following the method of He *et al.* using four overlapping synthetic oligonucleotides of approximately 75 bases in length (Table 10). The oligonucleotides were annealed pairwise and extended with the Klenow fragment of DNA polymerase I (New England Biolabs, Inc., Beverly, MA) for 15 min at room temperature, yielding two double-stranded fragments. The resulting fragments were denatured, annealed pairwise, and extended with Klenow, yielding a full-length fragment. The resulting product was amplified by the polymerase chain reaction (PCR) with outside primers E.HAT-5 (5'- TAT AAC GCG TCC ACC ATG GAC TCG - 3') (SEQ ID NO: 35) and E.HAT-6 (5'- TAT AGT CGA CGG ATT AAT ATA TCC -3') (SEQ ID NO: 36) using the Expand High Fidelity PCR System (Roche Molecular Biochemicals, Indianapolis, IN) by incubating at 94°C for 2 min, followed by 35 cycles of 94°C for 10 sec, 56°C for 10 sec and 72°C for 1 min, followed by incubating at 72°C for 10 min. The PCR-amplified fragment was gel-purified, digested with MluI and SalI, combined with a SalI-XbaI restriction fragment comprising the C-terminal half of the HAT-VH gene, and inserted into pVg1.D.Tt (Cole *et al*., *supra*). The resulting V region gene, designated E.HAT-VH, encodes the same mature heavy chain V region sequence as that described by Queen *et al*., *supra.* The modified heavy chain V region gene sequence was verified by nucleotide sequencing.

To facilitate DNA sequencing, the nucleotide sequence of the E.HAT-VH gene was modified using the overlap-extension PCR method (Higuchi, in "PCR Technology: Principles and Applications for DNA Amplification", Stockton Press, New York (1989), pp. 61-70) using the mutagenesis primers JXG1-4 (5'- GTG CAA GAG GAG GAG GAG TCT TGA C -3') (SEQ ID NO: 37) and JXG1-5 (5'- GTC AAA GAC TCC TCC TCC TCT TGC AC -3') (SEQ ID NO: 38). The first round of PCR used outside primer MBR3 (5'- CCA TAG AAG ACA CCG GGA CC -3') (SEQ ID NO: 39) and JXG1-5 for the left-hand fragment, and outside primer MD8 (5'- TCA CCT TAG CCC CCT CCC TG -3') (SEQ ID NO: 40) and JXG1-4 for the right-hand fragment. PCR was done using the Expand High Fidelity PCR System (Roche Molecular Biochemicals) by incubating at 95°C for 5 min, followed by 35 cycles of 95°C for 30 sec, 60°C for 30 sec and 72°C for 1 min, followed by incubating at 72°C for 10 min. The PCR products were gel purified, and then the second round of PCR to combine the left-hand and right-hand fragments was done as described above, using outside primers MBR3 and MD8. The PCR-amplified fragment was digested with MluI and XbaI, and then subcloned into pVg1.D.Tt (Cole *et al*., *supra*). The resulting V region gene, designated E.HAT(GGA)-VH, encodes the same mature heavy chain V region sequence as that described by Queen *et al*., *supra.* The modified heavy chain V region gene sequence was verified by nucleotide sequencing. The expression vector was further modified by replacing the bacterial replication origin with the high copy number bacterial replication origin from pUC18 (Yanisch-Perron *et al*., *supra*.).

Site-directed mutagenesis of the E.HAT-VH gene was done using the overlap-extension PCR method (Higuchi, ibid.). To generate the I48L, I48M, and I48V mutations, the mutagenesis primers DAC-48F (5'- CCC TGG ACA GGG TCT GGA ATG GNT GGG ATA TAT TAA TCC GTC GAC TGG GTA TAC TGA ATA C -3') (SEQ ID NO: 41) and DAC-N186-R (5'- CCA TTC CAG ACC CTG TCC AGG G -3') (SEQ ID NO: 42) were used, where N = A, C, G, or T. To generate the I51A, I51L, and I51V mutations, the mutagenesis primers DAC-51F (5'- CCC TGG ACA GGG TCT GGA ATG GAT TGG ATA TSY CAA TCC GTC GAC TGG GTA TAC TGA ATA C -3') (SEQ ID NO: 43) and DAC-N186-R were used, where S = C or G, and Y = C or T. To generate the T54A, T54V, and T54S mutations, the mutagenesis primers DAC-54F (5'- CCC TGG ACA GGG TCT GGA ATG GAT TGG ATA TAT TAA TCC GTC GKY CGG GTA TAC TGA ATA C -3') (SEQ ID NO: 44) and DAC-N186-R were used, where K = G or T. To generate the Y56A mutations, the mutagenesis primers DAC-56F (5'- CCC TGG ACA GGG TCT GGA ATG GAT TGG ATA TAT TAA TCC GTC GAC TGG GGC CAC TGA ATA C -3') (SEQ ID NO: 45) and DAC-N186-R were used. The first round of PCR used outside primer DAC-5END-F (5'- GTC AAC GCG TCC ACC ATG GAC TCG AG -3') (SEQ ID NO: 46) and DAC-N186-R for the left-hand fragment, and outside primer DAC-3END-R1 (5'- GTA CTC TAG AGG TTT TAA GGA CTC ACC TGA GGA GAC -3') (SEQ ID NO: 47) or DAC-3END-R2 (5'-GTA CTC TAG AGG TTT TAA GGA CTC ACC TGA -3') (SEQ ID NO: 48) and DAC-48F, DAC-51F, DAC-54F, or DAC-56F for the right-hand fragment. PCR was done using PfuTurbo DNA Polymerase (Stratagene, La Jolla, CA) by incubating at 94°C for 5 min, followed by 30 cycles of 94°C for 20 sec, 56°C for 30 sec and 72°C for 1 min, followed by incubating at 72°C for 10 min. The PCR products were gel purified, and then the second round of PCR to combine the left-hand and right-hand fragments was done as described above, using outside primers DAC-5END-F and DAC-3END-R1 or DAC-3END-R2. The full-length PCR products were gel purified, digested with MluI and XbaI, and subcloned into pVg1.D.Tt (Cole *et a*l., *ibid*.). Mutations were verified by nucleotide sequencing.

Site-directed mutagenesis of the E.HAT(GGA)-VH gene was done using the overlap-extension PCR method (Higuchi, *supra*). To generate the I48M/I51L double mutation, the mutagenesis primers DAC48MS1L (5'- CCC TGG ACA GGG TCT GGA ATG GAT GGG ATA TCT GAA TCC GTC GAC TGG GTA TAC TGA ATA C -3') (SEQ ID NO: 49) and DAC-N186-R were used. To generate the I48M/T54S double mutation, the mutagenesis primers DAC48M54S (5'- CCC TGG ACA GGG TCT GGA ATG GAT GGG ATA TAT TAA TCC GTC GTC CGG GTA TAC TGA ATA C -3') (SEQ ID NO: 50) and DAC-N186-R were used. To generate the I48V/T54S double mutation, the mutagenesis primers DAC48V54S (5'- CCC TGG ACA GGG TCT GGA ATG GGT GGG ATA TAT TAA TCC GTC GTC CGG GTA TAC TGA ATA C -3') (SEQ ID NO: 51) and DAC-N186-R were used. The first round of PCR was done as described above using outside primer DAC-5END-F and DAC-N186-R for the left-hand fragment, and outside primer DAC-3END-R1 or DAC-3END-R2 and DAC48M51L, DAC48M54S, or DAC48V54S for the right-hand fragment. The second round of PCR to combine the left-hand and right-hand fragments was done as described above, using outside primers DAC-5ENDF and DAC-3END-R2. The resulting full-length PCR products were gel purified, digested with MluI and XbaI, and subcloned into a modified form of pVg1.D.Tt (Cole *et al*., *supra*) containing the high copy number bacterial replication origin from pUC18 (Yanisch-Perron *et al*., *supra*). Mutations were verified by nucleotide sequencing.

### 8.5.1.8. Transient transfection

Human kidney cell line 293T/17 (American Type Culture Collection, Manassus, VA) was maintained in DMEM (BioWhittaker, Walkersville, MD) containing 10% Fetal Bovine Serum (FBS) (HyClone, Logan, UT), 0.1 mM MEM non-essential amino acids (Invitrogen Corporation) and 2 mM L-glutamine (Invitrogen Corporation), hereinafter referred to as 293 medium, at 37°C in a 7.5% CO₂ incubator. For expression and purification of monoclonal antibodies after transient transfection, 293T/17 cells were incubated in DMEM containing 2% Ultra-low IgG FCS (HyClone), 0.1 mM MEM non-essential amino acids and 2 mM L-glutamine, hereinafter referred to as low-IgG 293 medium.

Transient transfection of 293T/17 cells was carried out using Lipofectamine 2000 (Invitrogen Corporation) following the manufacturer's recommendations. Approximately 2 x 10⁷ cells per transfection were plated in a T-175 flask in 50 ml of 293 medium and grown overnight to confluence. The next day, 35 µg of light chain plasmid and 35 µg of heavy chain plasmid were combined with 3.75 ml of Hybridoma-SFM (HSFM) (Life Technologies, Rockville, MD). In a separate tube, 175 µl of Lipofectamine 2000 reagent and 3.75 ml of HSFM were combined and incubated for 5 min at room temperature. The 3.75 ml Lipofectamine 2000-HSFM mixture was mixed gently with the 3.75 ml DNA-HSFM mixture and incubated at room temperature for 20 min. One hour before the transfection, the medium covering the 293T/17 cells was aspirated and replaced with low-IgG 293 medium, and then the lipofectamine-DNA complexes were added dropwise to the cells, mixed gently by swirling, and the cells were incubated for 5 days at 37°C in a 7.5% CO₂ incubator before harvesting the supernatants.

### 8.5.1.9. Measurement of antibody expression by ELISA

Expression of antibodies was measured by sandwich ELISA. MaxiSorp ELISA plates (Nunc Nalge International, Rochester, NY) were coated overnight at 4°C with 100 µl/well of a 1:1000 dilution of AffiniPure goat anti-human IgG Fcγ-chain specific polyclonal antibodies (Jackson ImmunoResearch Laboratories, Inc., West Grove, PA) in 0.2 M sodium carbonate-bicarbonate buffer, pH 9.4, washed with Wash Buffer (PBS containing 0.1% Tween 20), and blocked for 1 hr at room temperature with 300 µl/well of SuperBlock Blocking Buffer in TBS (Pierce Chemical Company, Rockford, IL). After washing with Wash Buffer, supernatants were appropriately diluted in ELISA Buffer (PBS containing 1% BSA and 0.1% Tween 20) and 100 µl/well was applied to the ELISA plates. As a standard, humanized IgG1/κ antibody daclizumab (PDL BioPharma, Inc.) was used. After incubating the plates for 1 hr at room temperature, and washing with Wash Buffer, bound antibodies were detected using 100 µl/well of a 1:1000 dilution of HRP-conjugated goat anti-human kappa light chain specific polyclonal antibodies (Southern Biotechnology Associates, Inc., Birmingham, AL). After incubating for 1 hr at room temperature, and washing with Wash Buffer, color development was performed by adding 100 µl/well of ABTS Peroxidase Substrate/Peroxidase Solution B (KPL, Inc., Gaithersburg, MD). After incubating for 7 min at room temperature, color development was stopped by adding 100 µl/well of 2% oxalic acid. Absorbance was read at 415 nm using a VersaMax microplate reader (Molecular Devices Corporation, Sunnyvale, CA).

### 8.5.1.10. Purification of antibodies

Culture supernatants from transient transfections were harvested by centrifugation, and sterile filtered. The pH of the filtered supernatants was adjusted by addition of 1/50 volume of 1 M sodium citrate, pH 7.0. Supernatants were run over a 1 ml HiTrap Protein A HP column (GE Healthcare BioSciences Corporation, Piscataway, NJ) that was pre-equilibrated with 20 mM sodium citrate, 150 mM NaCl, pH 7.0. The column was washed with the same buffer, and bound antibody was eluted with 20 mM sodium citrate, pH 3.5. After neutralization by addition of 1/50 volume of 1.5 M sodium citrate, pH 6.5, the pooled antibody fractions were concentrated to ~0.5-1.0 mg/ml using a 15 ml Amicon Ultra-15 centrifugal filter device (30,000 dalton MWCO) (Millipore Corporation, Bedford, MA). Samples were then filter sterilized using a 0.2 µm Acrodisc syringe filter with HT Tuffryn membrane (Pall Corporation, East Hills, NY). The concentrations of the purified antibodies were determined by UV spectroscopy by measuring the absorbance at 280 nm (1 mg/ml = 1.4 A₂₈₀).

Five µg samples of purified antibodies were run under reducing or non-reducing conditions on NuPAGE Novex 4-12% Bis-Tris gels (Invitrogen Corporation) and stained using the SimplyBlue SafeStain Kit (Invitrogen Corporation) following the manufacturer's recommendations.

Fifty µg samples of purified antibodies were analyzed by gel filtration chromatography on a Beckman-Coulter HPLC (Beckman Coulter, Inc., Fullerton, CA) using a 7.8 mm x 30 cm Toso-Haas TSK G3000 SW_{XL} column (TosoHaas, Montgomeryville, PA) in PBS at 1.5 ml/min.

### 8.5.1.11. Antigen binding ELISA

MaxiSorp ELISA plates (Nunc Nalge International) were coated overnight at 4°C with 100 µl/well of a 50 ng/ml solution of recombinant human IL2-Rα (R&D Systems or PeproTech, Inc., Rocky Hill, NJ) in DPBS (Invitrogen Corporation), washed with Wash Buffer (PBS containing 0.1% Tween 20), and blocked for 30 min at room temperature with 200 µl/well of SuperBlock Blocking Buffer in TBS (Pierce Chemical Company). After washing with Wash Buffer, test antibodies (starting at 4 µg/ml and serially diluted 3-fold) in 100 µl/well of ELISA buffer (PBS containing 1% BSA and 0.1% Tween 20) were added in duplicate. After incubating the plates for 1 hr at room temperature, and washing with Wash Buffer, bound antibodies were detected using 100 µl/well of a 1:20,000 dilution of HRP-conjugated goat anti-human IgG (Southern Biotechnology Associates, Inc.) in ELISA buffer. After incubating for 1 hr at room temperature, and washing with Wash Buffer, color development was performed by adding 150 µl/well of TMB Peroxidase Substrate/Peroxidase Solution B (KPL, Inc.). After incubating for 8 min at room temperature, color development was stopped by adding 50 µl/well of 2 N sulfuric acid. Absorbance was read at 450 nm.

### 8.5.1.12. BIAcore analysis of antibody variants

Kinetics measurements for daclizumab wildtype (HYP and E.HAT) and mutant antibodies against human CD25 (R&D Systems) and cynomolgous CD25-HA (PDL BioPharma, Inc.) were performed using BIAcore 2000 and 3000 instruments (BIAcore International AB, Uppsala, Sweden). The daclizumab antibodies were captured with a goat anti-human Fcγ (GAHFc) reagent (Jackson ImmunoResearch Laboratories, Inc).

Prior to the kinetics experiment, capture volumes and concentrations of daclizumab were determined for this experimental series. A theoretical Rmax of 80 Resonance Units (RU) was selected for this kinetics study based on the formula for desired capture signal, R_{L} = Rmax / stoichiometry * MW_{Ligand}/MW_{Analyt}, where stoichiometry = 2, MW_{Ligand} = 150 kDa, MW_{Analyte} = 22 kDa. The predicted MW of 22 kDa for human CD25 was used in this study, which is consistent with what was used for previous studies by BIAcore. This value is close to the mass spectrometry determination of 27 kDa. The MW of cynomolgous CD25 was determined from Western blot experiments to be 32 kDa. Capture volume was determined by loading 50 µl of each antibody into the injection loop and injecting 5 µl increments of Dac at 1.5 µg/ml at a slow flow rate of 5 µl/min to determine the volume necessary to achieve the desired R_{L}. A final concentration of 0.76-0.96 µg/ml and a flow rate of 5 µl/min with an injection volume of 5 µl were used for the daclizumab antibodies studied.

For kinetics measurements, GAHFc was directly immobilized onto the sensor chip surface to capture daclizumab antibodies on individual flow cells, followed by injecting human or cynomolgous CD25 to observe their interaction with daclizumab in the buffer flow. For this capture approach, 30 µg/ml of GAHFc was immobilized to achieve a high response unit (20,000 RU) on each flow cell on the Research-grade CM5 sensor chip using the BIAcore amine coupling reagents (N-ethyl-N'-dimethylamino-propylcarbodiimide, EDC; N-hydroxysuccinimide, NHS; and ethanolamine HCl, pH 8.5). Daclizumab antibodies were captured using the specifications mentioned above. Binding assays to study the binding of daclizumab and CD25 were run at a flow rate of 30 µl/min at room temperature (25°C controlled internal temperature). A 3 min association phase of CD25 was followed by a 15 min injection of HBS-P running buffer (10 mM HEPES, 150 mM sodium chloride, 0.005% P-20 surfactant, pH 7.4) to monitor dissociation for each binding cycle, with a different CD25 concentration per cycle. The surface was regenerated with 20 mM HCl at 100 µl/min flow rate at the end of each cycle. The binding kinetics of each CD25 and daclizumab antibody pair was calculated from a global analysis of sensorgram data collected from eight different concentrations of CD25 (128, 64, 32, 16, 8, 4, 2, and 1 nM), using the BIAevaluate program. Double referencing was applied in each analysis to eliminate background responses from reference surface and buffer only control (0 nM). The affinity (K_{D}) resulting from association (kₐ) and dissociation (k_{d}) of each analyte (human or cynomolgous CD25) against each daclizumab antibody was obtained by simultaneously fitting the association and dissociation phases of the sensorgram from the analyte concentration series using the 1:1 Langmuir model from the BIAevaluate software. Each set of experiments was performed three times to assess the standard deviation of the data.

### 8.5.1.13. Preparation of Fab fragments

The parent antibody, E.HAT, and the four variant proteins were transiently expressed in 293T/17 cells. 293T/17 cells were transfected with antibody constructs using Lipofectamine (Invitrogen) according to the manufacturer's directions. Supernatants were harvested on day 7, and antibody was purified by protein A column affinity. Purified antibody was treated with immobilized papain (Pierce) according to the manufacturer's directions. Proteolysis was assessed by HPLC until completion, at which time the digested protein was separated by protein A column affinity to remove Fc fragments. Purity of the Fab preparations was assessed by SDS-PAGE electrophoresis, followed by anti-human Fc (gamma chain specific; Jackson Immunoresearch) western blotting. Prior to use, Fab preparations were heat-inactivated at 95°C for 15 minutes. This was necessary due to the significant anti-proliferation activity of the Fab proteins.

### 8.5.1.14. Fab Protein Proliferation Assay

Human PBMC in cell culture medium at 2 x 10⁵ per well were dispensed into flat-bottomed 96 well plates. Endotoxin-free heat-inactivated Fab proteins were added and the cultures were incubated at 37°C for 5 days. On day 5, 0.25 uCi of tritiated thymidine (GE Healthcare) was added to each well. Cultures were harvested 20-24 hours later using a Packard Cell Harvester. Scintillation counting was performed using the Wallac TriLux system (Uppsala, Finland). Data for each donor was converted to stimulation indices, and compiled.

### 8.5.2. Results

### 8.5.2.1. Identification of CD4⁺ T Cell Epitopes in the Daclizumab VH Regions

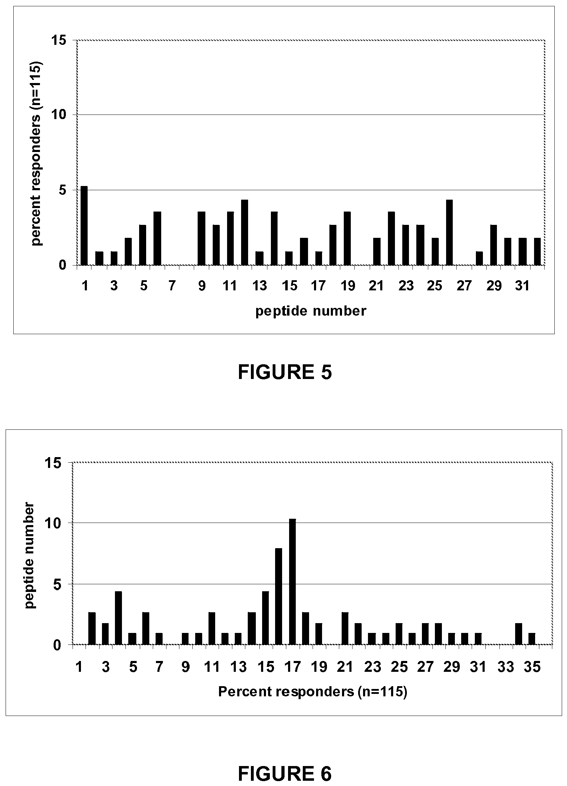

CD4⁺ T cell epitope peptides were identified by an analysis of the percent responses. The average percent response and standard deviation were calculated for all peptides tested describing the daclizumab heavy chain and light chain. A response rate greater than or equal to the average background response plus three standard deviations was considered a potential CD4⁺ T cell epitope. For the daclizumab light chain V region, 32 peptides were tested (Table 9) which resulted in an average background percent response of 2.12 ± 1.39% (Figure 5). Three standard deviations above background was determined to be 6.3%. No peptides displayed this level of response in the daclizumab light chain peptide dataset. For the daclizumab heavy chain V region, 36 peptides were tested (Table 9, right column and Figure 6). The average background percent response was 1.83 ± 2.12%. Three standard deviations above background was 8.18%. One peptide within the daclizumab heavy chain dataset, PH17, achieved a percent response of 10.3%. The peptide immediately adjacent to this peptide, PH16, reached a percent response of 7.8%. The average stimulation index was calculated for all peptides in the dataset. Heavy chain peptide PH17 had an average stimulation index value of 1.66 ± 0.18 s.e.m. Heavy chain peptide PH16 had an average stimulation index of 1.55 ± 0.11 s.e.m. Both of these values are significantly higher than the average stimulation index for all peptides in the two datasets (1.02 ± 0.02 for all 68 heavy chain and light chain peptides). Since the adjacent peptide (PH16) shares 12 amino acids with the epitope peptide (PH17), both peptides were selected for further study.

The HLA class II types were determined for all donors in the dataset. The HLA class II types of the responders to peptides PH16 and PH17 were examined for the presence of any relative enrichment. A proliferative response to peptide PH16 was found to associate with the presence of HLA-DQ6 (p < 0.04). There were no apparent associations of HLA types with a response to peptide PH17.

### 8.5.2.2. Identification of Reduced Immunogenicity Variants

The epitope peptide region (heavy chain peptides PH16 and PH17, see Table 9) is located at the framework 2/CDR2 junction. Amino acid sequence variants were selected with attention to residues known to contribute to CD25 specificity. Any CDR2 residue known to affect daclizumab affinity when substituted with an alanine residue was not considered for modification. Three residues, I51, T54 and Y56 (Kabat numbering) were selected for modification. In addition, the isoleucine at position 48 within the framework 2 region was selected for modification as it was a substitution in the framework region that had been back-mutated during the original humanization of the molecule. At position I51, leucine, valine and alanine were substituted. At position T54, alanine, valine and serine were substituted. For Y56, only an alanine substitution was tested. At position I48, valine, leucine and alanine were substituted. These modifications resulted in a total of 10 single-amino acid variants. Combinations of the selected modifications were also considered. A peptide set encompassing all possible single and double mutations was retested for functional activity in the CD4⁺ T cell assay (Table 11). Four peptide sequence variants were significantly reduced in their capacity to induce proliferative responses in a set of 78 community donor cell-derived assays as compared to the responses induced by the unmodified peptide sequence (boxed sequences). The parent peptide PH17 ("P" in Table 11) was tested twice in the 78-donor variant peptide dataset. The percent response to the parent peptide was 23.1 % and 19.2%, with stimulation indexes of 2.25 ± 0.21 and 2.03 ± 0.21. The stimulation index values are not different by a two-tailed paired T-test analysis. The four modifications that significantly reduced both proliferative and percent responses were I48M (Table 12), I48M I51L, I48M T54S and I48V T54S (Table 13 and Table 14). The most preferred variant was I48M I51L as it induced the lowest percent response of any variant tested, and no "non-responder" donors, that is, donors that do not mount a proliferative response of 2.95 or greater to the unmodified parent peptide, responded to the modified peptide.

### 8.5.2.3. CD25 binding activity of variant antibody molecules

The sequence modifications selected by functional testing were incorporated into the daclizumab heavy chain V region sequence. Variant antibody proteins and the unmodified daclizumab protein (E.HAT) were purified from supernatants of transiently transfected 293T/17 cells. To create comparable batches of the antibodies, 293T/17 cells were transfected and antibodies were purified from supernatants in parallel. Expression levels of approximately 30-50 µg/ml were typically observed. Purified antibodies were characterized by SDS polyacrylamide gel electrophoresis (SDS-PAGE) under non-reducing and reducing conditions. SDS-PAGE analysis under non-reducing conditions indicated that the purified antibodies had a molecular weight of about 150-160 kDa, while analysis under reducing conditions indicated that the purified antibodies were comprised of a heavy chain with a molecular weight of about 50 kDa and a light chain with a molecular weight of about 25 kDa. Gel filtration chromatography indicated that the purified antibodies were >97% monomeric IgG.

E.HAT protein, the E.HAT variants and a positive control batch of daclizumab High-Yield Process (HYP) (PDL BioPharma, Inc.) were tested for their binding potency in direct-binding recombinant human CD25 ELISA assays. Data shown are representative of similar analyses performed using different batches of the antibodies. The EC50 values for the antibodies were calculated in three separate experiments, and were benchmarked to the daclizumab HYP material. The potency values from the three experiments were averaged and are shown in Table 15 and Table 16. The values ranged from a low of 94% for the I48M variant (Table 16) to the high value of 136% for the same I48M variant in a second round of tests using a separate batch of purified antibody (Table 15). All values with the exception of a test of the I48M material fall within the 70-130% of daclizumab HYP material specification, indicating equivalent potency in this assay format.

### 8.5.2.4. Affinity testing of the variant antibody molecules

Modified antibody proteins were tested for binding affinity using a surface plasmon resonance assay format in a BIAcore device. Daclizumab HYP, E.HAT antibody and the E.HAT variant antibodies were immobilized on the sensor chip using an anti-human heavy chain antibody. Recombinant soluble human CD25 was flowed over the sensor chip and changes in mass were detected. The data was interpreted to yield kₐ, k_{d} and K_{D} values for all the proteins tested. The binding affinities were benchmarked to the daclizumab HYP binding affinity. Table 17 shows the relative affinity values for all 10 single amino acid mutant proteins. Affinity was measured in three separate experiments, benchmarked to the values for daclizumab HYP, and averaged. The relative K_{D} values for the antibodies range from 80% for variant I51A to 250% for variant Y56A. Affinity testing for the double mutant proteins was performed separately using the same protocol. Binding affinities were benchmarked to the values for daclizumab HYP. As shown in Table 18, the affinities of the double mutant proteins are similar to the unmodified parent antibodies. As a final test for the conservation of antigen-specificity and for practical development purposes, the E.HAT variant antibodies were tested for binding to cynomolgous monkey CD25. Binding affinity was tested using the BIAcore and was benchmarked to daclizumab HYP. As shown in Table 19, all of the variant antibodies had affinities for cynomolgous monkey CD25 that were similar to the unmodified parent antibodies.

### 8.5.2.5. Verification of reduced immunogenicity: Fab proliferation testing

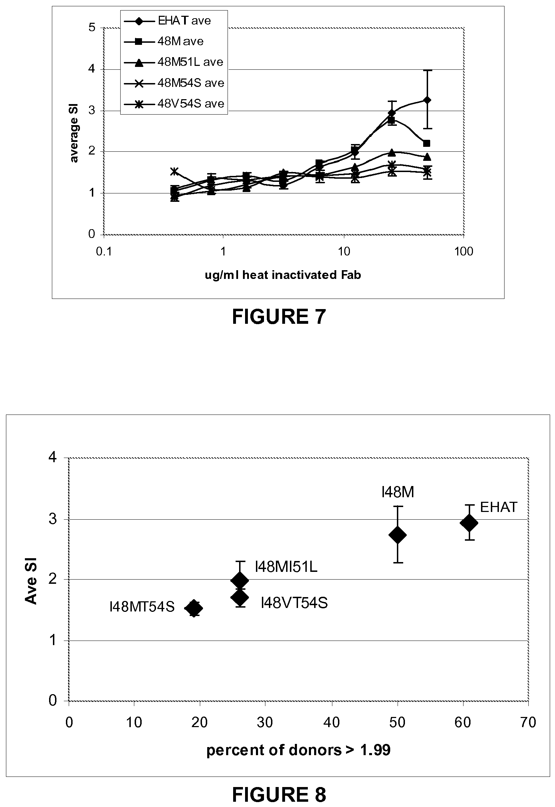

A total of thirty-one donors were tested parametrically with E.HAT, 48M, 48M54S, 48M51L, and 48T54S Fab fragments. The data was compiled and averaged for all donors (Figure 7). Not all donors were tested with all Fabs; the response to the 48M Fab was not different from the response rate to the parent E.HAT Fab, and therefore it was not tested after 16 donors were compiled. Additionally, not all donors were tested over the full range of concentrations due to limiting amounts of the proteins.

The average proliferative responses to 48M54S, 48M51L and 48V54S were comparable, and were lower than the average proliferative responses to the E.HAT and 48M Fab fragments. At 25 µg/ml the proliferative response to 48V54S and 48M54S were significantly lower than the response to E.HAT (two-tailed non-parametric t-test p < 0.01). The proliferative response to 48M51L was p = 0.06.

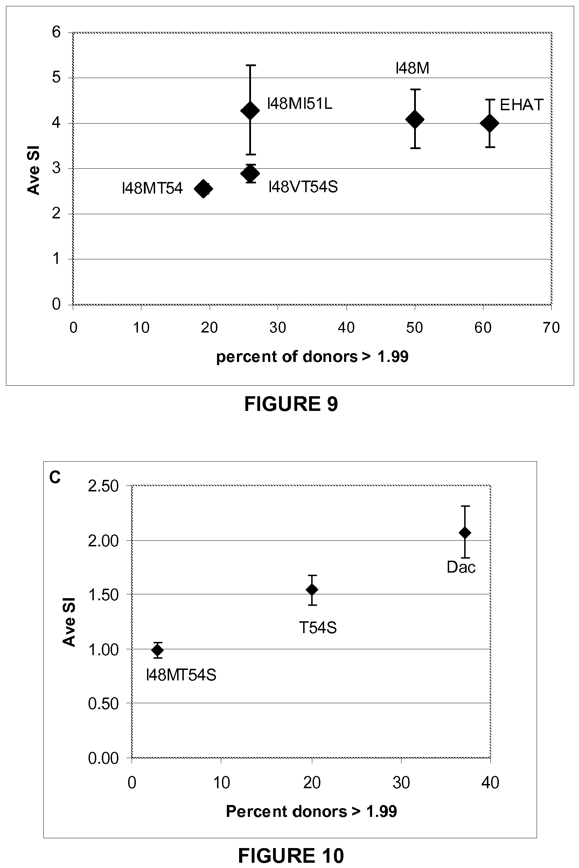

The data were re-analyzed to account for response rates among the tested donors. At the 25 µg/ml dose, any proliferative response greater than an SI = 1.99 was compiled as a positive response. Figure 8 displays the response rate on the x-axis with the corresponding average stimulation index for each Fab protein on the y-axis. This analysis reveals that fewer donor samples mounted proliferative responses to the doubly modified Fab proteins and that the overall response rates were lower. Finally, the magnitude of the average proliferative responses of the responders to each of the Fab proteins was analyzed and is shown in Figure 9. This data excludes stimulation indices from all donors whose proliferative responses were less than 1.99. The response to the E.HAT and 48M Fabs are 4.0 and 4.09, respectively. The double mutants induced fewer responses and for the 48M54S and 48V54S variants the average proliferative responses were lower. The average proliferative response to 48M51L is higher than the control, but this is due to an individual donor with a very high SI (SI = 10). The proliferative response to 48M54S was significantly different from E.HAT (p < 0.02 in 2 tailed unequal variance T-test) while the proliferative response to 48V54S was not significantly different (p = 0.06). In conclusion, the mutant proteins 48M54S and 48V54S induced fewer, weaker proliferative responses than the parent protein, E.HAT.

Finally, the T54S variant was tested as a single point mutation and the data is shown in Figure 10. This result shows that the combination of I48M and T54S results in the lowest overall *in vitro* immunogenic response.

### 8.6. EXAMPLE 5: IDENTIFICATION OF FC VARIANTS WITH REDUCED EFFECTOR FUNCTION

### 8.6.1. Overview

The fragment crystallizable ("Fc") region of an antibody is composed of two identical protein fragments, derived from the second and third constant domains of the antibody's two heavy chains. Fc regions bind to receptors on immune cells known as Fc receptors ("FcRs"), leading to both activating and inhibitory signals. For example, the FcγRIIIA (also known as CD16 or CD16a) is found on natural killer cells and macrophages, and has a low affinity for Fc regions. Binding of Fc ligand to an FcγRIIIA receptor can result in induction of antibody-dependent cell-mediated cytotoxicity (ADCC) and induction of cytokine release by macrophages. In contrast, the FcγRIIB receptor (also known as CD32b) is found on macrophages, neutrophils, B cells and eosinophils, and binding of Fc ligand to an FcγRIIB receptor inhibits cell activity.

By altering the Fc regions of antibodies, improvements can be made to increase antibody therapeutic efficacy, increase antibody half-life, and to reduce unwanted side effects. Hu1D10, a monoclonal antibody specific for the beta-chain of HLA-DR (Shi et al., 2002, Leuk Lymphoma. 43(6):1303-12) was used as a model system to generate Fc variants with reduced Fc effector function.

### 8.6.2. Binding of variants to FcγR-expressing cells

Hu1D10 IgG variant antibodies were expressed in soluble form, purified, and then used to assess binding to CHO cells expressing FcγRIIB. IgG variants were serially-diluted 3-fold starting at 20 µg/mL, or 133 nM, then added to 2x10⁵ cells/test. Anti-human kappa antibody was used to detect variant IgG binding. Samples were analyzed in a FACSCalibur and fluorescence was plotted against IgG concentration.

Fc domains are composed of two main domains, the CH2 domain and the CH3 domain, and have a small hinge region N-terminal to the CH2 domain. Variants with improved binding to FcγRIIB were identified having substitutions at position 263, position 266, position 273, or position 305 within the CH2 domain, wherein the numbering of the residues in the Fc domain is that of the EU index as in Kabat. These amino acid positions are are indicated by asterisk (*), dagger (†), double dagger (‡), and the number sign (#), respectively, in the Fc amino acid sequence (SEQ ID NO:17) in Figure 11.

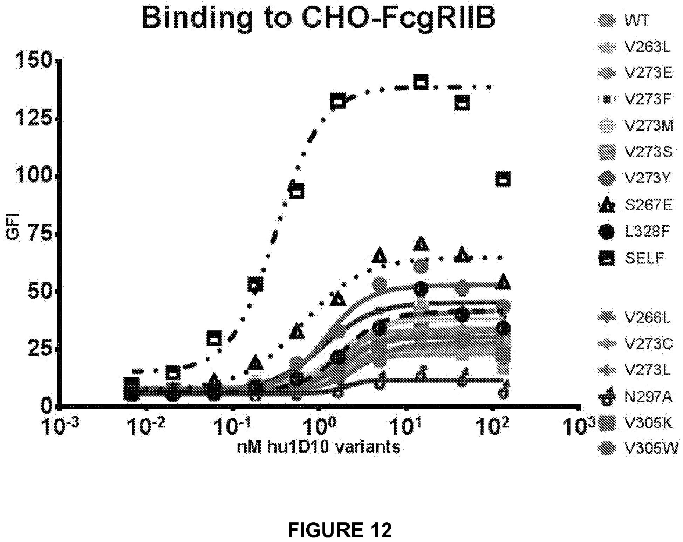

Figure 12 confirms that all the variants have a higher maximal binding to FcγRIIB than the wild-type antibody. V273F and V273Y had the best improvement of EC50 at 1.70- and 1.60-fold over wild-type, respectively.

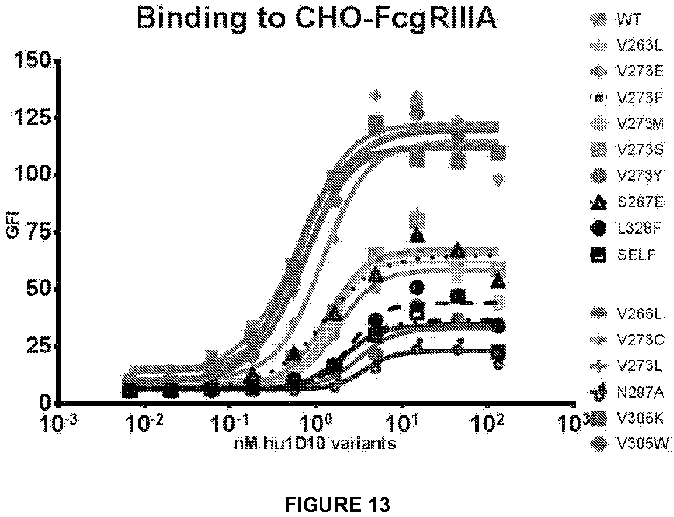

Hu1D10 IgG variants were purified and used to assess binding to FcγRIIIA CHO transfectants. IgG variants were serially-diluted 3-fold starting at 20ug/mL, or 133nM and then added to 2x10⁵ cells/test. A secondary stain of anti-human kappa antibody was used to detect variant IgG binding. Samples were analyzed in a FACSCalibur and fluorescence was plotted against IgG concentration in Figure 13. All variants bound equivalently or less well than wild-type Fc-containing antibody to FcγRIIIA. V273F and V273Y were the lowest binders at 0.30- and 0.19-fold over wild-type's EC50, respectively.

### 8.6.3. FACS-based antibody-dependent cell-mediated cytotoxicity

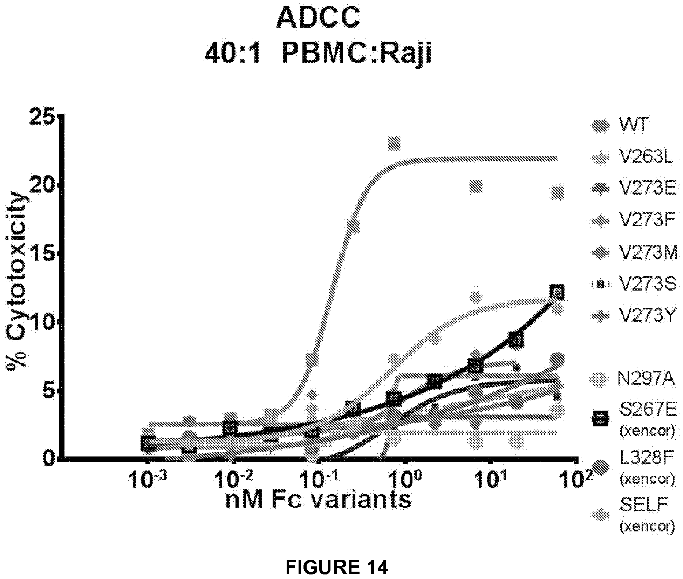

A non-radioactive antibody dependent cell cytotoxicity (ADCC) assay was optimized and used to test Hu1D10 IgG variants (Figure 14). Raji cells, and PBMC purified from freshly-drawn whole blood were used as target and effector cells, respectively, at a 1:40 ratio.

The Raji cells were washed and resuspended at 10⁶ cells/mL in PBS, then incubated with a 1:2000 dilution of CSFE (Cell Technology, Inc., part 4002) for 30 minutes. CFSE-loaded Raji cells were then washed and resuspended to 4x10⁵/mL in growth medium consisting of RPMI + 10% heat-inactivated FBS. 50 µL of cell suspension was added to each well of a V-bottom plate. 50 µL of three-fold serially diluted IgG variants was added to each well, starting at 18 µg/mL.

PBMCs were purified from freshly-drawn heparinized blood according to standard method using Ficoll-Paque. PBMCs were resuspended to 8x10⁶ cells/mL in growth media. 100 µL of cell suspension was added to each well of the target/IgG suspension and incubated at 37C for four hours. Cell suspensions were stained with 1:5 dilution of 7AAD (BD Biosciences, catalog number 559925) and incubated for 30 minutes. Samples were analyzed in a FACSCalibur.

Cytotoxicity was calculated as: (#dead cells / #all cells)*100. The percent cytotoxicity was graphed against IgG concentration to determine the EC50. Figure 13 shows the hu1D10 variants that did not elicit ADCC and compares them to substitutions that result in decreased binding to FcγRIIIA (S267E, L328F, double mutant "SELF") according to literature. V263L, V273E, V273F, V273M, V273S, and V273Y elicited comparable responses to L328F and lower ADCC responses than S267E and SELF.

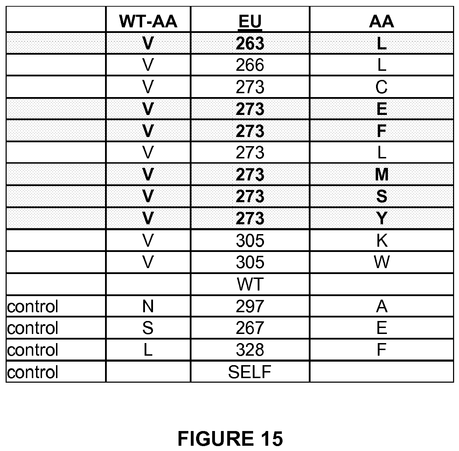

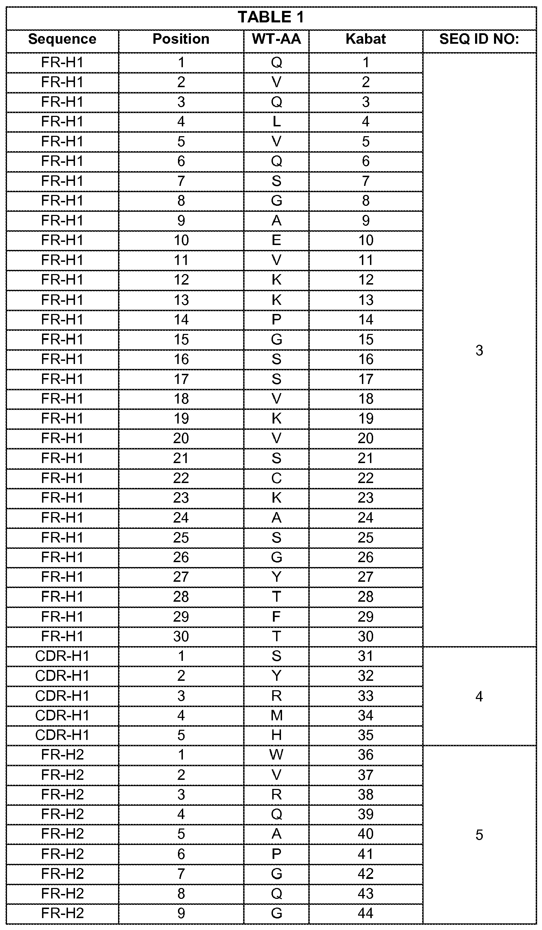

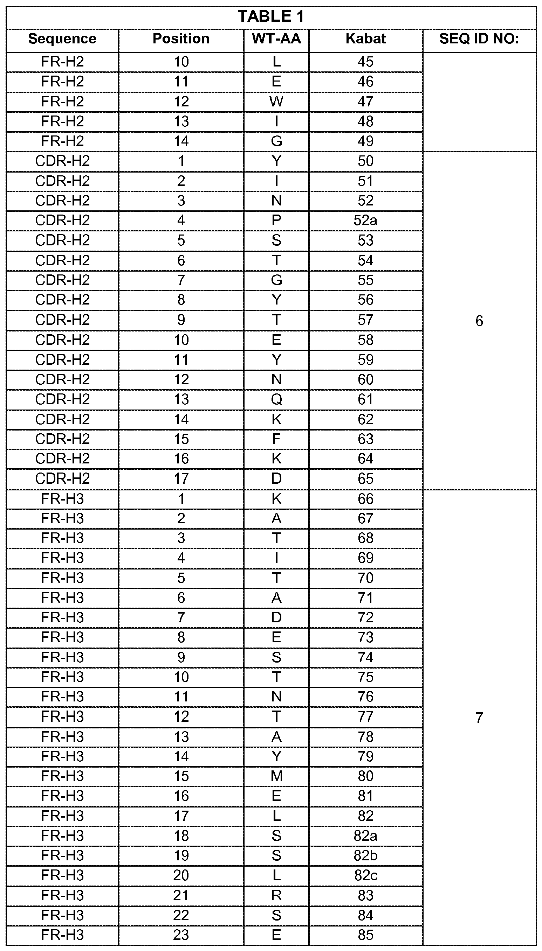

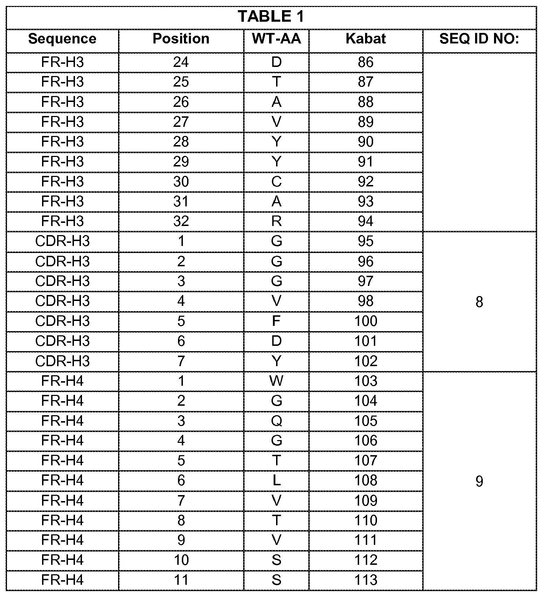

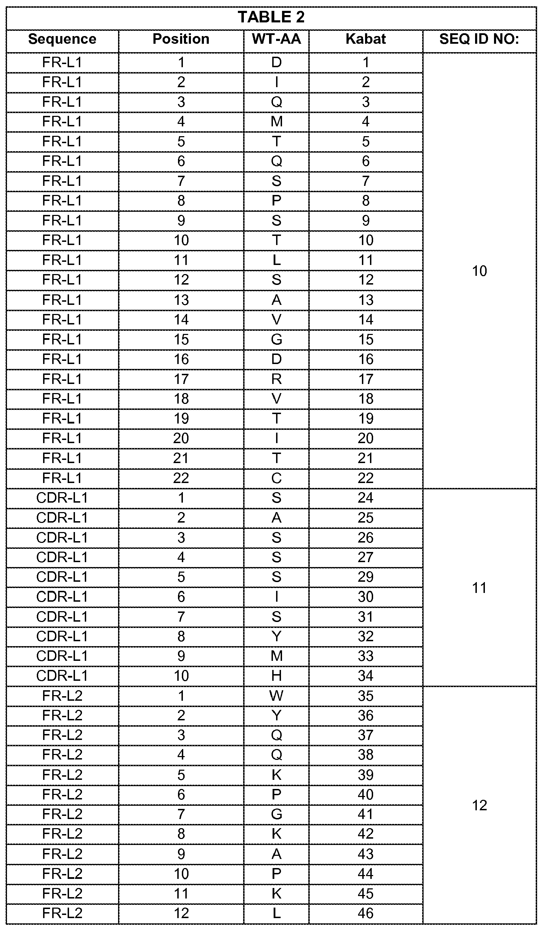

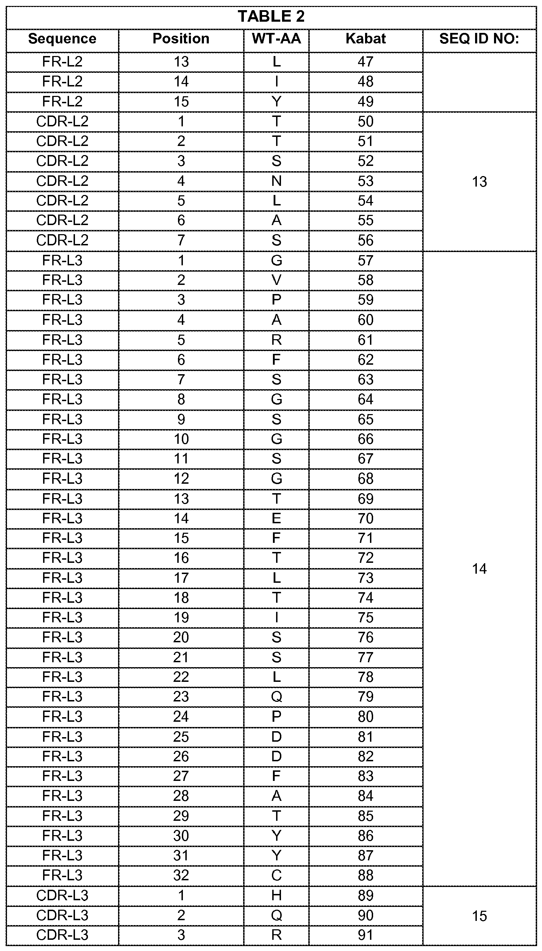

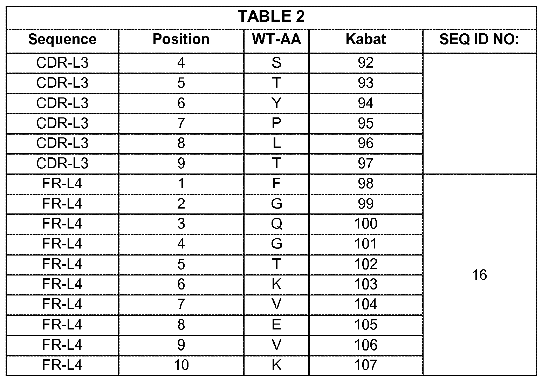

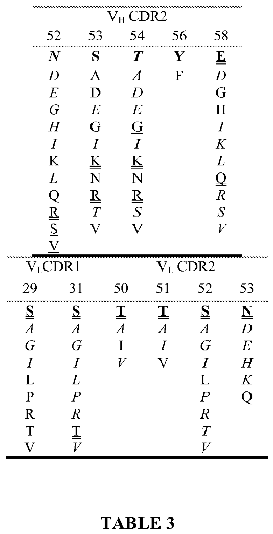

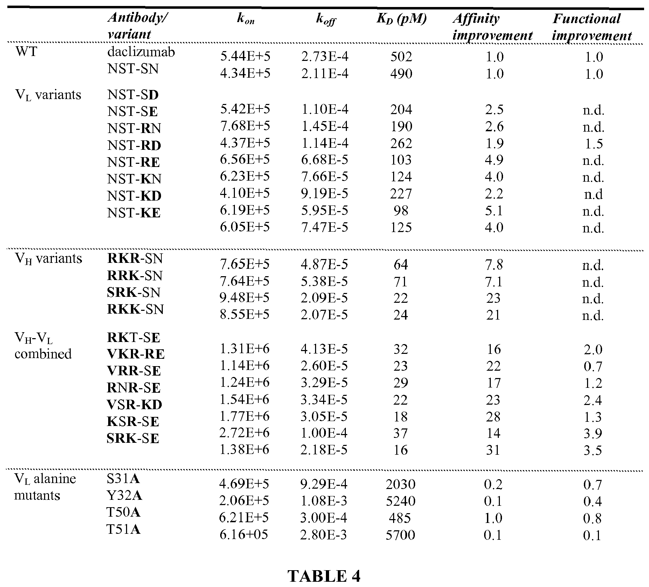

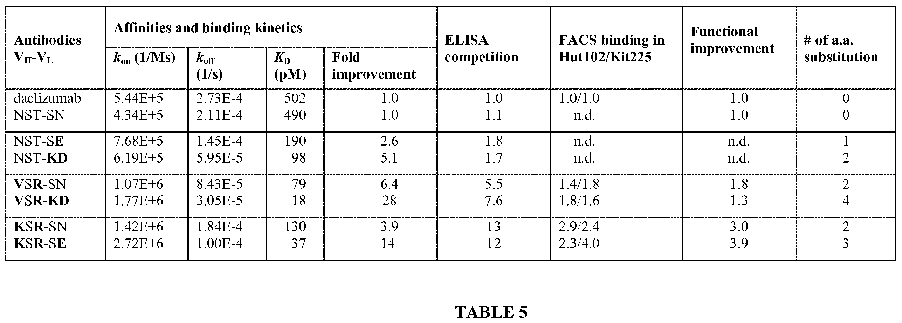

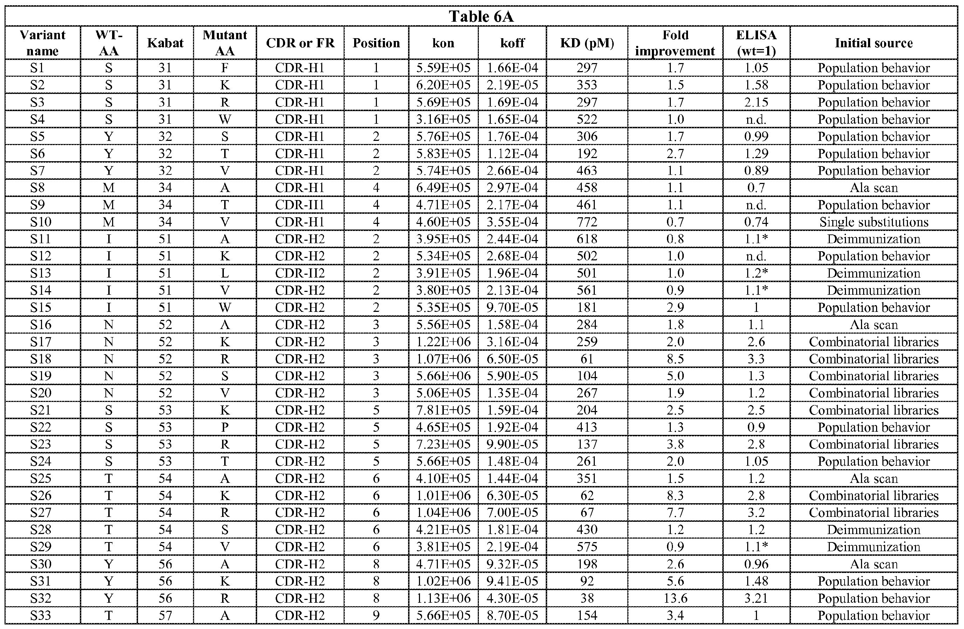

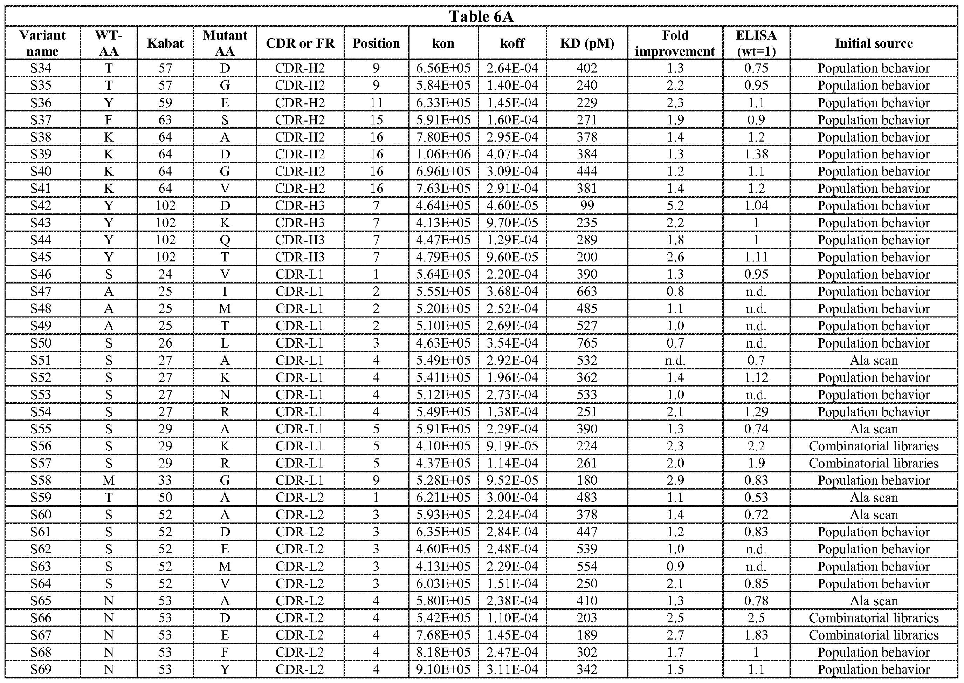

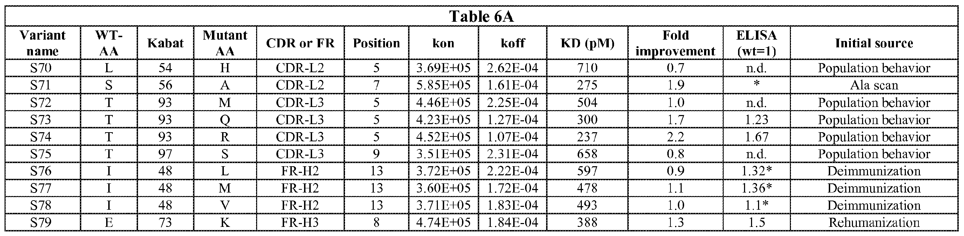

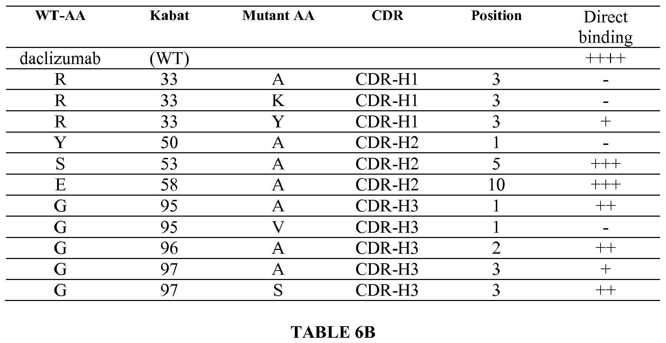

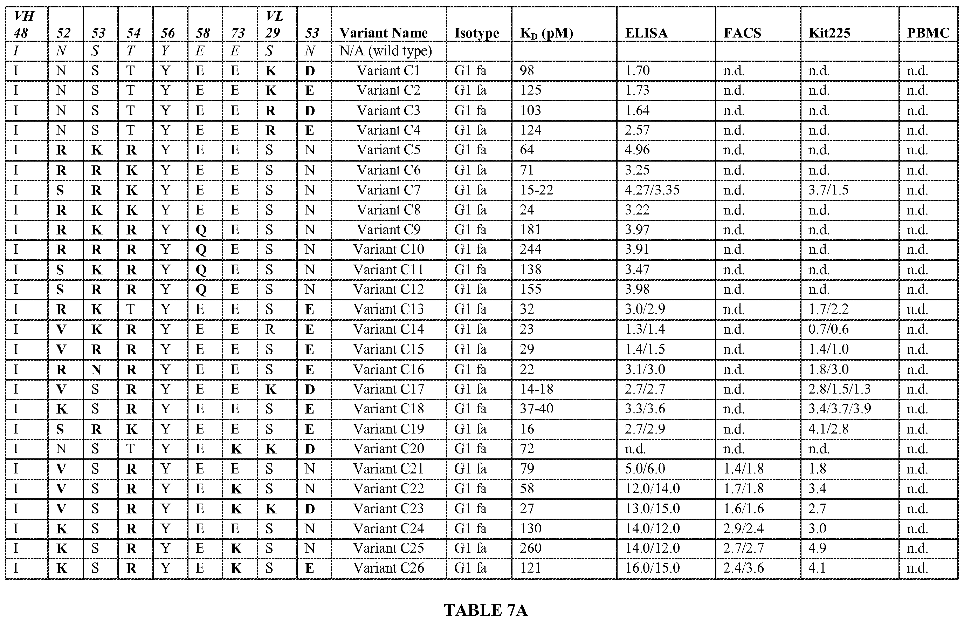

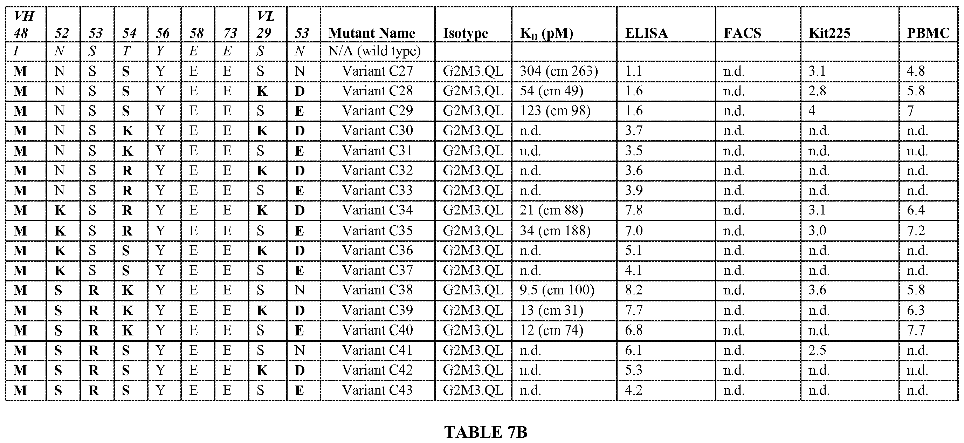

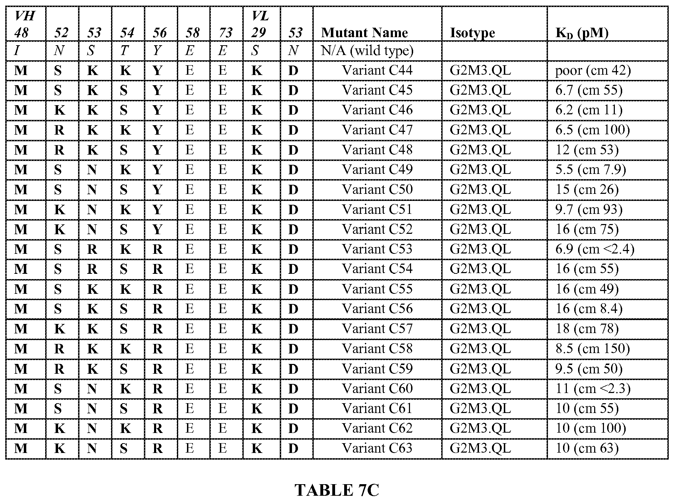

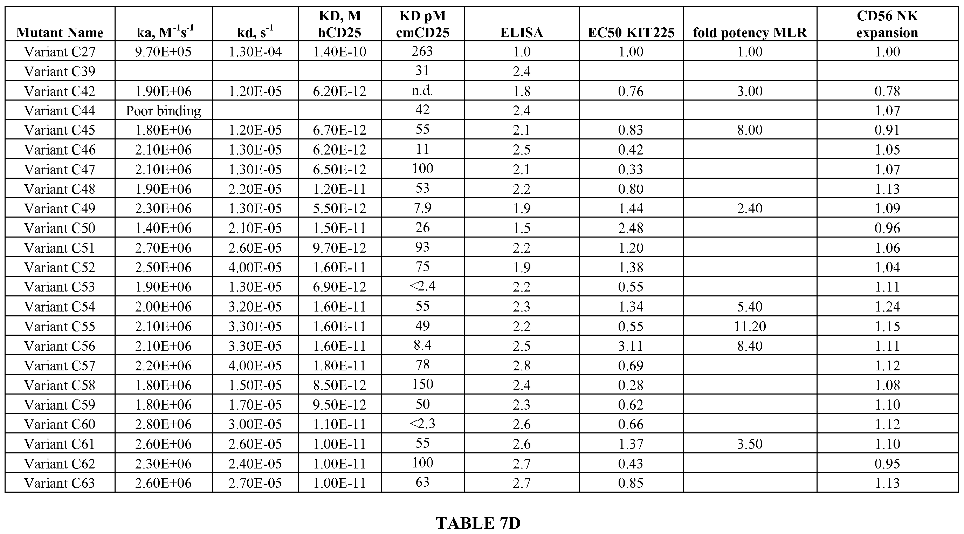

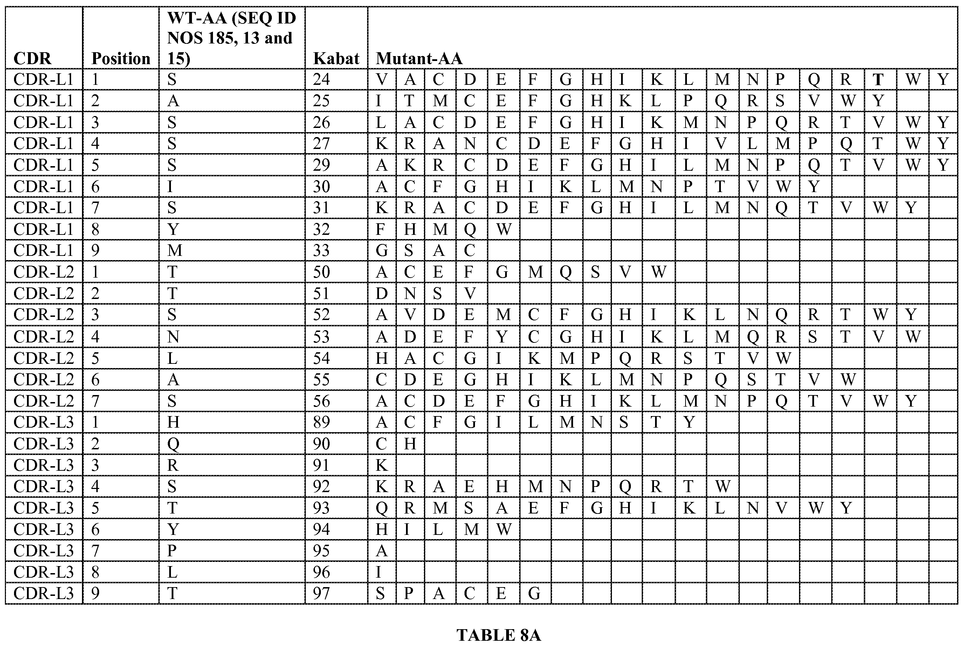

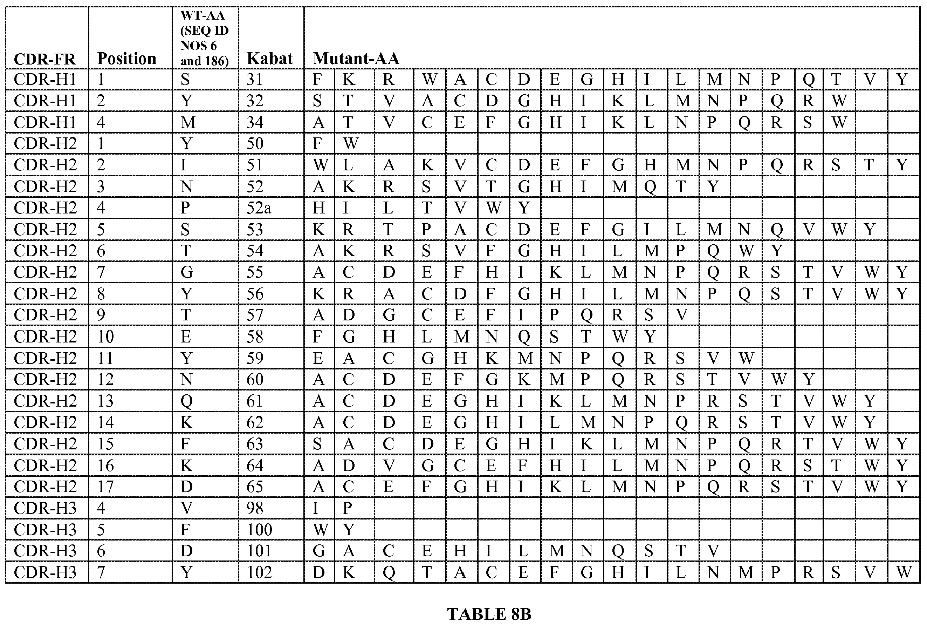

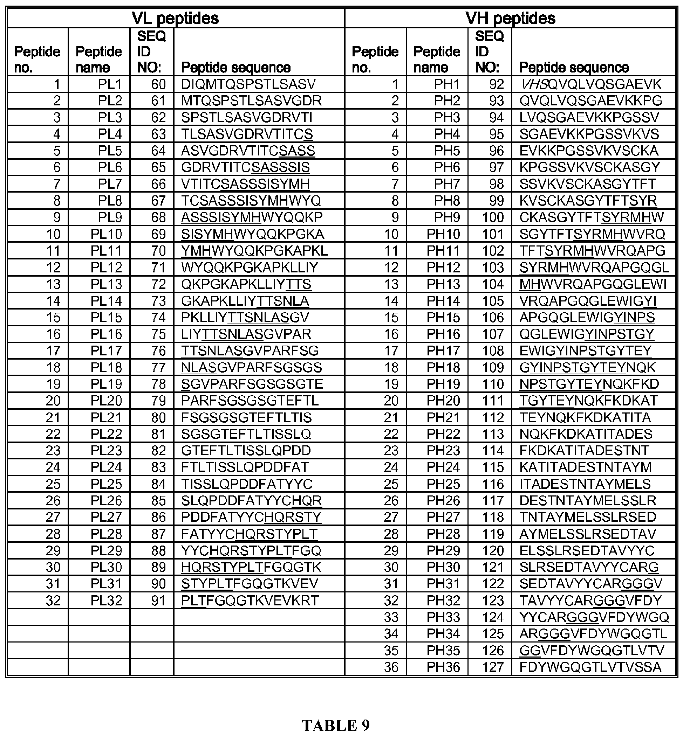

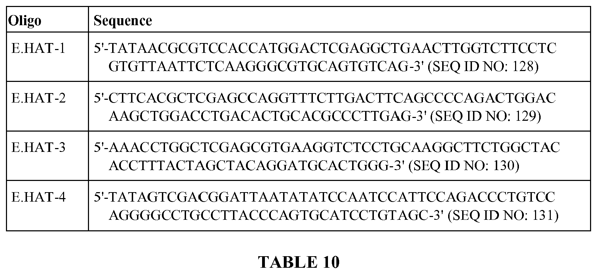

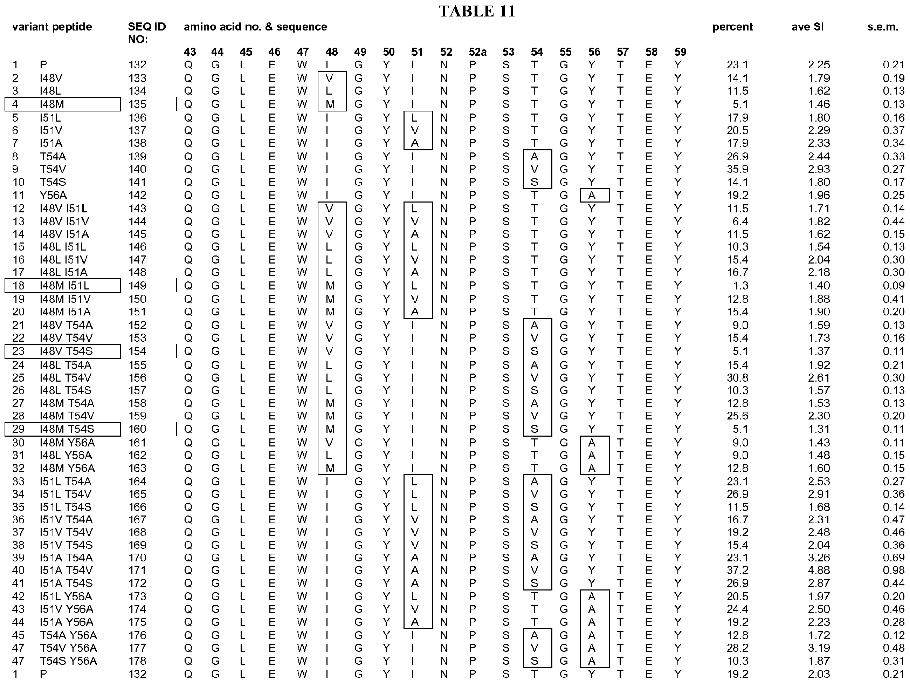

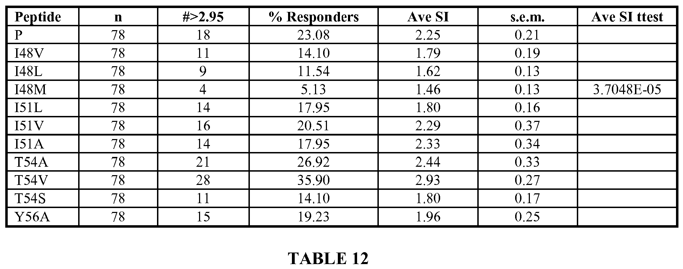

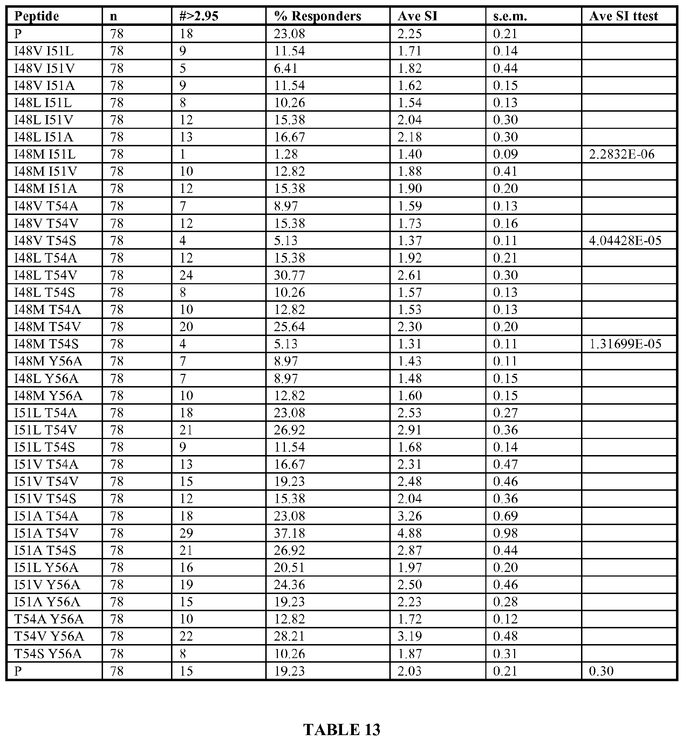

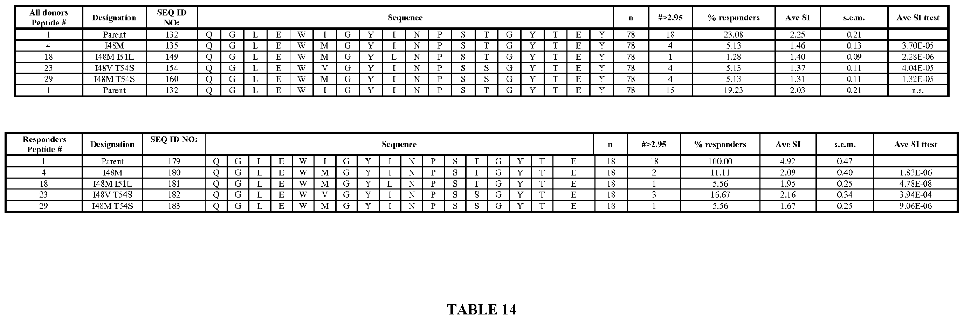

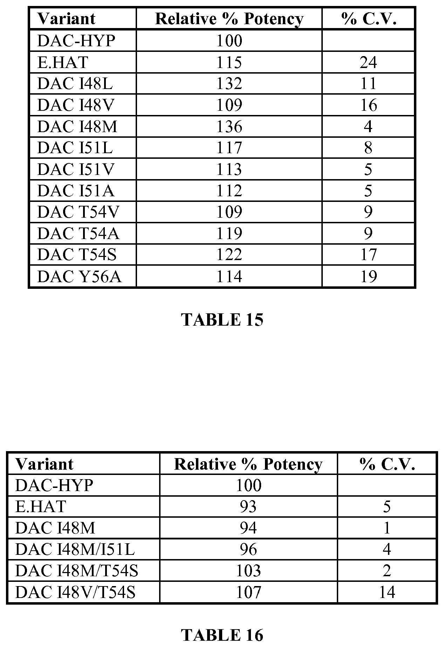

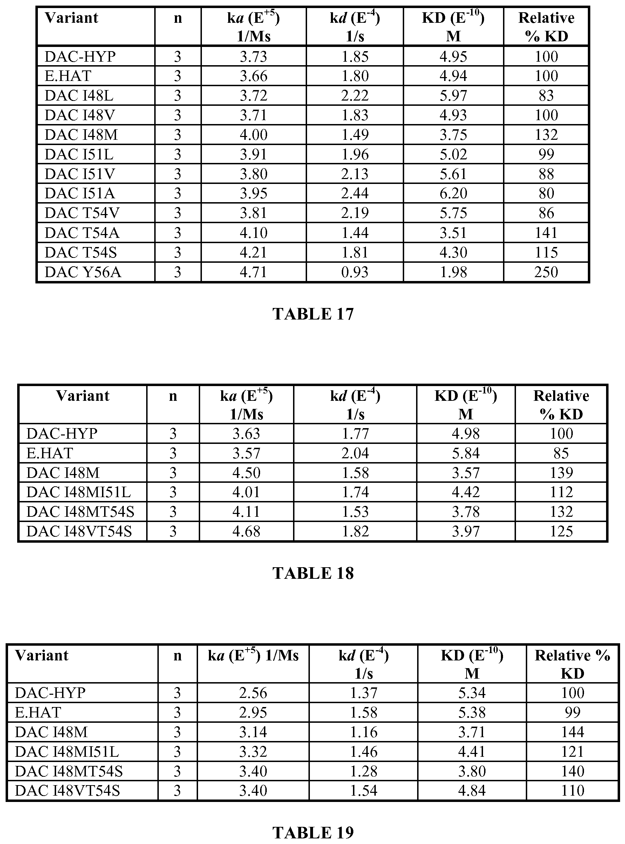

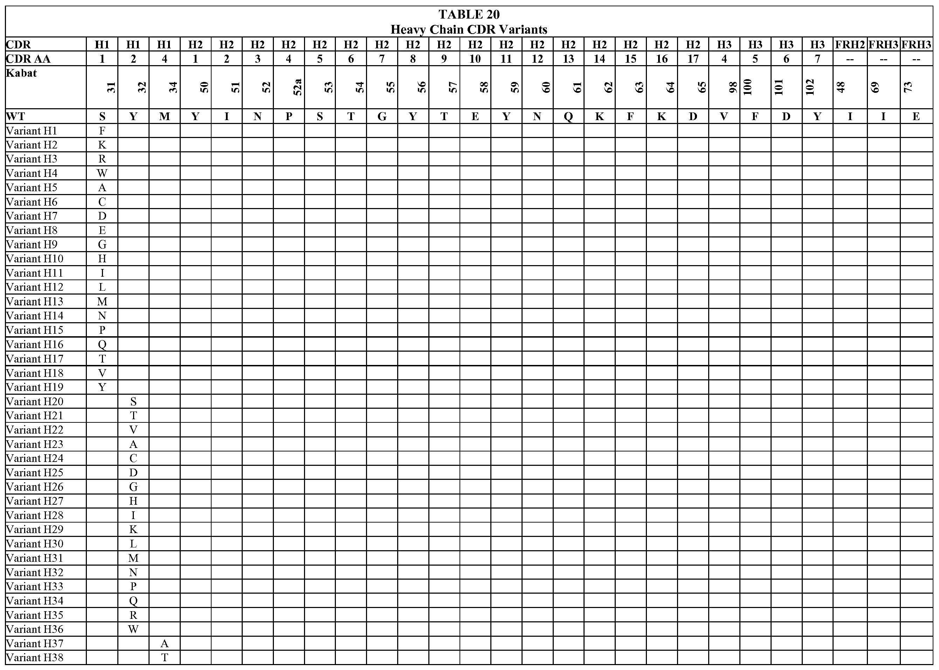

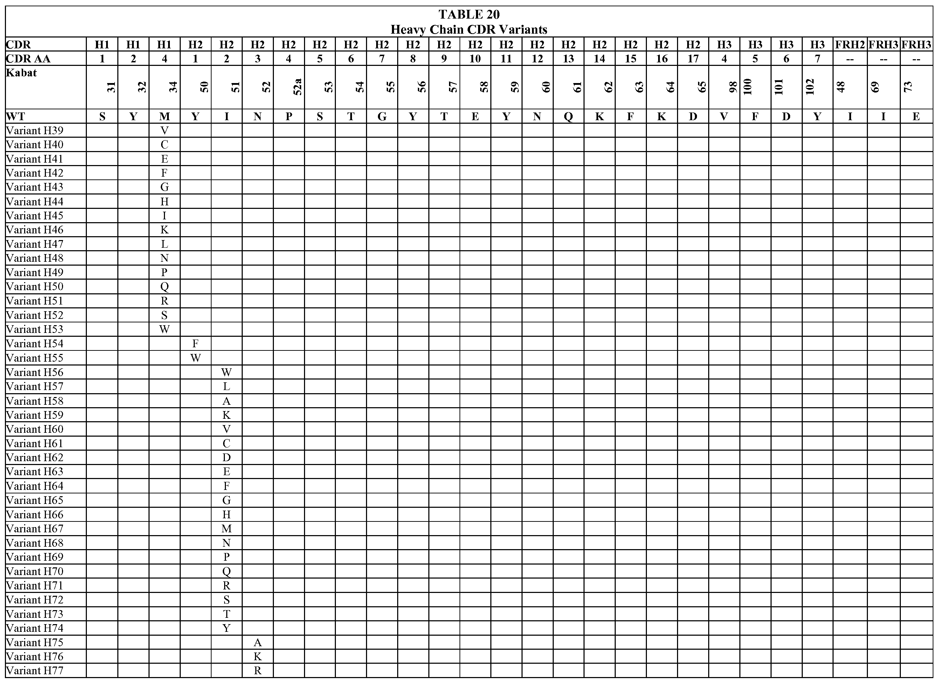

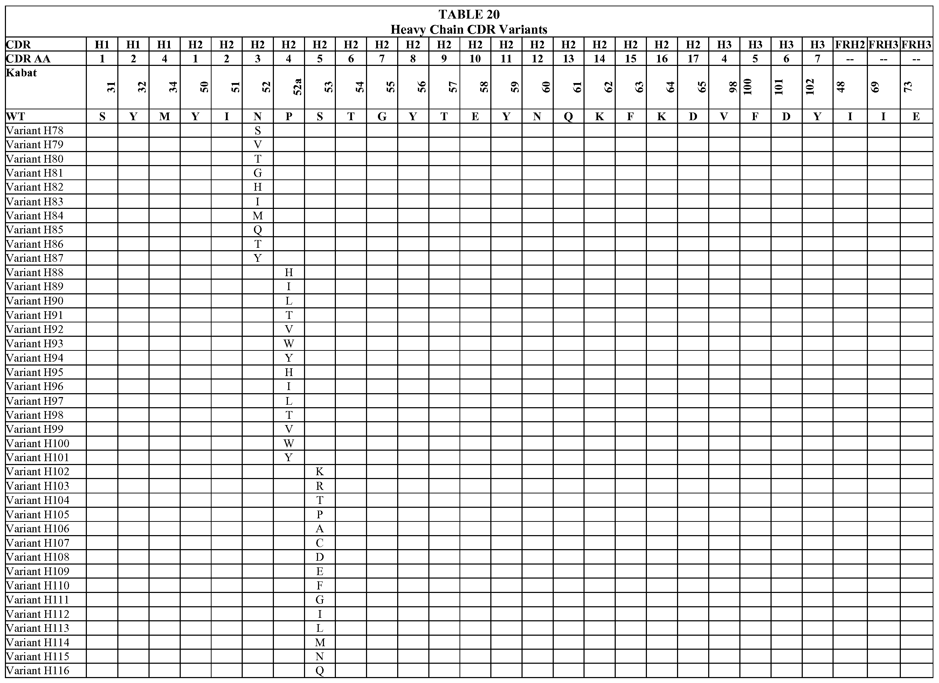

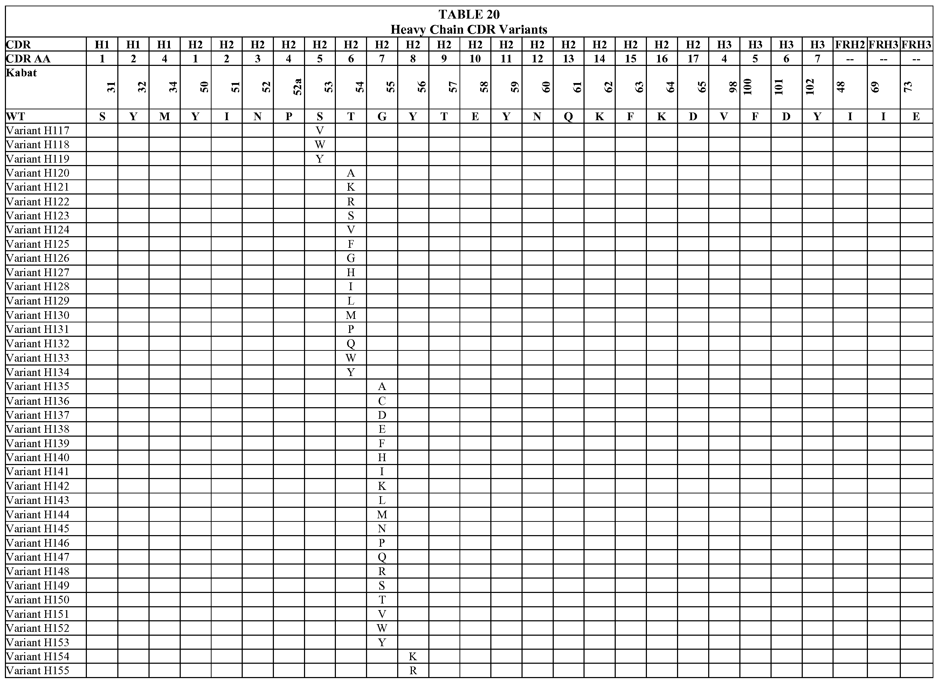

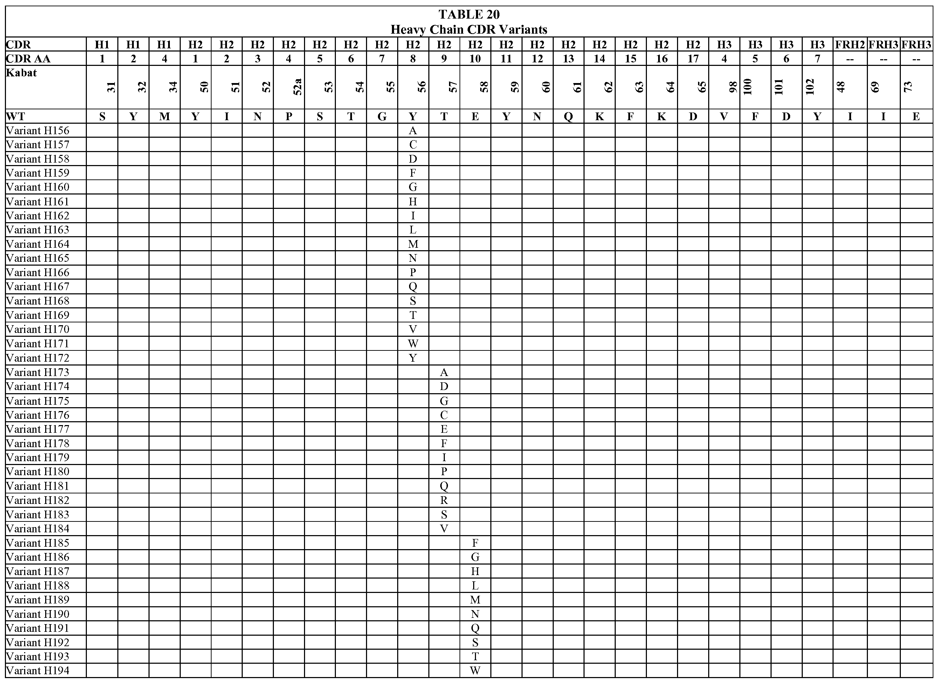

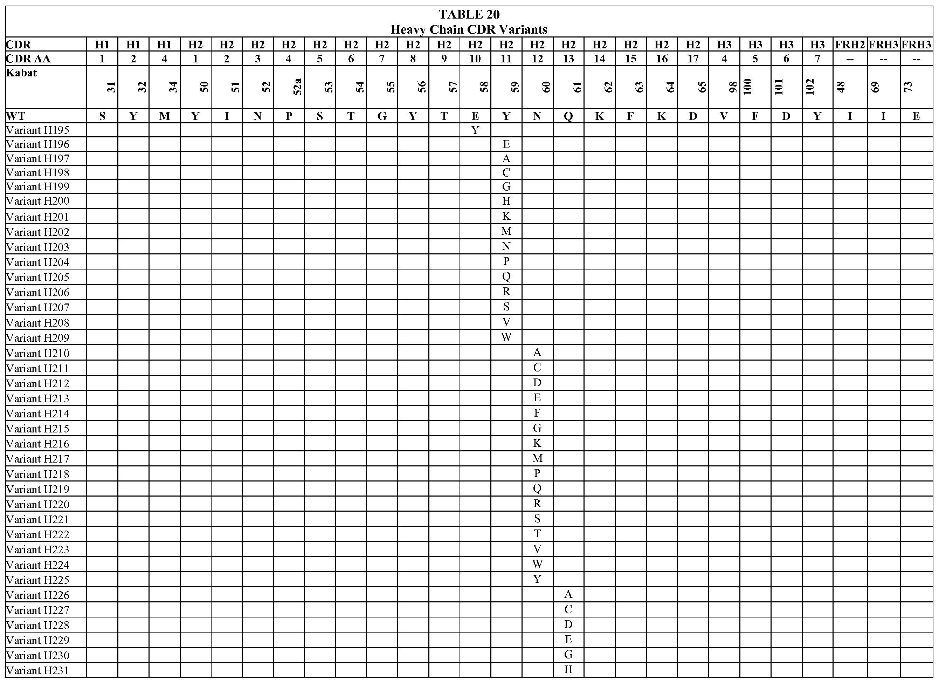

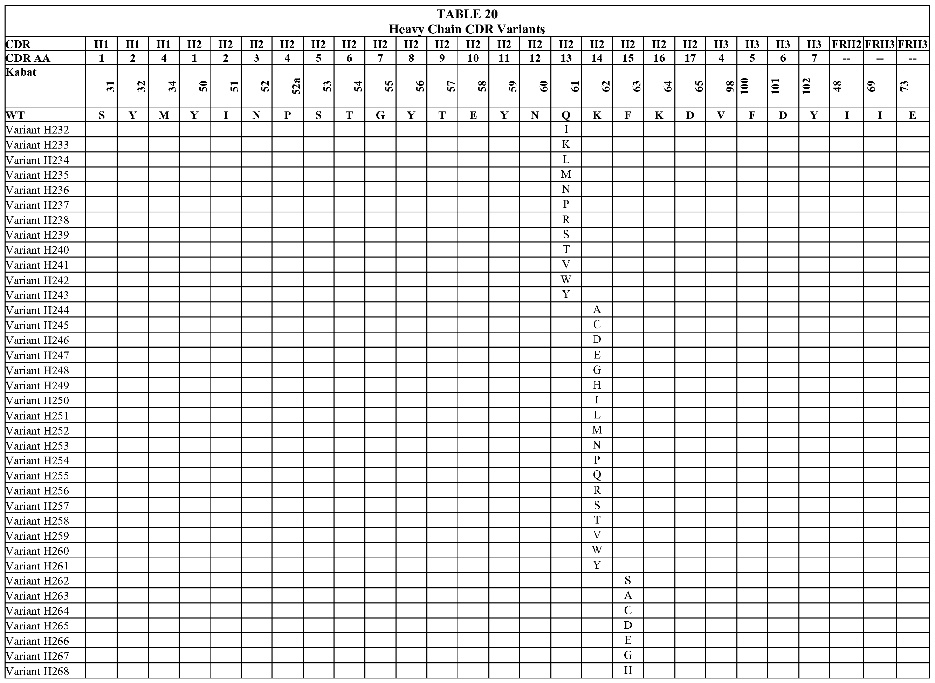

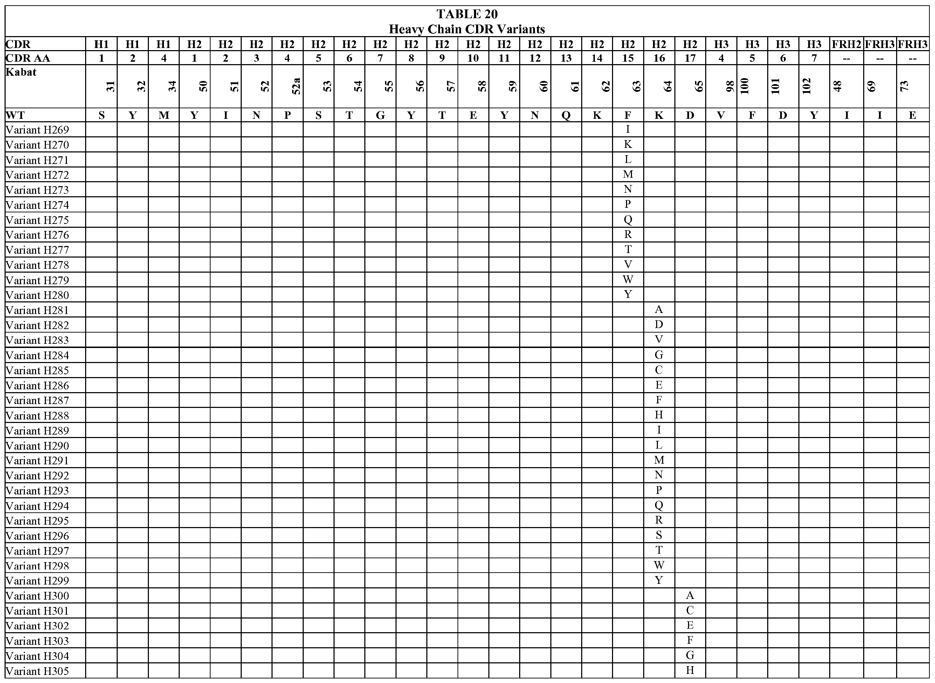

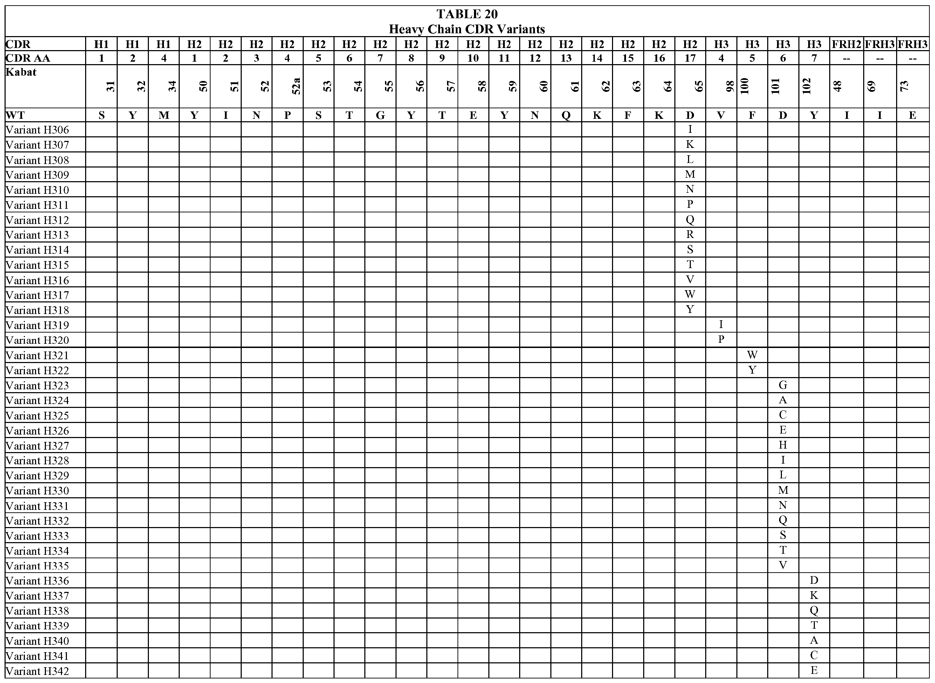

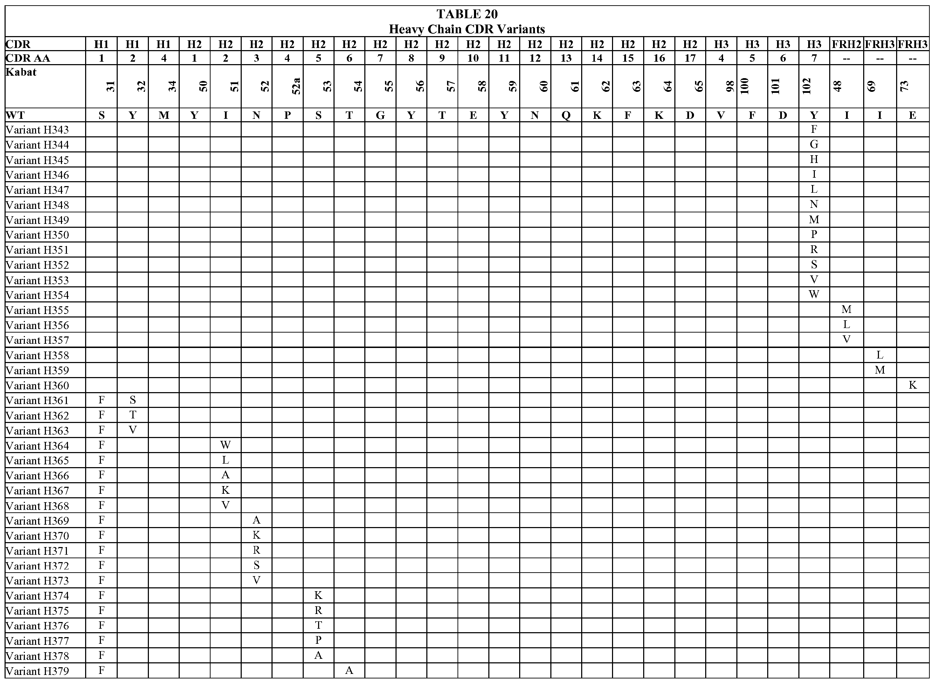

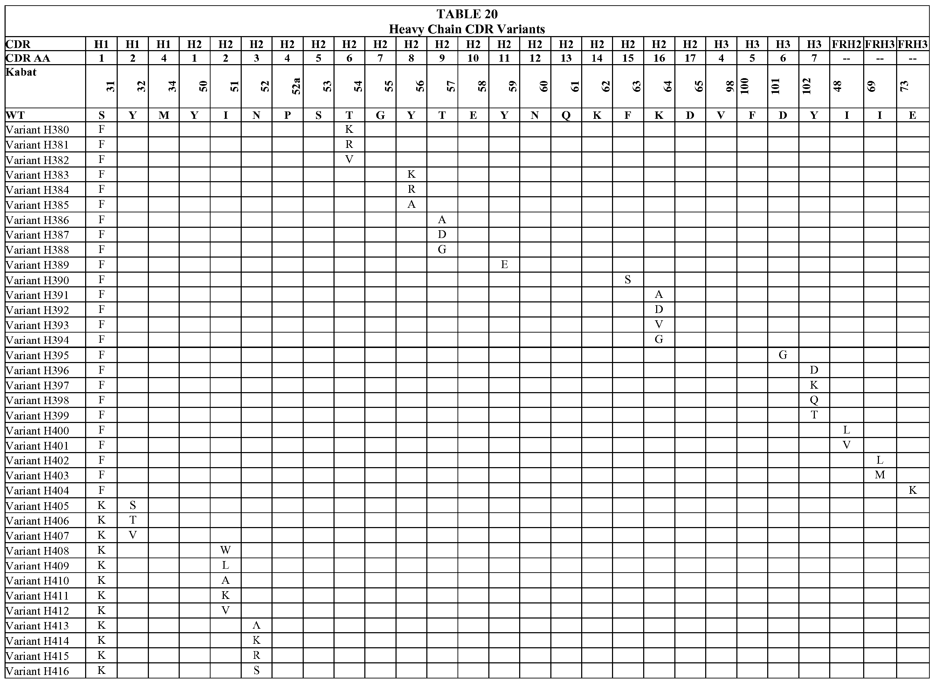

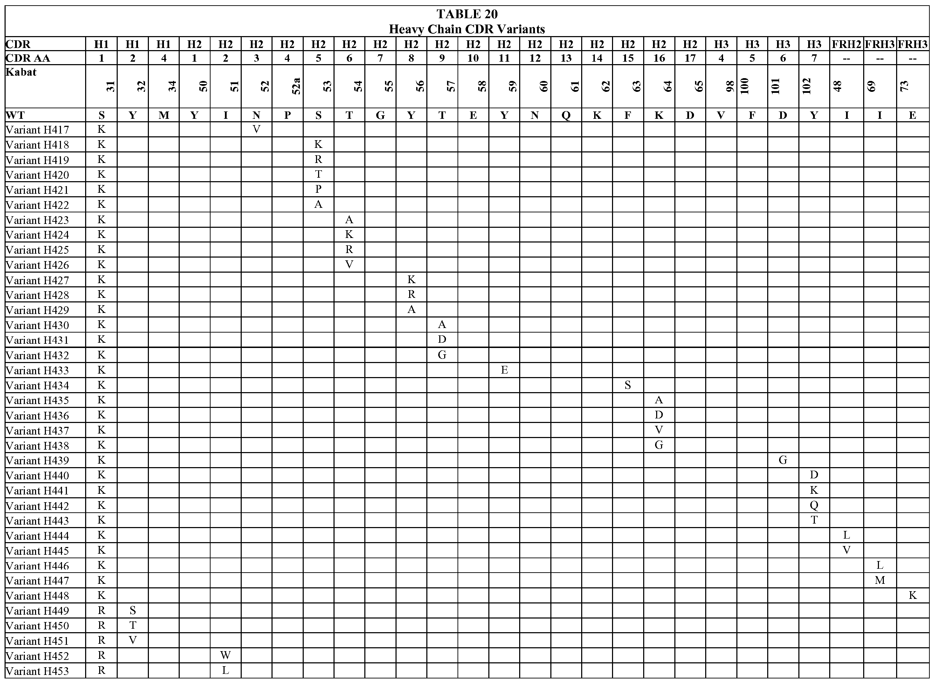

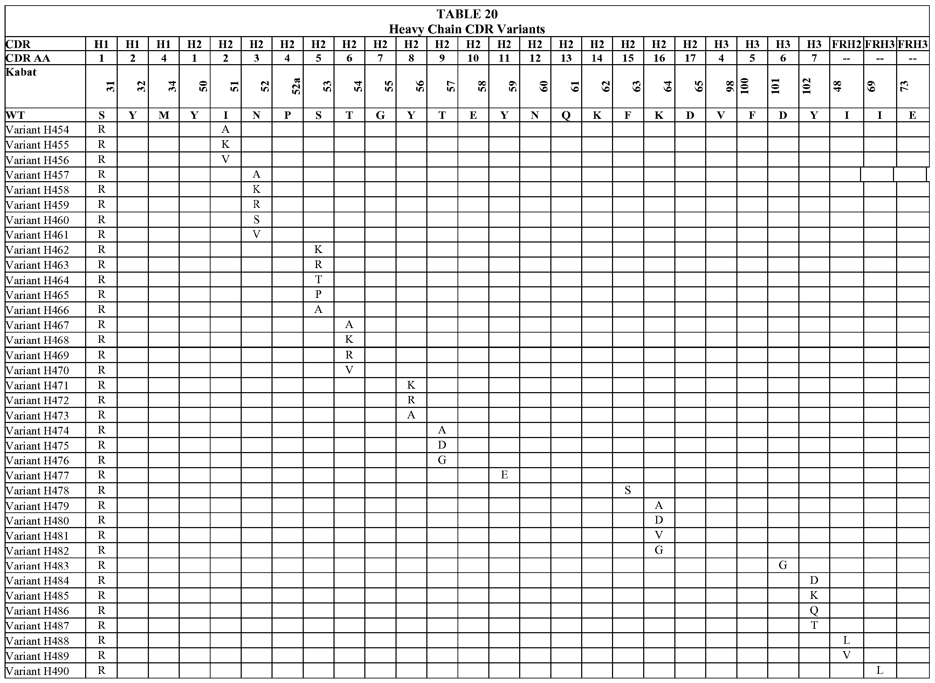

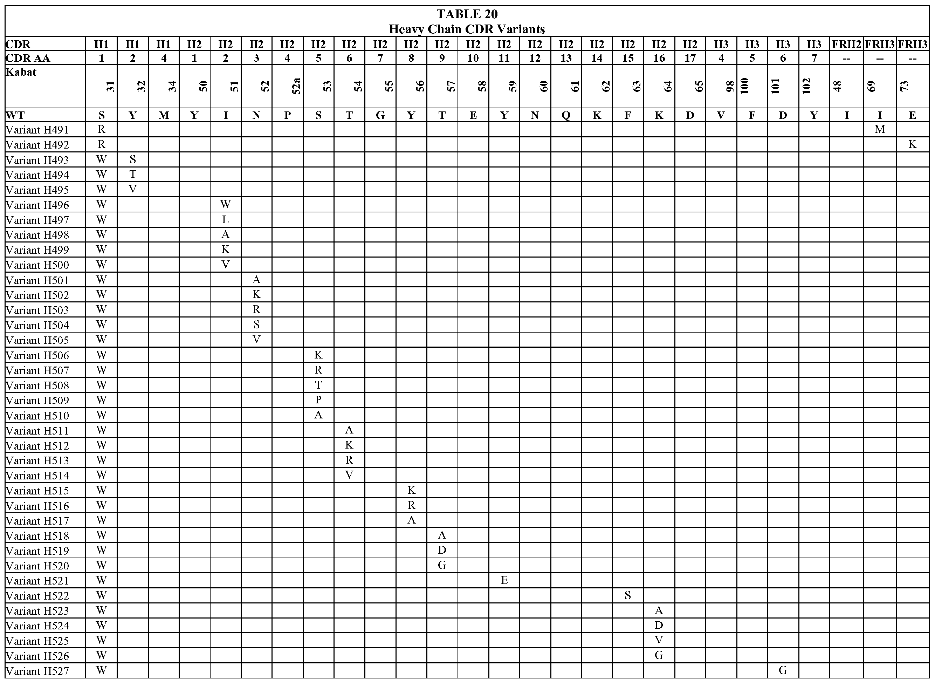

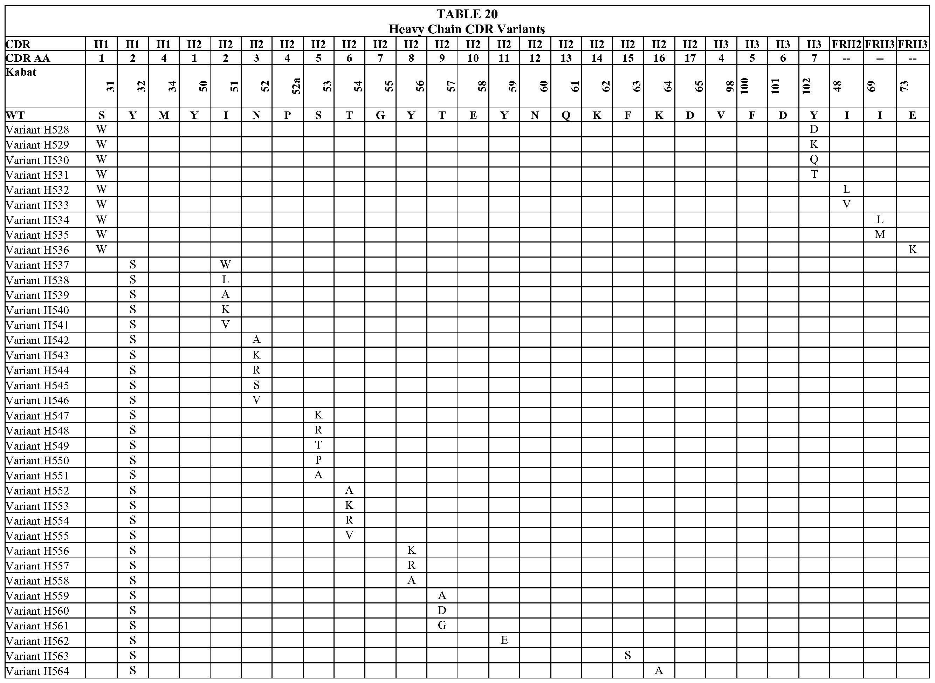

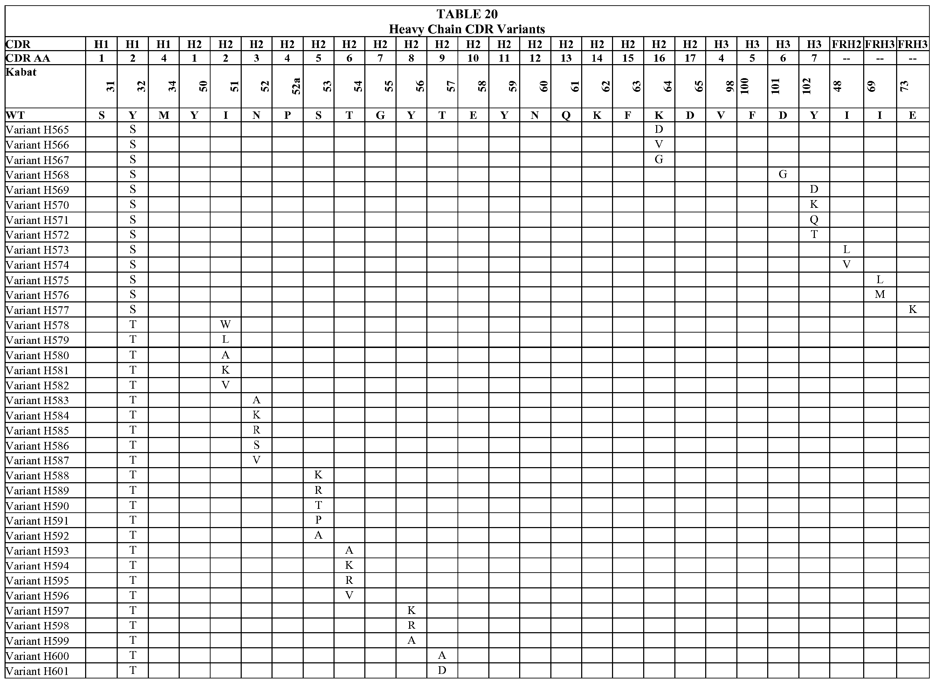

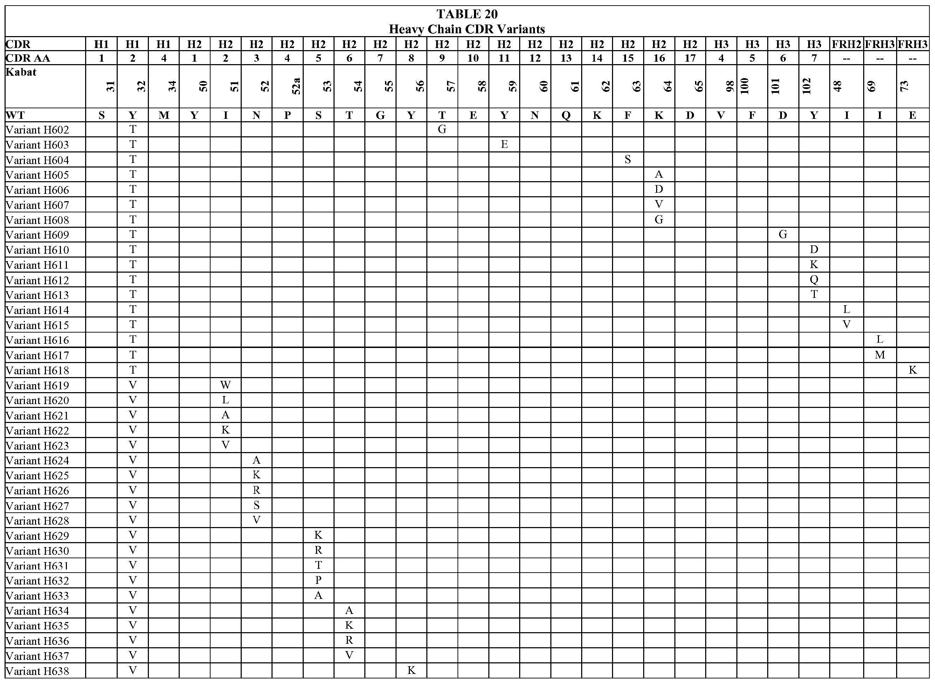

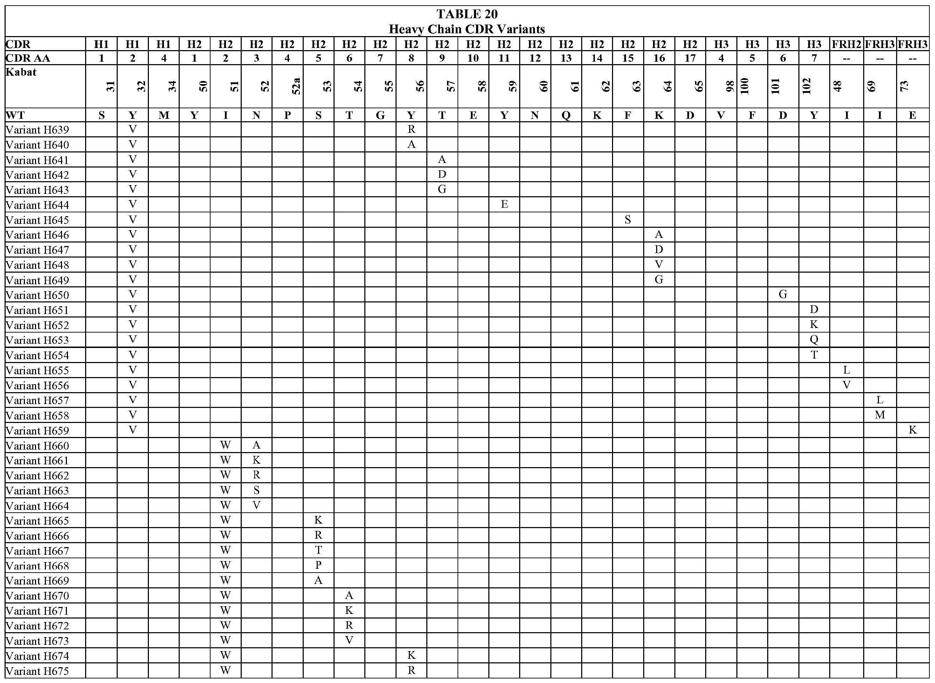

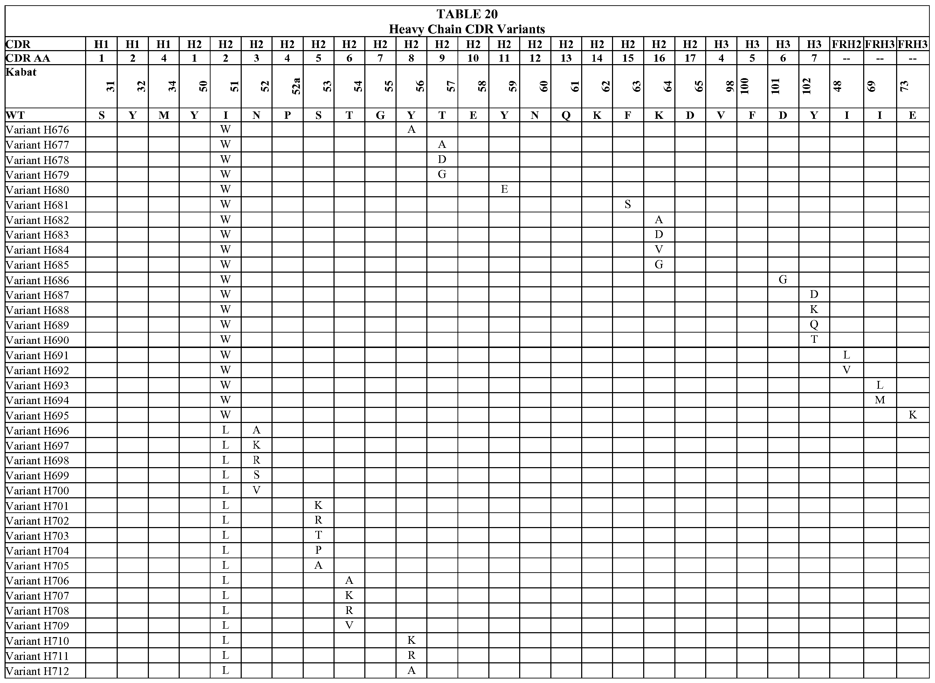

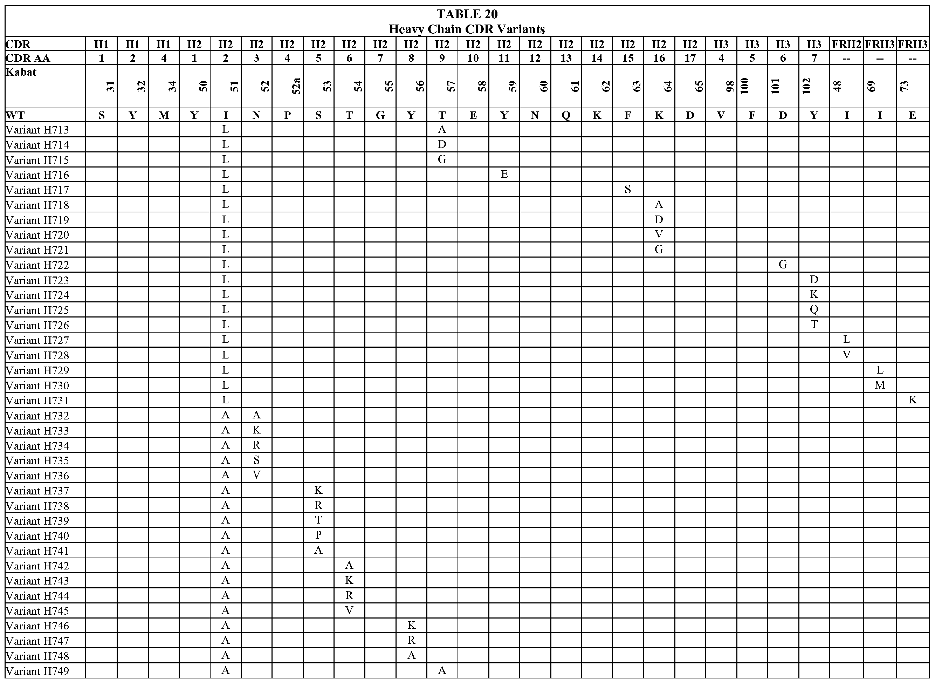

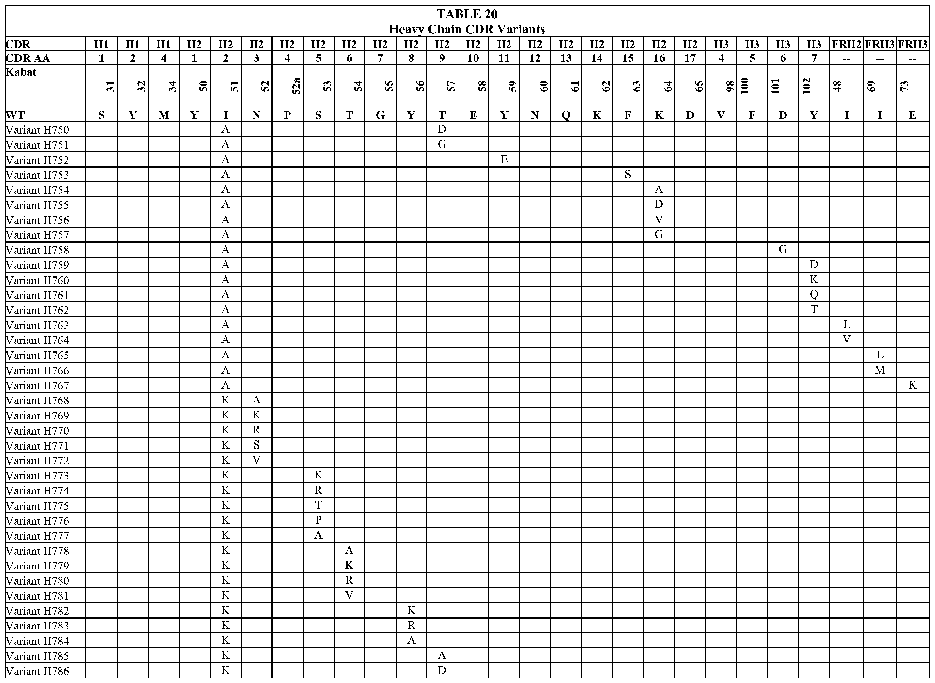

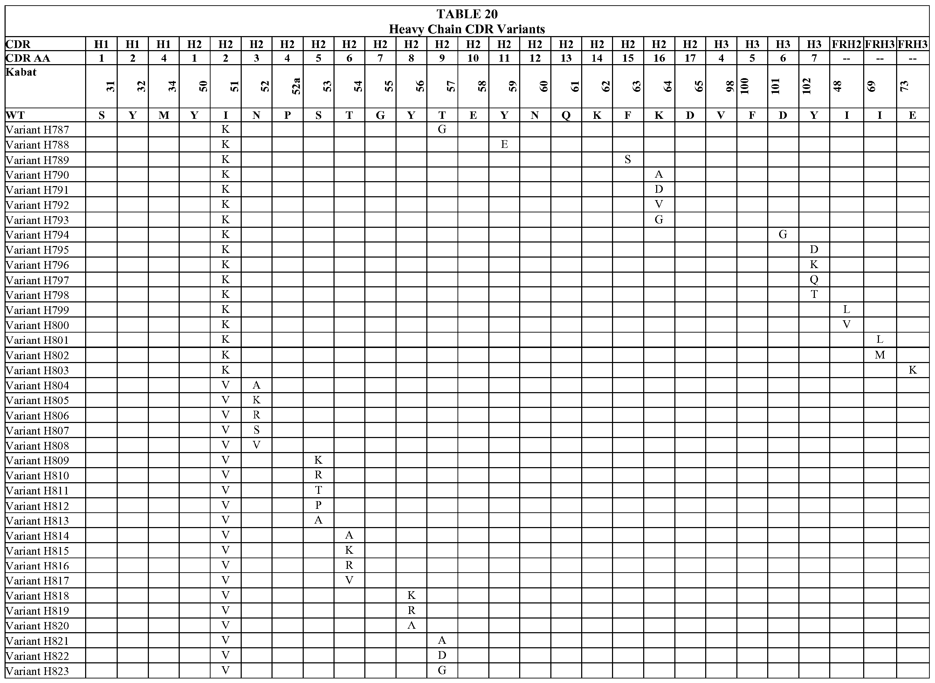

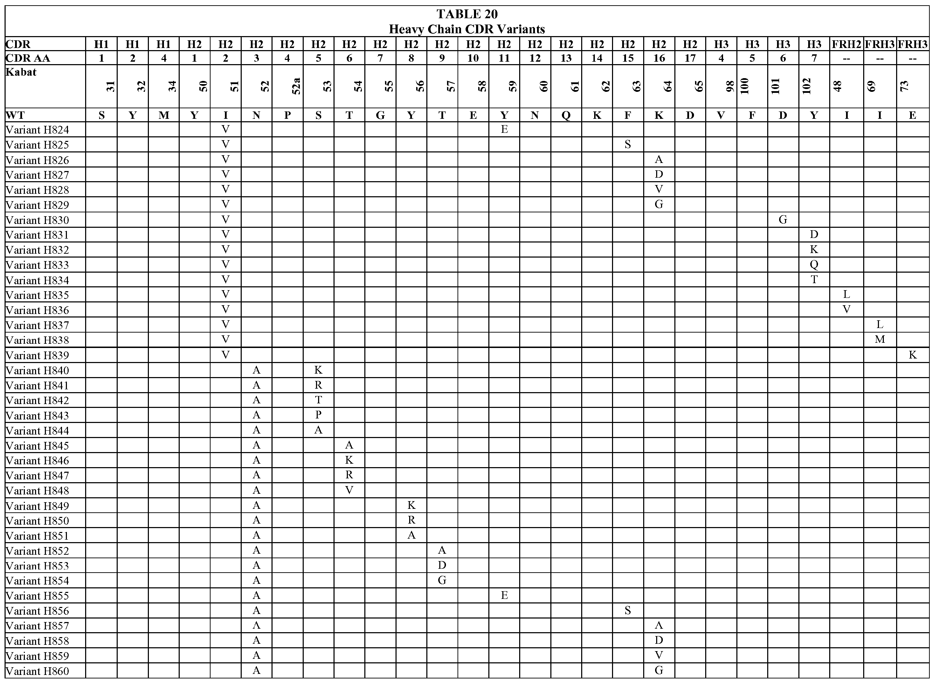

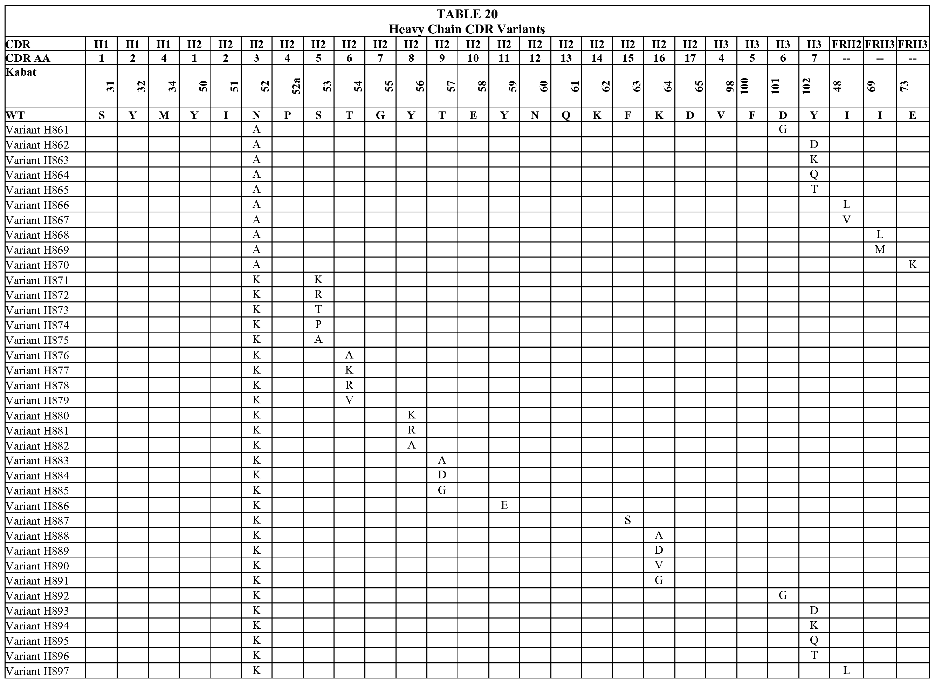

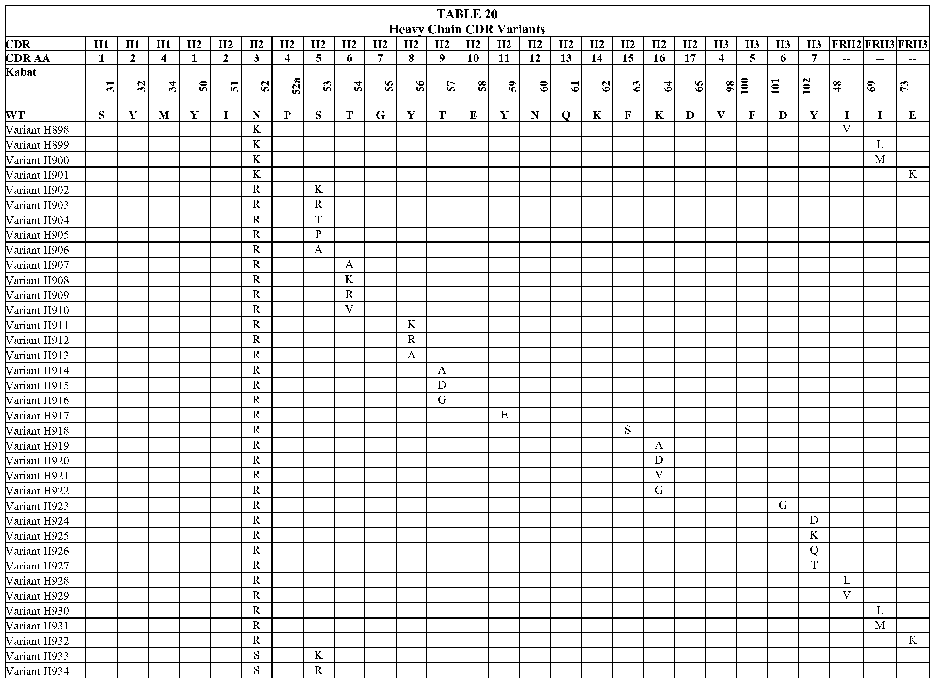

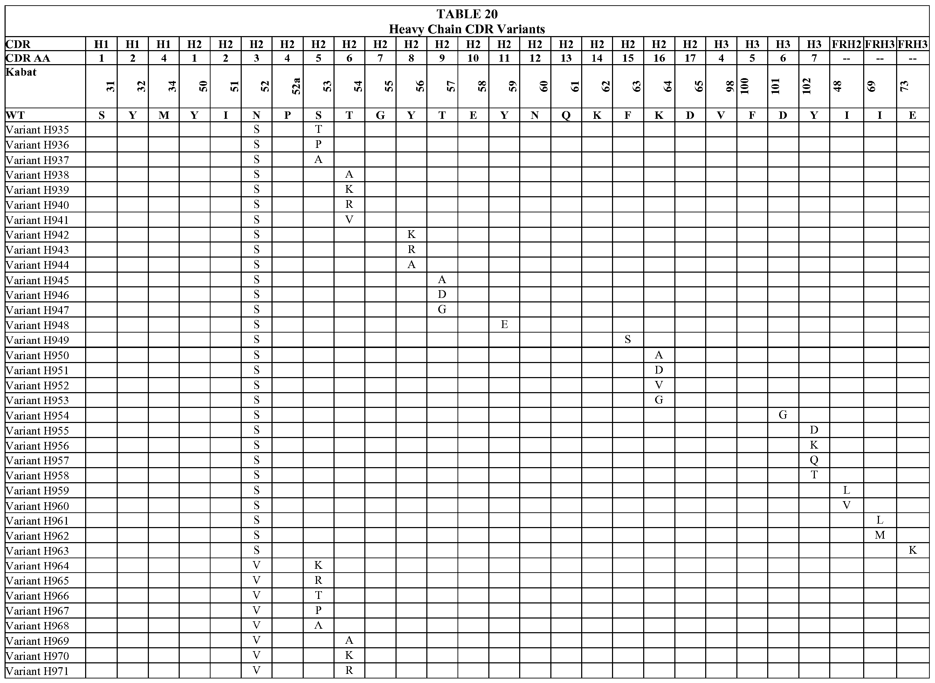

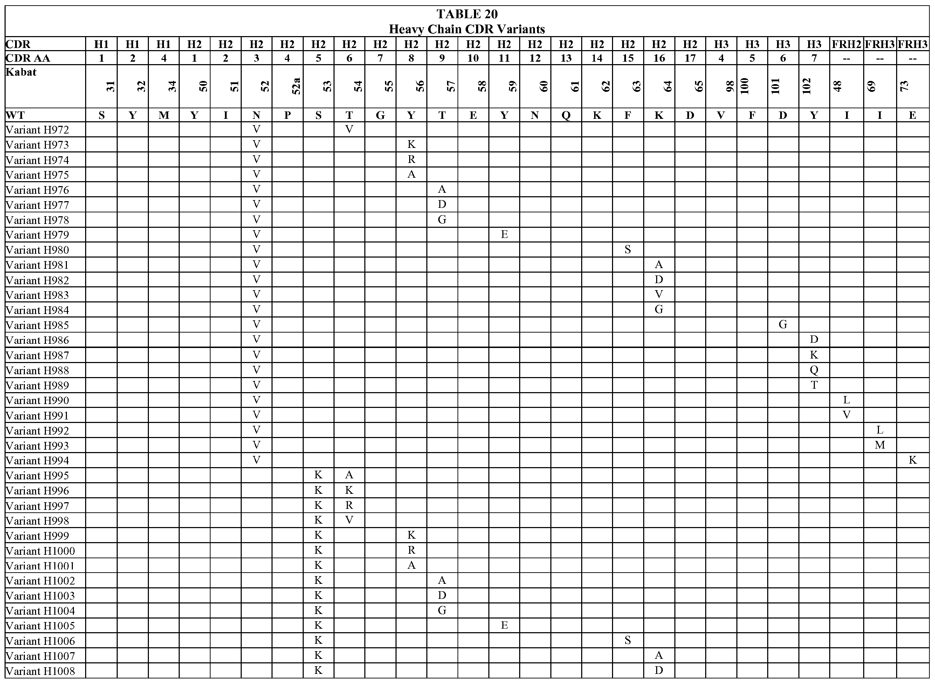

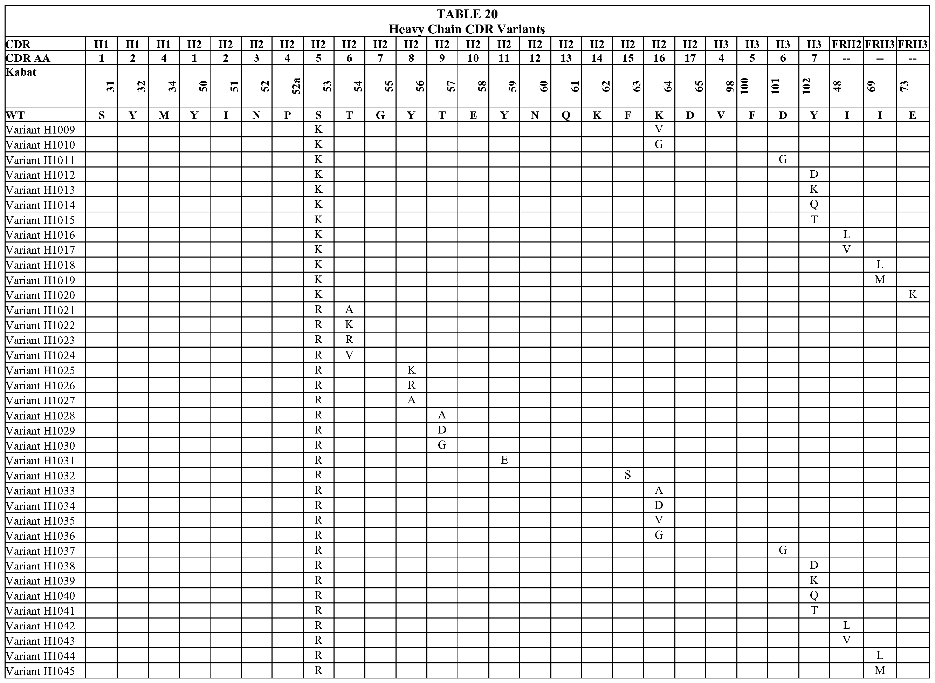

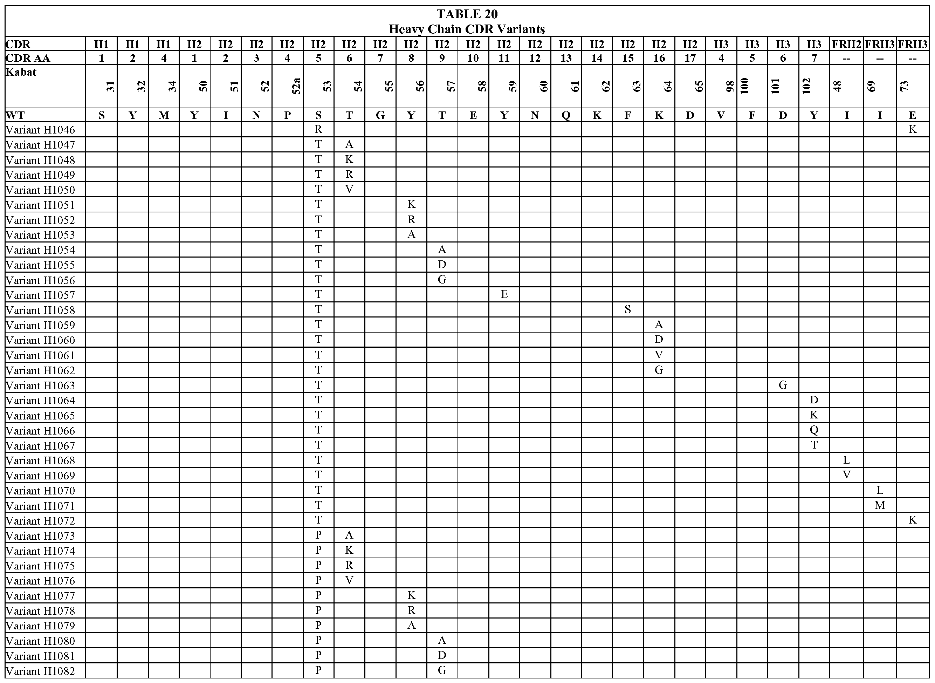

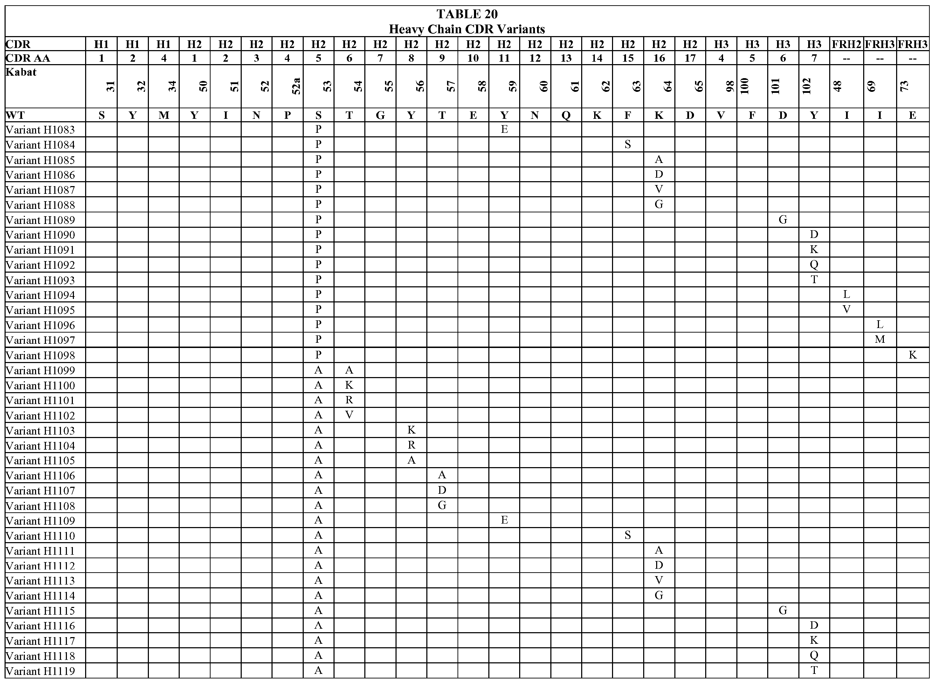

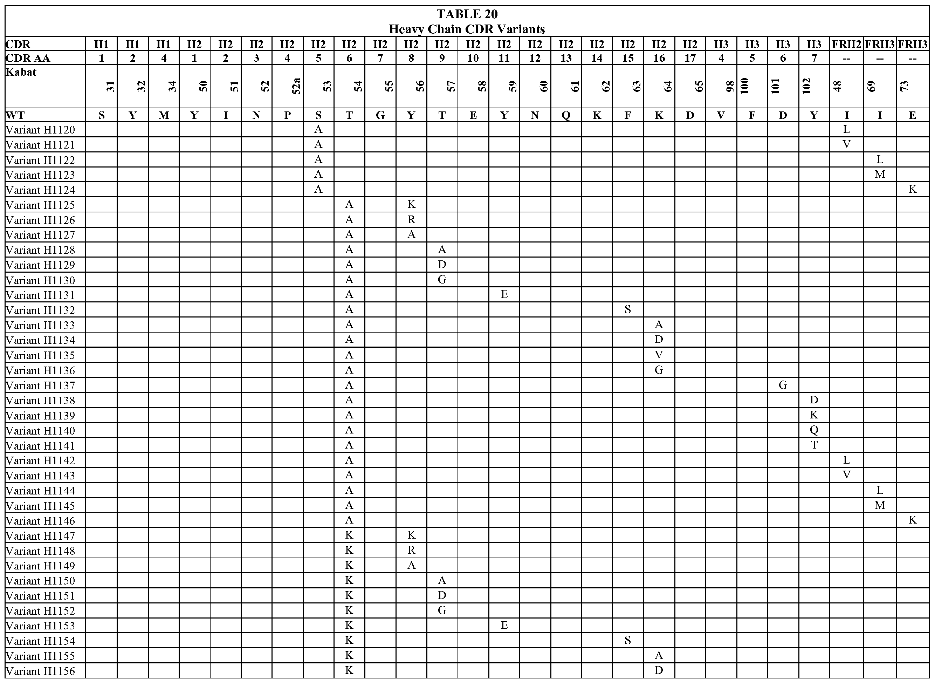

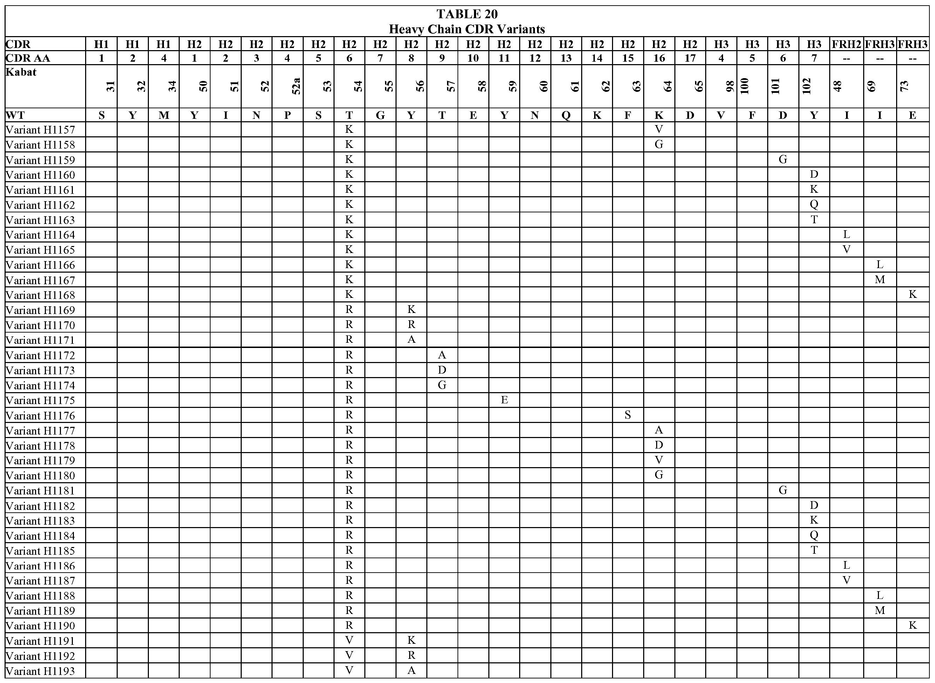

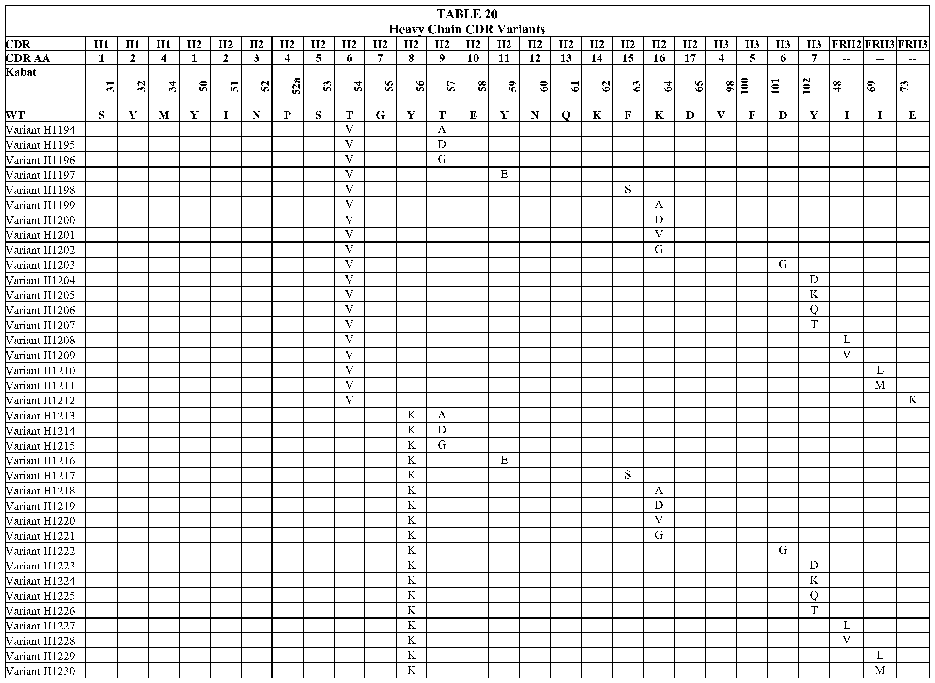

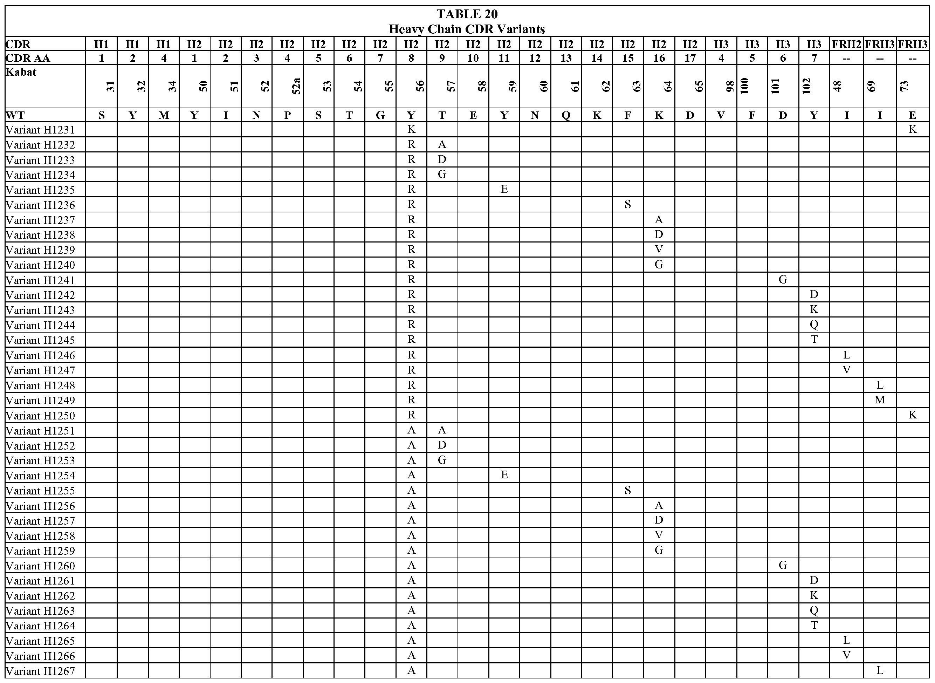

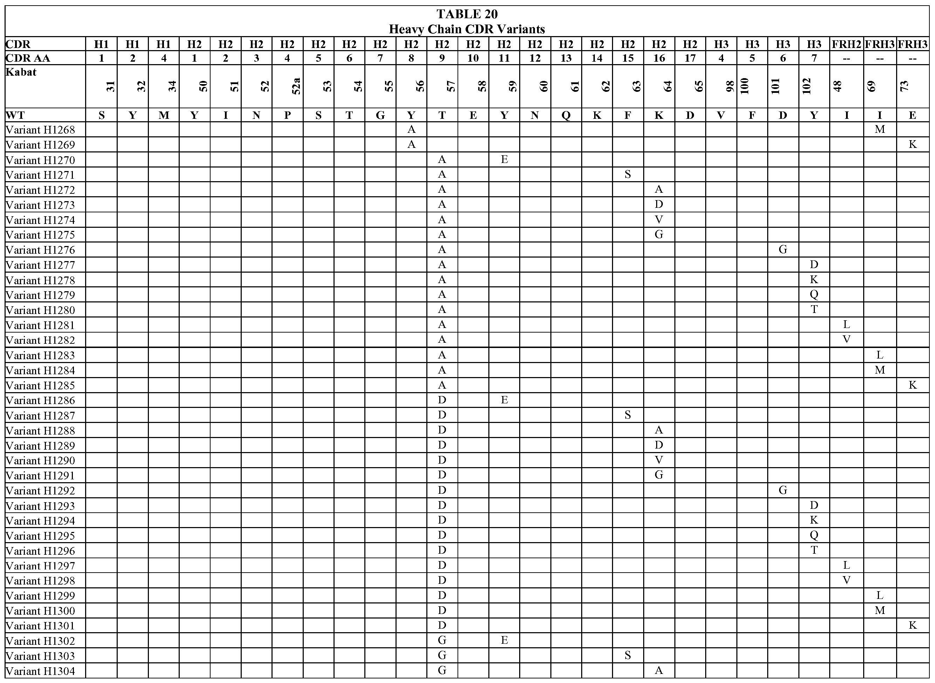

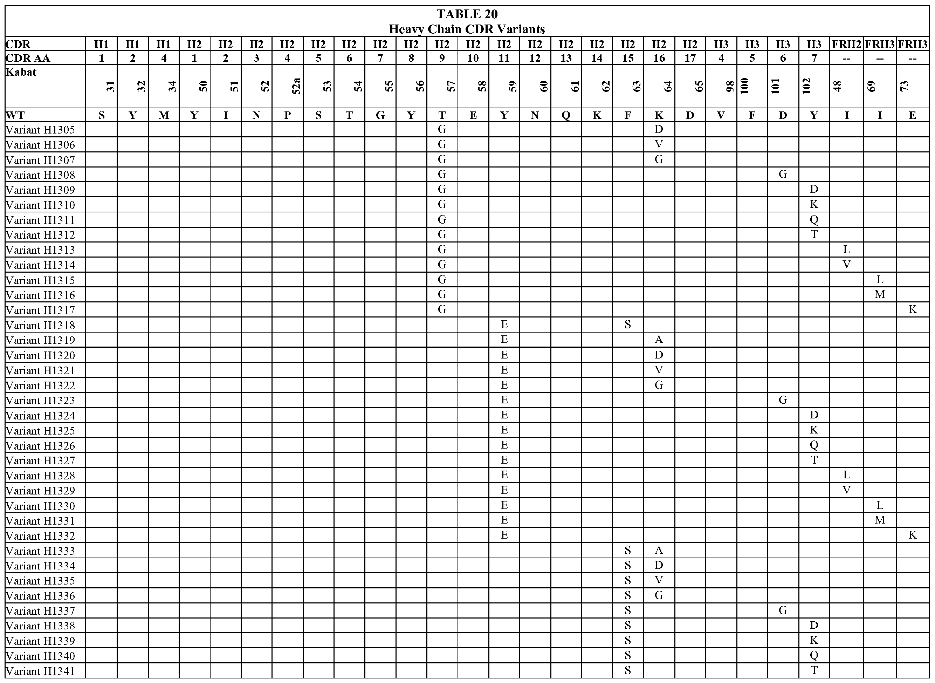

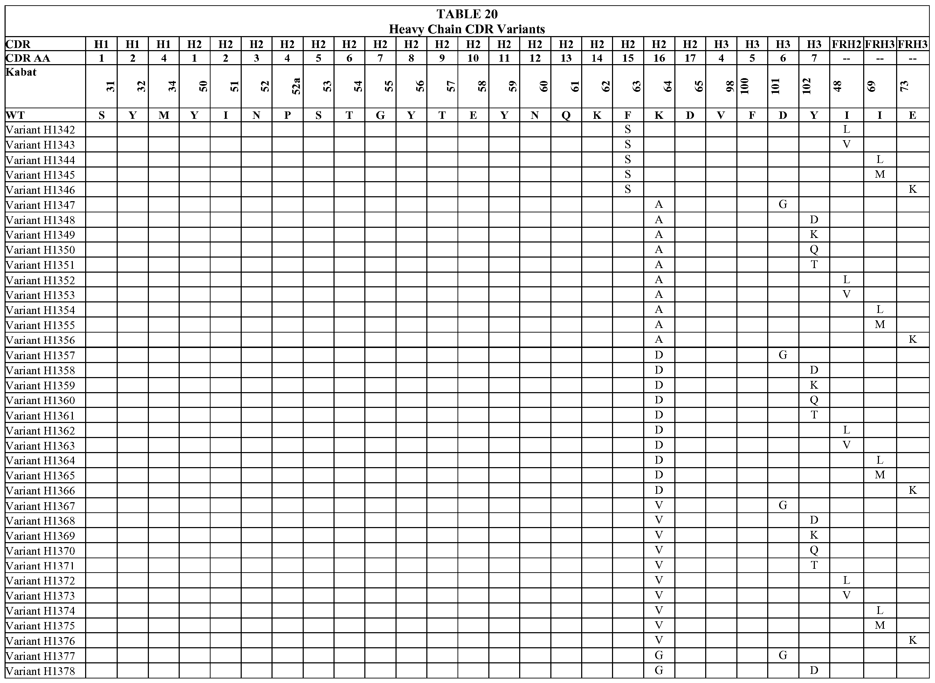

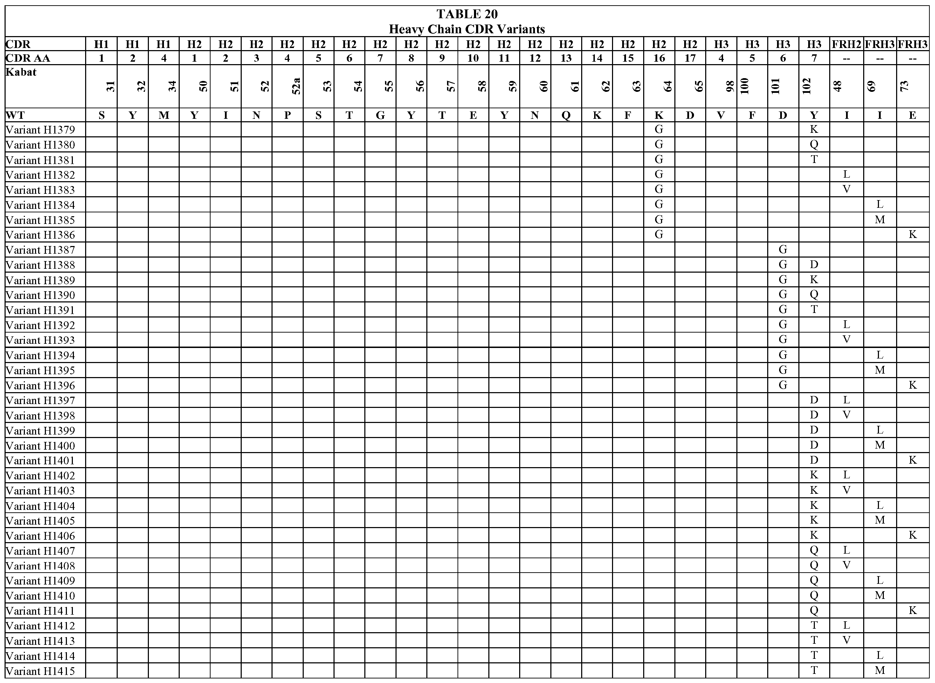

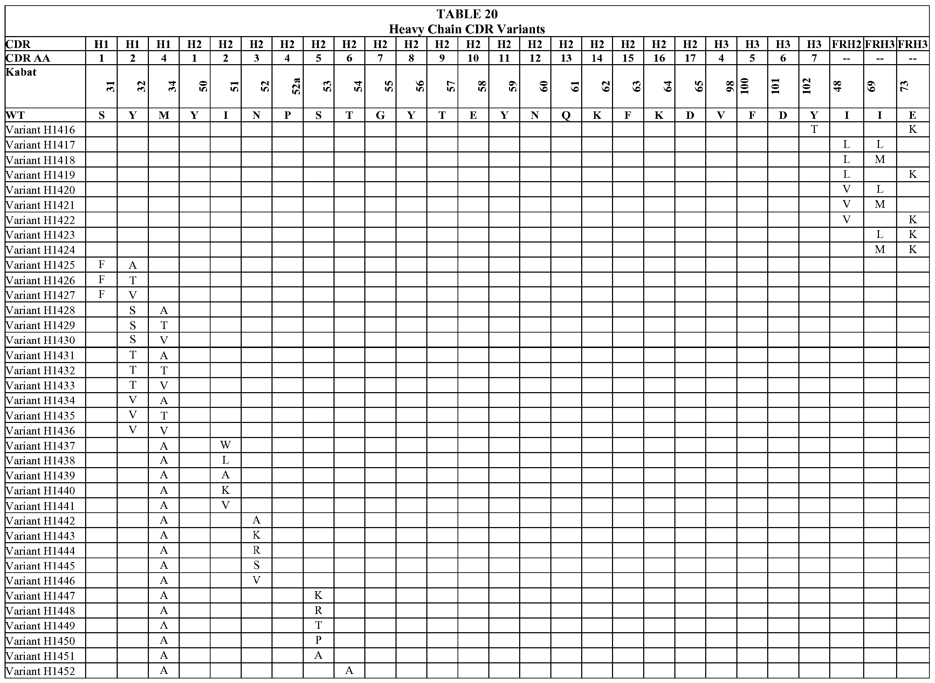

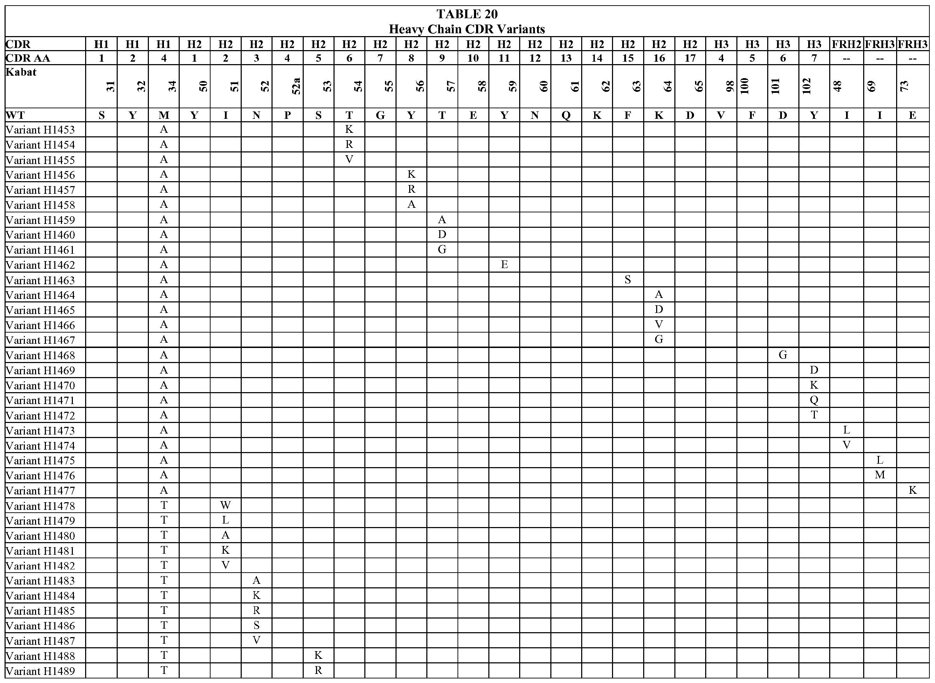

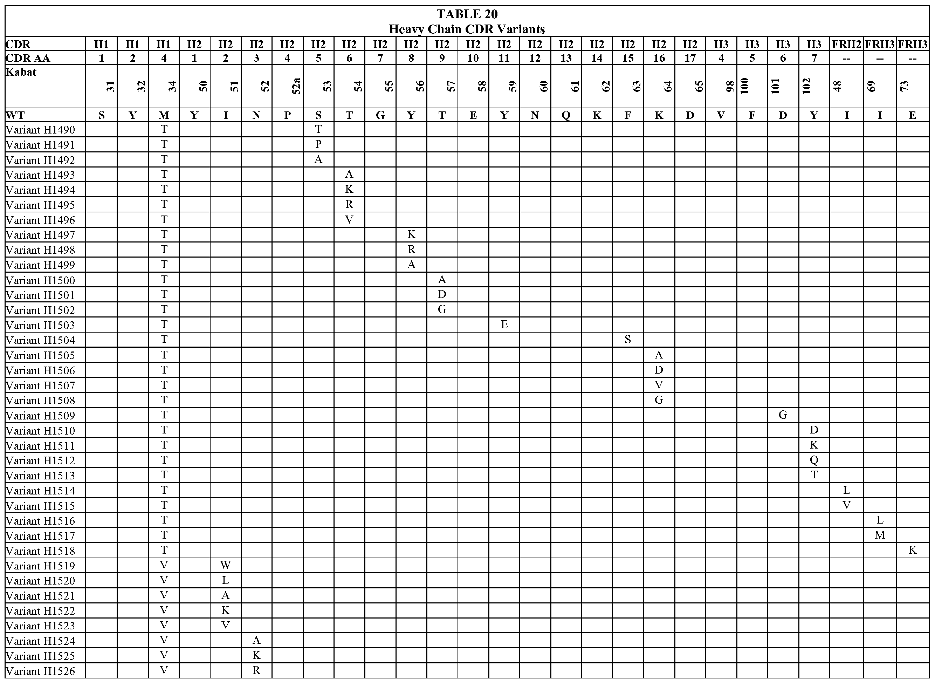

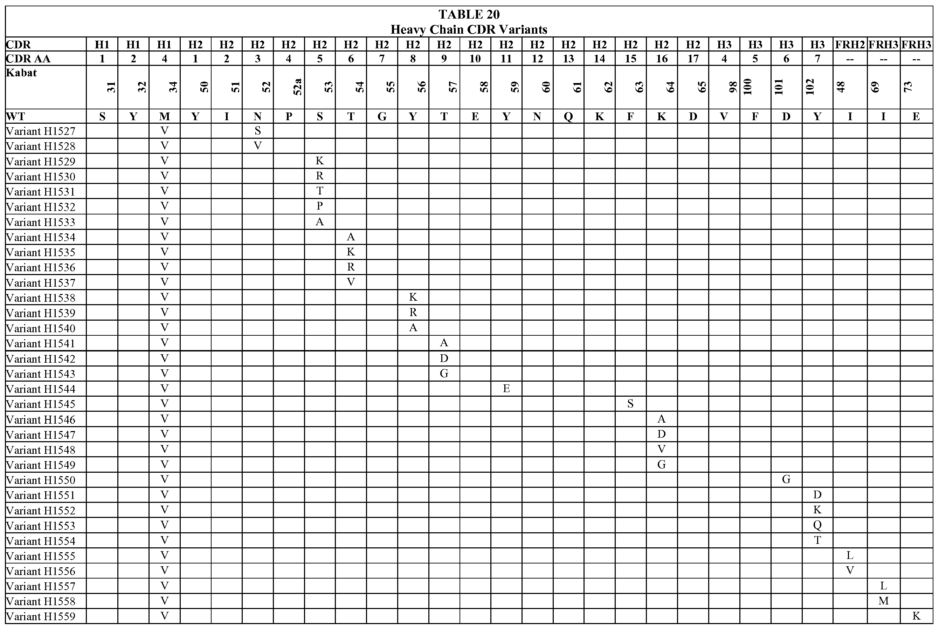

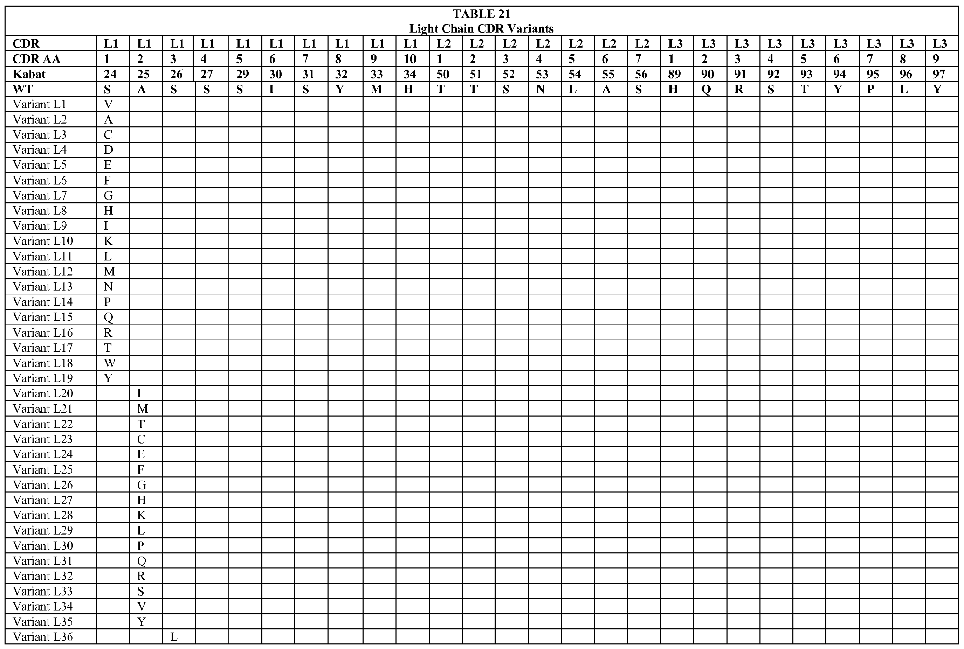

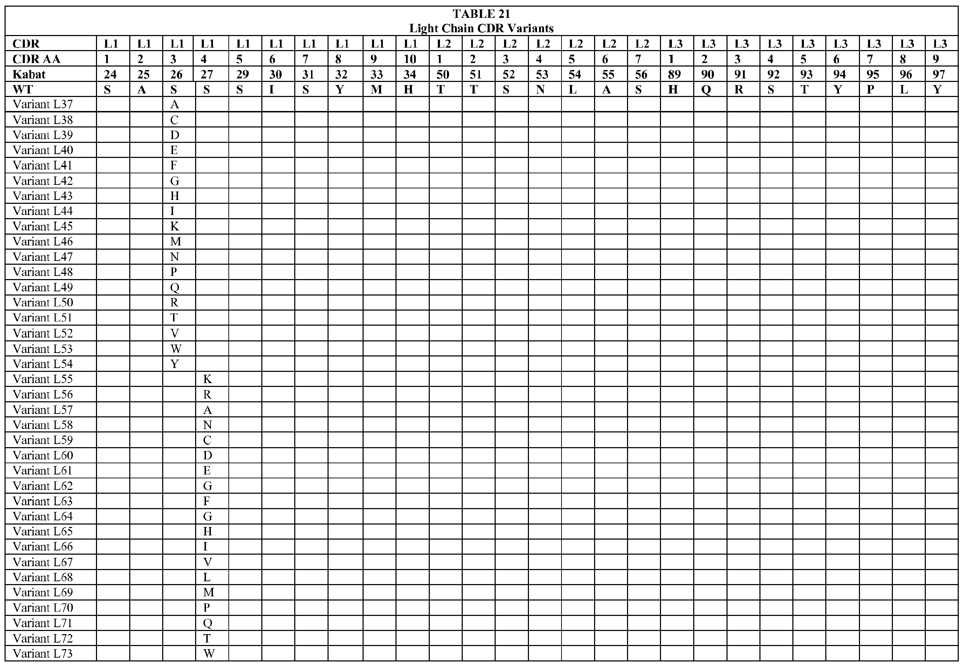

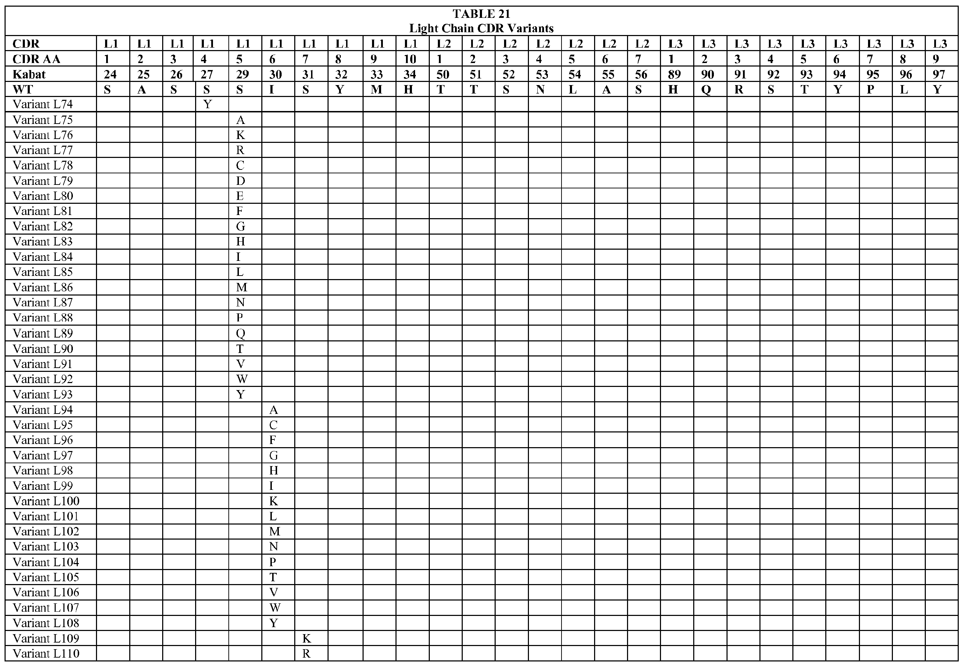

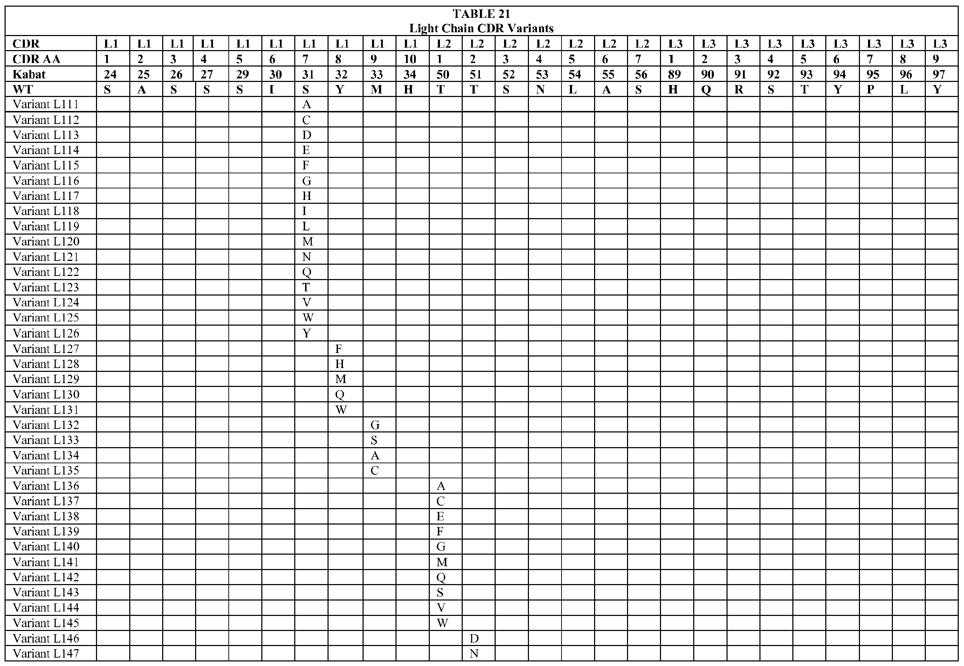

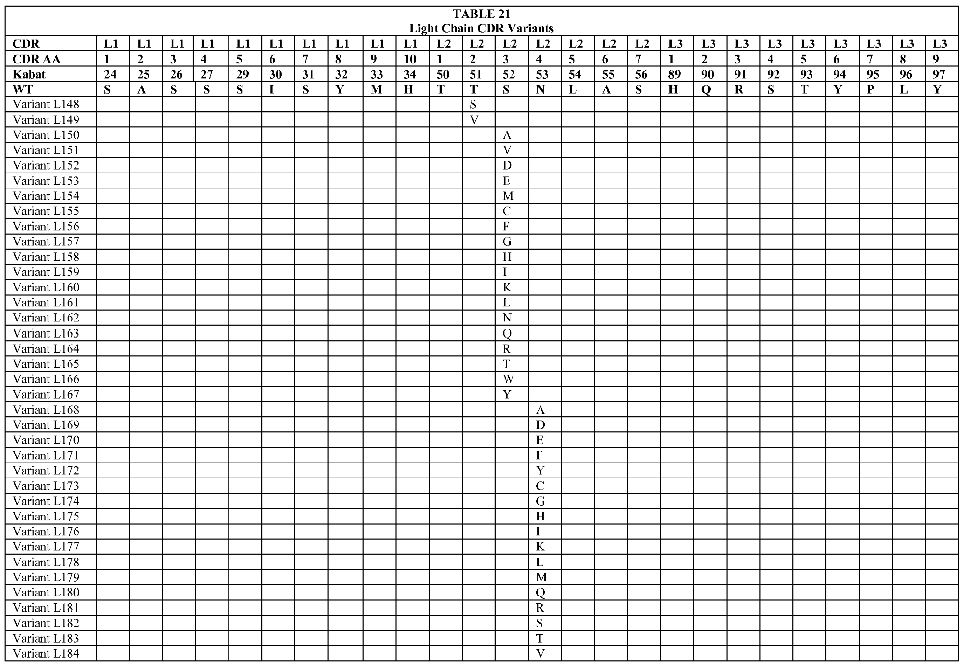

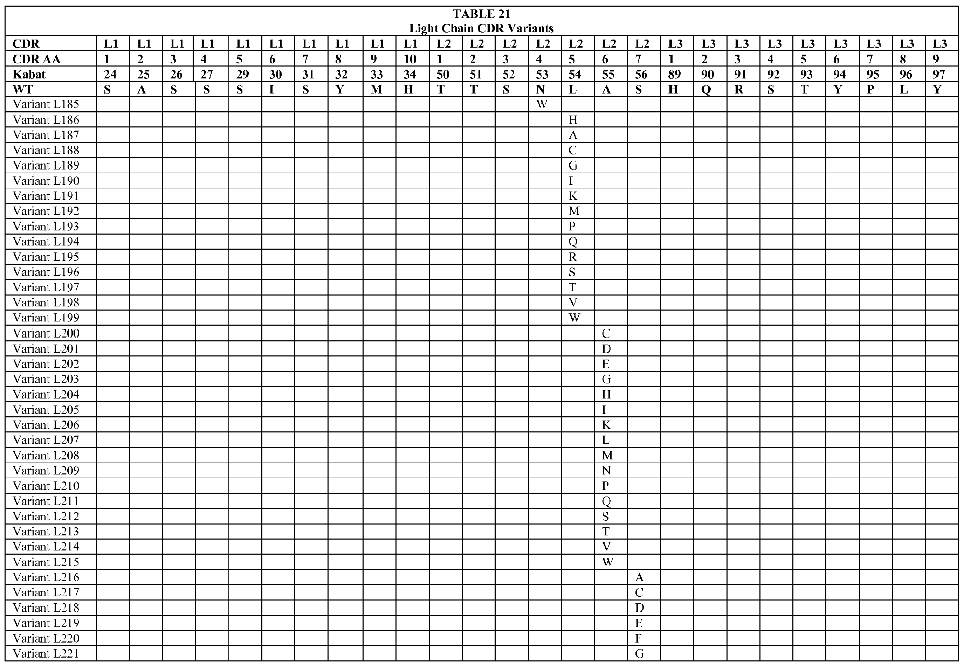

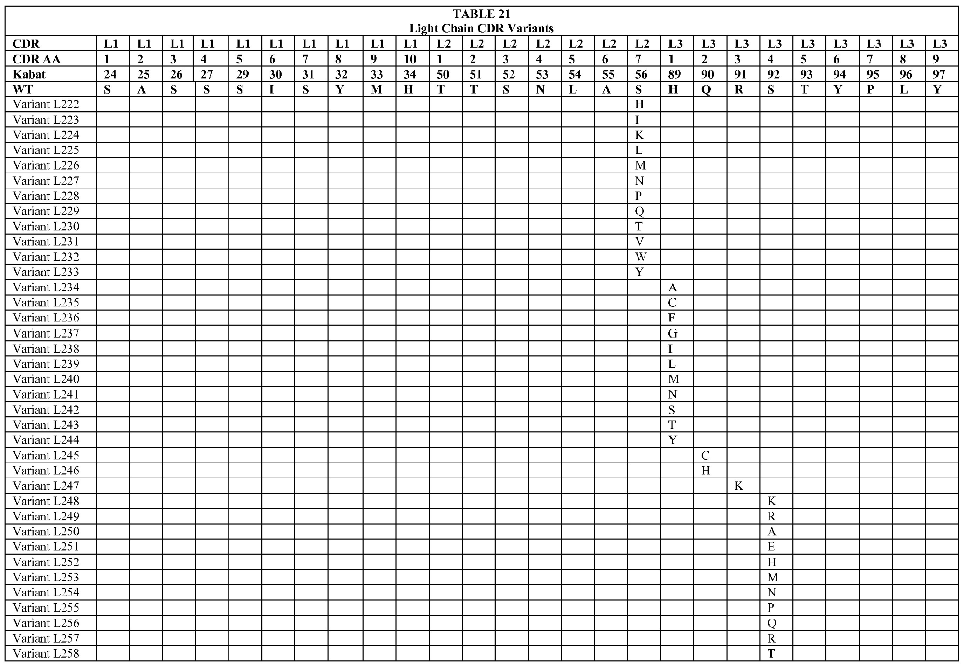

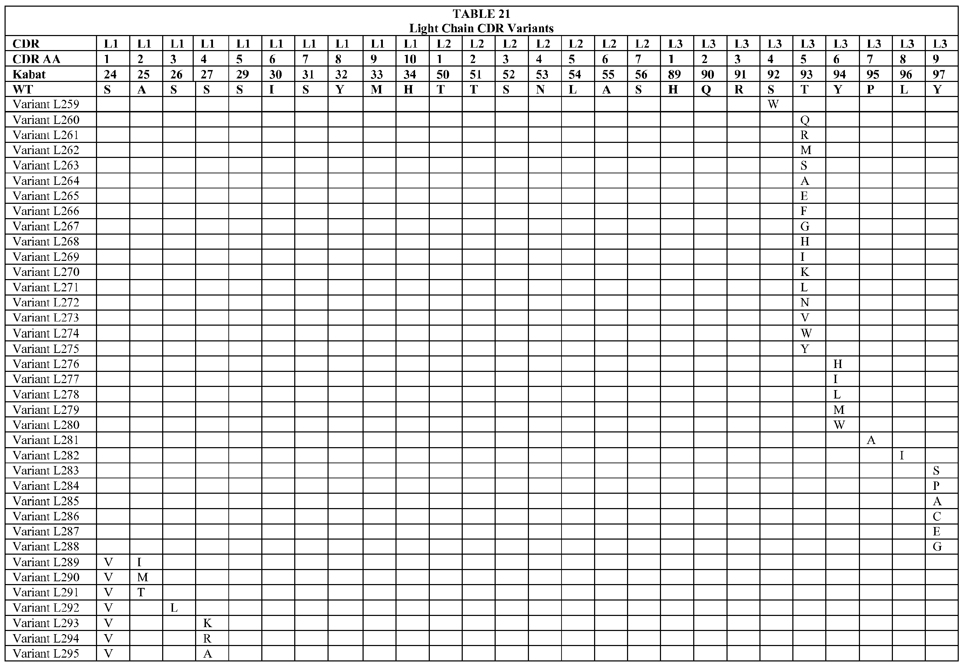

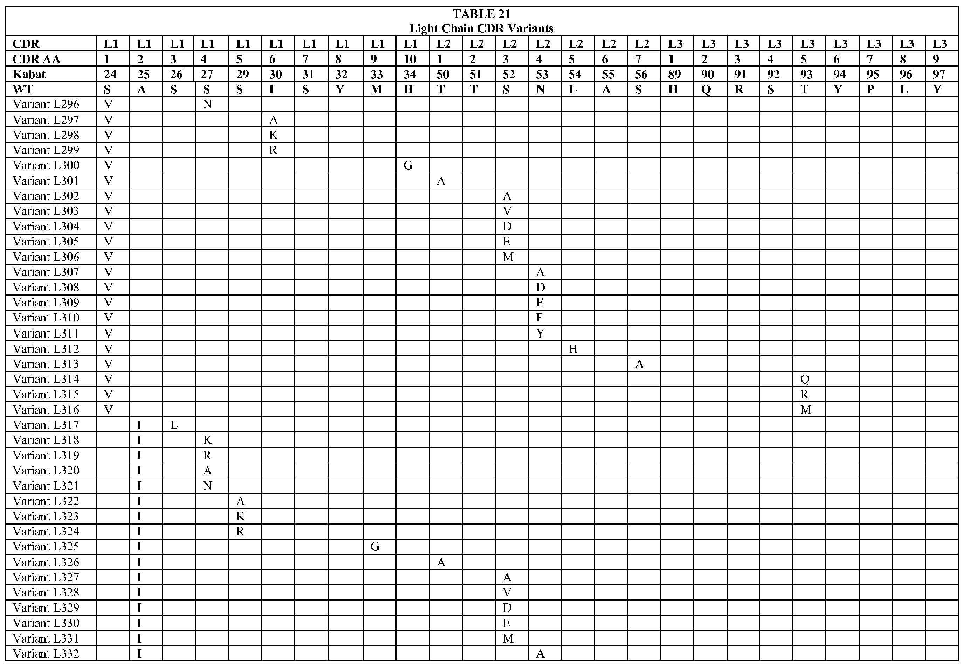

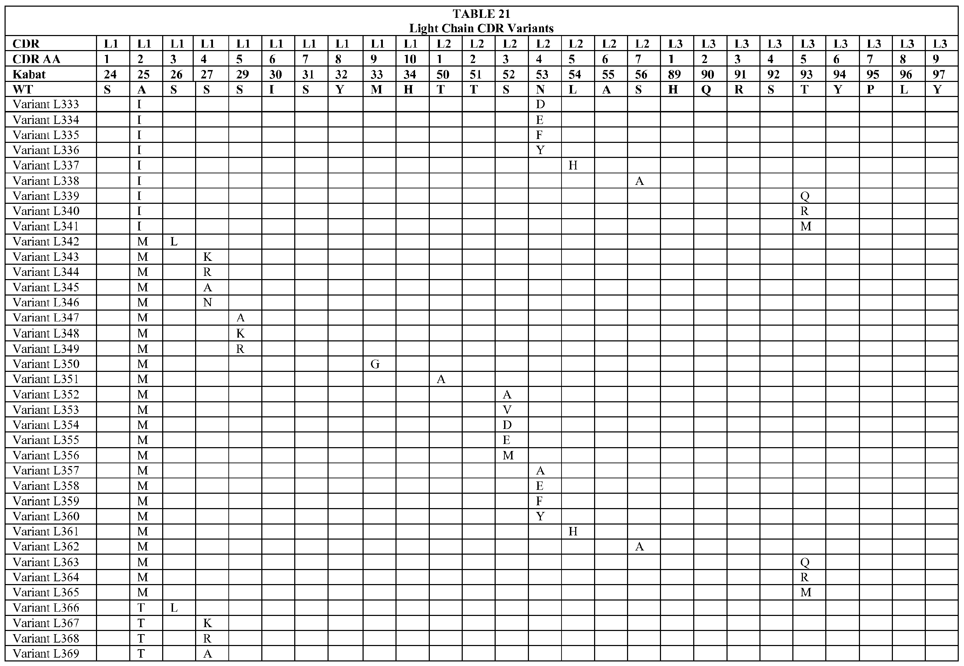

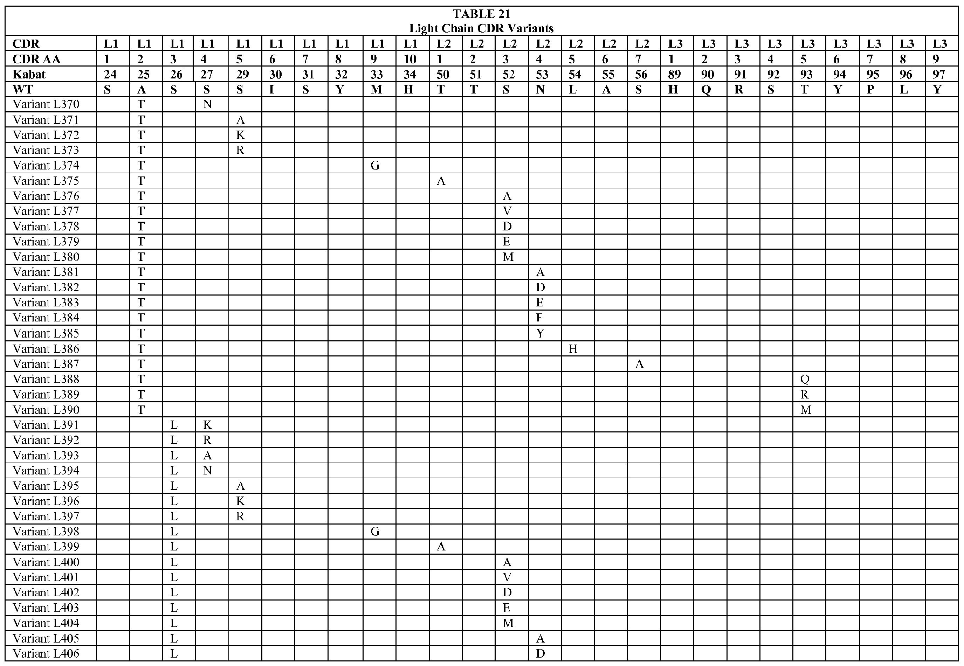

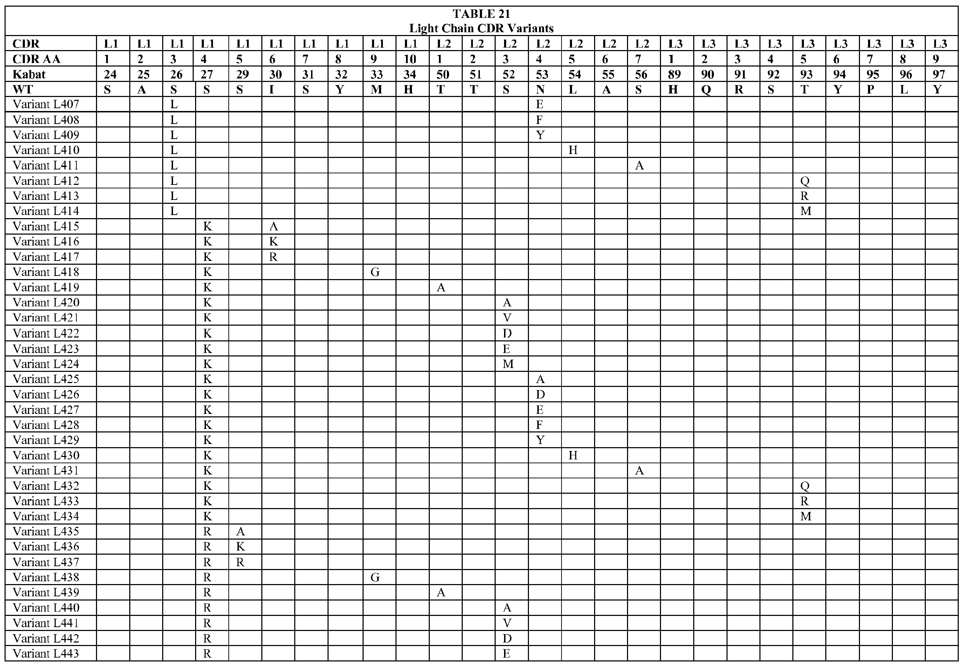

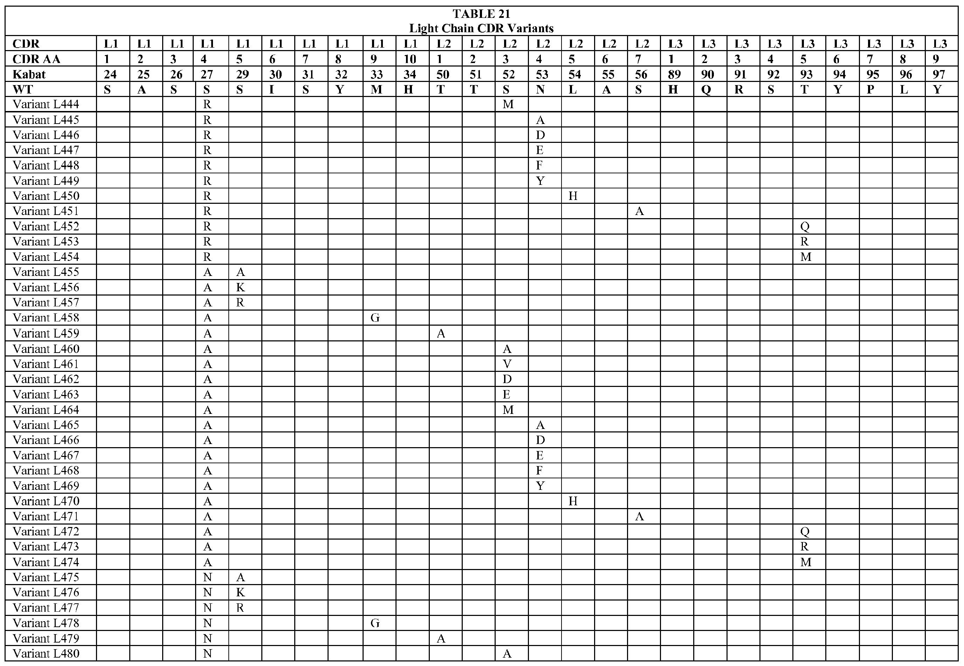

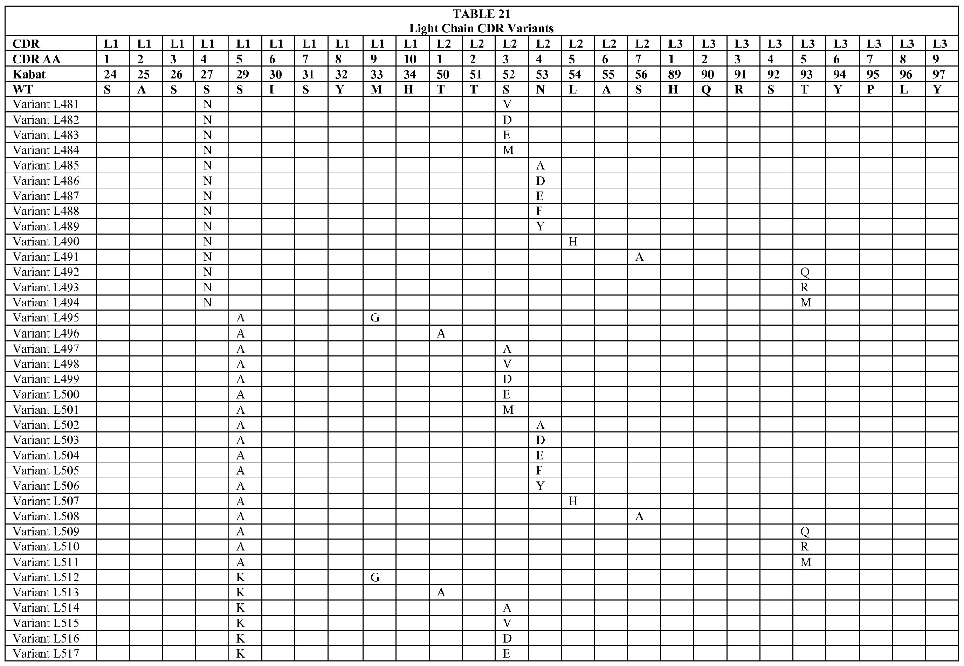

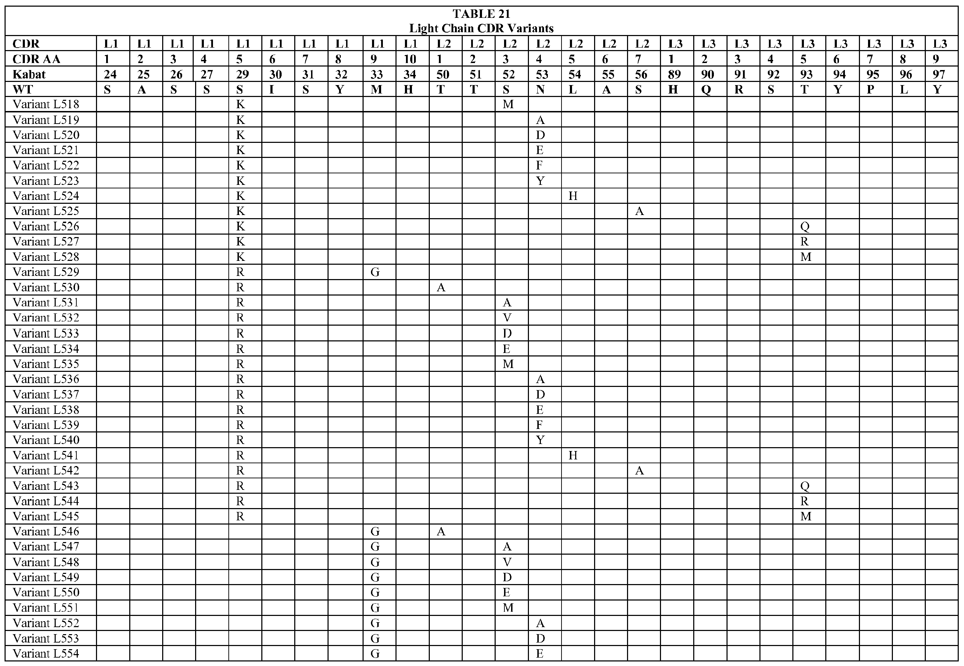

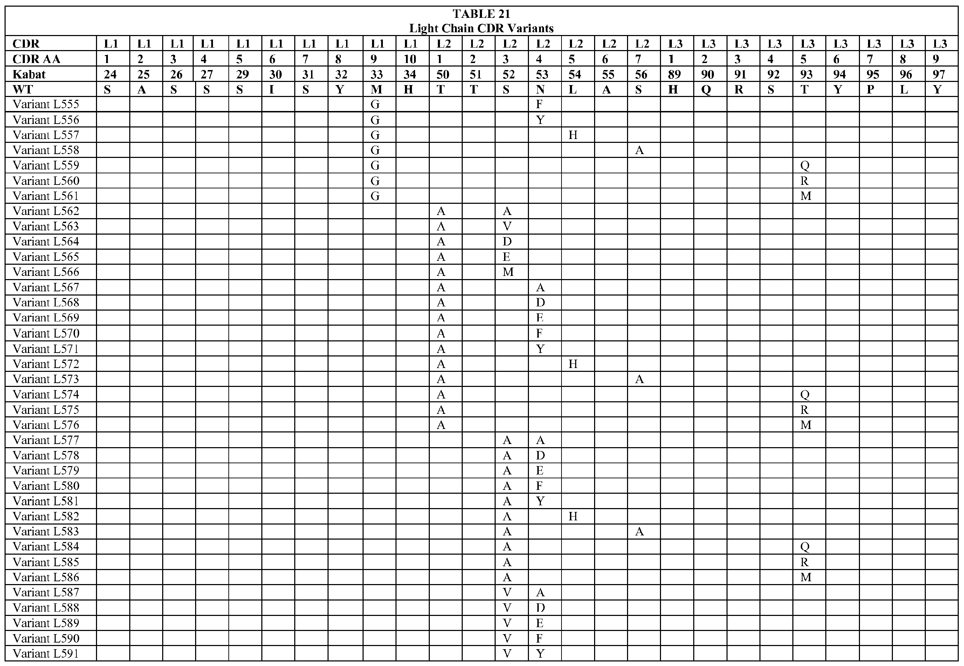

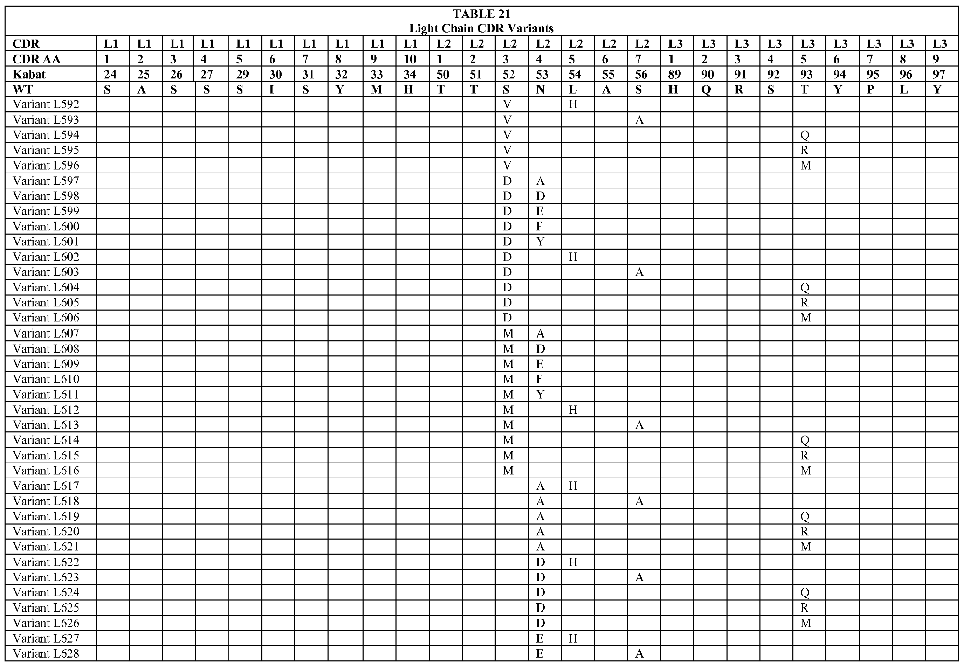

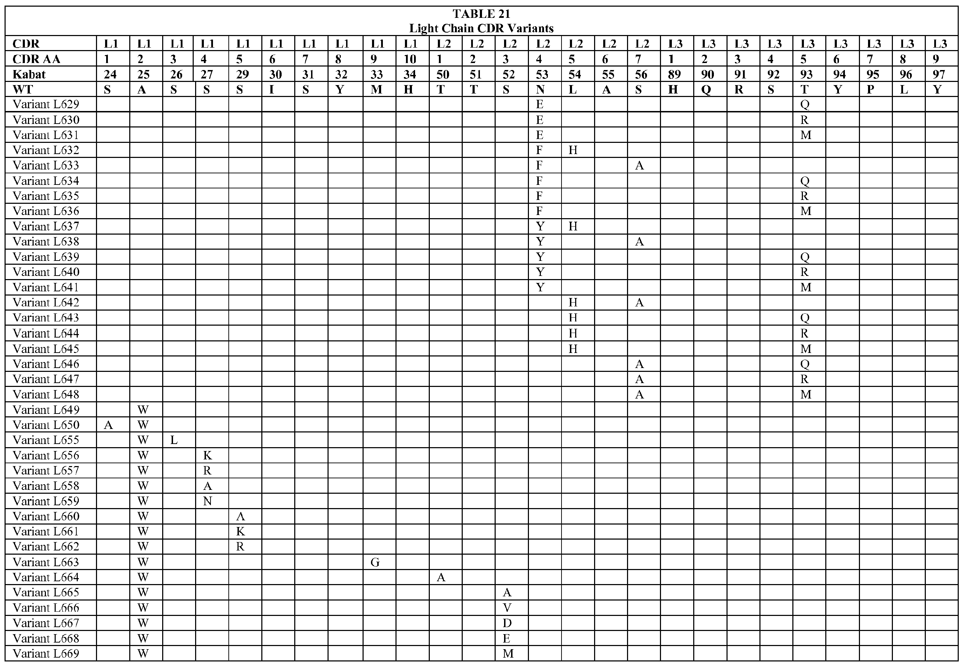

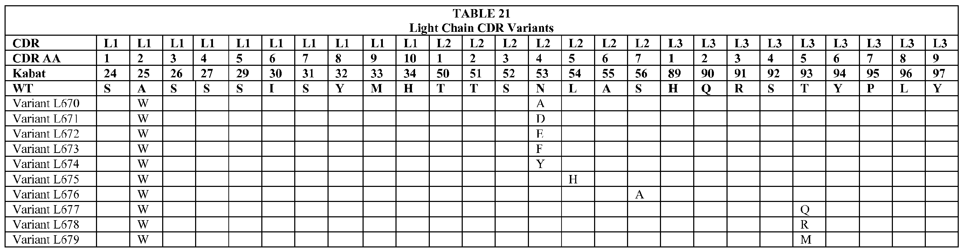

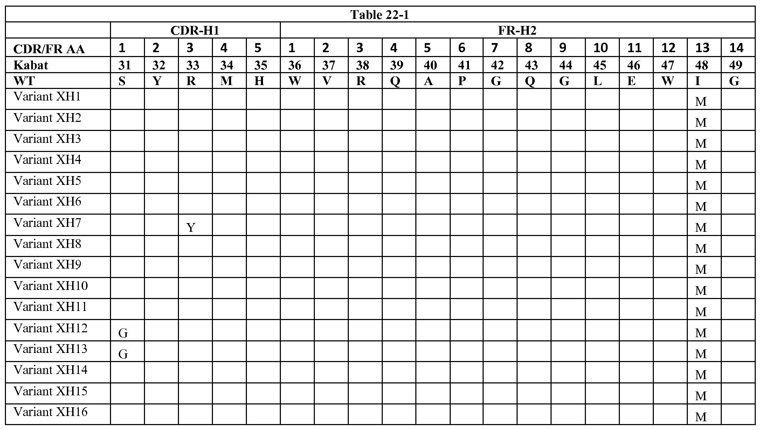

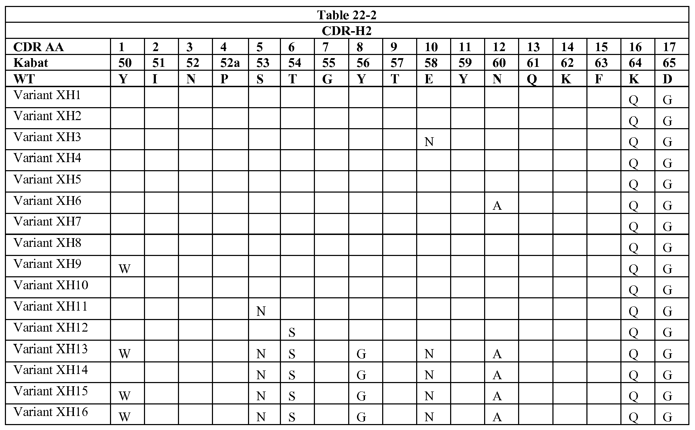

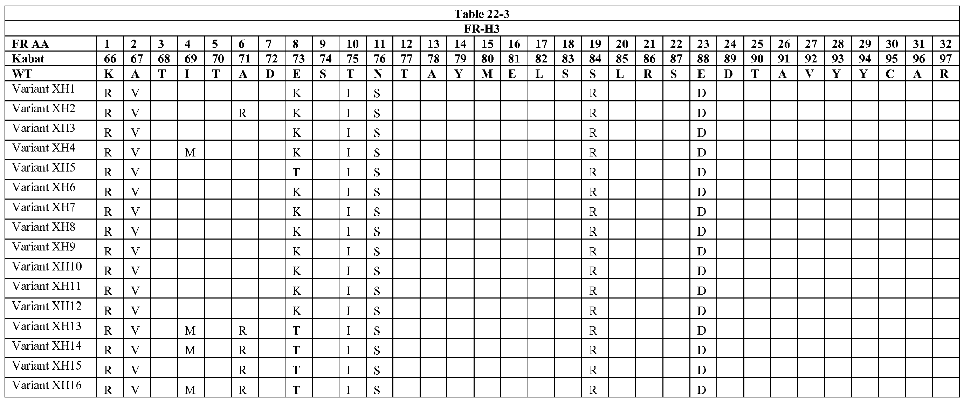

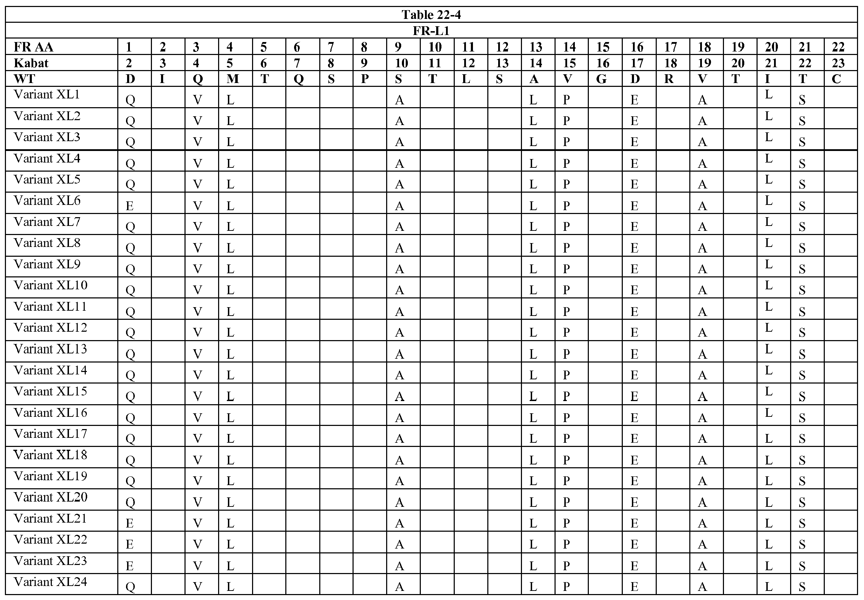

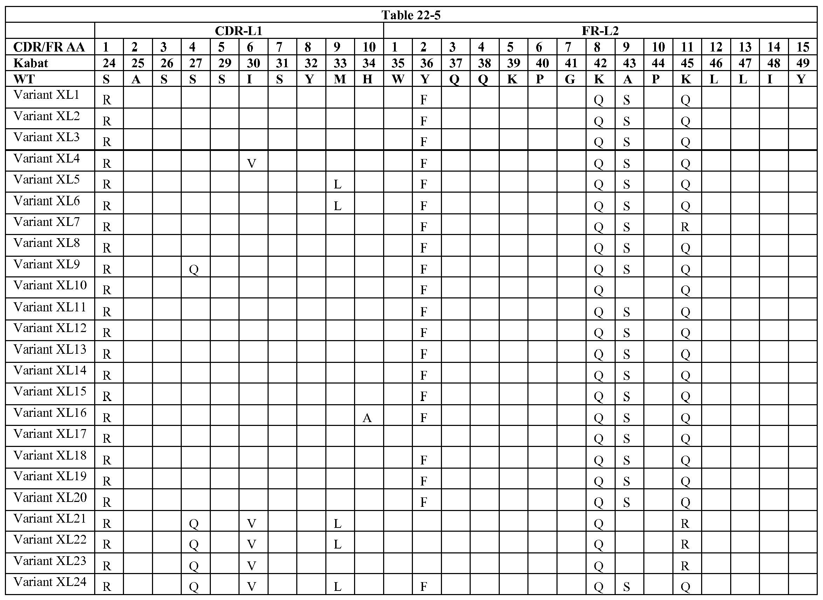

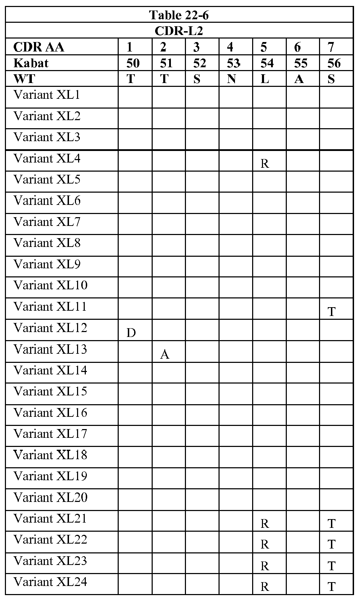

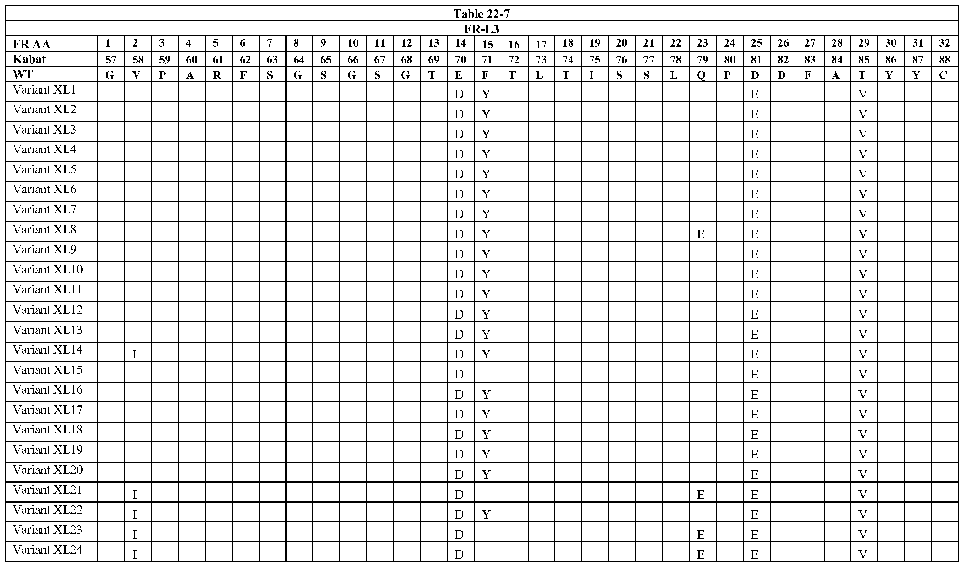

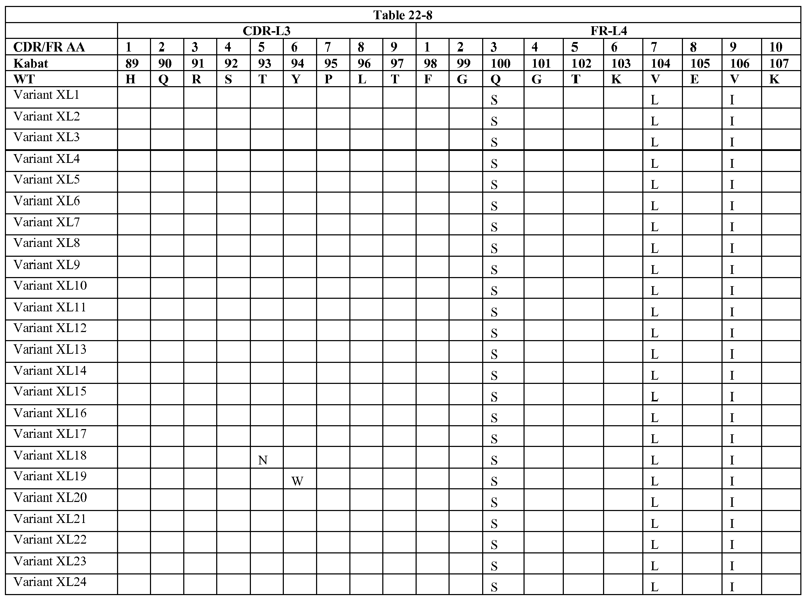

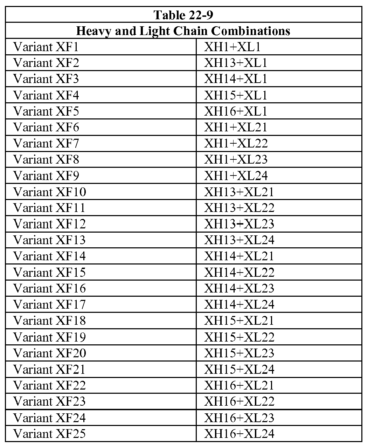

Figure 15 highlights variants with low-to-no ADCC activity with retained or improved FcγRIIB binding. Of the variants tested, V273F and V273Y showed the most improvement for FcγRIIB binding and the most decrease in FcγRIIIA binding.

### 9. SPECIFIC EMBODIMENTS, CITATION OF REFERENCES

All publications, patents, patent applications and other documents cited in this application are hereby incorporated by reference in their entireties for all purposes to the same extent as if each individual publication, patent, patent application or other document were individually indicated to be incorporated by reference for all purposes.

While various specific embodiments have been illustrated and described, it will be appreciated that various changes can be made without departing from the spirit and scope of the invention(s).

We set out below aspects of the invention:
1. A monoclonal anti-CD25 antibody or an anti-CD25 binding fragment of a monoclonal antibody, which:
   (a) binds to human CD25;
   (b) comprises CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3); and
   (c) has an IC₅₀ of up to 50% of the IC₅₀ of a corresponding antibody having CDRs of SEQ ID NOs:4, 6, 8, 11, 13, and 15 in an IL2-dependent T-cell proliferation assay.
2. The anti-CD25 antibody or anti-CD25 binding fragment of aspect 1, which has an IC₅₀ of up to 40% of the IC₅₀ of a corresponding antibody having CDRs of SEQ ID NOs:4, 6, 8, 11, 13, and 15 in an IL2-dependent T-cell proliferation assay.
3. The anti-CD25 antibody or anti-CD25 binding fragment of aspect 2, which has an IC₅₀ of up to 30% the IC₅₀ of a corresponding antibody having CDRs of SEQ ID NOs:4, 6, 8, 11, 13, and 15 in an IL2-dependent T-cell proliferation assay.
4. The anti-CD25 antibody or anti-CD25 binding fragments of any one of aspects 1 to 3, which comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
   (a) a V263 substitution that increases affinity towards FcγRIIB and decreases affinity towards FcγRIIIA;
   (b) a V266 substitution that increases affinity towards FcγRIIB and decreases affinity towards FcγRIIIA; and
   (c) a V273 substitution that increases affinity towards FcγRIIB and decreases affinity towards FcγRIIIA.
5. The anti-CD25 antibody or anti-CD25 binding fragments of any one of aspects 1 to 3, which comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
   (a) a V263 substitution that increases affinity towards FcγRIIB and decreases affinity towards FcγRIIIA; and
   (b) a V273 substitution that increases affinity towards FcγRIIB and decreases affinity towards FcγRIIIA.
6. The anti-CD25 antibody or anti-CD25 binding fragment of any one of aspects 1 to 3, which comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
   (i) the substitution V263L; and/or
   (ii) the substitution V266L; and/or
   (iii) a V273 substitution selected from V273C, V273E, V273F, V273L, V273M, V273S, V273Y; and/or
   (iv) a V305 substitution selected from V305K and V305W.
7. The anti-CD25 antibody or anti-CD25 binding fragment of aspect 6, which comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
   (i) the substitution V263L; and/or
   (ii) a V273 substitution selected from V273C, V273E, V273F, V273L, V273M, V273S, V273Y.
8. The anti-CD25 antibody or anti-CD25 binding fragment of any one of aspects 1 to 7, which comprises the amino acid substitutions (i) N52S, N52K, N52R or N52V and (ii) T54R, T54S or T54K in CDR-H2 as compared to CDR-H2 of SEQ ID NO:6, and, optionally, one or more of: (iii) S53R, S53K or S53N in CDR-H2 as compared to CDR-H2 of SEQ ID NO:6, (iv) Y56R in CDR-H2 as compared to CDR-H2 of SEQ ID NO:6, (v) E58Q in CDR-H2 as compared to CDR-H2 of SEQ ID NO:6, (vi) E73K in CDR-H2 as compared to CDR-H2 of SEQ ID NO:6, (vii) S29K in CDR-L1 as compared to CDR-L1 of SEQ ID NO:11 and (viii) N53D or N53E in CDR-L2 as compared to CDR-L2 of SEQ ID NO:13.
9. The anti-CD25 antibody or anti-CD25 binding fragment of any one of aspects 1 to 7, which comprises the amino acid substitutions (i) N52S, N52K, N52R or N52V, (ii) S53K, S53R or S53N and (iii) T54R, T54S or T54K in CDR-H2 as compared to CDR-H2 of SEQ ID NO:6, (iv) S29K in CDR-L1 as compared to CDR-L1 of SEQ ID NO:11 and (v) N53D or N53E in CDR-L2 as compared to CDR- L2 of SEQ ID NO:13, and, optionally, further comprises the amino acid substitution (iv) Y56R in CDR-H2 as compared to CDR-H2 of SEQ ID NO:6.
10. The anti-CD25 antibody or anti-CD25 binding fragment of any one of aspects 1 to 7, which comprises the amino acid substitutions (i) N52S, (ii) S53R or S53K, (iii) T54S or T54K, and (iv) Y56R in CDR-H2 as compared to CDR-H2 of SEQ ID NO:6, (v) S29K in CDR-L1 as compared to CDR-L1 of SEQ ID NO:11 and (vi) N53D in CDR-L2 as compared to CDR-L2 of SEQ ID NO:13.
11. The anti-CD25 antibody or anti-CD25 binding fragment of any one of aspects 1 to 7, which comprises the amino acid substitutions N52K and T54R in CDR-H2 as compared to CDR-H2 of SEQ ID NO:6.
12. The anti-CD25 antibody or anti-CD25 binding fragment of aspect 11, which further comprises the amino acid substitution N53E in CDR-L2 as compared to CDR-L2 of SEQ ID NO:13.
13. The anti-CD25 antibody or anti-CD25 binding fragment of any one of aspects 1 to 7, which comprises the amino acid substitutions N52S, S53R and T54K in CDR-H2 as compared to CDR-H2 of SEQ ID NO:6 and N53E in CDR-L2 as compared to CDR-L2 of SEQ ID NO:13.
14. The anti-CD25 antibody or anti-CD25 binding fragment of any one of aspects 1 to 13, which comprises framework regions with up to 4 amino acid substitutions as compared to frameworks of SEQ ID NO:3 (FR-H1), SEQ ID NO:5 (FR-H2), SEQ ID NO:7 (FR-H3), SEQ ID NO:9 (FR-H4), SEQ ID NO:10 (FR-L1), SEQ ID NO:12 (FR-L2), SEQ ID NO:14 (FR-L3) and SEQ ID NO:16 (FR-L4).
15. The anti-CD25 antibody or anti-CD25 binding fragment of aspect 14, which has reduced T cell immunogenicity as compared to an anti-CD25 antibody or anti-CD25 binding fragment comprising heavy and light variable regions of SEQ ID NO:1 and SEQ ID NO:2.
16. The anti-CD25 antibody or anti-CD25 binding fragment of any one of aspects 1-15, which comprises the amino acid substitution I48M in FR-H2 as compared to a FR-H2 of SEQ ID NO:5.
17. The anti-CD25 antibody or anti-CD25 binding fragment of any one of aspects 1-15, which lacks the amino acid substitution I48M in FR-H2 as compared to a FR-H2 of SEQ ID NO:5.
18. A monoclonal anti-CD25 antibody or an anti-CD25 binding fragment of a monoclonal antibody, which:
   (a) binds to human CD25;
   (b) comprises CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3);
   (c) comprises the amino acid substitutions (i) N52S, N52K, N52R or N52V and (ii) T54R, T54S or T54K in CDR-H2 as compared to CDR-H2 of SEQ ID NO:6, and, optionally, one or more of: (iii) S53R, S53K or S53N in CDR-H2 as compared to CDR-H2 of SEQ ID NO:6, (iv) Y56R in CDR-H2 as compared to CDR-H2 of SEQ ID NO:6, (v) E58Q in CDR-H2 as compared to CDR-H2 of SEQ ID NO:6, (vi) E73K in CDR-H2 as compared to CDR-H2 of SEQ ID NO:6, (vii) S29K in CDR-L1 as compared to CDR-L1 of SEQ ID NO:11 and (viii) N53D or N53E in CDR-L2 as compared to CDR-L2 of SEQ ID NO:13; and, optionally
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
19. A monoclonal anti-CD25 antibody or an anti-CD25 binding fragment of a monoclonal antibody, which:
   (a) binds to human CD25;
   (b) comprises CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3);
   (c) comprises the amino acid substitutions (i) N52S, N52K, N52R or N52V, (ii) S53K, S53R or S53N and (iii) T54R, T54S or T54K in CDR-H2 as compared to CDR-H2 of SEQ ID NO:6, (iv) S29K in CDR-L1 as compared to CDR-L1 of SEQ ID NO:11 and (v) N53D or N53E in CDR-L2 as compared to CDR-L2 of SEQ ID NO:13, and, optionally, further comprises the amino acid substitution (iv) Y56R in CDR-H2 as compared to CDR-H2 of SEQ ID NO:6; and, optionally
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
20. A monoclonal anti-CD25 antibody or an anti-CD25 binding fragment of a monoclonal antibody, which:
   (a) binds to human CD25;
   (b) comprises CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3);
   (c) comprises the amino acid substitutions (i) N52S, (ii) S53R or S53K, (iii) T54S or T54K, and (iv) Y56R in CDR-H2 as compared to CDR-H2 of SEQ ID NO:6, (v) S29K in CDR-L1 as compared to CDR-L1 of SEQ ID NO:11 and (vi) N53D in CDR-L2 as compared to CDR-L2 of SEQ ID NO:13; and, optionally
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
21. The anti-CD25 antibody or anti-CD25 binding fragment of any one of aspects 18 to 20, which comprises framework regions with up to 4 amino acid substitutions as compared to frameworks of SEQ ID NO:3 (FR-H1), SEQ ID NO:5 (FR-H2), SEQ ID NO:7 (FR-H3), SEQ ID NO:9 (FR-H4), SEQ ID NO:10 (FR-L1), SEQ ID NO:12 (FR-L2), SEQ ID NO:14 (FR-L3) and SEQ ID NO:16 (FR-L4).
22. The anti-CD25 antibody or anti-CD25 binding fragment of aspect 21, which has reduced T cell immunogenicity as compared to an anti-CD25 antibody or anti-CD25 binding fragment comprising heavy and light variable regions of SEQ ID NO:1 and SEQ ID NO:2.
23. The anti-CD25 antibody or anti-CD25 binding fragment of aspect 21 or aspect 22, which comprises the amino acid substitution I48M in FR-H2 as compared to a FR-H2 of SEQ ID NO:5.
24. The anti-CD25 antibody or anti-CD25 binding fragment of aspect 21 or aspect 22, which does not comprise a substitution at I48 in FR-H2 as compared to a FR-H2 of SEQ ID NO:5.
25. An monoclonal anti-CD25 antibody or an anti-CD25 binding fragment of a monoclonal antibody, which:
   (a) binds to human CD25;
   (b) comprises heavy and light chain variable regions having up to 12, up to 11, up to 10, up to 9, up to 8, up to 7, up to 6, up to 5 or up to 4 amino acid substitutions as compared to the heavy and light variable regions of SEQ ID NO:1 and SEQ ID NO:2, respectively;
   (c) has an IC₅₀ of up to 50% of the IC₅₀ of a corresponding antibody having the heavy and light variable regions of SEQ ID NO:1 and SEQ ID NO:2, respectively, in an IL2-dependent T-cell proliferation assay; and, optionally,
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
26. The anti-CD25 antibody or anti-CD25 binding fragment of aspect 25, which has an IC₅₀ of up to 40% of the IC₅₀ of a corresponding antibody having the heavy and light variable regions of SEQ ID NO:1 and SEQ ID NO:2, respectively, in an IL2-dependent T-cell proliferation assay.
27. The anti-CD25 antibody or anti-CD25 binding fragment of aspect 26, which has an IC₅₀ of up to 30% the IC₅₀ of a corresponding antibody having the heavy and light variable regions of SEQ ID NO:1 and SEQ ID NO:2, respectively, in an IL2-dependent T-cell proliferation assay.
28. The anti-CD25 antibody or anti-CD25 binding fragment of any one of aspects 25 to 27, which has reduced immunogenicity as compared to a corresponding antibody having the heavy and light variable regions of SEQ ID NO:1 and SEQ ID NO:2, respectively.
29. The anti-CD25 antibody or anti-CD25 binding fragment of aspect 28, which comprises the amino acid substitution I48M in FR-H2 as compared to a FR-H2 of SEQ ID NO:5.
30. The anti-CD25 antibody or anti-CD25 binding fragment of aspect 29, which further comprises the amino acid substitutions N52K and T54R in CDR-H2 as compared to CDR-H2 of SEQ ID NO:6 and S29K in CDR-L1 as compared to CDR-L1 of SEQ ID NO:11 and N53D in CDR-L2 as compared to CDR-L2 of SEQ ID NO:13.
31. The anti-CD25 antibody or anti-CD25 binding fragment of aspect 29, which further comprises the amino acid substitutions N52K and T54R in CDR-H2 as compared to CDR-H2 of SEQ ID NO:6 and N53E in CDR-L2 as compared to CDR-L2 of SEQ ID NO:13.
32. The anti-CD25 antibody or anti-CD25 binding fragment of aspect 29, which further comprises the amino acid substitutions N52S, S53R and T54K in CDR-H2 as compared to CDR-H2 of SEQ ID NO:6.
33. The anti-CD25 antibody or anti-CD25 binding fragment of aspect 28, which comprises the amino acid substitution T54S in CDR-H2 as compared to a CDR-H2 of SEQ ID NO:6.
34. The anti-CD25 antibody or anti-CD25 binding fragment of aspect 29, which further comprises the amino acid substitution T54S in CDR-H2 as compared to a CDR-H2 of SEQ ID NO:6.
35. The anti-CD25 antibody or anti-CD25 binding fragment of aspect 34, which further comprises the amino acid substitutions S29K in CDR-L1 as compared to CDR-L1 of SEQ ID NO:1 and N53D in CDR-L2 as compared to CDR-L2 of SEQ ID NO:13.
36. The anti-CD25 antibody or anti-CD25 binding fragment of aspect 34, which further comprises the amino acid substitution N53E in CDR-L2 as compared to CDR-L2 of SEQ ID NO:13.
37. The anti-CD25 antibody or anti-CD25 binding fragment of aspect 34, which further comprises the amino acid substitutions S53R and T54K in CDR-H2 as compared to CDR-H2 of SEQ ID NO:6.
38. The anti-CD25 antibody or anti-CD25 binding fragment of aspect 37, which further comprises the amino acid substitutions S29K in CDR-L1 as compared to CDR-L1 of SEQ ID NO:1 and N53D in CDR-L2 as compared to CDR-L2 of SEQ ID NO:13.
39. The anti-CD25 antibody or anti-CD25 binding fragment of aspect 37, which further comprises the amino acid substitution N53E in CDR-L2 as compared to CDR-L2 of SEQ ID NO:13.
40. A monoclonal anti-CD25 antibody or an anti-CD25 binding fragment of a monoclonal antibody, which:
   (a) binds to human CD25;
   (b) comprises CDRs having up to 8, up to 7, up to 6, up to 5, up to 4, up to 3 or up to 2 amino acid substitutions as compared to CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3);
   (c) has, as compared to an antibody with CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3),
      (i) heavy chains CDRs comprising at least one substitution present in any of the CDR variants H1-H354 as shown in Table 20; and/or
      (ii) light chain CDRs comprising at least one substitution present in any of the CDR variants L1-L288 and L649 as shown in Table 21; and, optionally,
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
41. The anti-CD25 antibody or anti-CD25 binding fragment of aspect 40, which has, as compared to an antibody with CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3), heavy chains CDRs comprising at least two substitutions present in any of the CDR variants H361-H369, H405-H443, H449-H487; H493-H531; H537-H572; H578-H613; H619-H654; H660-H690; H696-H726; H732-H762; H768-H798; H804-H834; H840-H865; H871-H896; H902-H927; H933-H958; H964-H989; H995-H1015; H1021-H1041; H107-H1067; H1073-H1093; H1099-H1119; H1125-H1141; H1147- H1163; H1169-H1185; H1191-H1207; H1213-H1226; H1232-H1245; H1251-H1264; H1270-H1280; H1286-H1296; H1302-H1312; H1316-H1327; H1333-H1341; H1347-H1351; H1357-H1361; H1367- H1371; H1377-H1381; H1387-H1391; H1425-H1476; H1478-H1517; and H1519-H1558 as shown in Table 20.
42. The anti-CD25 antibody or anti-CD25 binding fragment of aspect 40 or aspect 41, which has, as compared to an antibody with CDRs of SEQ ID NO:4 (CDR-H1), SEQ ID NO:6 (CDR-H2), SEQ ID NO:8 (CDR-H3), SEQ ID NO:11 (CDR-L1), SEQ ID NO:13 (CDR-L2) and SEQ ID NO:15 (CDR-L3), light chains CDRs comprising at least two substitutions present in any of the CDR variants L289- L648 and L650-L679 as shown in Table 21.
43. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1, and which comprises the heavy chain substitution I48M as compared to a heavy chain variable region of SEQ ID NO:1.
44. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID
   NO:1, and which comprises the heavy chain substitution I48V as compared to a heavy chain variable region of SEQ ID NO:1.
45. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1, and which comprises the heavy chain substitution I51L as compared to a heavy chain variable region of SEQ ID NO:1.
46. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1, and which comprises the heavy chain substitution T54S as compared to a heavy chain variable region of SEQ ID NO:1.
47. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1, and which comprises the heavy chain substitutions I48M and I51L as compared to a heavy chain variable region of SEQ ID NO:1.
48. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1, and which comprises the heavy chain substitutions I48V and T54S as compared to a heavy chain variable region of SEQ ID NO:1.
49. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1, and which comprises the heavy chain substitutions I48M and T54S as compared to a heavy chain variable region of SEQ ID NO:1.
50. The anti-CD25 antibody or anti-CD25 binding fragment of any one of aspects 40 to 42, whose heavy chain variable region has up to 12, up to 11, up to 10, up to 9, up to 8, up to 7, up to 6, up to 5 or up to 4 amino acid substitutions as compared to the heavy chain variable region of SEQ ID NO:1.
51. The anti-CD25 antibody or anti-CD25 binding fragment of any one of aspects 40 to 50, whose light chain variable region has up to 12, up to 11, up to 10, up to 9, up to 8, up to 7, up to 6, up to 5 or up to 4 amino acid substitutions as compared to the light chain variable region of SEQ ID NO:2.
52. A monoclonal anti-CD25 antibody or an anti-CD25 binding fragment of a monoclonal antibody, which:
   (a) binds to human CD25;
   (b) has a heavy chain variable region which has up to 12, up to 11, up to 10, up to 9, up to 8, up to 7, up to 6, up to 5 or up to 4 amino acid substitutions as compared to the heavy chain variable region of SEQ ID NO:1, said heavy chain comprising at least one substitution or combination of substitutions as compared to a heavy chain of SEQ ID NO:1 selected from:
      (i) I48M;
      (ii) I48V;
      (iii) 151L;
      (iv) T54S;
      (v) I48M and 151L;
      (vi) I48V and T54S; and
      (vii) I48M and T54S;
   (c) has a light chain variable region which has up to 12, up to 11, up to 10, up to 9, up to 8, up to 7, up to 6, up to 5 or up to 4 amino acid substitutions as compared to the heavy chain variable region of SEQ ID NO:2; and, optionally,
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO: 188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
53. A monoclonal anti-CD25 antibody or an anti-CD25 binding fragment of a monoclonal antibody, which:
   (a) binds to human CD25;
   (b) comprises heavy and light chain variable regions having up to 12, up to 11, up to 10, up to 9, up to 8, up to 7, up to 6, up to 5 or up to 4 amino acid substitutions as compared to the heavy and light variable regions of SEQ ID NO:1 and SEQ ID NO:2, respectively;
   (c) comprises the amino acid substitutions present in any of the combination variants C1-C19, C21 and C24-C63, as shown in Tables 7A-7C; and, optionally,
   (d) comprises a variant CH2 domain which has at least 70%, at least 75%, at least 80%, at least 85%, at least 90%, at least 95%, at least 96%, at least 97%, at least 98% or at least 99% sequence identity to the CH2 domain of SEQ ID NO:188, and which has relative to the CH2 domain of SEQ ID NO:188 one or more substitutions selected from:
      (i) the substitution V263L; and/or
      (ii) the substitution V266L; and/or
      (iii) a V273 substitution selected from V273C, V273E, V273F, V273L, V273M, V273S, V273Y; and/or
      (iv) a V305 substitution selected from V305K and V305W.
54. The anti-CD25 antibody or anti-CD25 binding fragment of any one of aspects 1 to 53, which comprises at least one light chain CDR substitution from Table 8A and/or at least one heavy chain CDR substitution from Table 8B.
55. The anti-CD25 antibody or anti-CD25 binding fragment of aspect 54, wherein at least one light chain CDR substitution from Table 8A includes one or more of:
   (a) S24V in CDR-L1;
   (b) A25I, A25T or A25M in CDR-L1;
   (c) S26L in CDR-L1;
   (d) S27K, S27R, S27A, or S27N in CDR-L1;
   (e) S29A, S29K or S29R in CDR-L1;
   (f) M33G in CDR-L1;
   (g) T50A in CDR-L2;
   (h) S52A, S52V, S52D, S52E or S52M in CDR-L2;
   (i) N53A, N53D, N53E, N53F or N53Y in CDR-L2;
   (j) L54H in CDR-L2;
   (k) S56A in CDR-L2;
   (l) T93Q, T93R, T93M in CDR-L3; and
   (m) T97S in CDR-L3.
56. The anti-CD25 antibody or anti-CD25 binding fragment of aspect 54 or aspect 55, wherein at least one heavy chain CDR substitution from Table 8B includes one or more of:
   (a) S31F, S31K, S31R or S31W in CDR-H1;
   (b) Y32S, Y32T or Y32V in CDR-H1;
   (c) M34A, M34T or M34V in CDR-H1;
   (d) I51W, I51L, I51A, I51K or I51V in CDR-H2;
   (e) N52A, N52K, N52R, N52S or N52V in CDR-H2;
   (f) S53K, S53T, S53P or S53A in CDR-H2;
   (g) T54A, T54K, T54S or T54V in CDR-H2;
   (h) Y56K, Y56R or Y56A in CDR-H2;
   (i) T57A, T57D or T57G in CDR-H2;
   (j) Y59E in CDR-H2;
   (k) F63S;
   (l) K64A, K64D, K64V or K64G in CDR-H2;
   (m) D101G in CDR-H3; and/or
   (n) Y102D, Y102K, Y102Q or Y102T in CDR-H3.
57. The anti-CD25 antibody or anti-CD25 binding fragment of any one of aspects 1 to 56 which comprises at least one light chain CDR substitution from Table 8A and/or at least one heavy chain CDR substitution from Table 8B in which a wild type non-histidine residue is substituted with histidine.
58. The anti-CD25 antibody or anti-CD25 binding fragment of any one of aspects 1 to 57 whose heavy and light chain variable regions comprise altogether at least 2, at least 3, at least 4 or at least 5 amino acid substitutions as compared to the heavy and light variable regions of SEQ ID NO:1 and SEQ ID NO:2.
59. The anti-CD25 antibody or anti-CD25 binding fragment of any one of aspects 1 to 54, whose six CDRs altogether have up to 8, up to 7, up to 6, up to 5, or up to 4 amino acid substitutions as compared to the CDR sequences SEQ ID NOs:4, 6, 8, 11, 13, and 15.
60. The anti-CD25 antibody or anti-CD25 binding fragment of aspect 54, wherein any individual CDR has no more than 3 amino acid substitutions as compared to the corresponding CDR sequence of an antibody having CDRs of SEQ ID NOs:4, 6, 8, 11, 13, and 15.
61. The anti-CD25 antibody or anti-CD25 binding fragment of aspect 54 or aspect 60, wherein any individual CDR other than CDR-H2 has no more than 2 amino acid substitutions as compared to the corresponding CDR sequence of an antibody having CDRs of SEQ ID NOs:4, 6, 8, 11, 13, and 15.
62. The anti-CD25 antibody or anti-CD25 binding fragment of any one of aspects 1 to 61, wherein any individual framework region has no more than 5 amino acid substitutions as compared to the corresponding framework sequence of an antibody having framework sequences of SEQ ID NOs:3, 5, 7, 9, 10, 12, 14 and 16.
63. The anti-CD25 antibody or anti-CD25 binding fragment of any one of aspects 1 to 62, wherein any individual framework region has no more than 4 amino acid substitutions as compared to the corresponding framework sequence of an antibody having framework sequences of SEQ ID NOs:3, 5, 7, 9, 10, 12, 14 and 16.
64. The anti-CD25 antibody or anti-CD25 binding fragment of any one of aspects 1 to 63, wherein any individual framework region has no more than 3 amino acid substitutions as compared to the corresponding framework sequence of an antibody having framework sequences of SEQ ID NOs:3, 5, 7, 9, 10, 12, 14 and 16.
65. The anti-CD25 antibody or anti-CD25 binding fragment of any one of aspects 1 to 64, wherein any individual framework region has no more than 2 amino acid substitutions as compared to the corresponding framework sequence of an antibody having framework sequences of SEQ ID NOs:3, 5, 7, 9, 10, 12, 14 and 16.
66. The anti-CD25 antibody or anti-CD25 binding fragment of any one of aspects 1 to, 65 wherein any individual framework region has no more than 1 amino acid substitution as compared to the corresponding framework sequence of an antibody having framework sequences of SEQ ID NOs:3, 5, 7, 9, 10, 12, 14 and 16.
67. The anti-CD25 antibody or anti-CD25 binding fragment of any one of aspects 1 to 66 whose V_{H} sequence does not consist of the V_{H} sequence of any of the variants XH1 to XH16 as shown Tables 22-1 to 22-3.
68. The anti-CD25 antibody or anti-CD25 binding fragment of any one of aspects 1 to 67 whose V₁ sequence does not consist of the V_{L} sequence of any of the variants XL1 to XL25 as shown in Tables 22-4 to 22-8.
69. The anti-CD25 antibody or anti-CD25 binding fragment of any one of aspects 1 to 66 whose V_{H} and V_{L} sequences do not consist of the V_{H} and V_{L} sequences of antibodies XF1 through XF15 as shown in Table 22-9.
70. The anti-CD25 antibody or anti-CD25 binding fragment of any one of aspects 1 to 69 which is a human or humanized antibody, or anti-CD25 binding fragment of a human or humanized antibody, respectively.
71. The anti-CD25 antibody or anti-CD25 binding fragment of any one of aspects 8 to 17, 21 to 24, 26 to 39, 41 to 51 and 54 to 70 to the extent such aspect is dependent from any one of aspects 4 to 7, 18 to 20, 25, 40, 52, and 53, wherein said CH2 domain is part of an Fc domain having up to 20, up to 15, up to 12, up to 10, up to 9, up to 8, up to 7, up to 6, up to 5 or up to 4 amino acid substitutions as compared to the CH2 domain of the Fc domain of SEQ ID NO:17.
72. The anti-CD25 antibody or anti-CD25 binding fragment of aspect 71, wherein the Fc domain has at least 70%, at least 75%, at least 80%, at least 85%, at least 90%, at least 95%, at least 96%, at least 97%, at least 98% or at least 99% sequence identity to the Fc domain of SEQ ID NO:17.
73. The anti-CD25 antibody or anti-CD25 binding fragment of any one of aspects 1 to 70 which is an IgG.
74. The anti-CD25 antibody or anti-CD25 binding fragment of aspect 73 which is an IgG₁.
75. The anti-CD25 antibody or anti-CD25 binding fragment of aspect 74 which is isotype IgG₁ fa.
76. The anti-CD25 antibody or anti-CD25 binding fragment of aspect 74 which is not isotype IgG₁ fa.
77. The anti-CD25 antibody or anti-CD25 binding fragment of aspect 73 which is an IgG₂.
78. The anti-CD25 antibody or anti-CD25 binding fragment of aspect 77 which is an IgG₂ M3.
79. The anti-CD25 antibody or anti-CD25 binding fragment of aspect 73 which is an IgG₄.
80. The anti-CD25 antibody or anti-CD25 binding fragment of any one of aspects 71 to 79 whose Fc domain comprises the substitution M428L.
81. The anti-CD25 antibody or anti-CD25 binding fragment of aspect 80 whose Fc domain further comprises the substitution T250Q.
82. The anti-CD25 antibody or anti-CD25 binding fragment of any one of aspects 1 to 81 which has an Fc domain comprising one or more substitutions selected from V263L, V266L, V273C, V273E, V273F, V273L, Y273M, V273S, V273Y, V305K, and V305W.
83. The anti-CD25 antibody or anti-CD25 binding fragment of any one of aspects 1 to 82 which includes one or more mutations in the Fc region that increases ADCC activity.
84. The anti-CD25 antibody or anti-CD25 binding fragment of any one of aspects 1 to 82 which includes one or more mutations in the Fc region that decreases ADCC activity.
85. The anti-CD25 antibody or anti-CD25 binding fragment of aspect 84, whose Fc domain includes one or more substitutions selected from V263L, V273E, V273F, V273M, V273S, and V273Y.
86. The anti-CD25 antibody or anti-CD25 binding fragment of any one of aspects 1 to 82 which is non-fucosylated.
87. The anti-CD25 antibody or anti-CD25 binding fragment of any one of aspects 1 to 82 which includes one or more mutations in the Fc region that increases binding to FcγR.
88. The anti-CD25 antibody or anti-CD25 binding fragment of any one of aspects 1 to 82 which includes one or more mutations in the Fc region that decreases binding to FcγR.
89. The anti-CD25 antibody or anti-CD25 binding fragment of any one of aspects 1 to 82 which includes one or more mutations in the Fc region that increases binding to FcRn.
90. The anti-CD25 antibody or anti-CD25 binding fragment of any one of aspects 1 to 89 which has an affinity to CD25 that is at least 1.2-fold, at least 1.5 fold, at least 2-fold, at least 3-fold, at least 4-fold or at least 5-fold greater than the affinity to CD25 of a corresponding antibody having V_{H} sequence corresponding to SEQ ID NO:1 and a V_{L} sequence corresponding to SEQ ID NO:2.
91. The anti-CD25 antibody or anti-CD25 binding fragment of any one of aspects 1 to 90 which has an affinity to CD25 that is up to 10-fold, up to 15-fold, up to 20-fold or up to 30-fold greater than the affinity to CD25 of a corresponding antibody having V_{H} sequence corresponding to SEQ ID NO:1 and a V_{L} sequence corresponding to SEQ ID NO:2.
92. The anti-CD25 antibody or anti-CD25 binding fragment of any one of aspects 1 to 91 which has an affinity to CD25 that is 3- to 15-fold, 5- to 10-fold, or 2- to 30-fold greater than the affinity to CD25 of a corresponding antibody having V_{H} sequence corresponding to SEQ ID NO:1 and a V_{L} sequence corresponding to SEQ ID NO:2.
93. The anti-CD25 antibody or anti-CD25 binding fragment of any one of aspects 1 to 92 which is purified.
94. The anti-CD25 antibody or anti-CD25 binding fragment of aspect 93 which is purified to at least 85%, at least 90%, at least 95% or at least 98% homogeneity.
95. An antibody-drug conjugate comprising an anti-CD25 antibody or anti-CD25 binding fragment according to any one of aspects 1 to 92.
96. A pharmaceutical composition comprising an anti-CD25 antibody or anti-CD25 binding fragment according to any one of aspects 1 to 92 or an antibody-drug conjugate according to aspect 95.
97. A nucleic acid comprising a nucleotide sequence encoding an anti-CD25 antibody or anti-CD25 binding fragment according to any one of aspects 1 to 92.
98. A vector comprising the nucleic acid of aspect 97.
99. A prokaryotic host cell transformed with a vector according to aspect 98.
100. A eukaryotic host cell transformed with a vector according to aspect 98.
101. A eukaryotic host cell engineered to express the nucleotide sequence of aspect 97.
102. The eukaryotic host cell of aspect 101 which is a mammalian host cell.
103. A method of producing an anti-CD25 antibody or anti-CD25 binding fragment comprising:
   (a) culturing the eukaryotic host cell of aspect 101 or aspect 102; and
   (b) recovering the anti-CD25 antibody or anti-CD25 binding fragment antibody.
104. A method of preventing organ transplant rejection, comprising administering to a human in need thereof a therapeutically effective amount of an anti-CD25 antibody or anti-CD25 binding fragment according to any one of aspects 1 to 94, an antibody-drug conjugate according to aspect 95, or a pharmaceutical composition according to aspect 96.
105. A method of treating asthma, multiple sclerosis, uveitis, ocular inflammation or human T cell leukemia virus-1 associated T-cell leukemia, comprising administering to a human in need thereof a therapeutically effective amount of an anti-CD25 antibody or anti-CD25 binding fragment according to any one of aspects 1 to 94, an antibody-drug conjugate according to aspect 95, or a pharmaceutical composition according to aspect 96.

## Claims

1. A monoclonal anti-CD25 antibody or an anti-CD25 binding fragment of a monoclonal antibody, which:
(a) binds to human CD25;
(b) comprises heavy and light chain variable regions having up to 12 amino acid substitutions as compared to the heavy and light chain variable regions of SEQ ID NO:1 and SEQ ID NO:2, respectively;
(c) comprises the amino acid substitutions present in any of the combination variants C1-C19, C21 and C24-C63, as shown in Tables 7A-7C; and,
(d) optionally, comprises a variant CH2 domain which has at least 70% sequence identity to the CH2 domain of SEQ ID NO:188, and which has relative to the CH2 domain of SEQ ID NO:188 one or more substitutions selected from:
(i) the substitution V263L; and/or
(ii) the substitution V266L; and/or
(iii) a V273 substitution selected from V273C, V273E, V273F, V273L, V273M, V273S, V273Y; and/or
(iv) a V305 substitution selected from V305K and V305W.

2. The monoclonal anti-CD25 antibody of claim 1, comprising:
(a) a set of heavy chain variable region amino acid substitutions as compared with SEQ ID NO:1, wherein the set consists of one of the following sets of amino acid substitutions:
(i) I48M and T54S;
(ii) I48M, N52S, S53R, T54S and Y56R; or
(iii) I48M, N52S, S53K, T54K and Y56R; and
(b) optionally, the light chain variable region amino acid substitutions S29K and N53D as compared with SEQ ID NO:2; and
(c) the CH2 domain of SEQ ID NO:188.

3. The monoclonal anti-CD25 antibody of claim 2, comprising:
(a) a set of heavy chain variable region amino acid substitutions as compared with SEQ ID NO:1, wherein the set consists of I48M and T54S,
(b) the light chain variable region of SEQ ID NO:2; and
(c) the CH2 domain of SEQ ID NO:188.

4. The monoclonal anti-CD25 antibody of claim 1, comprising:
(a) a set of heavy chain variable region amino acid substitutions as compared with SEQ ID NO:1, wherein the set consists of one of the following sets of amino acid substitutions:
(i) I48M, N52S, S53R, T54S and Y56R; or
(ii) I48M, N52S, S53K, T54K and Y56R; and
(b) light chain variable regions amino acid substitutions S29K and N53D as compared with SEQ ID NO:2; and
(c) the CH2 domain of SEQ ID NO:188.

5. The monoclonal anti-CD25 antibody of claim 1, wherein the antibody or antibody binding fragment has an Fc domain, said Fc domain comprising the substitutions T250Q and M428L, optionally wherein the antibody is a modified IgG2 M3 isotype antibody.

6. An antibody-drug conjugate comprising an anti-CD25 antibody or anti-CD25 binding fragment according to any of claims 1-5.

7. A pharmaceutical composition comprising an anti-CD25 antibody or anti-CD25 binding fragment according to any of claims 1-5.

8. A nucleic acid comprising a nucleotide sequence encoding an anti-CD25 antibody or anti-CD25 binding fragment according any of claims 1-5.

9. A vector comprising the nucleic acid of claim 8.

10. A prokaryotic host cell transformed with a vector according to claim 9.

11. A eukaryotic host cell transformed with a vector according to claim 9.

12. A eukaryotic host cell engineered to express the nucleotide sequence of claim 8.

13. A method of producing an anti-CD25 antibody or anti-CD25 binding fragment comprising:
(a) culturing the eukaryotic host cell of claim 11; and
(b) recovering the anti-CD25 antibody or anti-CD25 binding fragment antibody.

14. A monoclonal anti-CD25 antibody of claim 1, an antibody-drug conjugate of claim 6, or the pharmaceutical composition of claim 7 for use in preventing organ transplant rejection in a human patient in need thereof.

15. A monoclonal anti-CD25 antibody of claim 1, an antibody-drug conjugate of claim 6, or the pharmaceutical composition of claim 7 for treating asthma, multiple sclerosis, uveitis, ocular inflammation or human T cell leukemia virus-1 associated T-cell leukemia in a human patient in need thereof.
